(19) Europäisches Patentamt
European Patent Office
Office européen des brevets

(11) EP 4 303 221 A1

(12) **EUROPEAN PATENT APPLICATION**
published in accordance with Art. 153(4) EPC

(43) Date of publication:
**10.01.2024 Bulletin 2024/02**

(21) Application number: **22763275.9**

(22) Date of filing: **01.03.2022**

(51) International Patent Classification (IPC):
***C07D 498/22*** *(2006.01)* ***A61K 31/4995*** *(2006.01)*
***A61P 35/00*** *(2006.01)* ***C07D 491/22*** *(2006.01)*

(52) Cooperative Patent Classification (CPC):
**A61K 31/4995; A61P 35/00; C07D 491/22; C07D 498/22**

(86) International application number:
**PCT/JP2022/008664**

(87) International publication number:
**WO 2022/186221 (09.09.2022 Gazette 2022/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.03.2021 JP 2021033773**

(71) Applicant: **The University of Tokyo**
**Bunkyo-ku, Tokyo 113-8654 (JP)**

(72) Inventors:
- **OGURI Hiroki**
  **Tokyo 113-8654 (JP)**
- **TANIFUJI Ryo**
  **Tokyo 113-8654 (JP)**

(74) Representative: **Hoffmann Eitle**
**Patent- und Rechtsanwälte PartmbB**
**Arabellastraße 30**
**81925 München (DE)**

(54) **NOVEL TETRAHYDROISOQUINOLINE ALKALOID COMPOUND CONTAINING MACROCYCLE**

(57) [Problem] To provide: a novel tetrahydroisoquinoline (THIQ) alkaloid compound having a macrocyclic structure; an intermediate of the compound; and a method for producing the compound.

[Solution] Provided is a compound represented by formula (I), or a pharmaceutically acceptable salt thereof.

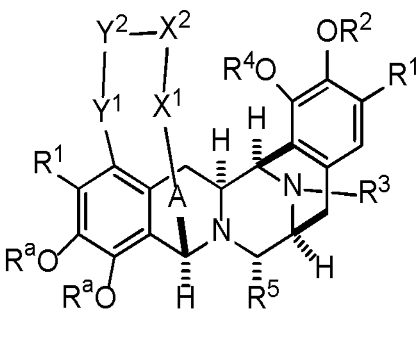

(I)

The present invention also provides: a method for synthesizing this compound; and an intermediate compound useful in this synthesis.



Processed by Luminess, 75001 PARIS (FR)

## Description

[Technical Field]

The present invention relates to a tetrahydroisoquinoline alkaloid compound containing a macrocyclic structure, an intermeaadiate thereof, and a method for producing the same.

[Background Art]

A tetrahydroisoquinoline alkaloid group represented by saframycin A is a compound having a complex pentacyclic framework in which a plurality of tetrahydroisoquinoline (THIQ) rings are linked, and it is known that the THIQ alkaloid group has strong antitumor activity by alkylating DNA double strands (for example, NPL 1). On the other hand, there is a problem of high toxicity to normal cells.

[C1]

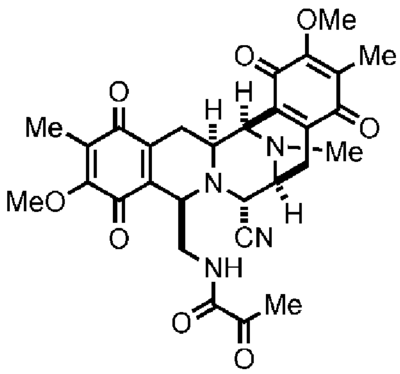

saframycin A

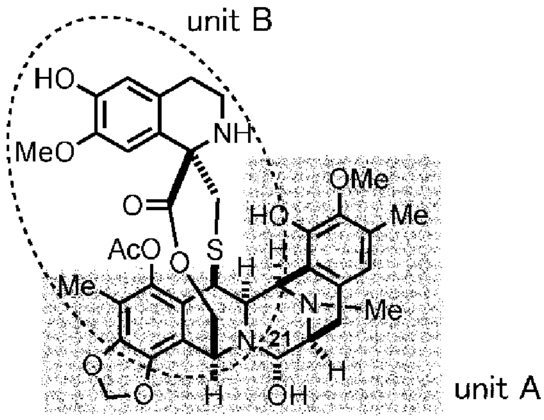


Ecteinascidin 743(1)
malignant soft tissue tumor drug
( Yondelis ®)

Ecteinascidin 743 (product name "Yondelis") is the only natural product group that has been clinically applied as an antitumor drug. Ecteinascidin 743 has a base framework (unit A) shared with saframycin A and also has a macrolactone ring (unit B) with a third THIQ linked. It is thought that the unit A recognizes DNA double strands at multiple points via hydrogen bonds and alkylates them in a sequence-selective manner, and on the other hand, the unit B inhibits approach of protein groups that interact with DNA such as a transcription factor and exhibits antitumor activity (NPL 2). In addition, the physiological activity of lurbinectedin (lurbinectedin, PM01183), which is a synthetic analog in which a THIQ moiety of Ecteinascidin 743 is modified, and Zalypsis (registered trademark "Zalypsis") analogs having no macrocycle are also being studied (NPL 3 and 4, etc.).

Regarding the THIQ alkaloid group that exhibits such excellent antitumor activity, although various total synthesis studies have been actively studied, starting materials are complicated due to their extremely complex polycyclic structures, and there is a problem of production efficiency for which a multiple-stage synthetic step is required. In particular, Ecteinascidin 743 is semi-synthesized from Cyanosafracin B obtained by mass culture by a method that requires a multiple stage process of 24 processes and the pattern of macrocycle analogs obtained by such a method is limited to being the same as the natural product Ecteinascidin 743.

On the other hand, methods for synthesizing analog groups of THIQ alkaloids that do not have a macrocycle are being studied in various countries around the world, but none of them has been marketed as drugs because they have strong toxicity to normal cells. Therefore, construction of a macrocycle framework is thought to be very important in order to exhibit cancer cell-selective toxicity, but no synthesis method that allows a macrocycle framework to be diversified has been reported at present.

[Citation List]

[Non Patent Literature]

[NPL 1] Scott, J. D.; Williams, R. M. Chem. Rev. 2002, 102, 1669.
[NPL 2] Le, V. H.; Inai, M.; Williams, R. M.; Kan, T. Nat. Prod. Rep. 2015, 32, 328.
[NPL 3] Daniele G. Soares, Miriana S. Machado, Celine J. Rocca, et al. Mol. Cancer. Ther. 2011, 10, 1481-1489.
[NPL 4] Bradley J. Petek, Robin L. Jones, Molecules 2014, 19, 12328-12335.

[Summary of Invention]

In view of such circumstances, a novel tetrahydroisoquinoline (THIQ) alkaloid compound containing a macrocyclic structure, a method for synthesizing the same, and an intermediate thereof are necessary.

A synthesis method that allows various modal macrocyclic structures linked to the THIQ framework to be constructed using Cyanosafracin B as a starting material has been found. Based on these findings, the present invention has been completed.

That is, one aspect of the present invention relates to a novel THIQ alkaloid compound containing a macrocyclic structure and a pharmaceutical composition containing the compound. In another aspect, the present invention relates to an intermediate compound suitable for obtaining the THIQ alkaloid compound containing a macrocyclic structure and a production method using the intermediate compound. The present invention is, for example, as follows.

[1] A compound represented by the following formula (I) or a pharmaceutically acceptable salt thereof:

[C2]

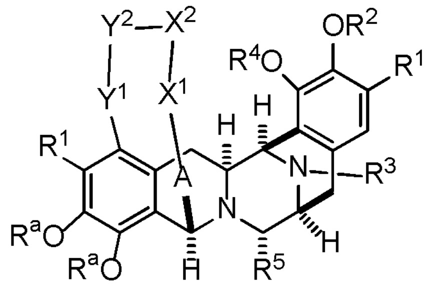

(I)

wherein A is a single bond or an optionally substituted $C_1$-$C_6$ alkylene group;

$X^1$ is a divalent group selected from the group consisting of an optionally substituted alkylene group, an optionally substituted alkenylene group, -$NR^bC(O)$-, -$C(O)NR^b$-, -C(O)O-, -OC(O)-, -$NR^bC(O)O$-, -$OC(O)NR^b$-, -OC(O)O-, a carbonyl group, -C(=S)-, -C(=$NR^b$)-, - $NR^b$-, a sulfonyl group, an ether group, a thioether group, an amino acid residue, and combinations thereof;

$Y^1$ is a divalent group selected from the group consisting of a single bond, an ether group, a thioether group, an optionally substituted $C_1$-$C_6$ alkylene group, and -$NR^b$-;

$Y^2$ is a divalent group selected from the group consisting of an optionally substituted alkylene group, an optionally substituted alkenylene group, -$NR^bC(O)$-, -$C(O)NR^b$-, -C(O)O-, -OC(O)-, -$NR^bC(O)O$-, -$OC(O)NR^b$-, -OC(O)O-, a carbonyl group, -C(=S)-, -C(=$NR^b$)-, - $NR^b$-, a sulfonyl group, an ether group, a thioether group, an amino acid residue, and combinations thereof;

$X^2$ is selected from the group consisting of -$L^1$-C(=$CR^f_2$)-$CR^f$=$CR^f$-$L^2$-, -$L^1$-$CR^fCR^f$-C(=$CR^f_2$)-$L^2$-, -$L^1$-$CR^f$=$CR^f$-$L^2$-, -$L^1$-$CR^f$=$CR^f$-$CR^f$=$CR^f$-$L^2$-, -$L^1$-$NR^b$-$CR^1_2$-C≡C-$L^2$-, -$L^1$-C≡C-$CR^f_2$-$NR^b$-$L^2$-, -$L^1$-C≡C-$L^2$-, and -$L^1$-C≡C-C≡C-$L^2$-,

[C3]

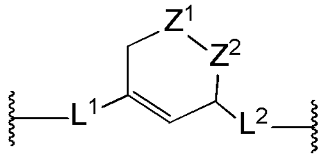 and 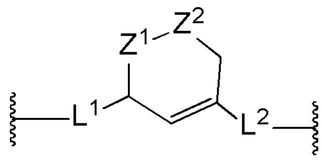

$L^1$ and $L^2$ each independently represent a single bond or a $C_1$-$C_6$ alkylene group,
$R^f$ each independently represents a hydrogen atom, an optionally substituted $C_1$-$C_{20}$ alkyl group or an optionally substituted aryl group,
$Z^1$ and $Z^2$ each independently represent -$NR^c$- or -$CR^dR^e$-, $R^c$, $R^d$, and $R^e$ are each independently a hydrogen atom or an optionally substituted $C_1$-$C_6$ alkyl group, or $R^c$ and $R^d$ together with $Z^1$ and $Z^2$ to which they are bonded form a 5- or 6-membered ring structure, and the ring structure may be substituted with 1 to 4 substituents;
$R^a$ is each independently a hydrogen atom or an optionally substituted $C_1$-$C_6$ alkyl group or respective $R^a$'s may be taken together to form a ring structure containing an oxygen atom to which they are bonded;
$R^b$ is each independently selected from among a hydrogen atom, an optionally substituted $C_1$-$C_{20}$ alkyl group, an optionally substituted aryl group, an optionally substituted allyl group, a propargyl group, and a nitrogen protecting group,
$R^1$ is a methyl group;
$R^2$ is a hydrogen atom or an optionally substituted $C_1$-$C_6$ alkyl group;
$R^3$ is a methyl group;
$R^4$ is selected from among a hydrogen atom, an optionally substituted $C_1$-$C_6$ alkyl group, an optionally substituted aryl group, an optionally substituted allyl group, a propargyl group, and a protecting group for a phenolic hydroxyl group, and
$R^5$ represents CN, a hydroxyl group or a leaving group containing an oxygen atom, a sulfur atom, a nitrogen atom, or a phosphorus atom.

[2] The compound according to [1], which is represented by the following formula (Ia) or a pharmaceutically acceptable salt thereof:

[C4]

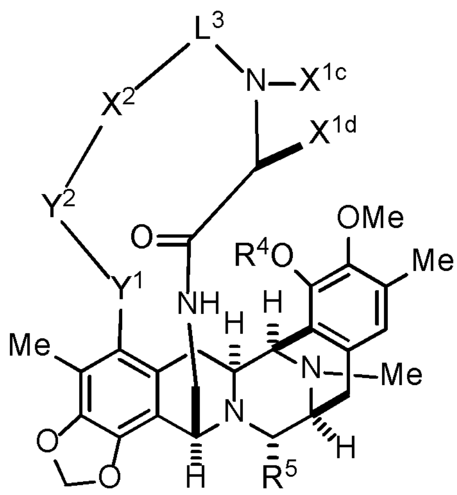

(Ia)

wherein

$X^2$, $Y^1$, $Y^2$, $R^4$, and $R^5$ are as defined in [1],
$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,
$X^{1d}$ is selected from among a hydrogen atom, a methyl group, and substituents corresponding to various natural/unnatural amino acid side chains,

$L^3$ is selected from the group consisting of a single bond, an optionally substituted alkylene group, an optionally substituted alkenylene group, a carbonyl group, -C(=S)-, - C(=$NR^b$)-, -C(O)O-, -C(O)$NR^b$-, -OC(O)-, -$NR^b$-, an ether group, a thioether group, and combinations thereof,

$R^b$ is selected from among a hydrogen atom, an optionally substituted $C_1$-$C_{20}$ alkyl group, an optionally substituted aryl group, an optionally substituted allyl group, a propargyl group, and a nitrogen protecting group, and Me represents a methyl group.

[3] The compound according to [1] or [2], which is represented by the following formula (Ic), formula (Id), formula (Ie), formula (If), formula (Ig), or formula (Ih), or a pharmaceutically acceptable salt thereof:

[C5]

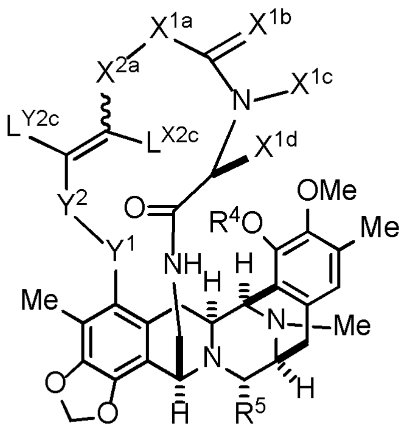

(Ic) ,

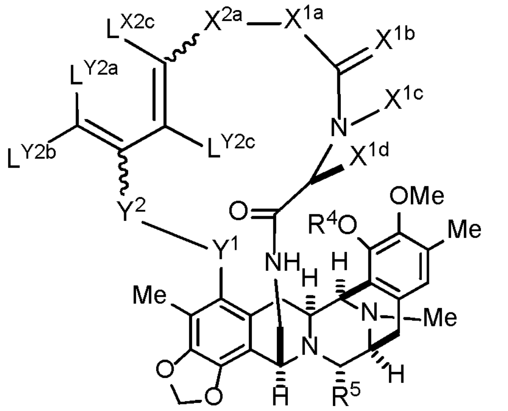

(Id) ,

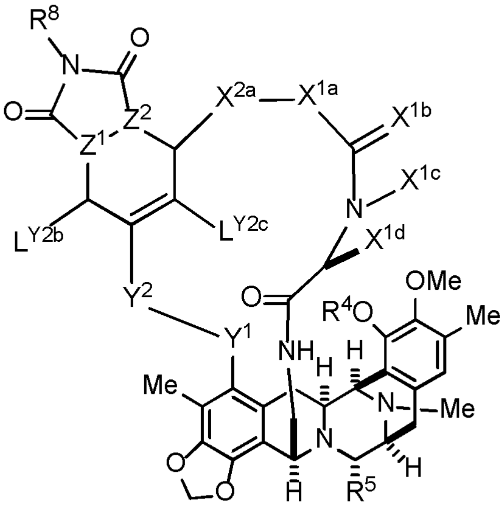

(Ie) ,

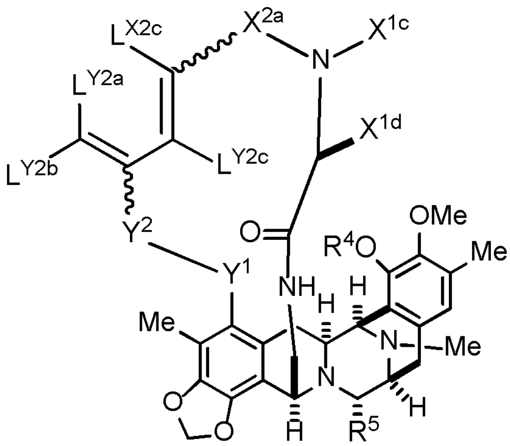

(If) ,

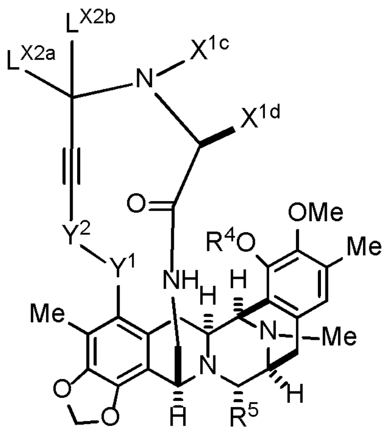

(Ig) ,

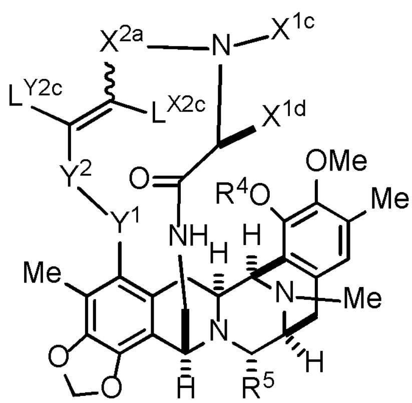

(Ih)

wherein

$X^{1a}$ represents an oxygen atom, a sulfur atom, $-NR^b-$, or an optionally substituted methylene group,
$X^{1b}$ represents an oxygen atom, a sulfur atom, $=NR^b$, or an optionally substituted methylene group,
$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,
$X^{1d}$ is selected from among a hydrogen atom, a methyl group, and substituents corresponding to various natural/unnatural amino acid side chains,
$X^{2a}$ represents an optionally substituted $C_1$-$C_6$ alkylene group,
$L^{X2a}$, $L^{X2b}$ and $L^{X2c}$ each independently represent a hydrogen atom, an optionally substituted $C_1$-$C_{20}$ alkyl group or an optionally substituted aryl group,
$Y^1$ is a divalent group selected from the group consisting of an ether group, a thioether group, an optionally substituted $C_1$-$C_6$ alkylene group, and $-NR^b-$;
$Y^2$ is a divalent group selected from the group consisting of an optionally substituted alkylene group, an optionally substituted alkenylene group, $-NR^bC(O)-$, $-C(O)NR^b-$, -C(O)O-, -OC(O)-, $-NR^bC(O)O-$, $-OC(O)NR^b-$, -OC(O)O-, a carbonyl group, -C(=S)-, $-C(=NR^b)-$, $-NR^b-$, a sulfonyl group, an ether group, a thioether group, and an amino acid residue,
$L^{Y2a}$, $L^{Y2b}$ and $L^{Y2c}$ each independently represent a hydrogen atom, an optionally substituted $C_1$-$C_{20}$ alkyl group or an optionally substituted aryl group,
$Z^1$ and $Z^2$ each independently represent N or $CR^e$,
$R^e$ is a hydrogen atom or an optionally substituted $C_1$-$C_6$ alkyl group,
$R^4$ is selected from among a hydrogen atom, an optionally substituted $C_1$-$C_6$ alkyl group, an optionally substituted aryl group, an optionally substituted allyl group, a propargyl group, and a protecting group for a phenolic hydroxyl group,
$R^5$ represents CN, a hydroxyl group, or a leaving group containing an oxygen atom, a sulfur atom, a nitrogen atom, or a phosphorus atom,
$R^8$ represents a hydrogen atom, an optionally substituted aryl group, an optionally substituted $C_1$-$C_{20}$ alkyl group, an optionally substituted allyl group, a propargyl group, or a nitrogen protecting group,
$R^b$ is each independently selected from among a hydrogen atom, an optionally substituted $C_1$-$C_{20}$ alkyl group, an optionally substituted aryl group, an optionally substituted allyl group, a propargyl group, and a nitrogen protecting group, and
Me represents a methyl group.

[4] The compound according to [3] or a pharmaceutically acceptable salt thereof,
wherein, in formula (Ic) to formula (Ih),

$X^{1a}$ represents an oxygen atom or $-NR^b-$,
$X^{1b}$ represents an oxygen atom,
$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,
$X^{1d}$ represents a methyl group,
$X^{2a}$ represents a $C_1$-$C_3$ alkylene group,
$L^{X2a}$, $L^{X2b}$ and $L^{X2c}$ each independently represent a hydrogen atom, an optionally substituted $C_1$-$C_8$ alkyl group, or an optionally substituted aryl group,
$Y^1$ represents an ether group;
$Y^2$ represents a $C_1$-$C_3$ alkylene group;
$L^{Y2a}$, $L^{Y2b}$ and $L^{Y2c}$ are each independently selected from among a hydrogen atom, an optionally substituted $C_1$-$C_8$ alkyl group, and an optionally substituted aryl group;
$Z^1$ and $Z^2$ each independently represent N or $CR^e$,
$R^e$ is a hydrogen atom or an optionally substituted $C_1$-$C_6$ alkyl group,
$R^4$ is selected from among a hydrogen atom, an optionally substituted $C_1$-$C_6$ alkyl group, an optionally substituted aryl group, an optionally substituted allyl group, a propargyl group, a propargyl group, and a protecting group for a phenolic hydroxyl group,
$R^5$ represents CN, a hydroxyl group, or a leaving group containing an oxygen atom, a sulfur atom, a nitrogen atom, or a phosphorus atom,
$R^8$ is selected from among a hydrogen atom, an optionally substituted phenyl group, an optionally substituted $C_1$-$C_{20}$ alkyl group, an allyl group, a propargyl group, and a nitrogen protecting group,
$R^b$ is each independently selected from among a hydrogen atom, an optionally substituted $C_1$-$C_{20}$ alkyl group,

an optionally substituted aryl group, an optionally substituted allyl group, a propargyl group, and a nitrogen protecting group, and

Me represents a methyl group.

[5] The compound according to [3] or [4] or a pharmaceutically acceptable salt thereof,
wherein, in formula (Ic) to formula (Ih),

$X^{1a}$ represents an oxygen atom,
$X^{1b}$ represents an oxygen atom,
$X^{1c}$ is selected from among a hydrogen atom, a methyl group and a propargyl group,
$X^{1d}$ represents a methyl group,
$X^{2a}$ represents a $C_1$-$C_3$ alkylene group,
$L^{X2a}$, $L^{X2b}$ and $L^{X2c}$ each independently represent a hydrogen atom,
$Y^1$ represents an ether group;
$Y^2$ represents a $C_1$-$C_3$ alkylene group;
$L^{Y2a}$, $L^{Y2b}$ and $L^{Y2c}$ each independently represent a hydrogen atom,
$Z^1$ and $Z^2$ each independently represent a nitrogen atom or CH,
$R^4$ represents a hydrogen atom,
$R^5$ represents CN,
$R^8$ represents a hydrogen atom or an optionally substituted phenyl group, and
Me represents a methyl group.

[5a] [1],
wherein the compound has a macrocyclic structure (a macrocyclic structure containing $Y^1$ and $Y^2$; that is, a macrocyclic structure in which the 1-position and 5-position of a tetrahydroisoquinoline framework are linked with a linking group ad formed together with carbon atoms at the 9-position and 10-position in the tetrahydroisoquinoline framework) with a 10- to 20-membered ring (preferably a 12- to 18-membered ring, and more preferably a 14-to 17-membered ring).

[6] The compound according to any one of [1] to [5], which is selected from the following group, or a pharmaceutically acceptable salt thereof:

[C6]

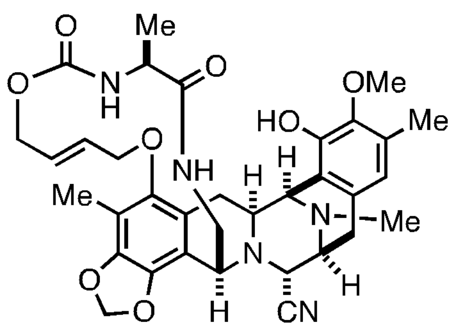
,
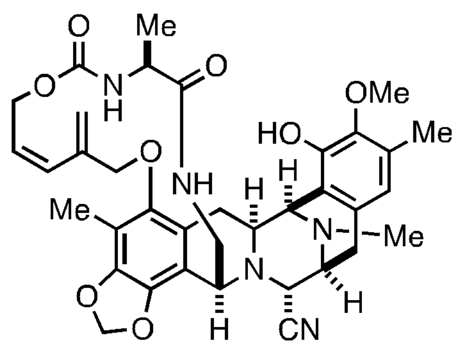
,

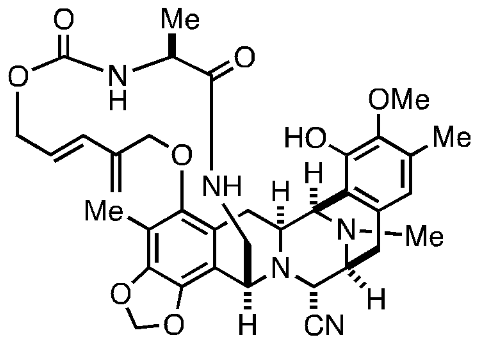

,

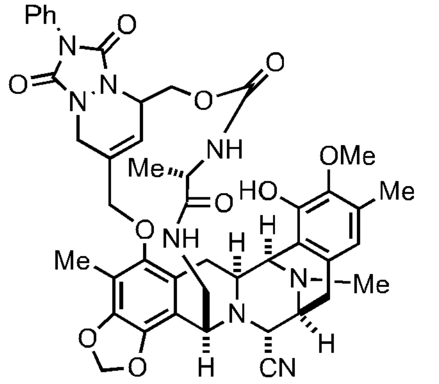

,

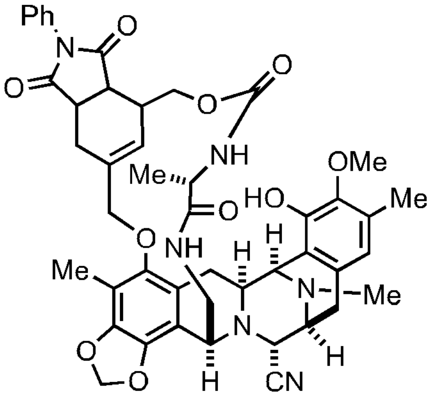

,

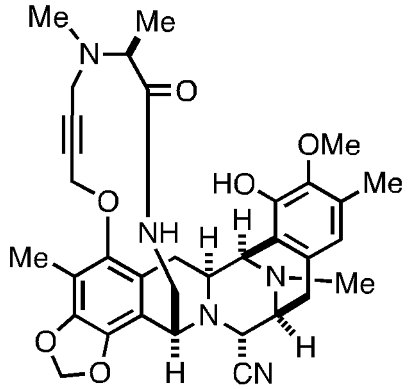

,

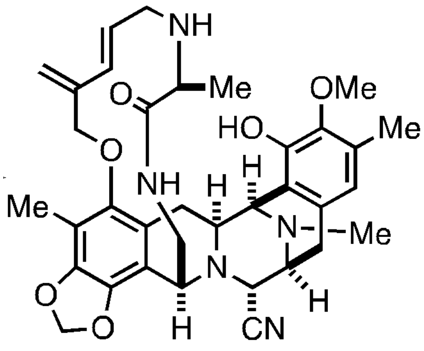

,

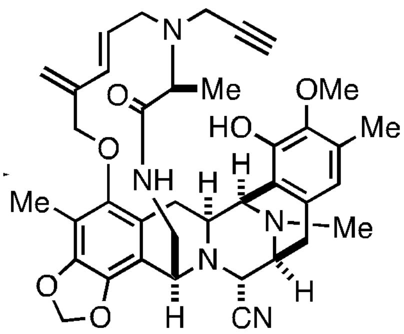

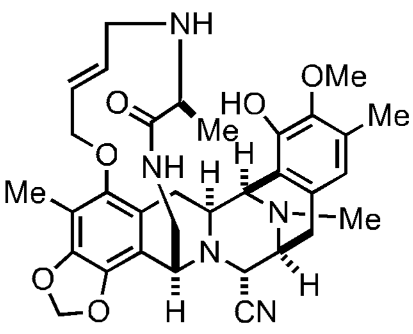

,

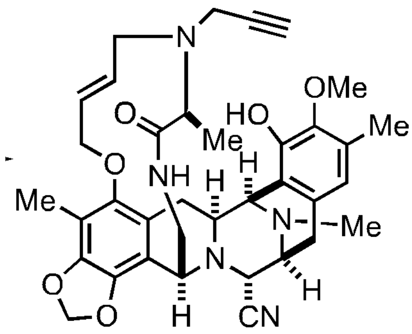

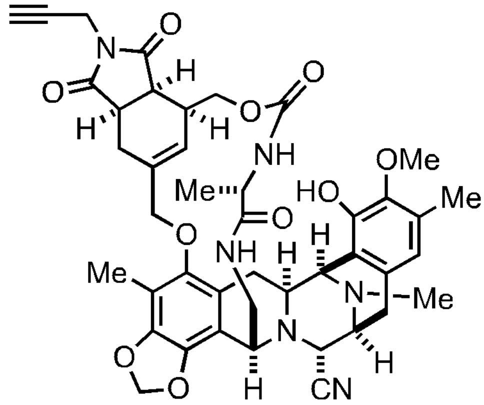

wherein Me represents a methyl group.

[7] A compound represented by the following formula (IIIc) or a pharmaceutically acceptable salt thereof:

[C7]

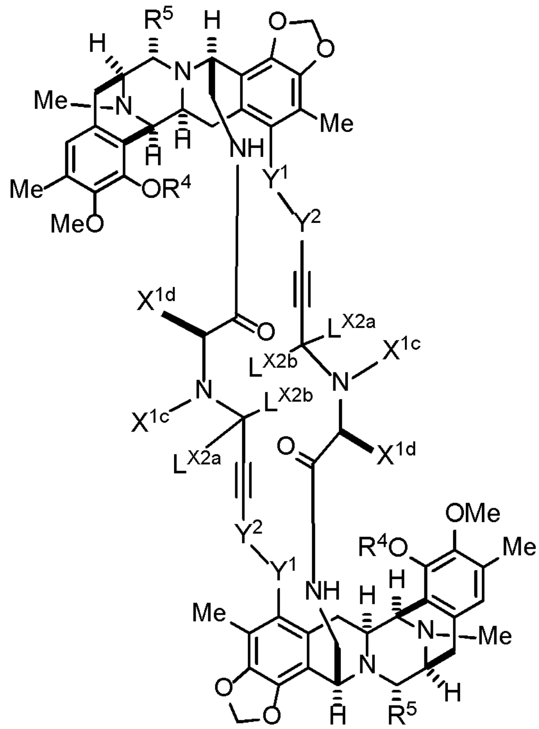

(IIIc)

wherein

$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,

$X^{1d}$ is selected from among a hydrogen atom, a methyl group, and substituents corresponding to various natural/unnatural amino acid side chains,

$L^{X2a}$ and $L^{X2b}$ each independently represent a hydrogen atom, an optionally substituted $C_1$-$C_{20}$ alkyl group or an optionally substituted aryl group,

$Y^1$ is a divalent group selected from the group consisting of an ether group, a thioether group, an optionally substituted $C_1$-$C_6$ alkylene group, and $-NR^b-$;

$Y^2$ is a divalent group selected from the group consisting of an optionally substituted alkylene group, an optionally

substituted alkenylene group, $-NR^bC(O)-$, $-C(O)NR^b-$, -C(O)O-, -OC(O)-, $-NR^bC(O)O-$, $-OC(O)NR^b-$, -OC(O)O-, a carbonyl group, -C(=S)-, $-C(=NR^b)-$, $-NR^b-$, a sulfonyl group, an ether group, a thioether group, and an amino acid residue,

$R^4$ is selected from among a hydrogen atom, an optionally substituted $C_1$-$C_6$ alkyl group, an optionally substituted aryl group, an optionally substituted allyl group, a propargyl group, and a protecting group for a phenolic hydroxyl group,

$R^5$ represents CN, a hydroxyl group, or a leaving group containing an oxygen atom, a sulfur atom, a nitrogen atom, or a phosphorus atom,

$R^b$ is selected from among a hydrogen atom, an optionally substituted $C_1$-$C_{20}$ alkyl group, an optionally substituted aryl group, an optionally substituted allyl group, a propargyl group, and a nitrogen protecting group, and Me represents a methyl group.

[8] The compound according to [7], which is the following compound, or a pharmaceutically acceptable salt thereof:

[C8]

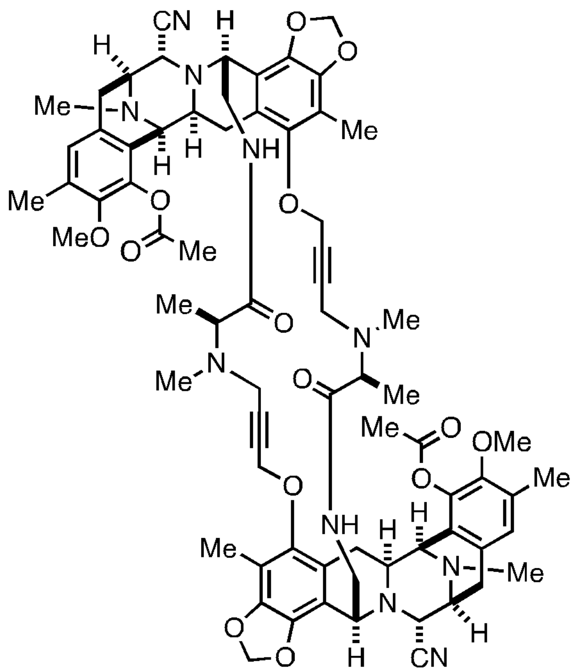

wherein Me represents a methyl group.

[9] A compound represented by the following formula (II) or a pharmaceutically acceptable salt thereof:

[C9]

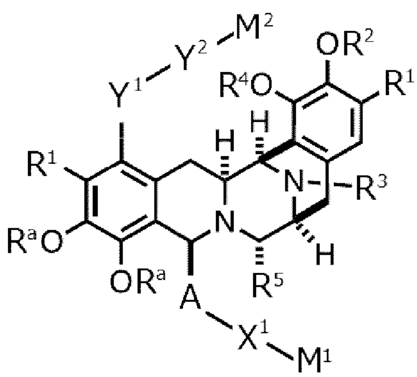

(II)

wherein

A is a single bond or an optionally substituted $C_1$-$C_6$ alkylene group;
$X^1$ is a divalent group selected from the group consisting of an optionally substituted alkylene group, an optionally substituted alkenylene group, $-NR^bC(O)-$, $-C(O)NR^b-$, -C(O)O-, -OC(O)-, $-NR^bC(O)O-$, $-OC(O)NR^b-$, -OC(O)O-, a carbonyl group, -C(=S)-, $-C(=NR^b)-$, $-NR^b-$, a sulfonyl group, an ether group, a thioether group, an amino acid residue, and combinations thereof;
$Y^1$ is a divalent group selected from the group consisting of a single bond, an ether group, a thioether group, an optionally substituted $C_1$-$C_6$ alkylene group, and $-NR^b-$;
$Y^2$ is a divalent group selected from the group consisting of an optionally substituted alkylene group, an optionally substituted alkenylene group, $-NR^bC(O)-$, $-C(O)NR^b-$, -C(O)O-, -OC(O)-, $-NR^bC(O)O-$, $-OC(O)NR^b-$, -OC(O)O-, a carbonyl group, -C(=S)-, $-C(=NR^b)-$, $-NR^b-$, a sulfonyl group, an ether group, a thioether group, an amino acid residue, and combinations thereof;
$M^1$ is selected from among a hydrogen atom, an optionally substituted alkyl group, an optionally substituted alkenyl group, an optionally substituted alkynyl group, $-N(R^b)_2$, an optionally substituted alkylene-$N(R^b)_2$, a hydroxyl group, a carbonyl group, a thiol group, and a halogen atom;
$M^2$ is selected from among a hydrogen atom, an optionally substituted alkyl group, an optionally substituted alkenyl group, an optionally substituted alkynyl group, $-N(R^b)_2$, an optionally substituted alkylene-$N(R^b)_2$, a hydroxyl group, a carbonyl group, a thiol group, and a halogen atom;
$R^b$ is each independently selected from among a hydrogen atom, an optionally substituted $C_1$-$C_6$ alkyl group, an optionally substituted aryl group, an optionally substituted allyl group, a propargyl group, and a nitrogen protecting group,
$R^a$ is each independently a hydrogen atom or an optionally substituted $C_1$-$C_6$ alkyl group or respective $R^a$'s may be taken together to form a ring structure containing an oxygen atom to which they are bonded;
$R^1$ is a methyl group;
$R^2$ is a hydrogen atom or an optionally substituted $C_1$-$C_6$ alkyl group;
$R^3$ is a methyl group;
$R^4$ is selected from among a hydrogen atom, an optionally substituted $C_1$-$C_6$ alkyl group, an optionally substituted aryl group, an optionally substituted allyl group, a propargyl group, and a protecting group for a phenolic hydroxyl group, and
$R^5$ represents CN, a hydroxyl group or a leaving group containing an oxygen atom, a sulfur atom, a nitrogen atom, or a phosphorus atom.

[10] The compound according to [9], which is represented by the following formula (IIc), formula (IId), formula (IIe), or formula (IIf) or a pharmaceutically acceptable salt thereof:

[C10]

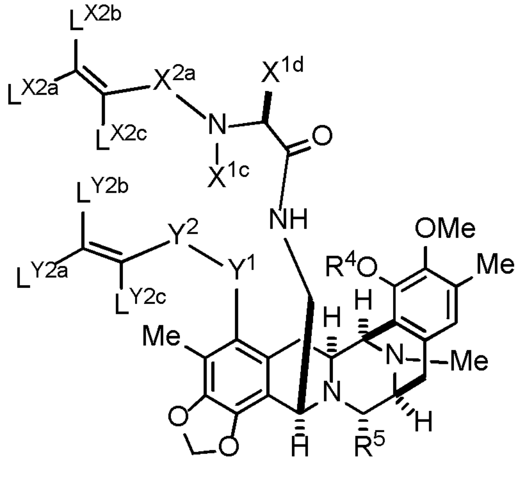

(IIc) ,

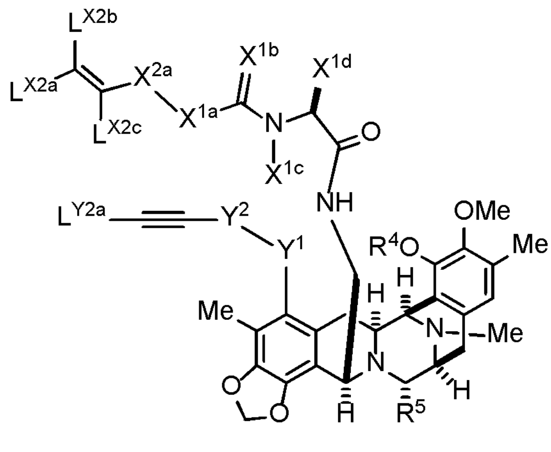

(IId) ,

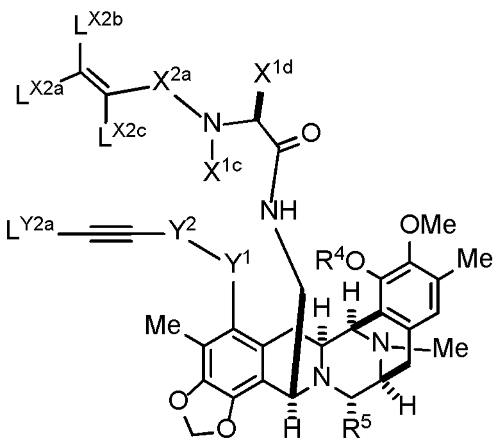

(IIe) ,

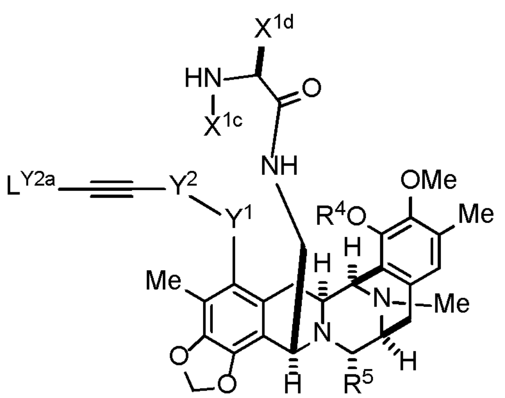

(IIf)

wherein

$X^{1a}$ represents an oxygen atom, a sulfur atom, $-NR^b-$, or an optionally substituted methylene group,
$X^{1b}$ represents an oxygen atom, a sulfur atom, $=NR^b$, or an optionally substituted methylene group,
$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,
$X^{1d}$ is selected from among a hydrogen atom, a methyl group, and substituents corresponding to various natural/unnatural amino acid side chains,
$X^{2a}$ represents an optionally substituted $C_1$-$C_6$ alkylene group,
$L^{X2a}$, $L^{X2b}$ and $L^{X2c}$ each independently represent a hydrogen atom, an optionally substituted $C_1$-$C_{20}$ alkyl group or an optionally substituted aryl group,
$Y^1$ is a divalent group selected from the group consisting of an ether group, a thioether group, an optionally substituted $C_1$-$C_6$ alkylene group, and $-NR^b-$;
$Y^2$ is a divalent group selected from the group consisting of an optionally substituted alkylene group, an optionally substituted alkenylene group, $-NR^bC(O)-$, $-C(O)NR^b-$, $-C(O)O-$, $-OC(O)-$, $-NR^bC(O)O-$, $-OC(O)NR^b-$, $-OC(O)O-$, a carbonyl group, $-C(=S)-$, $-C(=NR^b)-$, $-NR^b-$, a sulfonyl group, an ether group, a thioether group, and an amino acid residue,
$L^{Y2a}$, $L^{Y2b}$ and $L^{Y2c}$ each independently represent a hydrogen atom, an optionally substituted $C_1$-$C_{20}$ alkyl group or an optionally substituted aryl group,

$Z^1$ and $Z^2$ each independently represent N or $CR^e$,
$R^e$ is a hydrogen atom or an optionally substituted $C_1$-$C_6$ alkyl group,
$R^4$ is selected from among a hydrogen atom, an optionally substituted $C_1$-$C_6$ alkyl group, an optionally substituted aryl group, an optionally substituted allyl group, a propargyl group, and a protecting group for a phenolic hydroxyl group,
$R^5$ represents CN, a hydroxyl group, or a leaving group containing an oxygen atom, a sulfur atom, a nitrogen atom, or a phosphorus atom,
$R^b$ is selected from among a hydrogen atom, an optionally substituted $C_1$-$C_{20}$ alkyl group, an optionally substituted aryl group, an optionally substituted allyl group, a propargyl group, and a nitrogen protecting group, and
Me represents a methyl group.

[11] The compound according to [10] or a pharmaceutically acceptable salt thereof,
wherein, in formula (IIc) to formula (IIf),

$X^{1a}$ represents an oxygen atom or $-NR^b-$,
$X^{1b}$ represents an oxygen atom,
$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,
$X^{1d}$ represents a methyl group,
$X^{2a}$ represents a $C_1$-$C_3$ alkylene group,
$L^{X2a}$, $L^{X2b}$ and $L^{X2c}$ each independently represent a hydrogen atom, an optionally substituted $C_1$-$C_8$ alkyl group, or an optionally substituted aryl group,
$Y^1$ represents an ether group;
$Y^2$ represents a $C_1$-$C_3$ alkylene group;
$L^{Y2a}$, $L^{Y2b}$ and $L^{Y2c}$ are each independently selected from among a hydrogen atom, an optionally substituted $C_1$-$C_8$ alkyl group, and an optionally substituted aryl group;
$R^4$ is selected from among a hydrogen atom, an optionally substituted $C_1$-$C_6$ alkyl group, an aryl group, an allyl group, a propargyl group, a propargyl group, and a protecting group for a phenolic hydroxyl group,
$R^5$ represents CN, a hydroxyl group, or a leaving group containing an oxygen atom, a sulfur atom, a nitrogen atom, or a phosphorus atom,
$R^b$ is each independently selected from among a hydrogen atom, an optionally substituted $C_1$-$C_{20}$ alkyl group, an optionally substituted aryl group, an optionally substituted allyl group, a propargyl group, and a nitrogen protecting group, and
Me represents a methyl group.

[12] The compound according to [10] or [11] or a pharmaceutically acceptable salt thereof,
wherein, in formula (IIc) to formula (IIf),

$X^{1a}$ represents an oxygen atom,
$X^{1b}$ represents an oxygen atom,
$X^{1c}$ is selected from among a hydrogen atom, a methyl group, a propargyl group and a nitrogen protecting group,
$X^{1d}$ represents a methyl group,
$X^{2a}$ represents a $C_1$-$C_3$ alkylene group,
$L^{X2a}$, $L^{X2b}$ and $L^{X2c}$ each independently represent a hydrogen atom,
$Y^1$ represents an ether group;
$Y^2$ represents a $C_1$-$C_3$ alkylene group;
$L^{Y2a}$, $L^{Y2b}$ and $L^{Y2c}$ each independently represent a hydrogen atom,
$R^4$ represents a hydrogen atom or a protecting group for a phenolic hydroxyl group,
$R^5$ represents CN, and
Me represents a methyl group.

[13] The compound according to [9], which is selected from the following group:

[C11]

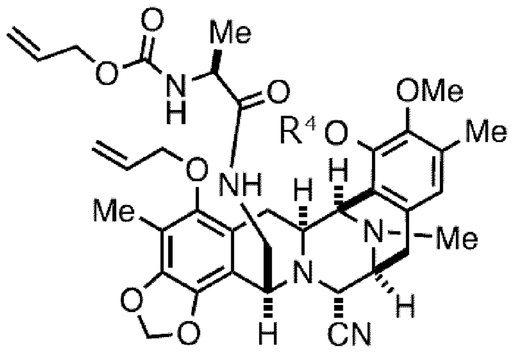

,

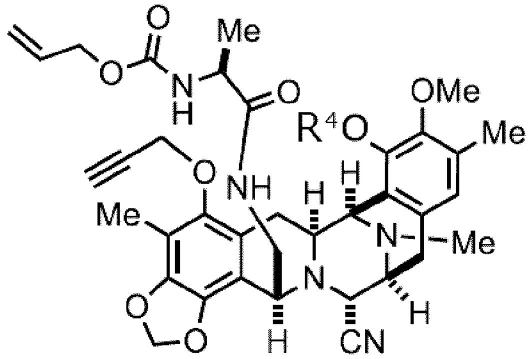

,

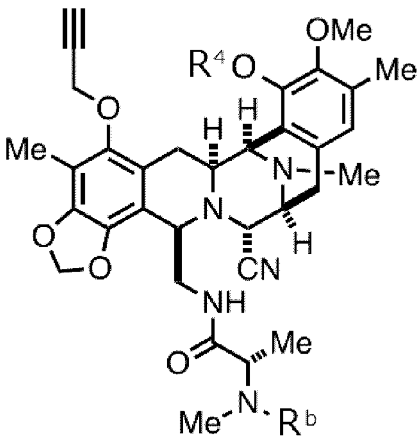

,

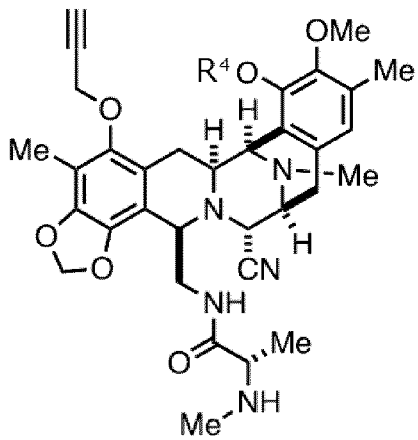

,

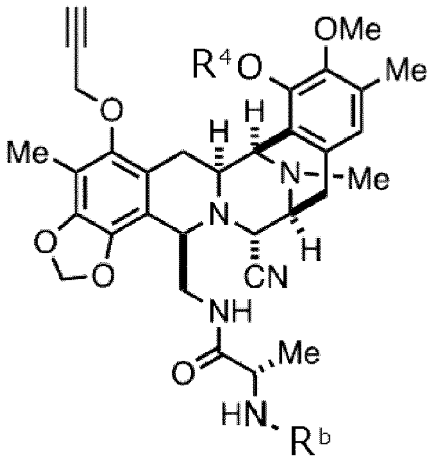

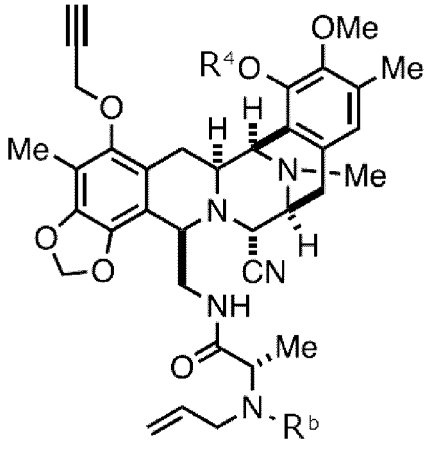

,

wherein Me represents a methyl group,

$R^4$ represents a hydrogen atom or a protecting group for a phenolic hydroxyl group, and
$R^b$ is each independently selected from among a hydrogen atom, an optionally substituted $C_1$-$C_{20}$ alkyl group, an optionally substituted aryl group, an optionally substituted allyl group, a propargyl group, and a nitrogen protecting group.

[14] A pharmaceutical composition containing the compound according to any one of [1] to [13] or a pharmaceutically

acceptable salt thereof, and a pharmaceutically acceptable carrier.

[14a] A pharmaceutical composition containing the compound according to any one of [1] to [13] or a pharmaceutically acceptable salt thereof, and a pharmaceutically acceptable carrier,

wherein the compound does not contain a protecting group for a phenolic hydroxyl group and does not contain a nitrogen protecting group.

[14b] A pharmaceutical composition containing the compound according to any one of [1] to [13] (provided that, in the formula, $R^4$ is a hydrogen atom, and $R^b$ is a substituent other than a nitrogen protecting group) or a pharmaceutically acceptable salt thereof, and a pharmaceutically acceptable carrier.

[15] A DNA alkylating agent containing the compound according to any one of [1] to [13] or a pharmaceutically acceptable salt thereof.

[15a] A DNA alkylating agent containing the compound according to any one of [1] to [13] or a pharmaceutically acceptable salt thereof,

wherein the compound does not contain a protecting group for a phenolic hydroxyl group and does not contain a nitrogen protecting group.

[15b] A DNA alkylating agent containing the compound according to any one of [1] to [13] (provided that, in the formula, $R^4$ is a hydrogen atom, and $R^b$ is a substituent other than a nitrogen protecting group) or a pharmaceutically acceptable salt thereof.

[16] An anti-cancer agent containing the compound according to any one of [1] to [13] or a pharmaceutically acceptable salt thereof.

[16a] An anti-cancer agent containing the compound according to any one of [1] to [13] or a pharmaceutically acceptable salt thereof, wherein the compound does not contain a protecting group for a phenolic hydroxyl group and does not contain a nitrogen protecting group.

[16b] An anti-cancer agent containing the compound according to any one of [1] to [13] (provided that, in the formula, $R^4$ is a hydrogen atom, and $R^b$ is a substituent other than a nitrogen protecting group) or a pharmaceutically acceptable salt thereof.

[17] The anti-cancer agent according to [16], [16a] or [16b],

wherein a target disease is selected from the group consisting of breast cancer, brain tumor, colorectal cancer, lung cancer, ovarian cancer, and gastric cancer.

[18] A method for producing a DNA alkylating agent or anti-cancer agent having a tetrahydroisoquinoline framework using the compound according to any one of [9] to [13].

[19] A use of the compound according to any one of [9] to [13], for producing a DNA alkylating agent or anti-cancer agent having a tetrahydroisoquinoline framework.

[20] A method for producing a tetrahydroisoquinoline alkaloid compound containing a macrocyclic structure, the method including any one step of the following steps (A) to (C): step (A): subjecting a compound represented by the following formula (IIc) to a ring-closing olefin metathesis reaction in the presence of a ruthenium catalyst or a tungsten catalyst to obtain a compound represented by formula (Ic).

[C12]

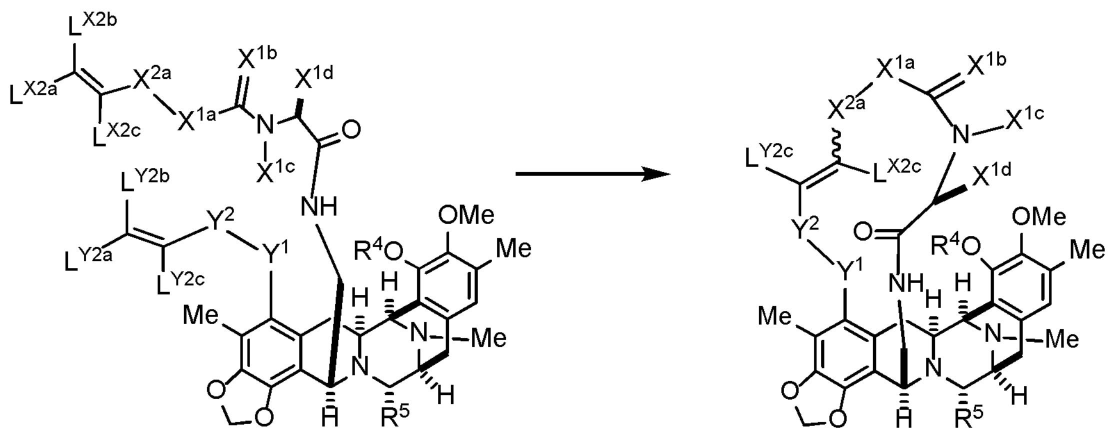

(IIc) (Ic)

step (B): subjecting a compound represented by the following formula (Iid) to a ring-closing enyne metathesis

reaction in the presence of a ruthenium catalyst or a tungsten catalyst to obtain a compound represented by formula (Id);

[C13]

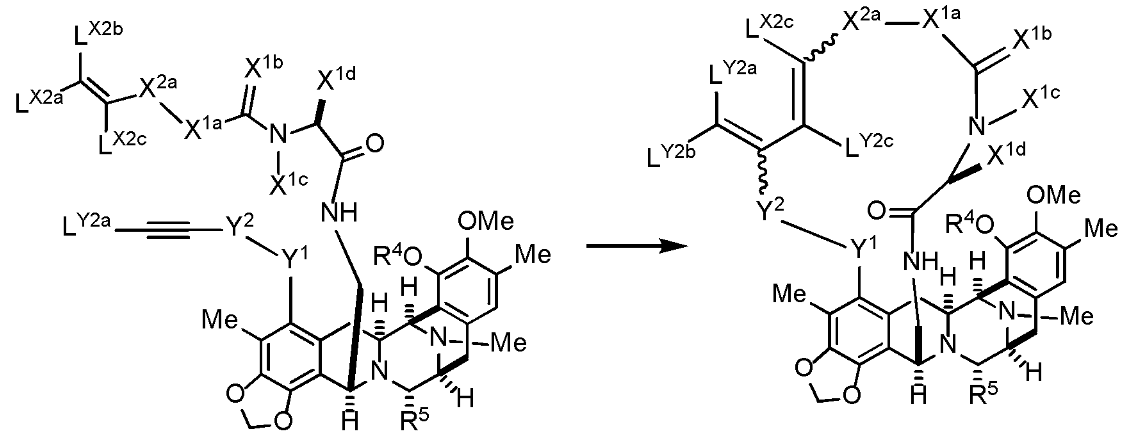

(IId) (Id)

step (C): subjecting a compound represented by the following formula (IIe) to a ring-closing enyne metathesis reaction in the presence of a ruthenium catalyst or a tungsten catalyst to obtain a compound represented by formula (If);

[C14]

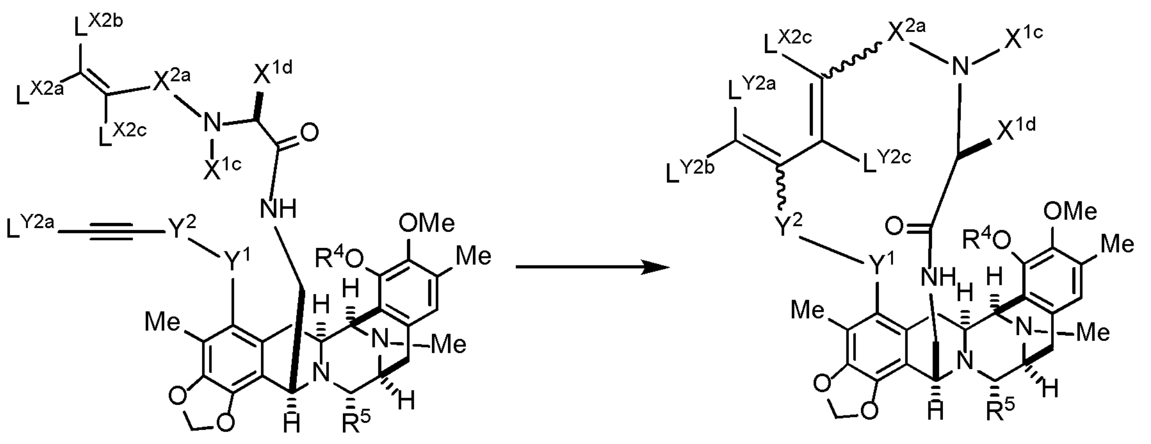

(IIe) (If)

wherein $X^{1a}$, $X^{1b}$, $X^{1c}$, $X^{1d}$, $X^{2a}$, $L^{X2a}$, $L^{X2b}$, $L^{X2c}$, $Y^1$, $Y^2$, $L^{Y2a}$, $L^{Y2b}$, $L^{Y2c}$, $R^4$, $R^5$ and Me are as defined in [10], and $R^4$ represents a protecting group for a phenolic hydroxyl group.

[21] A method for producing a tetrahydroisoquinoline alkaloid compound containing a macrocyclic structure, the method including the following step (D):

step (D): reacting a compound represented by formula (Id) with a compound represented by formula (IVa) to obtain a compound represented by formula (Ie):

[C15]

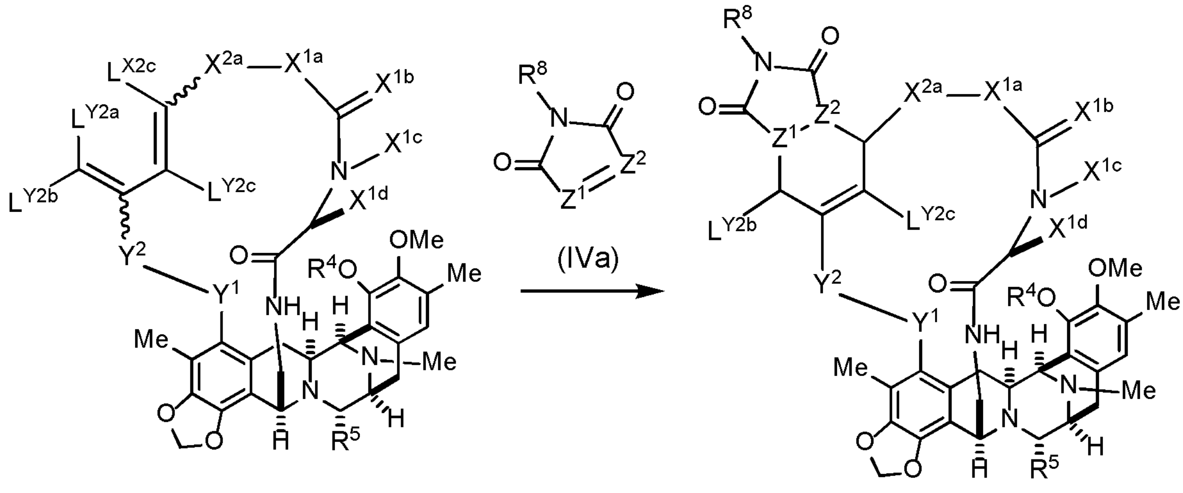

(Id) (IVa) (Ie)

wherein $X^{1a}$, $X^{1b}$, $X^{1c}$, $X^{1a}$, $X^{2a}$, $L^{X2c}$, $Y^1$, $Y^2$, $L^{Y2a}$, $L^{Y2b}$, $L^{Y2c}$, $R^4$, $R^5$, $R^8$, $Z^1$,$Z^2$ and Me are as defined in [10], and $R^4$ represents a protecting group for a phenolic hydroxyl group.

[22] A method for producing a tetrahydroisoquinoline alkaloid compound containing a macrocyclic structure, the method including the following step (E):

step (E): reacting a compound represented by the following formula (IIf) with a compound represented by formula (IVb) in the presence of a copper catalyst to obtain a compound represented by formula (Ig):

[C16]

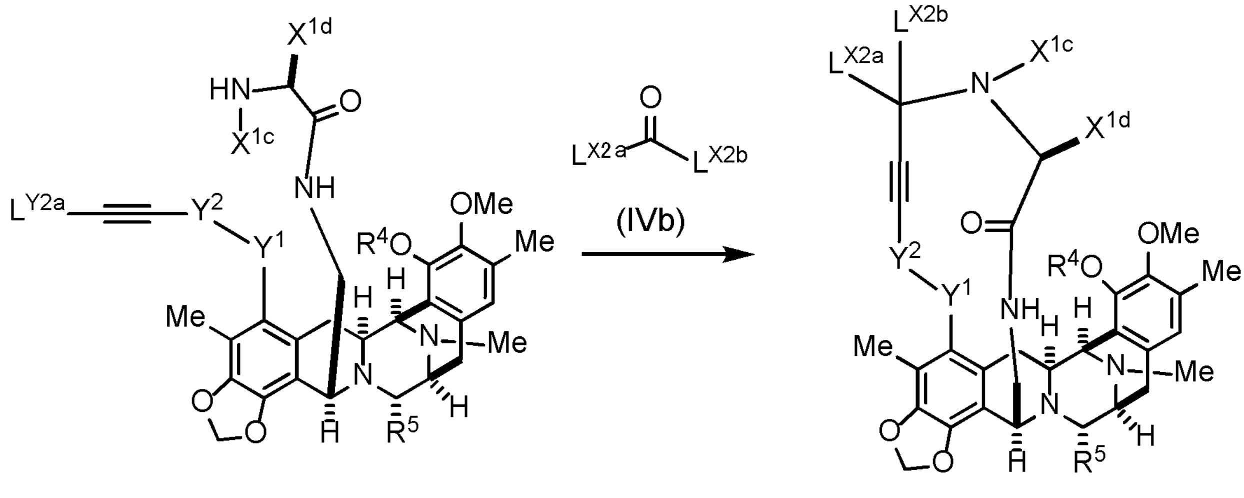

(IIf) (IVb) (Ig)

wherein $X^{1c}$, $X^{1d}$, $L^{X2a}$, $L^{X2b}$, $Y^1$, $Y^2$, $L^{Y2a}$, $R^4$, $R^5$, and Me are as defined in [10], and $R^4$ represents a protecting group for a phenolic hydroxyl group.

[23] A method for producing a tetrahydroisoquinoline alkaloid compound containing a macrocyclic structure, the method including the following step (F):

step (F): reacting a compound represented by the following formula (IIf) with a compound represented by formula (IVb) in the presence of a copper catalyst and a ligand to obtain a compound represented by formula (IIIc):

[C17]

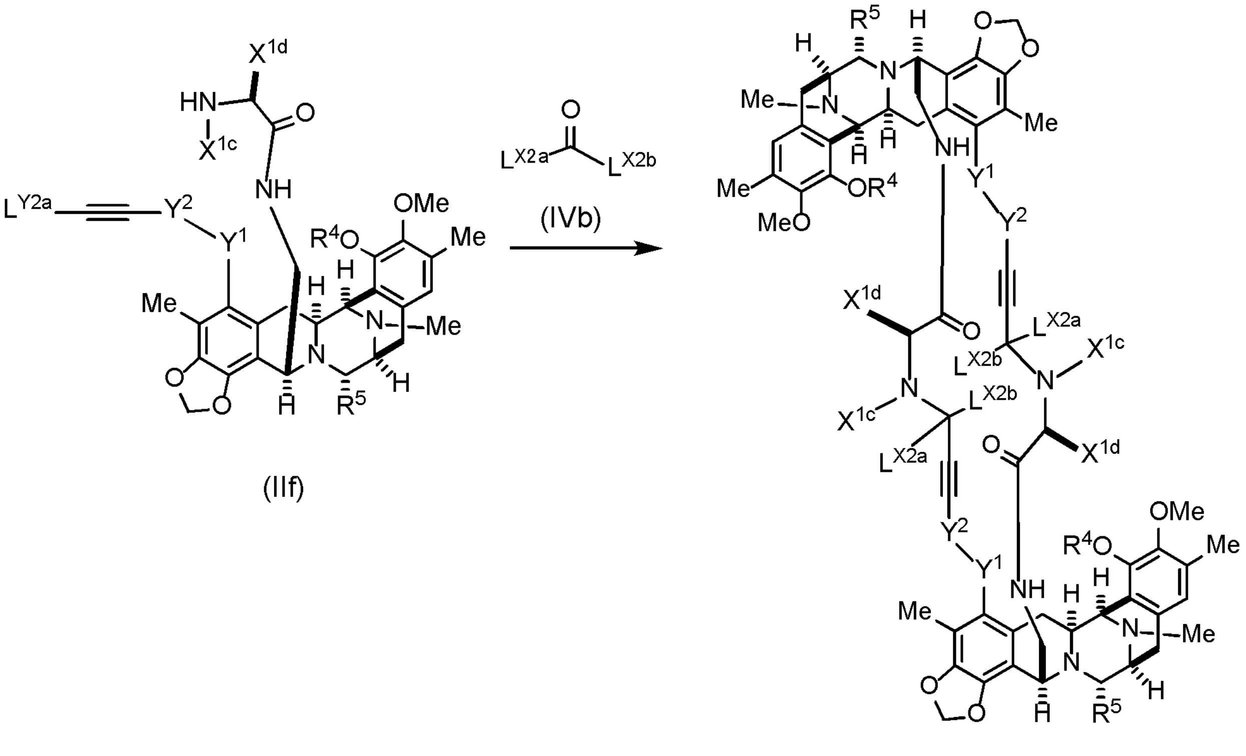

(IIf) (IVb) (IIIc)

wherein $X^{1c}$, $X^{1d}$, $L^{X2a}$, $L^{X2b}$, $Y^2$, $L^{Y2a}$, $R^4$, $R^5$, and Me are as defined in [10], and $R^4$ represents a protecting group for a phenolic hydroxyl group.

[24] A method for producing a tetrahydroisoquinoline alkaloid compound containing a macrocyclic structure, the method including the following step (G):

step (G): obtaining a compound of formula (Ii) according to a reaction of eliminating $CO_2$ from a compound in which $L^3$ is -OC(O)- in formula (Ia) (provided that a carbon atom of $L^3$ is bonded to a nitrogen atom of N-$X^{1c}$ in formula (Ia)):

[C18]

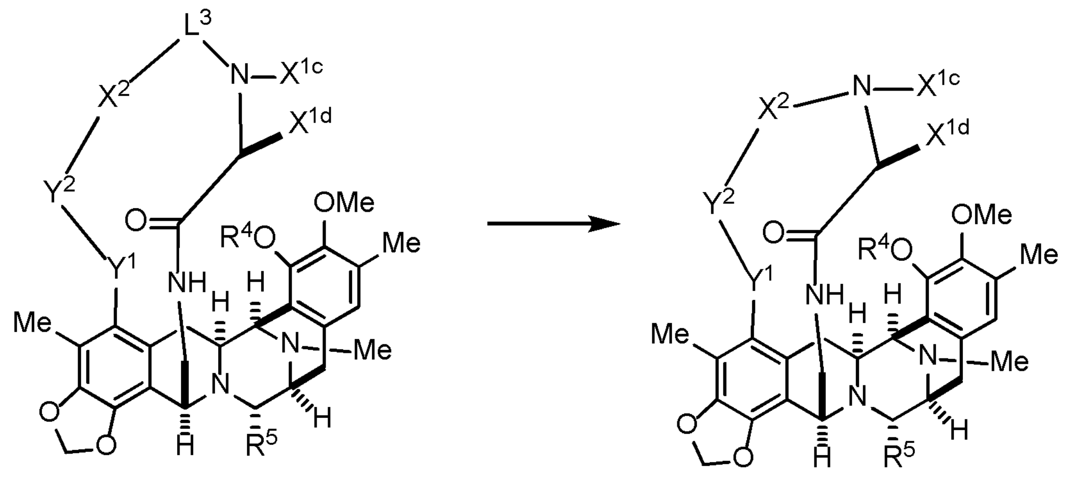

(Ia) (Ii)

wherein $X^{1c}$, $X^{1d}$, $X^2$, $Y^1$, $Y^2$, $R^4$ and Me are as defined in [2],
$L^3$ represents -OC(O)- (provided that a carbon atom of $L^3$ is bonded to a nitrogen atom of N-$X^{1c}$ in formula (Ia)).

[25] A method for producing a tetrahydroisoquinoline alkaloid compound containing a macrocyclic structure, the method including the following step (H):

step (H): obtaining a compound of formula (Ih) according to a reaction of eliminating $CO_2$ from a compound in which $X^{1a}$ and $X^{1b}$ are an oxygen atom (O) in formula (Ic):

[C19]

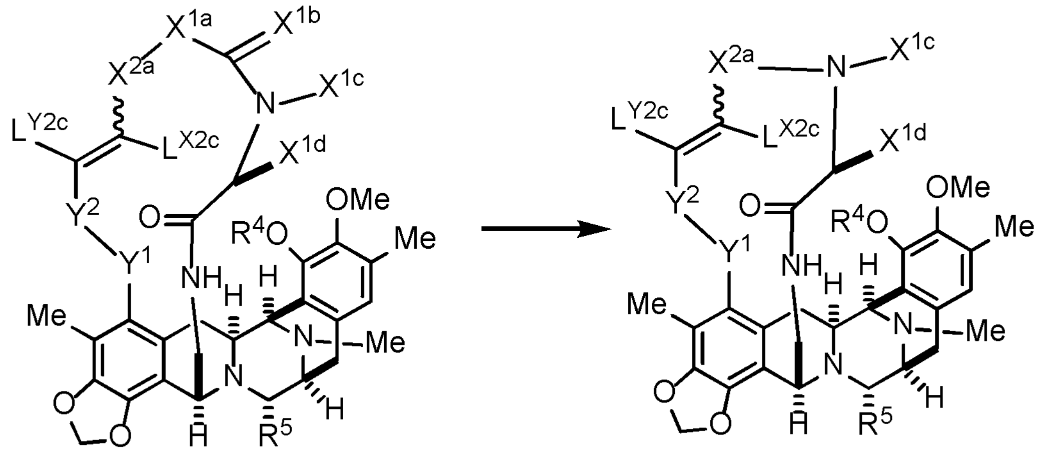

(Ic) (Ih)

wherein $X^{1c}$, $X^{1d}$, $X^{2a}$, $L^{X2c}$, $Y^1$, $Y^2$, $L^{Y2c}$, $R^4$, $R^5$, and Me are as defined in [3], and
$X^{1a}$ and $X^{1b}$ represent an oxygen atom (O).

The present invention has at least one or more of the following effects.

(1) In some embodiments, there is provided a novel compound having various modal macrocyclic structures linked to a THIQ framework.
(2) In some embodiments, the compound has an excellent DNA alkylating ability and antitumor activity.
(3) In some embodiments, since the compound has a functional group that can chemoselectively react with

various reagents in the molecule and can be derivatized into various analogs, it is possible to further improve the antitumor activity of a lead compound. Therefore, the present invention can contribute to the development of a novel antitumor drug.

(4) In some embodiments, there is provided a method for producing compounds having various modal macrocyclic structures linked to a THIQ framework from Cyanosafracin B. According to the production method, it is possible to efficiently synthesize various macrocyclic structures with few processes using Cyanosafracin B as a starting substance.

[Description of Embodiments]

Hereinafter, embodiments of the present invention will be described. The scope of the present invention is not limited by these descriptions, and embodiments other than the following examples can be appropriately changed and implemented without impairing the gist of the present invention.

1. Definition

X and Y in "$C_X$-$C_Y$" represent the number of carbon atoms. For example, "$C_1$-$C_4$" represents 1 to 4 carbon atoms.

In this specification, the "hydrocarbon group" is a group obtained by removing one, two or more hydrogen atoms from a linear, cyclic or branched saturated or unsaturated hydrocarbon having a specified number of carbon atoms. Specific examples thereof include an alkyl group, an alkenyl group, an alkynyl group, a cycloalkyl group, an aryl group, an alkylaryl group, an arylalkyl group, an alkylene group, an alkenylene group, an alkynylene group, a cycloalkylene group and combinations thereof.

The "unsaturated hydrocarbon group" is a hydrocarbon group having at least one unsaturated bond among hydrocarbon groups.

In this specification, the "alkyl group" is a saturated aliphatic hydrocarbon group that may be linear, branched, cyclic, or a combination thereof. A cyclic alkyl group is also called a cycloalkyl group. The number of carbon atoms in the alkyl group is not particularly limited, and it may be, for example, 1 to 20 ($C_1$-$C_{20}$), 1 to 15 ($C_1$-$C_{15}$), 1 to 10 ($C_1$-$C_{10}$), 1 to 8 ($C_1$-$C_8$), 1 to 6 ($C_1$-$C_6$), and 1 to 4 ($C_1$-$C_4$). Examples of $C_1$-$C_8$ alkyl group include a methyl, ethyl, n-propyl, isopropyl, n-butyl, isobutyl, sec-butyl, tert-butyl, n-pentyl, isopentyl, neopentyl, n-hexyl, isohexyl, n-heptyl, n-octyl, and cyclohexyl group.

In this specification, the "alkylene group" is a divalent group composed of a saturated aliphatic hydrocarbon that may be linear, branched, cyclic or a combination thereof. The number of carbon atoms in the alkylene group is, for example, 1 to 20 ($C_1$-$C_{20}$), 1 to 15 ($C_1$-$C_{15}$), 1 to 10 ($C_1$-$C_{10}$), 1 to 8 ($C_1$-$C_8$), 1 to 6 ($C_1$-$C_6$), 1 to 4 ($C_1$-$C_4$), or 1 to 3 ($C_1$-$C_3$).

In this specification, the "alkenyl group" is a monovalent group composed of an unsaturated hydrocarbon that may be linear, branched, cyclic or a combination thereof, which has at least one carbon-carbon double bond at an arbitrary position. The "alkenylene group" is a divalent group composed of an unsaturated hydrocarbon that may be linear, branched, cyclic, or a combination thereof, which has at least one carbon-carbon double bond at an arbitrary position. Examples of "alkenyl" and "alkenylene" include monoenes, dienes, trienes and tetraenes, but the present invention is not limited thereto. The number of carbon atoms in the alkenyl group or the alkenylene group is, for example, 2 to 20 ($C_2$-$C_{20}$), 2 to 15 ($C_2$-$C_{15}$), 2 to 10 ($C_2$-$C_{10}$), 2 to 8 ($C_2$-$C_8$), 2 to 6 ($C_2$-$C_6$), 2 to 4 ($C_2$-$C_4$), or 2 to 3 ($C_2$-$C_3$). Examples of $C_2$-$C_6$ alkenyl groups include a vinyl, propenyl, butenyl, pentenyl, and hexenyl group. Examples of $C_2$-$C_6$ alkenylene groups include a vinylene, propenylene, butenylene, pentenylene, and hexenylene group.

In this specification, the "alkynyl group" is a monovalent group composed of an unsaturated hydrocarbon that may be linear, branched, cyclic, or a combination thereof, which has at least one carbon-carbon triple bond at an arbitrary position. The "alkynylene group" is a divalent group composed of an unsaturated hydrocarbon that may be linear, branched, cyclic, or a combination thereof, which has at least one carbon-carbon triple bond at an arbitrary position. The number of carbon atoms in the alkynyl group or the alkynylene group is, for example, 2 to 15 ($C_2$-$C_{15}$), 2 to 10 ($C_2$-$C_{10}$), 2 to 6 ($C_2$-$C_6$), 2 to 4 ($C_2$-$C_4$), or 2 to 3 ($C_2$-$C_3$). Examples of $C_2$-$C_6$ alkynyl groups include an acetyl, ethynyl, propynyl (for example, propargyl, 1-propynyl), butynyl, pentynyl, and hexynyl group. Examples of $C_2$-$C_6$ alkynylene group include an acetylene, ethynylene, propynylene, butynylene, pentynylene, and hexynylene group.

In this specification, an alkyl group, alkylene group, alkenyl group, alkenylene group, alkynyl group, or alkynylene group may have one or more arbitrary substituents. The substituent may include, for example, an alkoxy group, a halogen atom (which may be a fluorine atom, a chlorine atom, a bromine atom, or an iodine atom), an amino group, a mono- or di-substituted amino group, a substituted silyl group, an acyl group, an aryl group, a heteroaryl group, and a heterocyclic group, but the present invention is not limited thereto. When an alkyl group has two or more substituents, these may be the same as or different from each other. The same applies to alkyl moieties of other substituents (for example, an alkoxy group, and an arylalkyl group, etc.) containing alkyl moieties.

In this specification, the "acyl group" is a group in which a carbonyl group (-CO-) is bonded to a terminal of an

alkyl group or an aryl group. The acyl group may be either an aliphatic acyl group or an aromatic acyl group, and may be an aliphatic acyl group having an aromatic group as a substituent. Specifically, an acetyl group may be exemplified.

In this specification, the "allyl group" represents a monovalent group represented by $CH_2$=CH-$CH_2$-.

In this specification, the "ether group" represents a divalent group represented by - oxygen atom (O)-.

In this specification, the "thioether group" represents a divalent group represented by - sulfur atom (S)-.

In this specification, the "sulfonyl group" represents a divalent group represented by - $S(=O)_2$-.

In this specification, the "carbonate group" represents a divalent group represented by -OC(O)O-.

In this specification, the "acetal group" represents a divalent group represented by R-C(OR)(OR)-R.

In this specification, the "carbamate group" represents a divalent group represented by -NRC(=O)O- or-OC(=O)NR-. R is an arbitrary substituent, and is typically a hydrogen atom, an alkyl group or an aryl group.

In this specification, the "amide group" represents a divalent group represented by - NRC(=O)- or -C(=O)NR-. R is an arbitrary substituent, and is typically a hydrogen atom, an alkyl group or an aryl group.

In this specification, the "ester group" represents a divalent group represented by - C(=O)O- or -OC(=O)-.

In this specification, the "silyl group" represents a monovalent group represented by $R_3$Si-. R is an arbitrary substituent and is typically an alkyl group or an aryl group.

In this specification, an "aryl" is an aromatic monocyclic or condensed polycyclic hydrocarbon. The aryl group is a monovalent or divalent group derived from an aryl. Examples thereof include a phenyl group and a naphthyl group.

The "alkylaryl group" is an aryl group to which one or more alkyl groups are bonded.

The "arylalkyl group" is an alkyl group to which an aryl ring is bonded.

In this specification, a "heteroaryl" is an aromatic monocycle or condensed polycycle containing one or more (for example, 1 to 5, or 1 to 3) heteroatoms selected from among an oxygen atom (O), a nitrogen atom (N) and a sulfur atom (S), and a carbon atom. The heteroaryl group is a monovalent or divalent group derived from a heteroaryl.

A "carbocycle" is a monocycle or condensed polycycle containing carbon atoms as ring-constituting atoms in the ring structure.

A "heterocycle" is a monocycle or condensed polycycle containing one or more (for example, 1 to 5, or 1 to 3) heteroatoms selected from among an oxygen atom (O), a nitrogen atom (N) and a sulfur atom (S) in addition to carbon atoms as ring-constituting atoms in the ring structure. The heterocycle includes a heteroaryl and a non-aromatic heterocycle. The heterocycle may be a saturated heterocycle or an unsaturated heterocycle. The "non-aromatic heterocycle" is a non-aromatic monocycle or condensed polycycle containing one or more (for example, 1 to 5, or 1 to 3) heteroatoms selected from among an oxygen atom (O), a nitrogen atom (N) and a sulfur atom (S) in addition to carbon atoms as ring-constituting atoms in the ring structure.

The heterocycle is typically a 3- to 20-membered monocyclic or polycyclic saturated, fully unsaturated or partially unsaturated heterocyclic group containing at least one (preferably 1 to 5, and more preferably 1 to 3) heteroatom selected from among an oxygen atom (O), a nitrogen atom (N) and a sulfur atom (S). The "heterocyclic group" is a monovalent or divalent group derived from a heterocycle.

In this specification, an aryl group, a heteroaryl group, a carbocycle, and a heterocyclic group may have one or more arbitrary substituents on the ring. Examples of such arbitrary substituents include an oxo group (=O), an alkyl group, an alkenyl group, an alkoxy group, a halogen atom, an amino group, a mono- or di-substituted amino group, a substituted silyl group, and an acyl group, but the present invention is not limited thereto. When an aryl group has two or more substituents, they may be the same as or different from each other. The same applies to aryl moieties of other substituents containing aryl moieties (for example, an aryloxy group and an arylalkyl group, etc.).

In this specification, the "alkoxy group" has a structure in which the alkyl group is bonded to an oxygen atom, and examples thereof include saturated alkoxy groups that may be linear, branched, cyclic or a combination thereof. The number of carbon atoms in the alkoxy group is not particularly limited, and it may be, for example, 1 to 6 ($C_1$-$C_6$) or 1 to 4 ($C_1$-$C_4$).

In this specification, the "halogen atom" is a fluorine atom (F), a chlorine atom (Cl), a bromine atom (Br), or an iodine atom (I).

In this specification, a "leaving group containing an oxygen atom, a sulfur atom, a nitrogen atom, or a phosphorus atom" is a substituent that readily dissociates from a compound with an electron pair that contributes to covalent bond formation. Examples thereof include a nitrile group, hydroxyl group, carboxylate group (-O-CO-R), methylsulfonyl group, trifluoromethanesulfonyl group, isocyanate, azide group, and diphenylphosphoryl group.

In this specification, the "amino acid" may be any compound as long as it is a compound having both an amino group and a carboxy group, including natural and unnatural amino acids. The amino acid may be a neutral amino acid, a basic amino acid, or an acidic amino acid, and in addition to amino acids that themselves function as transmitters such as neurotransmitters, amino acids that are components constituting polypeptide compounds such as biologically active peptides (including oligopeptides in addition to dipeptides, tripeptides, and tetrapeptides) and proteins can be used, and examples thereof include $\alpha$ amino acids, $\beta$ amino acids, and $\gamma$ amino acids. As the amino acid, it is preferable to use an optically active amino acid. For example, for $\alpha$ amino acids, either D- or L-amino acids may be used, and it may be

preferable to select optically active amino acids that function in vivo.

In this specification, the "amino acid residue" has the same partial structure remaining after a hydroxyl group is removed from a carboxy group of an amino acid, and has the same structure as a so-called N-terminal residue. However, this does not exclude cases in which a plurality of amino acid residues are linked together, and in such cases, the C-terminal amino acid residue may have a partial structure obtained by removing a hydroxyl group from a carboxy group of an amino acid as described above and removing a hydrogen atom from an amino group, and intermediate and N-terminal amino acid residues can be linked in the same manner as in general peptide chains.

In this specification, when a group is defined as "optionally substituted," the type of the substituent, the substitution position, and the number of substituents are not particularly limited, and when two or more substituents are provided, these may be the same as or different from each other. The number of substituents is typically, for example, 1 to 4, 1 to 3, 1 to 2, or 1. Examples of substituents include an alkyl group, an alkoxy group, a hydroxyl group, a carboxyl group, a halogen atom, a sulfo group, an amino group, an alkoxycarbonyl group, an oxo group (=O), an acyl group, a thiol group (-SH), a propargyl group, and an aryl group, but the present invention is not limited thereto. These substituents may further have a substituent. Examples of such groups include a halogenated alkyl group, but the present invention is not limited thereto.

In this specification, the term "ring structure" refers to a heterocycle or a carbocycle when it is formed by the combination of two substituents, and such a ring may be saturated, unsaturated, or aromatic. Therefore, it includes the cycloalkyl, cycloalkenyl, cycloalkynyl, aryl, heteroaryl, and non-aromatic heterocycle as defined above.

In this specification, certain substituents can form a ring structure with other substituents, and when such substituents are bonded together, it can be understood by those skilled in the art that a certain substitution, for example, bonding to hydrogen, is formed. Therefore, when it is described that certain substituents form a ring structure together, it can be understood by those skilled in the art that the ring structure can be formed according to a general chemical reaction and is easily produced. All such ring structures and procedures for forming them are within the range recognized by those skilled in the art. In addition, the heterocyclic structure may have an arbitrary substituent on the ring.

## 2. THIQ alkaloid compound containing macrocyclic structure

One aspect of the present invention relates to a tetrahydroisoquinoline (THIQ) alkaloid compound containing a macrocyclic structure. The compound of the present aspect has a ring-closed macrocyclic structure linked to the THIQ framework. More specifically, the compound of the present aspect has a macrocyclic structure in which the 1-position and 5-position of the THIQ framework are linked with a linking group and formed together with carbon atoms at the 9-position and 10-position in the THIQ framework. The macrocyclic structure may contain heteroatoms such as an oxygen atom, a nitrogen atom, and a sulfur atom in addition to carbon atoms as constituent atoms. In some embodiments, the macrocyclic structure includes an amino acid residue, a linear or branched unsaturated hydrocarbon group, a sulfonyl group, an ester group, an amide group, a carbamate group, a carbonate group, an ether group, an amino group, a thioether group, a carbonyl group and a combination thereof.

In some embodiments, the macrocyclic structure is formed by linking the 1-position and 5-position of the THIQ framework in one molecule with a linking group, and is typically a 10- to 20-membered ring (preferably a 12- to 18-membered ring, and more preferably a 14-to 17-membered ring).

In some embodiments, the macrocyclic structure is formed by linking the 1-position and 5-position of the THIQ framework of different molecules with a linking group. In some embodiments, the macrocyclic structure is formed by linking the 1-position of the THIQ framework of the first molecule and the 5-position of the THIQ framework of the second molecule with a first linking group and by linking the 5-position of the THIQ framework of the first molecule and the 1-position of the THIQ framework of the second molecule with a second linking group, and is typically a 15- to 40-membered ring (preferably a 24- to 36-membered ring, and more preferably a 28- to 30-membered ring).

In this specification, the number of members of the macrocyclic structure is a total number (minimum number) of atoms constituting the ring of the ring structure formed by linking the 1-position and 5-position of the THIQ framework with a linking group.

A compound according to one aspect of the present invention is a compound represented by the following formula (I).

[C20]

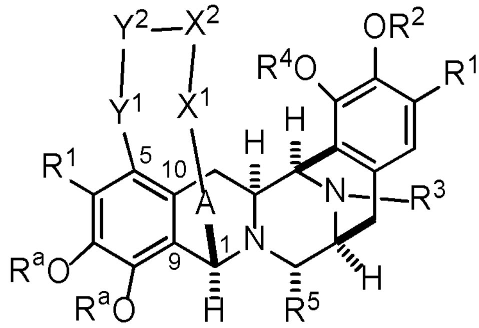

(I)

In formula (I), $X^2$ and $Y^2$ are linked to each other to form a ring structure (macrocyclic structure) containing $X^1$ and $Y^1$ to which they are bonded. That is, the "macrocyclic structure" in formula (I) is formed by linking to the THIQ framework in the manner of "A-$X^1$-$X^2$-$Y^2$-$Y^1$" starting from the 1-position and 5-position of the THIQ framework. Therefore, the "macrocyclic structure" in formula (I) has a cyclic structure linked in the manner of the carbon atom at the 1-position of the THIQ framework-A-$X^1$-$X^2$-$Y^2$-$Y^1$-carbon atom at the 5-position of the THIQ framework-carbon atom at the 10-position in the THIQ framework-carbon atom at the 9-position in the THIQ framework-.

In formula (I), the macrocyclic structure is, for example, a 10- to 20-membered ring (preferably a 12- to 18-membered ring, and more preferably a 14- to 17-membered ring). The number of members of the macrocyclic structure in formula (I) is a minimum total number of atoms constituting the ring composed of the carbon atom at the 1-position of the THIQ framework-A-$X^1$-$X^2$-$Y^2$-$Y^1$-carbon atom at the 5-position of the THIQ framework-carbon atom at the 10-position in the THIQ framework-carbon atom at the 9-position in the THIQ framework-.

In formula (I), A is a moiety starting from the macrocyclic structure linked to the 1-position of the THIQ framework, and A is a single bond or an optionally substituted $C_1$-$C_6$ alkylene group. Preferably, A may be a single bond, a methylene group, or an ethylene group. More preferably, A is a methylene group.

In this specification, when a substituent for A is described, the left side of the substituent described is bonded to the carbon atom at the 1-position of the THIQ framework, and the right side of the substituent described is bonded to $X^1$.

In formula (I), $X^1$ is a divalent group selected from the group consisting of an optionally substituted alkylene group, an optionally substituted alkenylene group, -$NR^bC(O)$-, -$C(O)NR^b$-, -C(O)O-, -OC(O)-, -$NR^bC(O)O$-, -$OC(O)NR^b$-, -OC(O)O-, a carbonyl group, - C(=S)-, -C(=$NR^b$)-, -$NR^b$-, a sulfonyl group, an ether group, a thioether group, an amino acid residue, and combinations thereof.

In this specification, when a substituent for $X^1$ is described, the left side of the substituent described is bonded to A, and the right side of the substituent described is bonded to $X^2$.

$X^1$ is a moiety having a spacer function. Preferably, $X^1$ preferably includes an amide group (-$NR^bC(O)$-, -$C(O)NR^b$-), an ester group (-C(O)O-, -OC(O)-), or an ether group, and more preferably includes an amide group (-$NR^bC(O)$-, -$C(O)NR^b$-) or an ester group (-C(O)O-, -OC(O)-).

In some embodiments, $X^1$ is selected from the group consisting of -$NR^bC(O)$-, - $C(O)NR^b$-, an alkylene group, -$NR^bC(O)O$-, -$OC(O)NR^b$-, -C(O)O-, -OC(O)-, -$NR^b$-, an amino acid residue, and combinations thereof.

The number of carbon atoms in the alkylene group and alkenylene group in $X^1$ is not particularly limited, and is selected in consideration of the number of members of the macrocyclic structure. In certain embodiments, the number of carbon atoms in the alkylene group and alkenylene group in $X^1$ is 1 to 6 ($C_1$-$C_6$) or 1 to 3 ($C_1$-$C_3$).

In formula (I), $Y^1$ is a moiety starting from the macrocyclic structure linked to the 5-position of the THIQ framework. $Y^1$ is a divalent group selected from the group consisting of a single bond, an ether group, a thioether group, an optionally substituted $C_1$-$C_6$ alkylene group, and -$NR^b$-.

In this specification, when a substituent for $Y^1$ is described, the left side of the substituent described is bonded to the carbon atom at the 5-position of the THIQ framework, and the right side of the substituent described is bonded to $Y^2$.

The number of carbon atoms in the alkylene group in $Y^1$ is not particularly limited, and is selected in consideration of the number of members of the macrocyclic structure. In certain embodiments, the number of carbon atoms in the alkylene group in $Y^1$ is 1 to 6 ($C_1$-$C_6$), or 1 to 3 ($C_1$-$C_3$).

In some embodiments, $Y^1$ is selected from the group consisting of an ether group, a thioether group, an optionally substituted $C_1$-$C_6$ alkylene group, and -$NR^b$-.

In some embodiments, $Y^1$ is selected from the group consisting of an ether group and - $NR^b$-.

In certain embodiments, $Y^1$ is an ether group.

In formula (I), $Y^2$ is a divalent group selected from the group consisting of an optionally substituted alkylene group, an optionally substituted alkenylene group, -$NR^bC(O)$-, -$C(O)NR^b$-, -C(O)O-, -OC(O)-, -$NR^bC(O)O$-, -$OC(O)NR^b$-,

-OC(O)O-, a carbonyl group, - C(=S)-, -C(=NR$^b$)-, -NR$^b$-, a sulfonyl group, an ether group, a thioether group, an amino acid residue, and combinations thereof.

In this specification, when a substituent for $Y^2$ is described, the left side of the substituent described is bonded to $Y^1$, and the right side of the substituent described is bonded to $X^2$.

In some embodiments, $Y^2$ is selected from the group consisting of an optionally substituted $C_1$-$C_6$ alkylene group, -NR$^b$C(O)-, -C(O)NR$^b$-, -C(O)O-, -OC(O)-, -NR$^b$C(O)O-, - OC(O)NR$^b$-, -NR$^b$-, an amino acid residue, and combinations thereof.

In some embodiments, $Y^2$ is selected from the group consisting of an optionally substituted $C_1$-$C_6$ alkylene group, -C(O)O-, -OC(O)-, -NR$^b$C(O)-, -C(O)NR$^b$-, and combinations thereof.

In certain embodiments, $Y^2$ is an optionally substituted $C_1$-$C_6$ (preferably $C_1$-$C_3$, and more preferably $C_1$-$C_2$) alkylene group. In certain embodiments, $Y^2$ is a $C_1$-$C_3$ (preferably $C_1$-$C_2$) alkylene group. In one embodiment, $Y^2$ is a methylene group.

In some embodiments, at least one of $X^1$ and $Y^2$ includes an amino acid residue.

In the above embodiment, the type of the amino acid residue that can be contained in $X^1$ and $Y^2$ is not particularly limited. In some embodiments, the amino acid residue is an amino acid residue selected from the group consisting of alanine, cysteine, serine, tryptophan, threonine, lysine, arginine, propargylglycine, allylglycine, ornithine, histidine, and combinations thereof. In certain embodiments, the amino acid residue is an alanine or cysteine residue. In one embodiment, the amino acid residue is an alanine residue.

In some embodiments, $X^1$ contains an amino acid residue. In certain embodiments, $X^1$ contains an alanine or cysteine residue. In one embodiment, $X^1$ contains an alanine residue.

In formula (1), $X^2$ is a divalent group selected from the group consisting of -L$^1$-C(=CR$^f_2$)-CR$^f$=CR$^f$-L$^2$-, -L$^1$-CR$^f$=CR$^f$-C(=CR$^f_2$)-L$^2$-, -L$^1$-CR$^f$=CR$^f$-L$^2$-, -L$^-$CR$^f$=CR$^f$-CR$^f$=CR$^f$-L$^2$-, -L$^1$-NR$^b$-CR$^f_2$-C≡C-L$^2$-, -L$^l$-C=C-CRf2-NR$^b$–L$^2$- ,, -L$^1$-C≡C-L$^2$-, and -L$^1$-C≡C-C≡C-L$^2$-.

[C21]

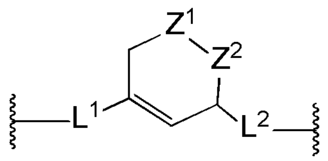
and
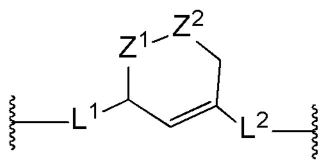

In this specification, when a substituent for $X^2$ is described, the left side of the substituent described is bonded to $X^1$, and the right side of the substituent described is bonded to $Y^2$. That is, $L^1$ is bonded to $X^1$, and $L^2$ is bonded to $Y^2$.

$L^1$ and $L^2$ each independently represent a single bond or a $C_1$-$C_6$ alkylene group.

R$^f$ each independently represents a hydrogen atom, an optionally substituted $C_1$-$C_{20}$ alkyl group or an optionally substituted aryl group.

$Z^1$ and $Z^2$ each independently represent -NR$^c$- or -CR$^d$R$^e$-, R$^c$, R$^d$, and R$^e$ are each independently a hydrogen atom or an optionally substituted $C_1$-$C_6$ alkyl group, or R$^c$ and R$^d$ together with $Z^1$ and $Z^2$ to which they are bonded form a 5- or 6-membered ring structure (ring Q), and the ring structure (ring Q) may be substituted with 1 to 4 substituents. In some embodiments, the ring structure (ring Q) may be substituted with 1 to 4 (preferably 1 to 3) substituents that are independently selected from among a methyl group, an oxo group, a phenyl group, and a propargyl group. The ring structure (ring Q) may be a carbocycle or a heterocycle containing 1 to 4 (preferably 1 to 3) heteroatoms as ring-constituting atoms.

When $L^1$ and $L^2$ are an alkylene group, the number of carbon atoms may be appropriately set according to the number of members constituting the macrocyclic structure. As an example, when $L^1$ and $L^2$ are an alkylene group, the number of carbon atoms is 1 to 6 ($C_1$-$C_3$ alkylene group), 1 to 3 ($C_1$-$C_3$ alkylene group), 1 to 2 ($C_1$-$C_2$ alkylene group), or 1 (methylene group).

In some embodiments, $L^1$ and $L^2$ are a single bond, a methylene group, or an ethylene group.

In some embodiments, $X^2$ is selected from the group consisting of -C(=CH$_2$)-CH=CH-CH$_2$-, -CH$_2$-CH=CH-C(=CH$_2$)-, -CH=CH-CH$_2$-, -CH$_2$-CH=CH-, -C=C-CH$_2$, -CH$_2$-C≡C-, - NH-CH$_2$-C≡C-, and -C≡C-CH$_2$-NH-.

[C22]

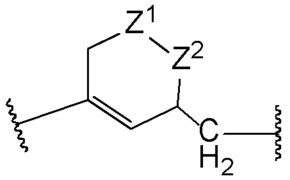

and

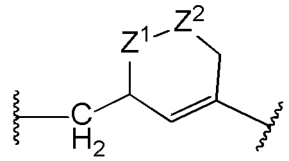

$Z^1$ and $Z^2$ each independently represent $-NR^c-$ or $-CR^dR^e-$, $R^c$, $R^d$, and $R^d$ are each independently a hydrogen atom or a $C_1$-$C_3$ alkyl group (for example, a methyl group), or $R^c$ and $R^d$ together with $Z^1$ and $Z^2$ to which they are bonded form a 5- or 6-membered ring structure (ring Q), and the ring structure (ring Q) may be substituted with 1 to 4 (preferably 1 to 3) substituents that are independently selected from among a methyl group, an oxo group, a phenyl group, and a propargyl group. The ring structure (ring Q) may be substituted with 1 to 3 substituents selected from among an oxo group and a phenyl group.

In certain embodiments, $X^2$ is selected from the group consisting of

[C23]

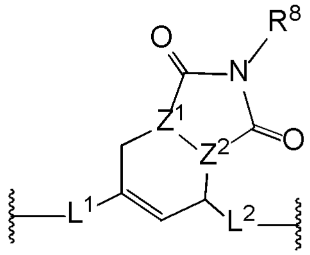

, or

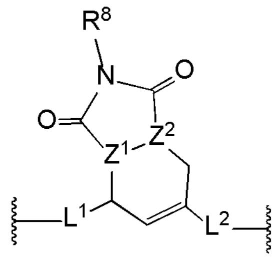

.

In certain embodiments, $X^2$ is selected from the group consisting of

[C24]

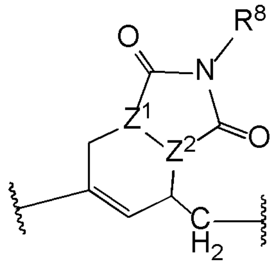

and

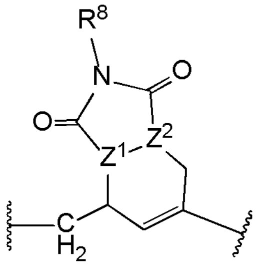

.

In one embodiment, $Z^1$ and $Z^2$ each independently represent N or CH, and $R^8$ represents a hydrogen atom, a phenyl group or a propargyl group.

In the above embodiment, $R^8$ represents a hydrogen atom, an optionally substituted aryl group, an optionally substituted $C_1$-$C_{20}$ alkyl group, an optionally substituted allyl group, a propargyl group, or a nitrogen protecting group.

In some embodiments, $R^8$ is selected from among a hydrogen atom, an optionally substituted phenyl group, an optionally substituted $C_1$-$C_{20}$ alkyl group, an allyl group, a propargyl group, and a nitrogen protecting group.

In certain embodiments, $R^8$ represents a hydrogen atom or an optionally substituted phenyl group.

In formula (I), $R^b$ is each independently selected from among a hydrogen atom, an optionally substituted $C_1$-$C_{20}$ alkyl group, an optionally substituted aryl group, an optionally substituted allyl group, a propargyl group, and a nitrogen protecting group.

In some embodiments, $R^b$ is each independently selected from among a hydrogen atom, an optionally substituted $C_1$-$C_6$ alkyl group, an optionally substituted aryl group, an optionally substituted allyl group, a propargyl group, and a nitrogen protecting group.

In some embodiments, $R^b$ is a hydrogen atom, an optionally substituted $C_1$-$C_6$ alkyl group (for example, methyl group, ethyl group), or a propargyl group.

In certain embodiments, $R^b$ is a hydrogen atom, a methyl group, or a propargyl group.

In one embodiment, $R^b$ is a nitrogen protecting group.

In formula (I), $R^a$ is each independently a hydrogen atom or an optionally substituted $C_1$-$C_6$ alkyl group. In addition, when $R^a$ is an alkyl group, respective $R^a$'s may be taken together to form a ring structure containing an oxygen atom to which they are bonded. Preferably, respective $R^a$'s be taken together to form a ring structure containing an oxygen atom to which they are bonded, and the ring structure can be a 5- to 9-membered ring.

In formula (I) $R^1$ is a methyl group.

In formula (I), $R^2$ is a hydrogen atom or an optionally substituted $C_1$-$C_6$ alkyl group. In one embodiment, $R^2$ is a methyl group.

In formula (I), $R^3$ is a methyl group.

In formula (I), $R^4$ is selected from among a hydrogen atom, an optionally substituted $C_1$-$C_6$ alkyl group, an optionally substituted aryl group, an optionally substituted allyl group, a propargyl group, and a protecting group for a phenolic hydroxyl group.

In some embodiments, $R^4$ represents a hydrogen atom.

In some embodiments, $R^4$ represents a protecting group for a phenolic hydroxyl group.

In formula (I), $R^5$ represents a cyano group (CN), a hydroxyl group or a leaving group containing an oxygen atom, a sulfur atom, a nitrogen atom, or a phosphorus atom.

In some embodiments, $R^5$ represents CN or a hydroxyl group.

In one embodiment, $R^5$ represents CN.

In some embodiments, a combination in which A is a methylene group, $X^1$ contains an amide group (-$NR^bC(O)$-, -$C(O)NR^b$-) or an ester group (-C(O)O-, -OC(O)-), $R^b$ is a hydrogen atom, a methyl group, or a propargyl group, and $Y^1$ is an ether group can be used.

In some embodiments, the compound of formula (I) is represented by the following formula (Ia). The compound of the embodiment can be produced with few processes from Cyanosafracin B as a starting substance.

[C25]

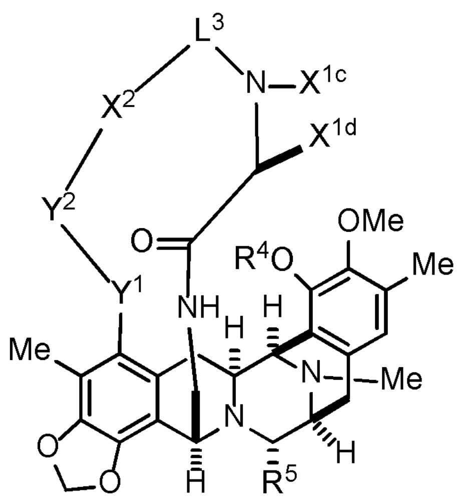

(Ia)

In formula (Ia), $X^2$, $Y^1$, $Y^2$, $R^4$, and $R^5$ are as defined in formula (I), and the above specific aspects and preferable embodiments can be used.

In formula (Ia), Me represents a methyl group.

In formula (Ia), $X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group.

In some embodiments, $X^{1c}$ is selected from among a hydrogen atom, an optionally substituted $C_1$-$C_6$ alkyl group (for example, a methyl group and an ethyl group), an optionally substituted aryl group (for example, a phenyl group), an optionally substituted allyl group, a propargyl group, and a nitrogen protecting group.

In some embodiments, $X^{1c}$ is a hydrogen atom, an optionally substituted $C_1$-$C_6$ alkyl group (for example, a methyl group and an ethyl group), or a propargyl group.

In certain embodiments, $X^{1c}$ is a hydrogen atom, a methyl group, or a propargyl group.

In formula (Ia), $X^{1d}$ is selected from among a hydrogen atom, a methyl group, and substituents corresponding to various natural/unnatural amino acid side chains. Substituents corresponding to various natural/unnatural amino acid side chains are not particularly limited, and may be groups corresponding to side chains of amino acids bonded to the $\alpha$ carbon of amino acids. For example, the substituent corresponding to the side chain of alanine is a methyl group. The substituent corresponding to the side chain of cysteine is $-CH_2-SH$.

In certain embodiments, $X^{1d}$ represents a methyl group.

In formula (Ia), $L^3$ is selected from the group consisting of a single bond, an optionally substituted alkylene group, an optionally substituted alkenylene group, a carbonyl group, - C(=S)-, $-C(=NR^b)-$, -C(O)O-, $-C(O)NR^b-$, -OC(O)-, $-NR^b-$, an ether group, a thioether group, and combinations thereof.

In some embodiments, $L^3$ is selected from the group consisting of a single bond, an optionally substituted alkylene group, an optionally substituted alkenylene group, carbonyl group, -C(=S)-, $-C(=NR^b)-$, -C(O)O-, $-C(O)NR^b-$, -OC(O)-, $-NR^b-$, an ether group, and a thioether group.

In some embodiments, $L^3$ is selected from the group consisting of a single bond, an optionally substituted $C_1$-$C_6$ (preferably $C_1$-$C_3$, and more preferably $C_1$-$C_2$) alkylene group, - C(O)O-, and -OC(O)-.

In some embodiments, $L^3$ is selected from the group consisting of a single bond, - C(O)O-, and -OC(O)-.

In certain embodiments, $L^3$ is a single bond.

In certain embodiments, $L^3$ is -C(O)O- or -OC(O)-.

In certain embodiments, $L^3$ is -OC(O)- (provided that a carbon atom of $L^3$ is bonded to a nitrogen atom of N-$X^{1c}$ in formula (Ia)).

In formula (Ia), $R^b$ is as defined in formula (I), and the above specific aspects and preferable embodiments can be used.

In some embodiments, the compound of formula (I) is represented by the following formula (Ic), formula (Id), formula (Ie), formula (If) or formula (Ig). In one embodiment, the compound of formula (I) is represented by the following formula (Ic). In one embodiment, the compound of formula (I) is represented by formula (Id). In one embodiment, the compound of formula (I) is represented by formula (Ie). In one embodiment, the compound of formula (I) is represented by formula (If). In one embodiment, the compound of formula (I) is represented by formula (Ig). In one embodiment, the compound of formula (I) is represented by the following formula (Ih). The compound of this embodiment can be produced with few processes from Cyanosafracin B as a starting substance.

[C26]

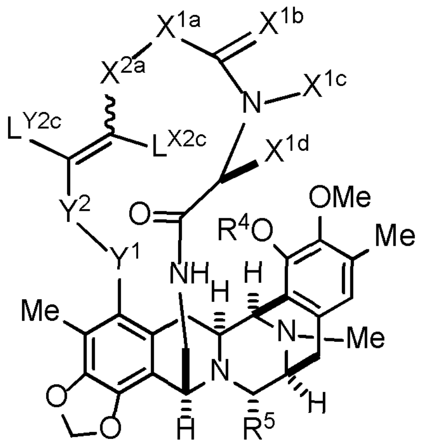

(Ic) ,

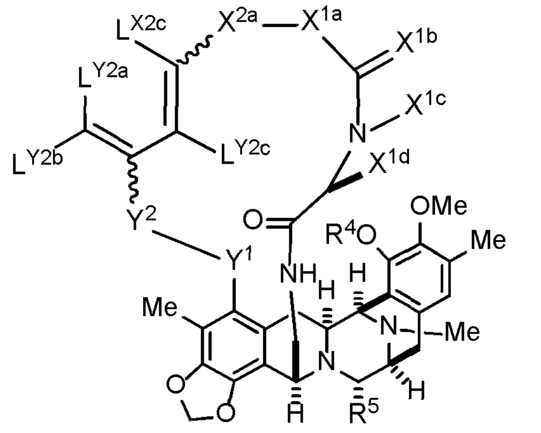

(Id) ,

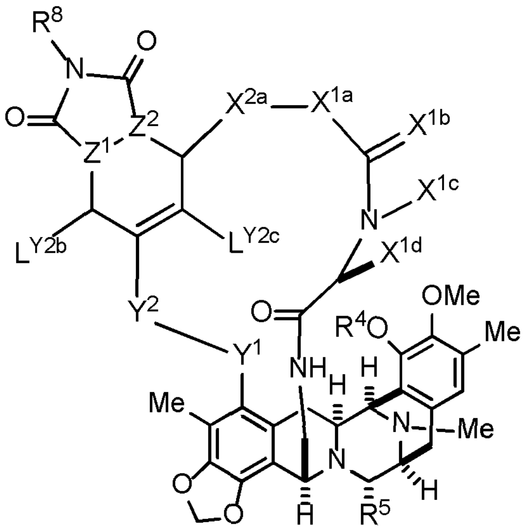

(Ie) ,

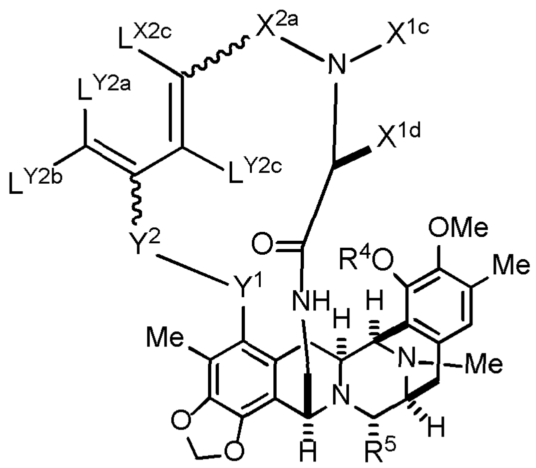

(If) ,

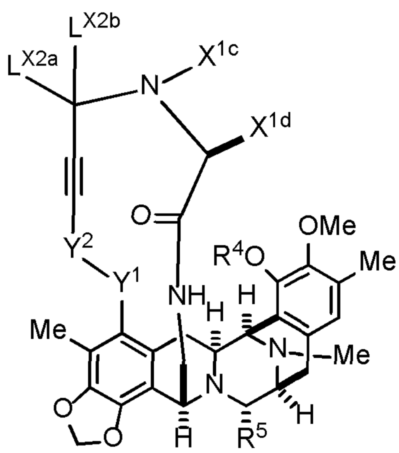

(Ig) ,

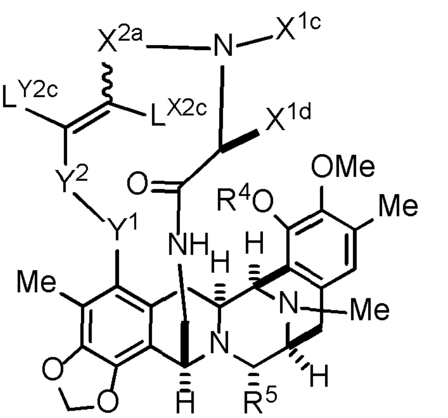

(Ih)

In this specification,

[C27]



represents a bond on an sp2 carbon atom, and indicates that it may be any conformation of a stereoisomer such as cis, trans, and conformational isomers (s-cis/s-trans).

In formula (Ic), formula (Id), formula (Ie), formula (If), formula (Ig) and formula (Ih) (hereinafter referred to as formulae (Ic) to (Ih)), $R^4$, $R^5$, $X^{1c}$ and $X^{1d}$ are as defined in formula (Ia), and the above specific aspects and preferable embodiments can be used.

In formulae (Ic) to (Ih), Me represents a methyl group.

In formula (Ic), formula (Id), and formula (Ie), $X^{1a}$ represents an oxygen atom, a sulfur atom, $-NR^b-$, or an optionally substituted methylene group ($-CH_2-$). Examples of substituents for a methylene group include substituents corresponding to various natural/unnatural amino acid side chains.

In some embodiments, $X^{1a}$ represents an oxygen atom or $-NR^b-$

In certain embodiments, $X^{1a}$ is an oxygen atom.

In formula (Ic), formula (Id), and formula (Ie), $X^{1b}$ represents an oxygen atom, a sulfur atom, $=NR^b$, or an optionally substituted methylene group ($=CH_2$). Examples of substituents for a methylene group include an optionally substituted $C_1$-$C_6$ alkylene group and an aryl group.

In some embodiments, $X^{1b}$ is an oxygen atom.

In formula (Ic), formula (Id), formula (Ie), formula (If), and formula (Ih), $X^{2a}$ represents an optionally substituted $C_1$-$C_6$ alkylene group.

In some embodiments, $X^{2a}$ is a $C_1$-$C_3$ alkylene group.

In one embodiment, $X^{2a}$ is a methylene group.

In formulae (Ic) to (Ih), $Y^1$ is a divalent group selected from the group consisting of an ether group, a thioether group, an optionally substituted $C_1$-$C_6$ alkylene group, and $-NR^b-$.

In some embodiments, $Y^1$ is selected from the group consisting of an ether group and - $NR^b-$.

In certain embodiments, $Y^1$ is an ether group.

In formulae (Ic) to (Ih), $Y^2$ is a divalent group selected from the group consisting of an optionally substituted alkylene group, an optionally substituted alkenylene group, $-NR^bC(O)-$, $-C(O)NR^b-$, -C(O)O-, -OC(O)-, $-NR^bC(O)O-$, $-OC(O)NR^b-$, -OC(O)O-, a carbonyl group, - C(=S)-, $-C(=NR^b)-$, $-NR^b-$, a sulfonyl group, an ether group, a thioether group, and an amino acid residue.

In some embodiments, $Y^2$ is selected from the group consisting of an optionally substituted $C_1$-$C_6$ alkylene group, an optionally substituted $C_2$-$C_6$ alkenylene group, - $NR^bC(O)-$, $-C(O)NR^b-$, -C(O)O-, -OC(O)-, $-NR^bC(O)O-$, $-OC(O)NR^b-$, -OC(O)O-, a carbonyl group, -C(=S)-, $-C(=NR^b)-$, $-NR^b-$, a sulfonyl group, an ether group, a thioether group, and an amino acid residue.

In some embodiments, $Y^2$ is selected from the group consisting of an optionally substituted $C_1$-$C_6$ alkylene group, $-NR^bC(O)-$, $-C(O)NR^b-$, -C(O)O-, -OC(O)-, $-NR^bC(O)O-$, - $OC(O)NR^b-$, $-NR^b-$, an amino acid residue, and combinations thereof.

In some embodiments, $Y^2$ is selected from the group consisting of an optionally substituted $C_1$-$C_6$ alkylene group, -C(O)O-, -OC(O)-, $-NR^bC(O)-$, $-C(O)NR^b-$, and combinations thereof.

In certain embodiments, $Y^2$ is an optionally substituted $C_1$-$C_6$ (preferably $C_1$-$C_3$, and more preferably $C_1$-$C_2$) alkylene group.

In certain embodiments, $Y^2$ is a $C_1$-$C_3$ (preferably $C_1$-$C_2$) alkylene group.

In one embodiment, $Y^2$ is a methylene group.

In formula (Ig), $L^{X2a}$ and $L^{X2b}$ each independently represent a hydrogen atom, an optionally substituted $C_1$-$C_{20}$ alkyl group or an optionally substituted aryl group. In some embodiments, $L^{X2a}$ and $L^{X2b}$ each independently represent a hydrogen atom, an optionally substituted $C_1$-$C_8$ alkyl group, or an optionally substituted aryl group.

In one embodiment, $L^{X2a}$ and $L^{X2b}$ are a hydrogen atom.

In formula (Ic), formula (Id), formula (Ie), formula (If) and formula (Ih), $L^{X2c}$ represents a hydrogen atom, an optionally substituted $C_1$-$C_{20}$ alkyl group or an optionally substituted aryl group.

In some embodiments, $L^{X2c}$ represents a hydrogen atom, an optionally substituted $C_1$-$C_8$ alkyl group, or an optionally substituted aryl group.

In one embodiment, $L^{X2c}$ is a hydrogen atom.

In formula (Id), formula (Ie), and formula (If), $L^{Y2a}$ represents a hydrogen atom, an optionally substituted $C_1$-$C_{20}$ alkyl group or an optionally substituted aryl group.

In some embodiments, $L^{Y2a}$ is selected from among a hydrogen atom, an optionally substituted $C_1$-$C_8$ alkyl group, and an optionally substituted aryl group.

In one embodiment, $L^{Y2a}$ is a hydrogen atom.

In formula (Ic), formula (Id), formula (Ie), and formula (If), $L^{Y2b}$ represents a hydrogen atom, an optionally substituted $C_1$-$C_{20}$ alkyl group or an optionally substituted aryl group.

In some embodiments, $L^{Y2b}$ is selected from among a hydrogen atom, an optionally substituted $C_1$-$C_8$ alkyl group, and an optionally substituted aryl group.

In one embodiment, $L^{Y2b}$ is a hydrogen atom.

In formula (Ic), formula (Id), formula (Ie), formula (If) and formula (Ih), $L^{Y2c}$ represents a hydrogen atom, an optionally substituted $C_1$-$C_{20}$ alkyl group or an optionally substituted aryl group.

In some embodiments, $L^{Y2c}$ is selected from among a hydrogen atom, an optionally substituted $C_1$-$C_8$ alkyl group, and an optionally substituted aryl group.

In one embodiment, $L^{Y2c}$ is a hydrogen atom.

In formula (Ie), $Z^1$ and $Z^2$ each independently represent N or $CR^e$, and $R^e$ is a hydrogen atom or an optionally substituted $C_1$-$C_6$ alkyl group.

In certain embodiments, $Z^1$ and $Z^2$ each independently represent a nitrogen atom or CH.

In formula (Ie), $R^8$ represents a hydrogen atom, an optionally substituted aryl group, an optionally substituted $C_1$-$C_{20}$ alkyl group, an optionally substituted allyl group, a propargyl group, or a nitrogen protecting group.

In certain embodiments, $R^8$ represents a hydrogen atom or an optionally substituted phenyl group.

In one embodiment, $R^8$ is a phenyl group.

In formulae (Ic) to (Ih), $R^b$ is as defined in formula (I), and the above specific aspects and preferable embodiments can be used.

Specific examples of compounds of the present invention represented by formula (I) include compounds having the following structures. However, the present invention is not limited thereto. The numbers shown below the compounds are numbers of compounds synthesized in examples.

[C28]

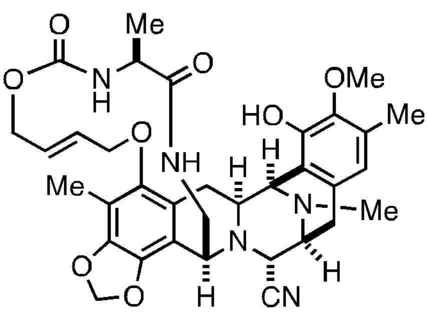

,

7

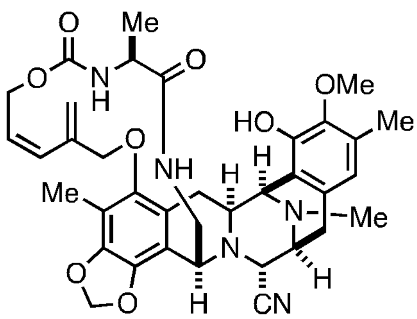

,

14

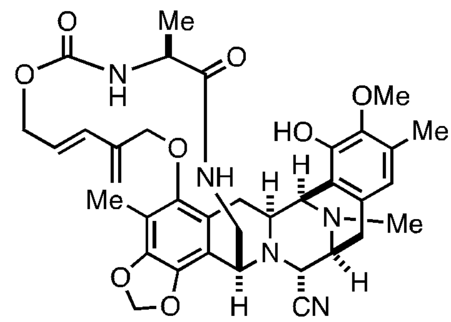

,

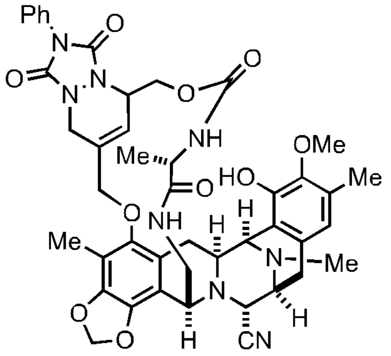

,

13

16

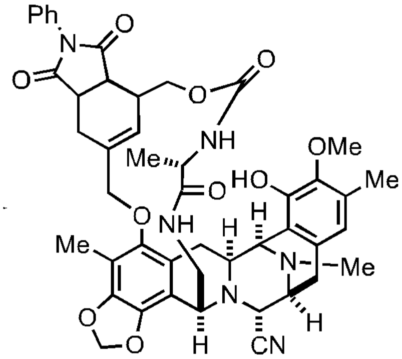

,

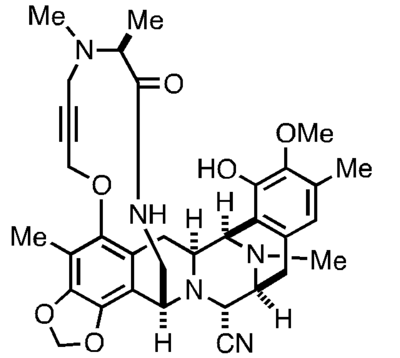

,

18

29

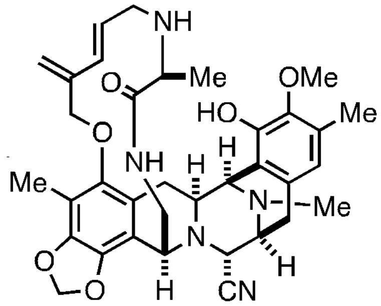

33

,

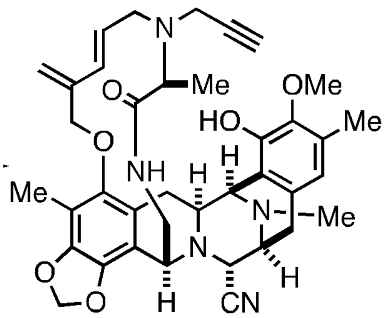

35

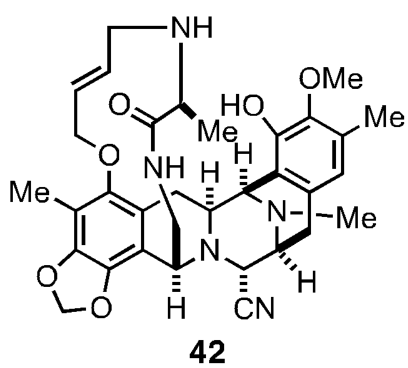

42

,

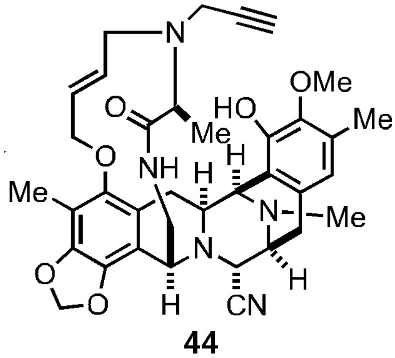

44

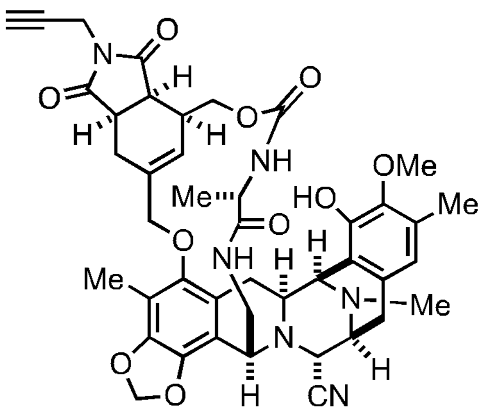

**47**

wherein Me represents a methyl group.

Another aspect of the present invention relates to a compound represented by the following formula (IIIc).

[C29]

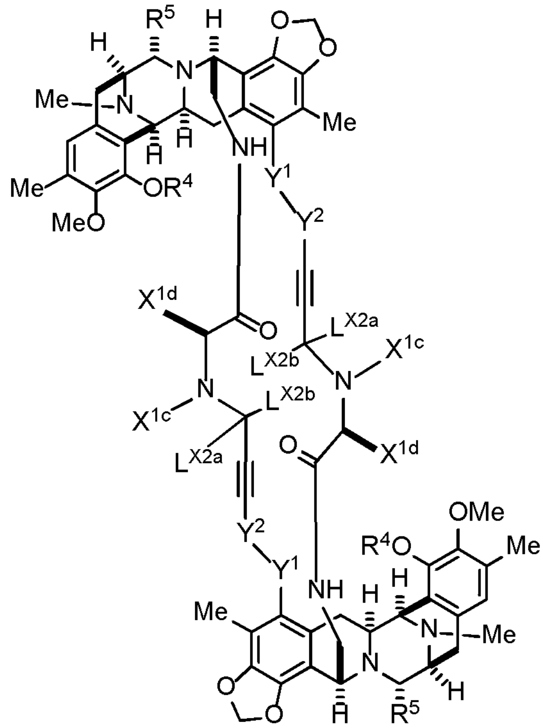

(IIIc)

In the compound of formula (IIIc), the 1-position and 5-position of the THIQ framework of two different molecules are linked with a linking group to form a macrocyclic structure. Therefore, the compound of formula (IIIc) is also called a dimer compound of the compound of formula (I). The macrocyclic structure in formula (IIIc) is, for example, a 15-to 40-membered ring (preferably a 24- to 36-membered ring, and more preferably a 28- to 30-membered ring).

In formula (IIIc), $X^{1c}$, $X^{1d}$, $L^{X2a}$, $L^{X2b}$, $Y^1$, $Y^2$, $R^4$, $R^5$, and $R^b$ are as defined in formula (Ig), and the above specific aspects and preferable embodiments can be used. Me represents a methyl group.

Specific examples of compounds of the present invention represented by formula (IIIc) include compounds having the following structures. However, the present invention is not limited thereto. The compounds of the present invention represented by formula (IIIc) are expected to exhibit a DNA alkylating ability and anticancer activity in the same

manner as the group of compounds described in examples. The numbers shown below the compounds are numbers of compounds synthesized in examples.

[C30]

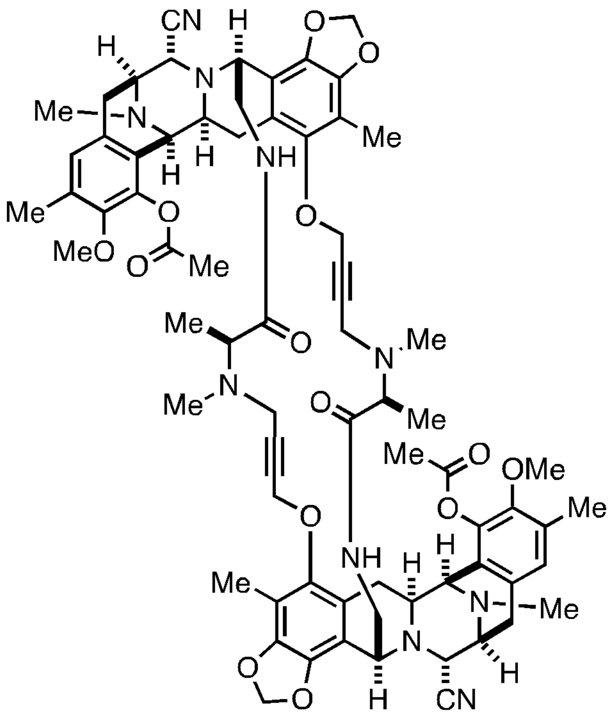

**28**

Compounds of the present invention represented by formula (I), formula (Ia), formulae (Ic) to (Ih), formula (IIIc) and formulae (II), (Iic) to (IIf) described below (hereinafter referred to as "compounds of the present invention") may exist as salts. The salt is not particularly limited as long as it is a pharmaceutically acceptable salt, and examples thereof include base addition salts, acid addition salts, and amino acid salts. Examples of base addition salts include metal salts such as sodium salts, potassium salts, calcium salts, and magnesium salts, ammonium salts, and organic amine salts such as triethylamine salts, piperidine salts, and morpholine salts, and examples of acid addition salts include mineral salts such as hydrochloric acid salts, sulfuric acid salts, and nitrates and organic acid salts such as methanesulfonates, p-toluenesulfonate, citrates, and oxalates. Examples of amino acid salts include glycine salts. However, the present invention is not limited to these salts.

The compounds of the present invention may have one, two or more asymmetric carbon atoms depending on the type of substituents, and stereoisomers such as optical isomers or diastereoisomers may be present. Asymmetric carbon atoms may have the (R) or (S) configuration. All optical isomers or diastereoisomers produced by specific configurations of asymmetric carbon atoms present in the molecule and mixtures thereof are included in the scope of the present invention. Stereoisomerism about double bonds (geometric isomerism) is also possible, and in some embodiments, molecules may exist as (E) isomers or (Z) isomers. If the molecule contains several double bonds, it may have stereoisomerism at each double bond. In addition, in certain cases, molecules may also exist as conformation isomers. Diastereoisomers, geometric isomers, conformation isomers, and mixtures thereof are included in the scope of the present invention. All stereoisomers in the pure form, any mixture of stereoisomers, racemates and the like are included in the scope of the present invention.

In addition, the compounds of the present invention may exist as hydrates or solvates, and all of these substances are included in the scope of the present invention. The type of the solvent that forms a solvate is not particularly limited, and examples thereof include solvents such as ethanol, acetone, and isopropanol.

The compounds of the present invention may exist in isotopically labeled forms. All pharmaceutically acceptable salts and isotopically labeled forms of compounds referred to herein and mixtures thereof are included in the scope of the present invention.

The forms in which the compounds of the present invention are protected are included in the scope of the

present invention. Suitable protecting groups are well known to those skilled in the art. A general review of protecting groups in organic chemistry is provided by P.G.M. Watts, "Protecting Groups in Organic Synthesis," 5th edition, Wiley InterScience; and P.J. Kocienski, "Protecting groups" 3rd edition, Georg Thieme Verlag.

In some embodiments, hydroxyl groups of compounds of the present invention may be in a form protected with, for example, a protecting group for a phenolic hydroxyl group. In some embodiments, amino groups of compounds of the present invention may be in a form protected with a nitrogen protecting group.

In this specification, the nitrogen protecting group is not particularly limited. Specific examples of nitrogen protecting groups include carbamate-based protecting groups including a tert-butoxycarbonyl (Boc) group, and allyloxycarbonyl (Alloc) groups; acyl-based protecting groups; sulfonyl-based protecting groups such as a 2-nitrobenzenesulfonyl (Ns) group; and benzyl-based protecting groups.

In this specification, the protecting group for a phenolic hydroxyl group is not particularly limited. Specific examples of phenolic hydroxyl groups include methoxymethyl (MOM) groups, ethoxyethyl (EE) groups, and tetrahydropyranyl (THP) groups; silyl-based protecting groups including tert-butyldimethylsilyl (TBS) groups; acyl-based protecting groups including acetyl (Ac) groups; acetal-based protecting groups; carbonate-based protecting groups; and sulfonyl-based protecting groups.

In some embodiments, hydroxyl groups of compounds represented by formula (I), and formulae (Ic) to (Ih) may be in a form protected with, for example, a protecting group for a phenolic hydroxyl group.

In some embodiments, in formula (I) and formulae (Ic) to (Ih), $R^4$ is a phenolic protecting group.

In certain embodiments, in formula (I) and formulae (Ic) to (Ih), $R^4$ is selected from among a methoxymethyl (MOM) group, an ethoxyethyl (EE) group, a tetrahydropyranyl (THP) group; a silyl-based protecting group including a tert-butyldimethylsilyl (TBS) group; and an acetyl (Ac) group.

In some embodiments, amino groups of compounds represented by formula (I) and formulae (Ic) to (Ih) may be in a form protected with a nitrogen protecting group.

In some embodiments, in formula (I) and formulae (Ic) to (Ih), $R^b$ is a nitrogen protecting group.

In certain embodiments, in formula (I) and formulae (Ic) to (Ih), $R^b$ is selected from among a tert-butoxycarbonyl (Boc) group, an allyloxycarbonyl (Alloc) group, and a 2-nitrobenzenesulfonyl (Ns) group.

In some embodiments, in formulae (Ic) to (Ih), $X^{1c}$ is a nitrogen protecting group.

In certain embodiments, in formulae (Ic) to (Ih), $X^{1c}$ is selected from among a tert-butoxycarbonyl (Boc) group, an allyloxycarbonyl (Alloc) group, and a 2-nitrobenzenesulfonyl (Ns) group.

In some embodiments, compounds represented by formula (I), and formulae (Ic) to (Ih) do not contain a protecting group.

In some embodiments, in formula (I), $R^b$ is not a nitrogen protecting group, and $R^4$ is not a protecting group for a phenolic hydroxyl group.

In some embodiments, in formulae (Ic) to (Ih), $R^b$ is not a nitrogen protecting group, $X^{1c}$ is not a nitrogen protecting group, and $R^4$ is not a protecting group for a phenolic hydroxyl group.

3. THIQ alkaloid compound containing no macrocyclic structure

Another aspect of the present invention relates to a compound represented by the following formula (II). The compound of formula (II) is a compound obtained by introducing side chain moieties at the 1-position and 5 position of the THIQ framework from Cyanosafracin B as a raw material and optionally modifying the substituents.

[C31]

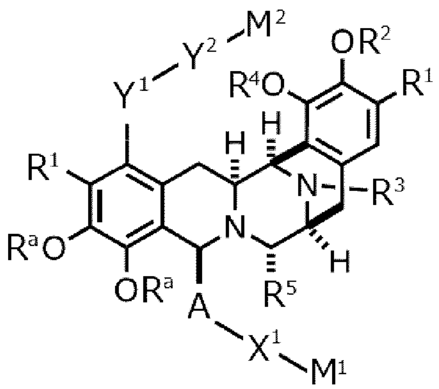

(II)

In some embodiments, these compounds are intermediate compounds suitably used for synthesizing a THIQ alkaloid compound containing a macrocyclic structure represented by formula (I), formula (Ia), formulae (Ic) to (Ih), and formula (IIIc). The compound represented by formula (II) has a functional group that can chemoselectively react with various reagents in the molecule and can be derivatized into various analogs. In some embodiments, the compound of formula (II) has an excellent DNA alkylating ability and thus has excellent antitumor activity.

In formula (II), A, $Y^1$, $Y^2$, $X^1$, $R^a$, $R^1$, $R^2$, $R^3$, and $R^5$ are the same as those in formula (I), and the above definitions and descriptions for preferable embodiments regarding formula (I) are applied without change.

In formula (II), $M^1$ is selected from among a hydrogen atom, an optionally substituted alkyl group, an optionally substituted alkenyl group, an optionally substituted alkynyl group, - $N(R^b)_2$, a hydroxyl group, a carbonyl group, a thiol group, and a halogen atom.

In formula (II), $M^2$ is selected from among a hydrogen atom, an optionally substituted alkyl group, an optionally substituted alkenyl group, an optionally substituted alkynyl group, - $N(R^b)_2$, a hydroxyl group, a carbonyl group, a thiol group, and a halogen atom.

In some embodiments, $M^1$ is a hydrogen atom, an optionally substituted alkenyl group, an optionally substituted alkynyl group or $-NHR^b$, and $M^2$ is a hydrogen atom, an optionally substituted alkenyl group, an optionally substituted alkynyl group or $-NHR^b$.

In certain embodiments, $M^1$ and $M^2$ have the following combinations.

(i) $M^1$ is an optionally substituted alkenyl group, and $M^2$ is an optionally substituted alkynyl group.
(ii) $M^1$ is an optionally substituted alkynyl group, and $M^2$ is an optionally substituted alkenyl group.
(iii) $M^1$ is an optionally substituted alkenyl group, and $M^2$ is an optionally substituted alkenyl group.
(iv) $M^1$ is $NHR^b$, and $M^2$ is an optionally substituted alkynyl group.
(v) $M^1$ is an optionally substituted alkynyl group, and $M^2$ is $NHR^b$.

In formula (II), for $R^b$, the above definition and description for preferable embodiments regarding formula (I) are applied without change.

In some embodiments, the compound of formula (II) is represented by the following formula (Iic), formula (Iid), formula (IIe), or formula (IIf). In one embodiment, the compound of formula (II) is represented by the following formula (IIc). In one embodiment, the compound of formula (II) is represented by formula (IId). In one embodiment, the compound of formula (II) is represented by formula (IIe). The compounds of these embodiments can be produced with few processes from Cyanosafracin B as a starting substance, and can be used to produce the compound containing a macrocyclic structure of formula (I) or formula (IIIc).

[C32]

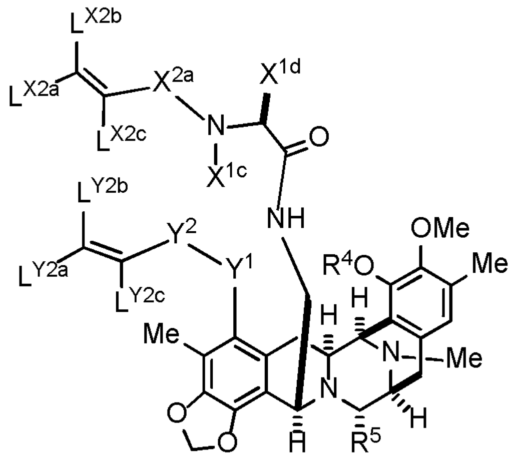

(IIc) ,

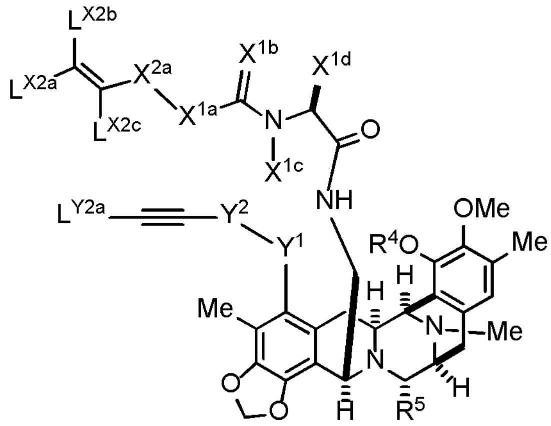

(IId) ,

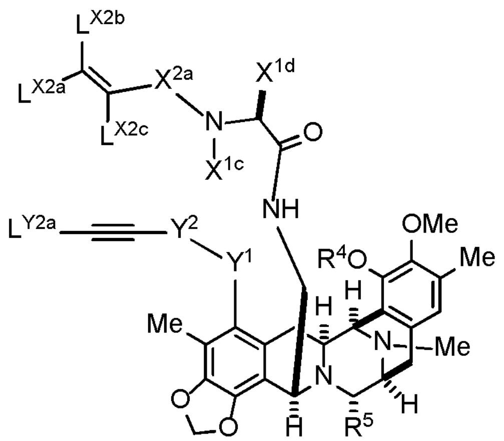

(IIe) ,

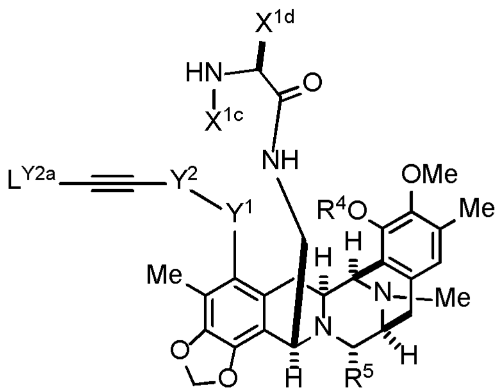

(IIf)

In formula (Iic), formula (Iid), formula (IIe), and formula (IIf) (also referred to as formulae (IIc) to (IIf)), $X^{1c}$, $X^{1d}$, $X^{2a}$, $L^{X2a}$, $L^{X2b}$, $L^{X2c}$, $Y^1$, $Y^2$, $R^4$, $R^5$, $L^{Y2a}$, $L^{Y2b}$, $L^{Y2c}$, $R^b$, $R^4$, and $R^5$ are as defined in formulae (I), (Ia), (Ic) to (Ih), and (II), the above specific aspects and preferable embodiments can be used. Me represents a methyl group.

Specific examples of compounds represented by formula (II) include those having the following structures. However, the present invention is not limited thereto.

[C33]

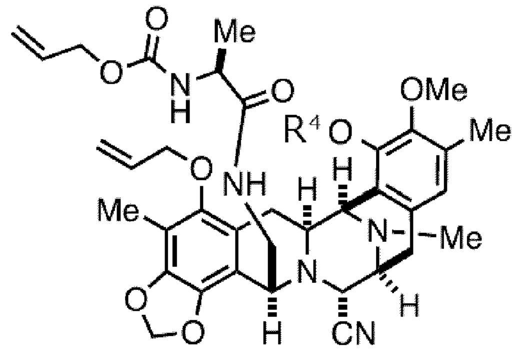

,

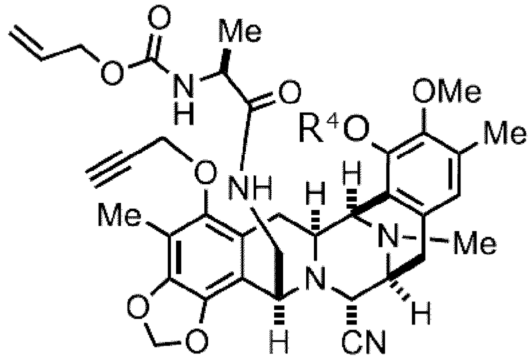

,

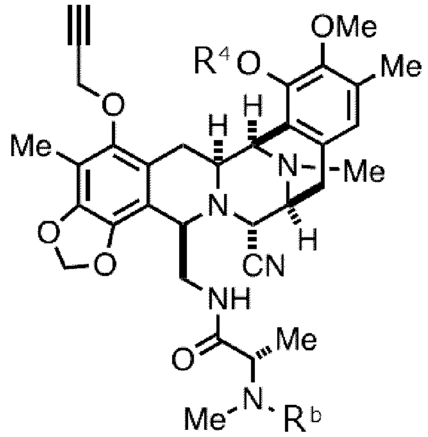

,

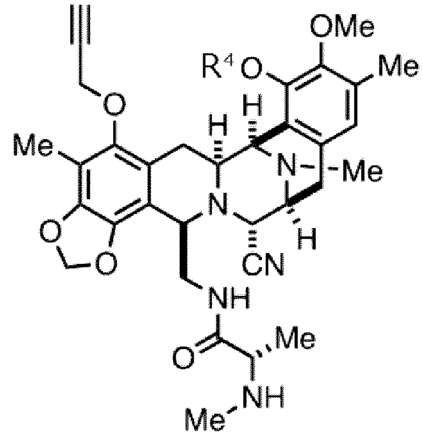

,

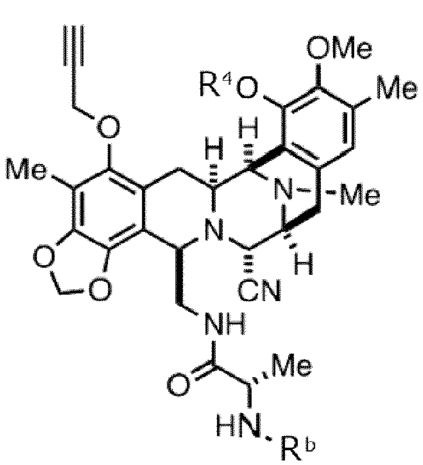

,

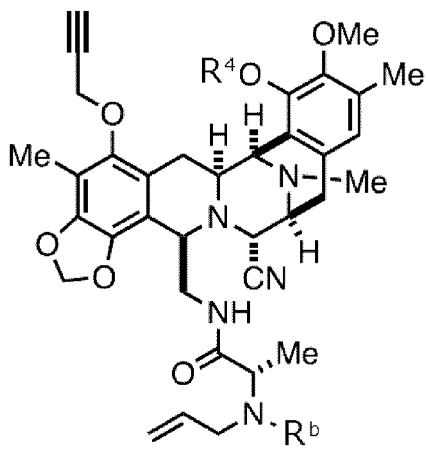

In the formula, Me represents a methyl group, $R^4$ represents a hydrogen atom or a protecting group for a phenolic hydroxyl group (for example, a methoxymethyl (MOM) group, a tert-butyldimethylsilyl (TBS) group, and an acetyl (Ac) group, etc.), $R^b$ each independently represents a hydrogen atom, an optionally substituted $C_1$-$C_{20}$ alkyl group, an optionally substituted aryl group, an optionally substituted allyl group, a propargyl group, or a nitrogen protecting group (for example, a tert-butoxycarbonyl (Boc) group, an allyloxycarbonyl (Alloc) group, and a 2-nitrobenzenesulfonyl (Ns) group).

In one embodiment, examples of compounds represented by formula (II) include those having the following structures. The numbers shown below the compounds are numbers of compounds synthesized in examples.

[C34]

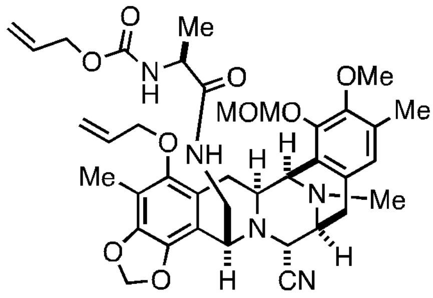
,
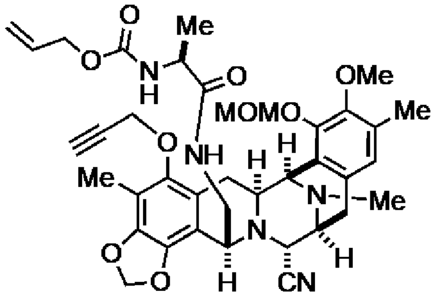
,

5

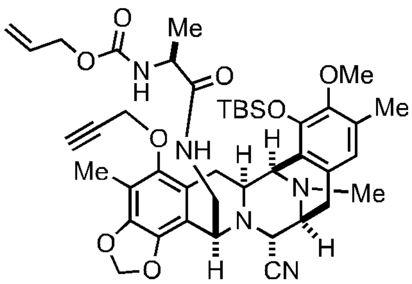
,
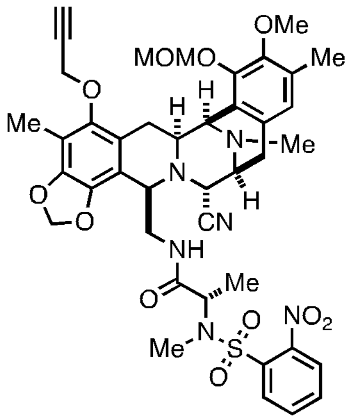
,

10

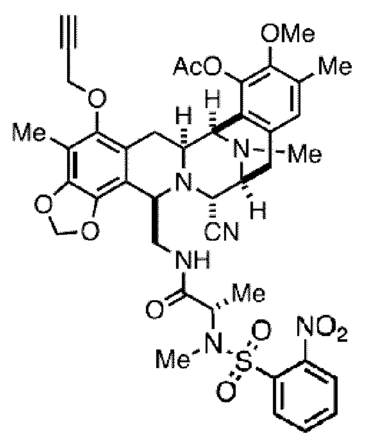

,

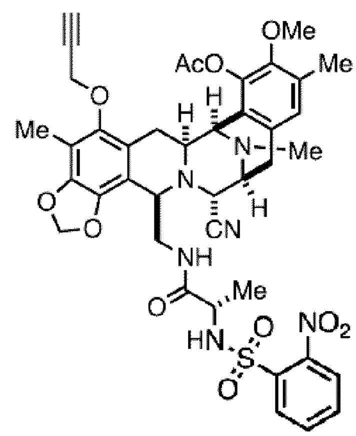

,

25 24

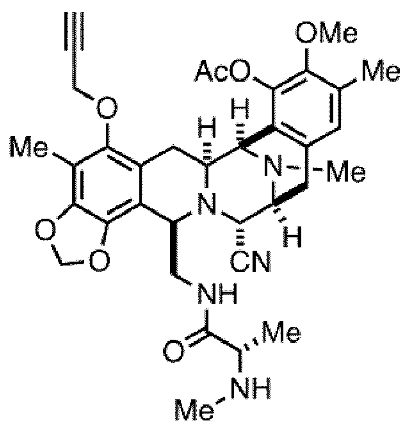

,

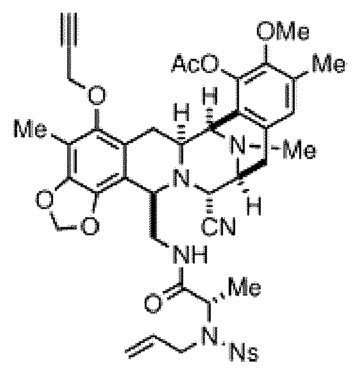

26 30

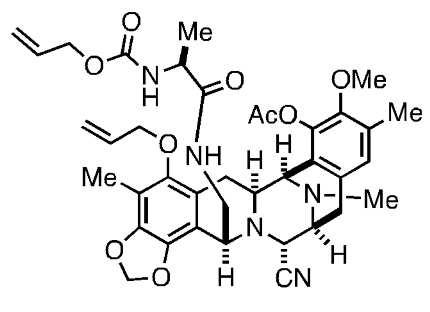

**38**

wherein Me represents a methyl group, MOM represents a methoxymethyl group, Ac represents an acetyl group, Ns represents a 2-nitrobenzenesulfonyl group, and TBS represents a tert-butyldimethylsilyl group.

In one embodiment, examples of compounds represented by formula (II) include those having the following structures.

[C35]

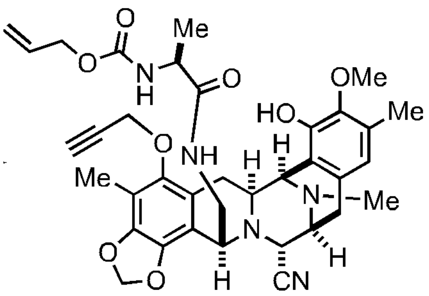

36

,

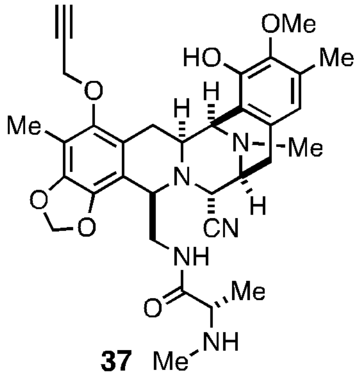

37

In some embodiments, hydroxyl groups of compounds represented by formula (II) and formulae (IIc) to (IIf) may be in a form protected with, for example, a protecting group for a phenolic hydroxyl group.

In some embodiments, in formula (II) and formulae (IIc) to (IIf), $R^4$ is a phenolic protecting group.

In certain embodiments, in formula (II) and formulae (IIc) to (IIf), $R^4$ is selected from among a methoxymethyl (MOM) group, an ethoxyethyl (EE) group, and a tetrahydropyranyl (THP) group; a silyl-based protecting group including a tert-butyldimethylsilyl (TBS) group; and an acetyl (Ac) group.

In some embodiments, amino groups of compounds represented by formula (II) and formulae (IIc) to (IIf) may be in a form protected with a nitrogen protecting group.

In some embodiments, in formula (II) and formulae (IIc) to (IIf), $R^b$ is a nitrogen protecting group.

In certain embodiments, in formula (II) and formulae (IIc) to (IIf), $R^b$ is selected from among a tert-butoxycarbonyl (Boc) group, an allyloxycarbonyl (Alloc) group, and a 2-nitrobenzenesulfonyl (Ns) group.

In some embodiments, in formulae (IIc) to (IIf), $X^{1c}$ is a nitrogen protecting group.

In certain embodiments, in formulae (IIc) to (IIf), $X^{1c}$ is selected from among a tert-butoxycarbonyl (Boc) group, an allyloxycarbonyl (Alloc) group, and a 2-nitrobenzenesulfonyl (Ns) group.

In some embodiments, compounds represented by formula (II) and formulae (IIc) to (IIf) do not contain a protecting group.

In some embodiments, in formula (II), $R^b$ is not a nitrogen protecting group, and $R^4$ is not a protecting group for a phenolic hydroxyl group.

In some embodiments, in formulae (IIc) to (IIf), $R^b$ is not a nitrogen protecting group, $X^{1c}$ is not a nitrogen protecting group, and $R^4$ is not a protecting group for a phenolic hydroxyl group.

4. Pharmaceutical composition of present invention

One aspect of the present invention relates to compounds represented by formula (I), formula (Ia), formulae (Ic) to (Ih), formula (IIIc) and formulae (Ii), (IIc) to (IIf) described below (compounds of the present invention) or a pharmaceutically acceptable salt thereof, and a pharmaceutical composition containing a pharmaceutically acceptable carrier. The term "composition" for the pharmaceutical composition includes not only a product including an active component and an inactive component constituting a carrier but also any product produced directly or indirectly as a result of associating, complexing, or aggregating any two or more components, as a result of dissociating one or more components, or as a result of reacting or interacting other types of one or more components.

In some embodiments, compounds of the present invention contained in the pharmaceutical composition do not contain a protecting group. In some embodiments, compounds of the present invention contained in the pharmaceutical composition do not contain a protecting group for a phenolic hydroxyl group and do not contain a nitrogen protecting group. In some embodiments, in compounds of the present invention contained in the pharmaceutical composition, in the formula, $R^4$ is a hydrogen atom, and when there is $R^b$, $R^b$ is a substituent other than a nitrogen protecting group.

In this specification, for the "pharmaceutically acceptable carrier," carriers commonly used in the related art can be used, and for example, excipients such as lactose, sucrose, glucose, starch, and crystalline cellulose; for example, binders such as hydroxypropylcellulose, methylcellulose, gelatin, tragacanth, gum arabic, and sodium alginate, and for example, disintegrating agents such as starch, carboxymethyl cellulose, and calcium carbonate; and for example, lubricants such as magnesium stearate, talc, and stearic acid can be used.

The pharmaceutical composition contains an effective amount of a compound of the present invention so that

suitable medication is obtained.

The term "effective amount" or "therapeutically effective amount" of compounds of the present invention refers to an amount of an active compound of the present invention at which a biological response or medical response of a subject is elicited, symptoms are ameliorated, conditions are alleviated, disease progression is slowed or delayed, or a disease is prevented.

In this specification, "subject" refers to an animal. Examples of subjects include primates (for example, human), cows, sheep, goats, horses, dogs, cats, rabbits, rats, mice, fish, birds, and the like, and the present invention is not limited to these animals. In preferable embodiments, the subject is a mammal, and most preferably a human.

As shown in examples below, compounds of the present invention have an excellent DNA alkylating ability and have excellent antitumor activity accordingly, which was found first through the present invention. Although not necessarily limited to the following mechanism of action, compounds of the present invention recognize DNA double strands at multiple points in the THIQ ring moiety unit (nucleic acid alkylation moiety) and alkylate them in a sequence-selective manner. It is speculated that, since the three-dimensional structure of DNA alkylated with a THIQ alkaloid changes greatly, excellent antitumor activity is exhibited by modulating and controlling the mode of interaction between nuclear proteins (nucleic acid repair enzyme groups, transcription factors, and the like) and DNA, and protein functions. In the present invention, while maintaining the nucleic acid alkylation moiety structure of the antitumor agent represented by Ecteinascidin 743 (Yondelis), since macrocyclic moieties with various numbers of members can be freely introduced and modified, the mode of interaction with nuclear proteins at macrocyclic moieties positioned on the side opposite to nucleic acid alkylation moieties is highly likely to be rationally modified and controlled.

Therefore, the pharmaceutical composition of the present invention containing a compound of the present invention as an active component can be a DNA alkylating agent or an anti-cancer agent, or can be used for cancer treatment. In some embodiments, there is provided a method for treating cancer in a subject in need, including administering an effective amount of a compound of the present invention or a pharmaceutical composition containing the same to a subject in need. In some embodiments of the present invention, there is provided a compound of the present invention for use as a medicament (for example, an antitumor agent and an anti-cancer agent) or a composition containing the same.

Here, "treatment" in the present invention need only maintain or inhibit progress or metastasis of symptoms related to cancer or malignant tumors, and does not necessarily mean complete curing. In addition, "cancer" is not particularly limited, and includes any malignant tumor including sarcoma, and preferably, use for treating solid cancer is preferable. Examples thereof include breast cancer, brain tumor, colorectal cancer, lung cancer, ovarian cancer, and gastric cancer.

The pharmaceutical composition of the present invention may be in any form of liquid, solid, powder or gel, and examples thereof include tablets, pills, powders, capsules (soft capsules and hard capsules), granules, lozenges, chewable agents, internal liquid agents, injection agents (intravascular administration, intramuscular administration, subcutaneous administration, intradermal administration, etc.), and suppositories. As necessary, tablets may be coated with a general coating, and examples thereof include sugar-coated tablets and film-coated tablets, and double-layered tablets, and multi-layered tablets may also be used. In addition, granules and powders can also be coated with a general coating.

The pharmaceutical composition of the present invention may optionally contain additives that can be used in general pharmaceuticals in addition to the above carriers based on the application form. As such additives, for example, formulation components for various preparations such as stabilizers, diluents, pH buffers, solubilizers, dissolution adjuvants, isotonic agents, and wetting agents can be used, and the amounts of these formulations can be appropriately selected by those skilled in the art.

The compounds and pharmaceutical compositions of the present invention can be administered orally or parenterally, and for example, can be administered orally in dosage forms such as powders, granules, tablets, capsules, syrups, and suspensions as described above, or can be parenterally administered as injection agents or drip agents, for example, in dosage forms such as emulsions and suspensions.

In addition, the compounds and pharmaceutical compositions of the present invention can be administered at an appropriate dosage depending on the type, sex, age, weight, symptoms or other factors of a target animal. A preferable daily dosage per adult is for example, 0.02 to 200 mg/kg weight/day, and preferably 0.2 to 20 mg/kg weight/day, in terms of the amount of an active component. In the above formulation, the administration period of the composition for preventing or treating the bone disease, which can be administered according to any administration program, can be arbitrarily determined according to the age and symptoms. For example, it can be administered continuously, three times a day, twice a day, once a day, once every two days, once every three days, once a week, or at any period and interval.

In another embodiment of the present invention, there is provided a kit including an effective amount of a compound of the present invention and a pharmaceutically acceptable carrier. In some embodiments, the kit is used for treating cancer.

5. Method for producing compounds of the present invention

Typically, as shown in examples below, the compounds of the present invention can be obtained by introducing appropriate substituents at the 1-position and 5-position of the THIQ framework from Cyanosafracin B as a raw material and performing a cyclization reaction thereon to form a macrocyclic structure. In examples in this specification, since a method for producing a representative compound included in compounds of the present invention represented by general formula (I), general formula (II) or general formula (III) is specifically described, those skilled in the art can well understand that, based on the disclosure of this specification and knowledge of synthetic chemistry publicly known in the related art, it is possible to synthesize any compound included in general formula (I), general formula (II) or general formula (III) by appropriately selecting starting materials, reagents, and reaction conditions as necessary. In any of processes for preparing compounds according to various embodiments of the present invention, it may be necessary and/or desirable to protect functional groups or reactive groups on any of related molecules. Such protection can be achieved using conventional protecting groups. The protecting group can be removed at a favorable subsequent stage using a method well-known in the related art.

Since the synthetic schemes described in this specification are an example, the present invention is not limited by the chemical reactions and conditions described in these schemes and examples. Various starting substances used in schemes and examples are commercially available or can be prepared by those skilled in the art based on knowledge of synthetic chemistry publicly known in the related art.

One aspect of the present invention relates to a method for producing a tetrahydroisoquinoline (THIQ) alkaloid compound containing a macrocyclic structure (hereinafter referred to as a "macrocycle-containing THIQ compound"). In preferable embodiments, a tetrahydroisoquinoline (THIQ) alkaloid compound containing a macrocyclic structure is produced using Cyanosafracin B as a starting substance via the compound represented by formula (II). By synthesizing using the compound represented by formula (II), it is possible to efficiently produce compounds having various modal macrocyclic structures linked to the THIQ framework with few processes (for example, 6 to 10 processes).

In certain embodiments, the macrocycle-containing THIQ compound is the compound represented by formula (I). In certain embodiments, the macrocycle-containing THIQ compound is the compound represented by formula (Ia). In certain embodiments, the macrocycle-containing THIQ compound is the compound represented by formula (Ic), formula (Id), formula (Ie), formula (If), or formula (Ig). In certain embodiments, the macrocycle-containing THIQ compound is the compound represented by formula (Ih).

In certain embodiments, the macrocycle-containing THIQ compound is the compound represented by formula (IIIc).

In some embodiments, the macrocycle-containing THIQ compound can be obtained using Cyanosafracin B as a raw material 1) by introducing an appropriate substituent into the side chain at the 1-position of a THIQ framework, phenolizing a p-benzoquinone moiety at the 5-position of the THIQ framework, and introducing an appropriate substituent to obtain the compound represented by formula (II); and then 2) by forming a macrocyclic structure using an appropriate catalyst. Hereinafter, a method for synthesizing the compound represented by formula (I) or formula (IIIc) from Cyanosafracin B via the compound represented by formula (II) will be described.

In some embodiments, a method for producing a compound represented by formula (I) or formula (IIIc) includes (1) a step of synthesizing the compound of formula (II) from Cyanosafracin B, (2) a step of reacting the compound represented by formula (II) to form a macrocyclic structure, and optionally, (3) a step of modifying the macrocyclic structure and optionally, (4) a step of deprotecting a protecting group.

The compound of formula (II) is a compound at the stage in which side chain moieties for forming a macrocyclic structure are introduced at the 1-position and 5-position of the THIQ framework. When a ring structure is formed after appropriate substituents are introduced into "$M^1$" and "$M^2$" in formula (II) according to a desired macrocyclic structure, it is possible to obtain a THIQ alkaloid compound containing a macrocyclic structure of formula (I) or formula (III).

(1) Step of synthesizing compound of formula (II)

(i) introduction of side chain ($Y^1$-$Y^2$-$M^2$) into 5-position of THIQ framework

A hydroxyl group can be introduced at the 5-position by phenolizing the oxo moiety of p-benzoquinone at the 5-position of the THIQ framework of Cyanosafracin B. Phenolizing at the 5-position of the THIQ framework can typically be performed by performing conversion into a phenolic hydroxyl group with visible light emission. Since Cyanosafracin B contains an amino group and a phenolic hydroxyl group, it is preferable to perform the reaction when these are protected with an amino protecting group or a hydroxyl group-protecting group when a photocyclization reaction is performed. Then, after phenolizing, when the side chain (-$Y^2$-$M^2$) is introduced at the 5-position, a compound in which $Y^1$ has an ether group is obtained.

Next, when a phenolic hydroxyl group at the 5-position of the THIQ framework is reacted with an alkenyl halide

such as allyl bromide or alkynyl halide such as a propargyl bromide, it is possible to introduce a side chain containing an alkenyl group or an alkynyl group at the 5-position of the THIQ framework.

In order to obtain a structure other than an ether group for $Y^1$ in formula (1), for example, a phenol is converted into a trifluoromethanesulfonate, a halogen, boron, zinc, or zinc tin and various cross-coupling reactions are then performed, and thus a side chain containing an alkyl group, an alkenyl group, an alkynyl group, an amino group, a thiol group or the like can be introduced.

In addition, an amino group can also be introduced by direct amination using a photoredox catalyst (NPL Margrey, K. A.; Levens, A.; Nicewicz, D. A. Angew. Chem. Int. Ed. 2017, 56, 15644.).

In addition, in order to introduce an ester group, an amide group, an ether group, a thioether group, a carbamate group, a carbonyl group, an alkyl group, a sulfonyl group, an amino group, an amino acid residue or the like, halogenating reagents containing these (for example, allyl chloroformate or carboxybenzyl chloride) can be used.

(ii) Introduction of side chain ($-A-X^1-M^1$) to 1-position of THIQ framework

The side chain ($-CH_2-CH-C(=O)-CH(CH_3)-NH_2$) at the 1-position of the THIQ framework in Cyanosafracin B can be modified and used as a side chain at the 1-position of the THIQ framework.

For example, when an amino group positioned at the side chain terminal at the 1-position of the THIQ framework of Cyanosafracin B is reacted with an alkenyl halide such as allyl bromide or alkynyl halide such as a propargyl bromide, it is possible to introduce a side chain containing an alkenyl group or an alkynyl group. Alternatively, when the terminal amino group is reacted with cesium carbonate or the like, it is possible to introduce a side chain containing an ester group (-COO-). In addition, in order to introduce an ester group, an amide group, an ether group, a thioether group, a carbamate group, a carbonyl group, an alkyl group, a sulfonyl group, an amino group, an amino acid residue or the like, halogenating reagents containing these (for example, allyl chloroformate or carboxybenzyl chloride) can be used.

Alternatively, after a side chain at the 1-position derived from Cyanosafracin B is removed by Edman degradation, by condensing or alkylating the obtained primary amine, it is possible to adjust the length of the alkyl chain of A in formula (1) and it is possible to obtain a structure in which A is a single bond. After removal, it is possible to introduce side chains having various functional groups in the same manner as above.

(iii) Modification of substituent of THIQ framework

8-position of THIQ framework ($R^a$)

A hydroxyl group can be introduced at the 8-position by phenolizing the oxo moiety of p-benzoquinone at the 8-position of the THIQ framework of Cyanosafracin B. The hydroxy group introduced at the 8-position can react with a methoxy group at the 9-position of the THIQ framework of Cyanosafracin B to form a dioxolane ring. The phenolizing and dioxolane ring formation can typically be performed according to a photocyclization reaction with visible light emission.

Modification of $R^2$, $R^4$, and $R^5$

Modification of $R^2$, $R^4$, and $R^5$ can be removed using a method well-known in the related art.

For example, for $R^4$, any substituted alkyl group, alkenyl group, alkynyl group, or aryl group can be introduced by reacting an electrophilic agent on the phenolic hydroxyl group.

For example, for $R^5$, a thioether, aminal, or hemiaminal structure can be constructed by adding a nucleophilic agent after an acid is reacted to form an iminium cation.

(2) Step of forming macrocyclic structure

Formation of the macrocyclic structure in the above (2) can typically be performed by a ring-closing olefin/alkyne metathesis reaction or enyne metathesis cyclization using a ruthenium catalyst or a tungsten catalyst represented by a Grubbs catalyst. Alternatively, in formation of the macrocyclic structure, a three-component connection reaction of amine, alkyne, and aldehyde/ketone using a copper catalyst can be used.

(2-1) Intramolecular cyclization

(i) ring-closing olefin metathesis reaction and enyne metathesis cyclization

In some embodiments, there is provided a production method including obtaining a compound represented by formula (I) by subjecting a compound represented by formula (II) to a ring-closing olefin/alkyne metathesis reaction or

enyne metathesis reaction using a ruthenium catalyst or a tungsten catalyst represented by a Grubbs catalyst.

[C36]

(Scheme 1)

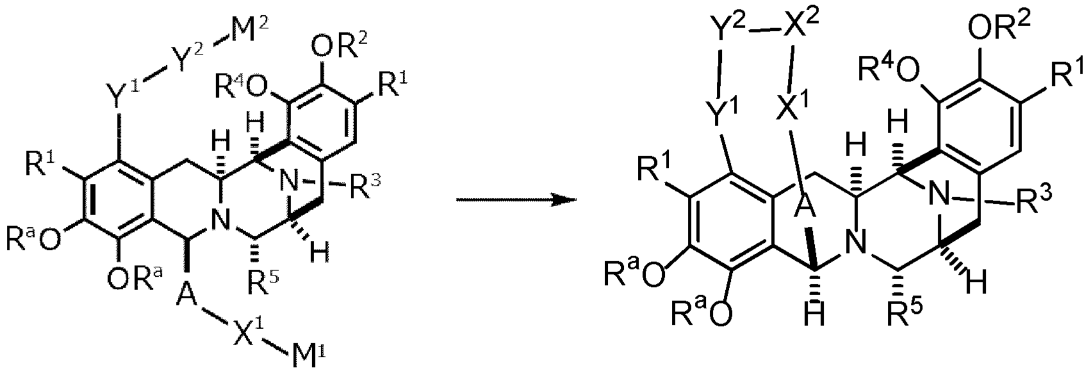

(II)

(I)

In the embodiment, A, $X^1$, $Y^1$, $Y^2$, $R^a$, $R^1$, $R^2$, $R^3$, and $R^5$ are as defined in formula (II), the above definitions and descriptions for preferable embodiments regarding formula (II) are applied without change. $R^4$ is a protecting group for a phenolic hydroxyl group.

In the embodiment, the combination of $M^1$ and $M^2$ is any one of the following (i) to (iii).

(i) $M^1$ is an optionally substituted alkenyl group, and $M^2$ is an optionally substituted alkynyl group.
(ii) $M^1$ is an optionally substituted alkynyl group, and $M^2$ is an optionally substituted alkenyl group.
(iii) $M^1$ is an optionally substituted alkenyl group, and $M^2$ is an optionally substituted alkenyl group.

$X^2$ is a corresponding group formed by the reaction of $M^1$ and $M^2$.

In the case of (i), between a double bond of $M^1$ and a triple bond of $M^2$, a ring-closing enyne metathesis reaction occurs, and a structure in which $X^2$ is $-L^1-CR^f=CR^f-C(=CR^f_2)-L^2-$ is formed. $L^1$ is a moiety that is bonded to $X^1$ and derived from $M^1$ other than a double bond, $L^2$ is a moiety that is bonded to $Y^2$ and derived from $M^2$ other than a triple bond, and $R^f$ is a substituent of double bond and triple bond moieties. Therefore, the production method can be used to produce a compound in which $X^2$ is $-L^1-CR^f=CR^f-C(=CR^f_2)-L^2-$ in formula (I). For example, examples of the compound of formula (II) in the case of (i) include Example Compounds 10 and 30 below.

In the case of (ii), between a triple bond of $M^1$ and a double bond of $M^2$, a ring-closing enyne metathesis reaction occurs, and a structure in which $X^2$ is $-L^1-C(=CR^f_2)-CR^f=CR^f-L^2-$ is formed. $L^1$ is a moiety that is bonded to $X^1$ and derived from $M^1$ other than a triple bond, $L^2$ is a moiety that is bonded to $Y^2$ and derived from $M^2$ other than a double bond, and $R^f$ is a substituent of double bond and triple bond moieties. Therefore, the production method can be used to produce a compound in which $X^2$ is $-L^1-C(=CR^f_2)-CR^f=CR^f-L^2-$ in formula (I).

In the case of (iii), between a double bond of $M^1$ and a double bond of $M^2$, a ring-closing olefin metathesis reaction occurs, and a structure in which $X^2$ is $-L^1-CR^f=CR^f-L^2-$ is formed. $L^1$ is a moiety that is bonded to $X^1$ and derived from $M^1$ other than a double bond, $L^2$ is a moiety that is bonded to $Y^2$ and derived from $M^2$ other than a double bond, and $R^f$ is a substituent of double bond and triple bond moieties. Therefore, the production method can be used to produce a compound in which $X^2$ is $-L^1-CR^f=CR^f-L^2-$ in formula (I). For example, as the compound of formula (II) in the case of (iii), Example Compound 5 below is exemplified.

In some embodiments, there is provided a production method, which is a method for producing a tetrahydroisoquinoline alkaloid compound containing a macrocyclic structure, the method including any one step of the following steps (A) to (C).

step (A): subjecting a compound represented by the following formula (IIc) to a ring-closing olefin/alkyne metathesis reaction or an enyne metathesis reaction using a ruthenium catalyst or a tungsten catalyst represented by a Grubbs catalyst to obtain a compound represented by formula (Ic).

[C37]

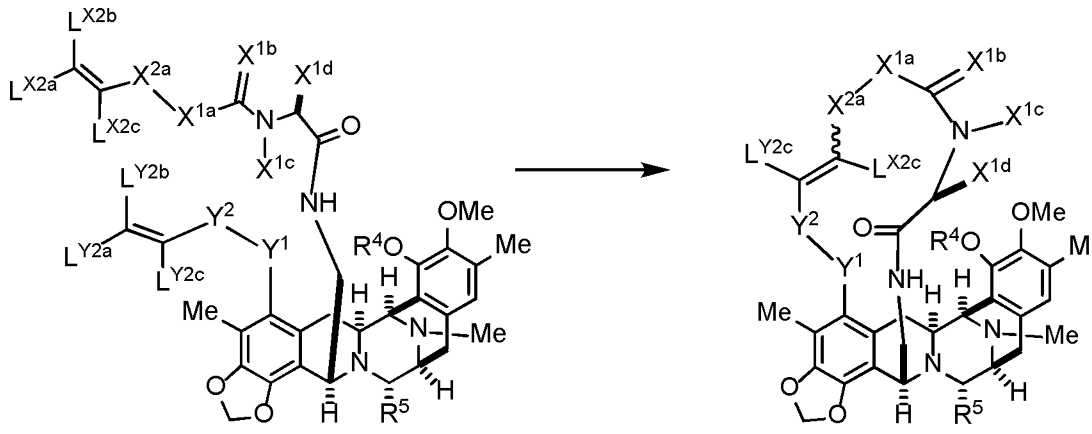

(IIc) (Ic)

step (B): subjecting a compound represented by the following formula (IId) to a ring-closing olefin/alkyne metathesis reaction or an enyne metathesis reaction using a ruthenium catalyst or a tungsten catalyst represented by a Grubbs catalyst to obtain a compound represented by formula (Id).

[C38]

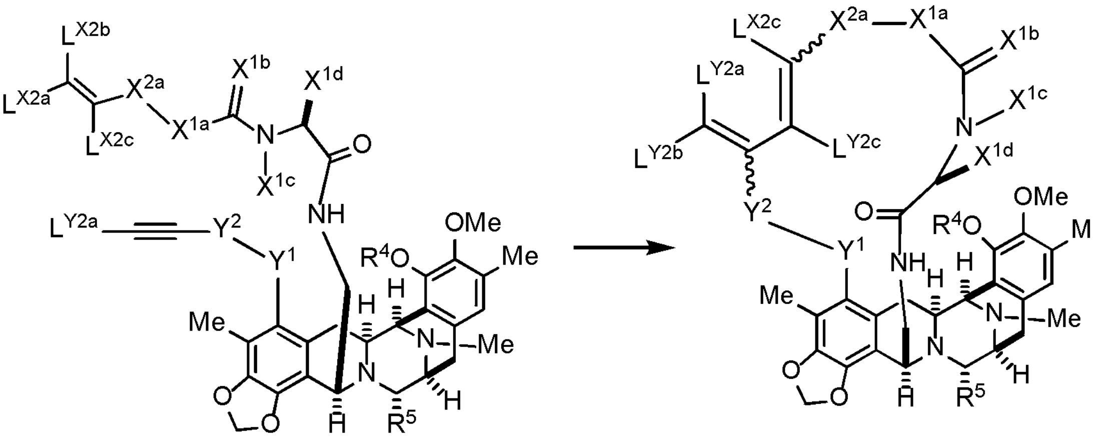

(IId) (Id)

step (C): subjecting a compound represented by the following formula (IIe) to a ring-closing olefin/alkyne metathesis reaction or an enyne metathesis reaction using a ruthenium catalyst or a tungsten catalyst represented by a Grubbs catalyst to obtain a compound represented by formula (If).

[C39]

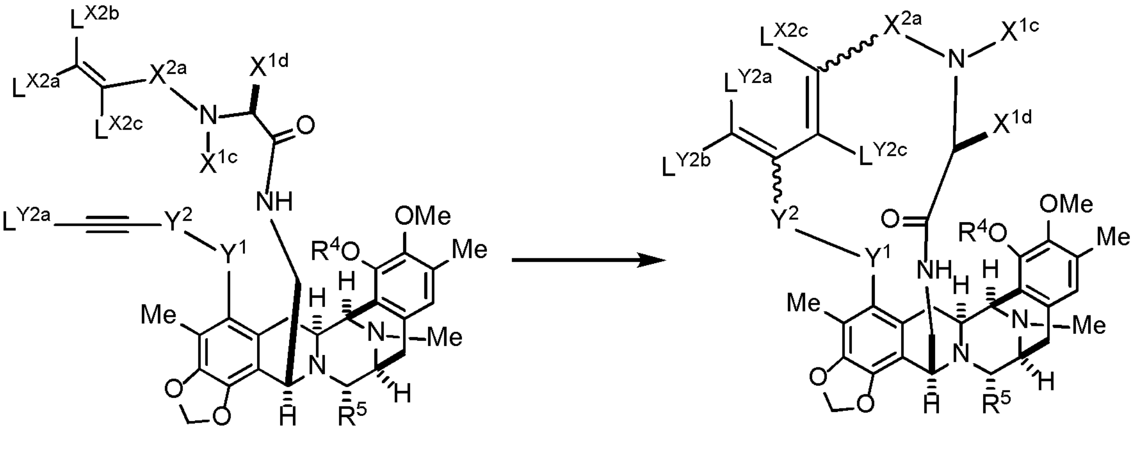

(IIe) (If)

In the above steps (A) to (C), $X^{1a}$, $X^{1b}$, $X^{1c}$, $X^{1d}$, $X^{2a}$, $L^{X2a}$, $L^{X2b}$, $L^{X2c}$, $Y^1$, $Y^{2a}$, $L^{Y2a}$, $L^{Y2b}$, $L^{Y2c}$, $R^4$, $R^5$ and Me are as defined in formulae (IIc) to (IIe) and formulae (Ic) to (If), and the above definitions and descriptions for preferable embodiments regarding formulae (IIc) to (IIe) and formulae (Ic) to (If) are applied without change. $R^4$ represents a protecting group for a phenolic hydroxyl group.

In the above reaction, as the Grubbs catalyst (Grubbs), any of ruthenium catalysts represented by a first generation Grubbs catalyst and a second generation Grubbs catalyst and tungsten catalysts that can be applied to alkyne-type substrates can be used. In addition, as the Grubbs catalyst (Grubbs), new catalysts derived from the first generation Grubbs catalyst and the second generation Grubbs catalyst such as Grubbs catalyst (registered trademark) M101: dichloro(3-phenyl-1H-inden-1-ylidene)bis(tricyclohexylphosphine)ruthenium (II) can also be used.

The reaction is typically performed in a reaction solvent such as an aromatic hydrocarbon-based solvent, for example, toluene, benzene, and xylene, dichloroethane, and dichloromethane. The reaction temperature is generally 0 to 100°C and preferably 20 to 60°C, and the reaction time is generally 1 to 48 hours.

(ii) three-component connection reaction of amine, alkyne, and aldehyde

In some embodiments, there is provided a production method including obtaining a compound represented by formula (I) by reacting the compound represented by formula (II) with aldehyde or ketone in the presence of a copper catalyst.

[C40]

(Scheme 2)

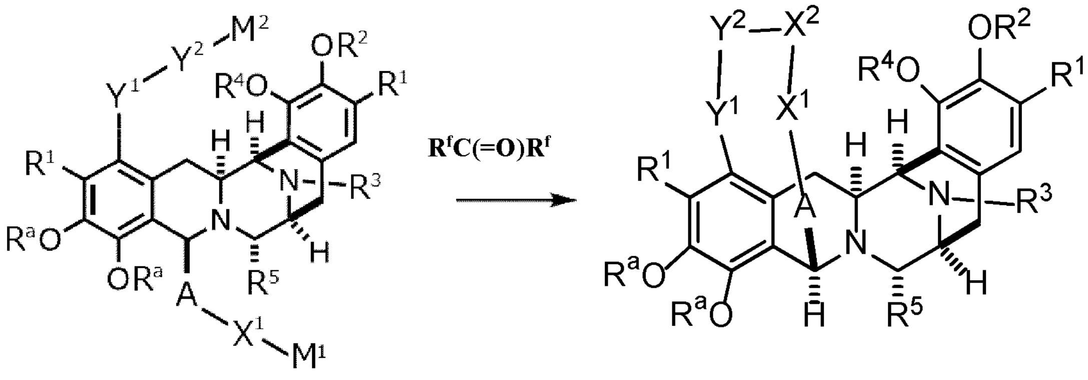


(II) (I)

In the embodiment, A, $X^1$, $Y^1$, $Y^2$, $R^a$, $R^1$, $R^2$, $R^3$, $R^5$, and $R^f$ are as defined in formula (II) and formula (I). $R^4$ is a protecting group.

In the embodiment, the combination of $M^1$ and $M^2$ is, for example, any one of the following (i) to (ii).

(i) $M^1$ is $NHR^b$ or an optionally substituted alkylene-$N(R^b)_2$, and $M^2$ is an optionally substituted alkynyl group.
(ii) $M^1$ is an optionally substituted alkynyl group, and $M^2$ is $NHR^b$ or an optionally substituted alkylene-$N(R^b)_2$.

$X^2$ is a corresponding group formed by the reaction of $M^1$ and $M^2$.

In the case of (i), among an amino group ($-NHR^b$) of $M^1$, an alkynyl group of $M^2$, and an aldehyde ($HCOR^f$), a three-component connection reaction occurs, and a structure in which $X^2$ is an alkynylene-derived from $-L^1-NR^b-CR^f_2-M^2$ is formed. $L^1$ is a single bond or a moiety derived from an optionally substituted alkylene of $M^1$. Therefore, the production method can be used to produce a compound in which $X^2$ is $-L^1-NR^b-CR^f_2-C{\equiv}C-L^2-$ in formula (I). For example, as the compound of formula (II) in the case of (i), the following Example Compound 26 may be exemplified.

In the case of (ii), among an alkynyl group (triple bond) of $M^1$, an amino group ($-NHR^b$) of $M^2$, and an aldehyde ($HCOR^f$), a three-component connection reaction occurs, and a structure in which $X^2$ is $-M^1$-derived alkynylene-$CHR^f-NR^b-L^2-$ is formed. $L^2$ is a single bond or a moiety derived from an optionally substituted alkylene of $M^2$. Therefore, the production method can be used to produce a compound in which $X^2$ is $-L^1-C{\equiv}C-CR^f_2-NR^b-L^2-$in formula (I).

In some embodiments, there is provided a production method, which is a method for producing a tetrahydroisoquinoline alkaloid compound containing a macrocyclic structure, the method including the following step (E).

step (E): reacting a compound represented by the following formula (IIf) with a compound represented by formula (IVb) in the presence of a copper catalyst to obtain a compound represented by formula (Ig).

[C41]

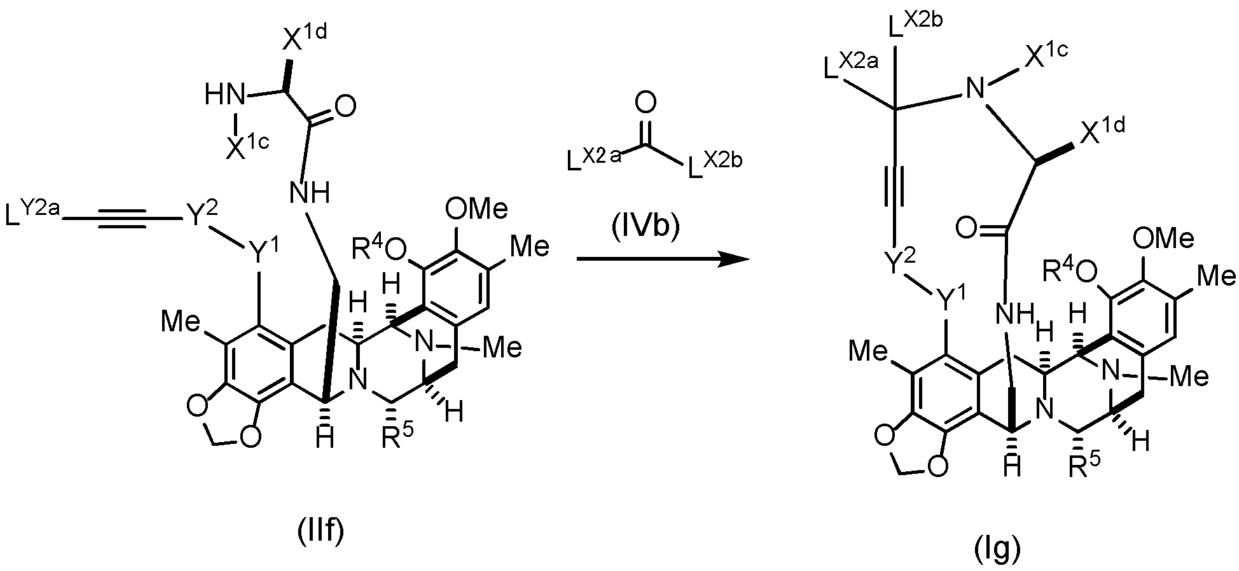


In the above step (E), $X^{1c}$, $X^{1d}$, $L^{X2a}$, $L^{X2b}$, $L^{X2c}$, $Y^1$, $Y^2$, $L^{Y2a}$, $R^4$, $R^5$, and Me are as defined in formulae (IIf) and (Ig), and the above definitions and descriptions for preferable embodiments regarding formula (IIf) are applied without change. $R^4$ represents a protecting group for a phenolic hydroxyl group.

In the above reaction, any copper catalyst may be used as long as it is used in a three-component connection reaction, and for example, copper(I) halide such as CuBr can be used.

The reaction is typically performed in a reaction solvent such as dimethyl dicarbonate or toluene. The reaction temperature is generally 0 to 100°C and preferably 20 to 60°C, and the reaction time is generally 1 to 48 hours.

(2-2) Bimolecular cyclization

The macrocyclic structure in a dimer compound represented by formula (III) is formed using two compounds represented by formula (II) and reacting them between $M^1$ at the 1-position of the THIQ framework of the first compound (II) and $M^2$ at the 5-position of the THIQ framework of the second compound (II) and between $M^2$ at the 5-position of the THIQ framework of the first compound (II) and $M^1$ at the 1-position of the THIQ framework of the second compound

(II) so that two compounds are linked via two linking groups $X^2$.

Such a macrocyclic structure can also be formed from two compounds represented by formula (II) using a three-component connection reaction of amine, alkyne, and aldehyde using a copper catalyst.

In some embodiments, there is provided a production method, which is a method for producing a tetrahydroisoquinoline alkaloid compound containing a macrocyclic structure, the method including the following step (F).

step (F): reacting a compound represented by the following formula (IIf) with a compound represented by formula (IVb) in the presence of a copper catalyst and a ligand to obtain a compound represented by formula (IIIc):

[C42]

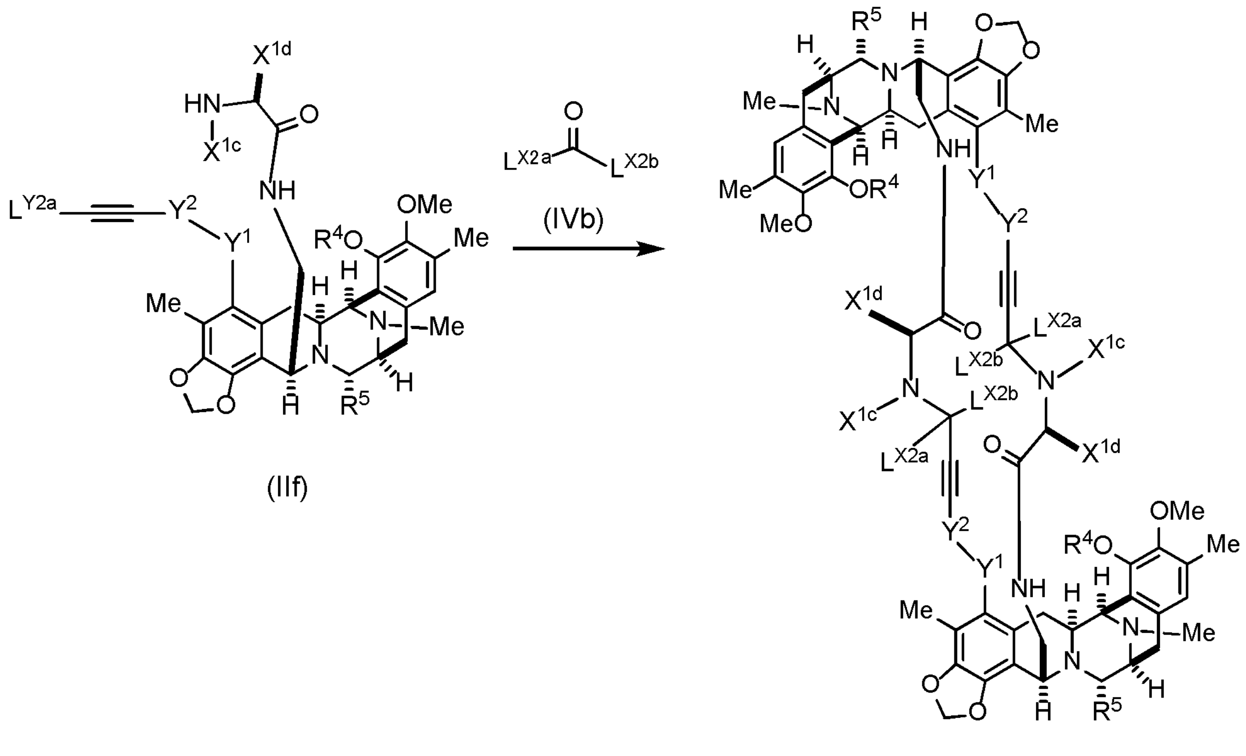

(IIf)

(IVb)

(IIIc)

In the above step (F), $X^{1c}$, $X^{1d}$, $L^{X2a}$, $L^{X2b}$, $L^{X2c}$, $Y^1$, $Y^2$, $L^{Y2a}$, $R^4$, $R^5$, and Me are as defined in formulae (IIf) and (IIIc), and the above definitions and descriptions for preferable embodiments regarding formula (IIf) are applied without change. $R^4$ represents a protecting group for a phenolic hydroxyl group.

Any copper catalyst may be used as long as it is used in a three-component coupling reaction, and for example, copper(I) halide such as CuBr can be used.

Examples of ligands include bulky ligands such as (R,M)-PINAP and Pybox(Pyridine-2,6-bis(oxazolines))-based ligands.

The reaction is typically performed in a reaction solvent such as dimethyl dicarbonate or toluene. The reaction temperature is generally 0 to 100°C and preferably 20 to 60°C, and the reaction time is generally 1 to 48 hours.

Alternatively, it is possible to obtain a dimer compound by performing a ring-closing metathesis reaction using two compounds represented by formula (II) in the presence of a Grubbs catalyst. The reaction conditions in this case are the same as conditions in the ring-closing metathesis reaction in formula (I).

(3) Step of modifying macrocyclic structure

After the macrocyclic structure is formed, as necessary, if a functional group that is contained in the macrocyclic structure and can be modified is used, or a functional group that can be modified is introduced into the macrocyclic structure, the substituents and structures are changed using the functional group, it is possible to produce compounds having various macrocyclic structures.

(3-1) [4+2]-cycloaddition reaction using dienophile

In some embodiments, when a dienophile represented by the following formula: $Z^1=Z^2$ is added to a compound in which $X^2$ is $-L^1-C(=CR^f_2)-CR^f=CR^f-L^2-$ or $-L^1-C(=CR^f_2)-CR^f=CR^f-L^2-$ in formula (I) according to a [4+2]-cycloaddition reaction, a compound in which $X^2$ has a cyclic structure represented by the following (z1) or (z2) in formula (I) is obtained. Therefore, the step can be used to produce a compound in which $X^2$ is a group is a group represented by the following (z1) or (z2) in formula (I).

[C43]

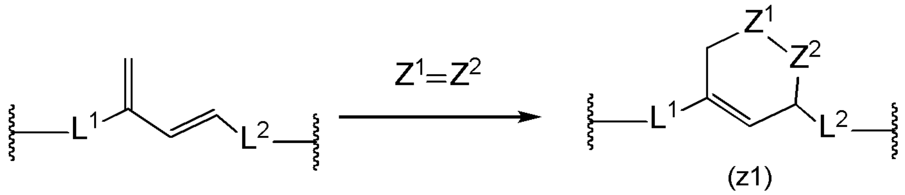

(z1)

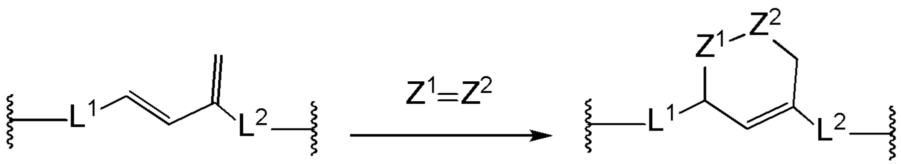

(z2)

$Z^1$, $Z^2$, $L^1$ and $L^2$ are as defined in formula (1).

In one embodiment, the dienophile is a substituted maleimide or substituted 1,2,4-triazoline-3,5-dione, and a compound with $X^2$ in formula (I) is obtained according to a [4+2]-cycloaddition reaction.

[C44]

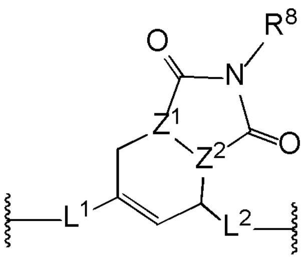

, or

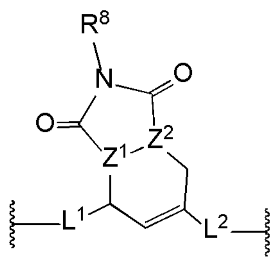

In some embodiments, there is provided a production method, which is a method for producing a tetrahydroisoquinoline alkaloid compound containing a macrocyclic structure, the method including the following step (D).

step (D): reacting a compound represented by formula (Id) with a compound represented by formula (IVa) to obtain a compound represented by formula (Ie).

[C45]

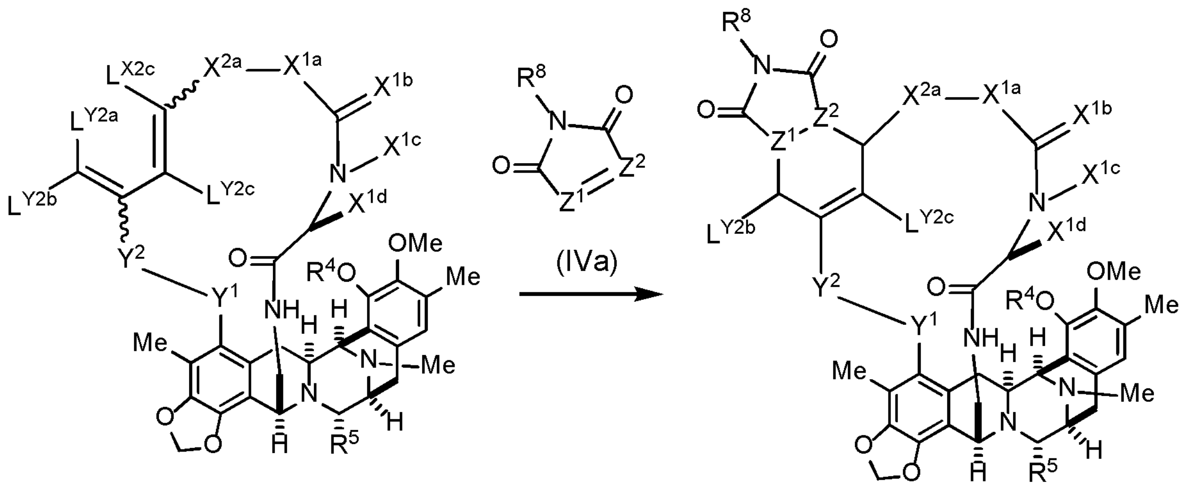

(IVa)

(Id)

(Ie)

In the above step (D), $X^{1a}$, $X^{1b}$, $X^{1c}$, $X^{1d}$, $X^{2a}$, $L^{X2c}$, $Y^1$, $Y^2$, $L^{Y2a}$, $L^{Y2b}$, $L^{Y2c}$, $R^4$, $R^5$, $R^8$, $Z^1$, $Z^2$ and Me are as defined in formulae (Id) and (Ie), and the above definitions and descriptions for preferable embodiments regarding formulae (Id) and (Ie) are applied without change. $R^4$ represents a protecting group for a phenolic hydroxyl group.

Conditions for the [4+2]-cycloaddition reaction using a dienophile are not particularly limited. Typically, the reaction is performed in a reaction solvent such as dichloromethane, dichloroethane, and tetrahydrofuran (THF). The reaction temperature is generally 0 to 100°C and preferably 20 to 60°C, and the reaction time is generally 1 to 48 hours.

(3-2) Eliminating $CO_2$ from carbamate

In some embodiments, it is possible to obtain a compound in which a cyclic structure of a macrocycle is changed according to the reaction of eliminating $CO_2$ from the carbamate group. For example, according to the reaction of eliminating $CO_2$ from a compound in which $X^1$ is $-NR^bC(O)O-$ or $-OC(O)NR^b-$ in formula (I) and a compound in which $Y^2$ is - $NR^bC(O)O-$ or $-OC(O)NR^b-$ in formula (I) (compound having a carbamate group), a compound in which $X^1$ is $-NR^b-$ or $-NR^b-$ in formula (I) and a compound in which $Y^2$ is $-NR^b-$ or $-NR^b-$ in formula (I) are obtained respectively.

In some embodiments, there is provided a production method, which is a method for producing a tetrahydroisoquinoline alkaloid compound containing a macrocyclic structure, the method including the following step (G).

step (G): obtaining a compound of formula (Ii) according to a reaction of eliminating $CO_2$ from a compound in which $L^3$ is -OC(O)- in formula (Ia) (provided that a carbon atom of $L^3$ is bonded to a nitrogen atom of N-$X^{1c}$ in formula (Ia)).

[C46]

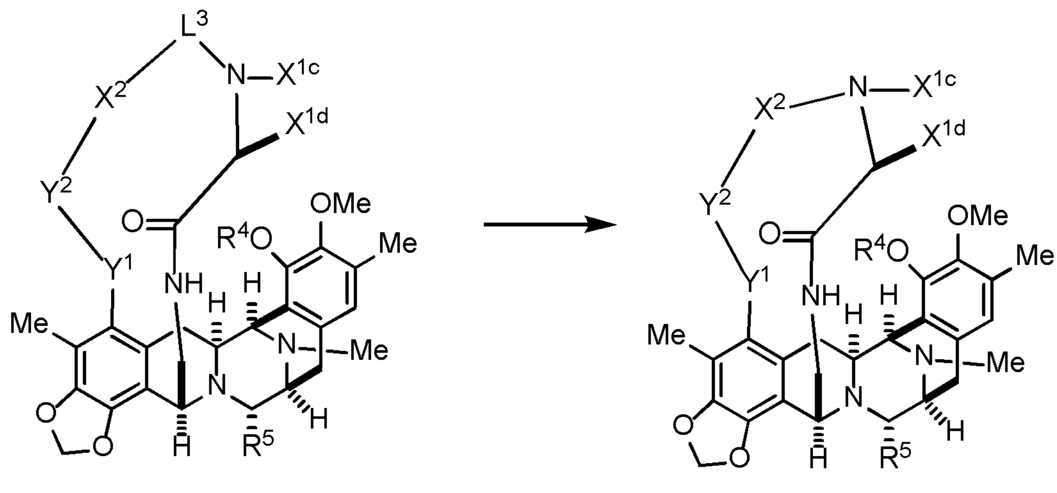

(Ia) (Ii)

In the above step (G), for $X^{1c}$, $X^{1d}$, $X^2$, $Y^1$, $Y^2$, $R^4$ and Me, the above definitions and descriptions for preferable embodiments regarding formula (Ia) are applied without change. $L^3$ is -OC(O)- (provided that a carbon atom of $L^3$ is bonded to a nitrogen atom of N-$X^{1c}$ in formula (Ia)).

In some embodiments, there is provided a production method, which is a method for producing a tetrahydroisoquinoline alkaloid compound containing a macrocyclic structure, the method including the following step (H). In the step (H), C(=O)O (that is, a moiety corresponding to C(=$X^{1b}$)$X^{1a}$) constituting a macrocycle is eliminated.

step (H): obtaining a compound of formula (Ih) according to a reaction of eliminating $CO_2$ from a compound in which $X^{1a}$ and $X^{1b}$ are an oxygen atom (O) in formula (Ic).

[C47]

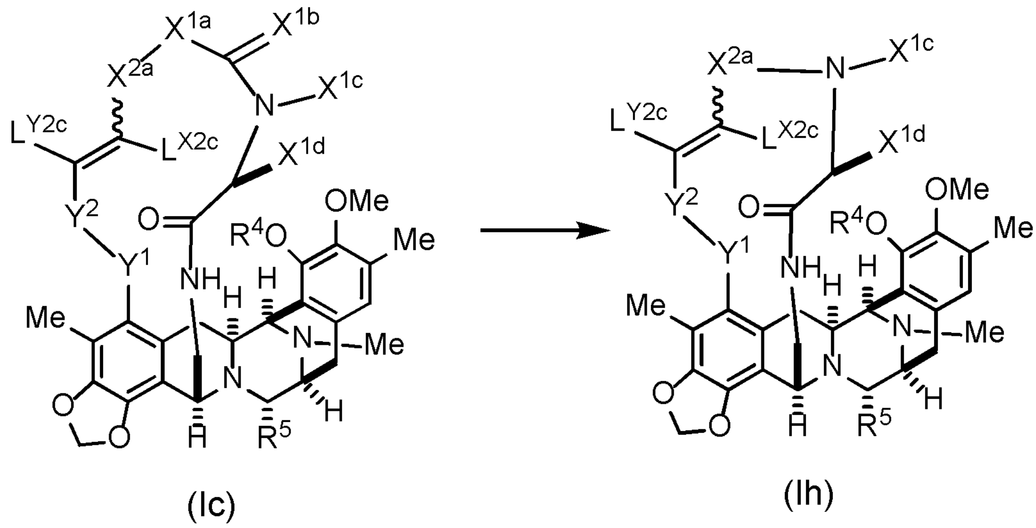

(Ic) (Ih)

In the above step (H), $X^{1c}$, $X^{1d}$, $X^{2a}$, $L^{X2c}$, $Y^1$, $Y^2$, $L^{Y2c}$, $R^4$, $R^5$, and Me are as defined in formulae (Ic) and (Ih), and the above definitions and descriptions for preferable embodiments regarding formulae (Ic) and (Ih) are applied without change. $X^{1a}$ and $X^{1b}$ represent an oxygen atom (O).

The reaction conditions for eliminating $CO_2$ from the carbamate are not particularly limited. Typically, in the presence of a 0-valent palladium catalyst, and optionally, a ligand such as phosphine, the reaction is performed in a reaction solvent such as toluene, dichloromethane, dichloroethane, or tetrahydrofuran (THF). The reaction temperature is generally 0 to 100°C and preferably 20 to 60°C, and the reaction time is generally 1 to 48 hours. The 0-valent palladium

catalyst is not particularly limited, and examples thereof include tris(dibenzylideneacetone)dipalladium ($Pd_2(dba)_3$), tetrakis(triphenylphosphine)palladium ($Pd(PPh_3)_4$), palladium acetate, bis(triphenylphosphine)palladium dichloride, trifluoropalladium acetate, bis(triphenylphosphine)palladium diacetate, bis(tri-o-tolyl phosphine)palladium dichloride, [1,2-bis(diphenylphosphino)ethane]palladium dichloride and combinations thereof. Examples of phosphine ligands include triphenylphosphine ($PPh_3$), tri-tert-butylphosphine, SEGPHOS (registered trademark) and combinations thereof.

(4) Step of deprotecting protecting group

A protecting group such as a nitrogen protecting group or phenolic hydroxyl group-protecting group introduced during the reaction procedure can be removed at a favorable subsequent stage using a method well-known in the related art.

For example, when a tert-butyloxycarbonyl group is used as a protecting group for a nitrogen group, deprotection is preferably performed under acidic conditions, and examples of acids include hydrochloric acid, acetic acid, trifluoroacetic acid, sulfuric acid, and tosic acid.

For example, when an acyl-based protecting group is used as a protecting group for a phenolic hydroxyl group, deprotection can be performed under reduction conditions (for example, in the presence of a reducing agent such as DIBAL (diisobutylaluminum hydride) or LAH (lithium aluminium hydride)) or basic conditions (for example, in the presence of NaOH or $K_2CO_3$/MeOH).

In one embodiment, when the protecting group contained in the compound of formula (I) is deprotected, a compound that does not contain a protecting group in formula (I) is obtained.

In one embodiment, when the protecting group contained in the compound of formula (IIIc) is deprotected, a compound that does not contain a protecting group in formula (IIIc) is obtained.

As described above, since the intermediate compound represented by formula (II) can be suitably used to synthesize a THIQ alkaloid compound containing a macrocyclic structure of formula (I), in another aspect, the present invention includes a method for producing a DNA alkylating agent or anti-cancer agent having a tetrahydroisoquinoline framework using an intermediate compound represented by formula (II). Similarly, in still another aspect, the present invention includes a use of the intermediate compound represented by formula (II) for producing a DNA alkylating agent or anti-cancer agent having a tetrahydroisoquinoline framework.

As described above, the compound represented by formula (II) can be suitably used for synthesizing a tetrahydroisoquinoline alkaloid compound containing a macrocyclic structure. Therefore, according to one aspect of the present invention, there is also provided a method for producing a tetrahydroisoquinoline alkaloid compound containing a macrocyclic structure using the compound represented by formula (II).

(5) Other intermediate compounds

In some embodiments, a compound in which $X^2$ is $-L^1-C(=CR^f_2)-CR^f=CR^f-L^2-$ or $-L^1-CR^f=CR^f-C(=CR^f_2)-L^2-$ in formula (I) is provided. Such a compound can be produced by the method (i) or (ii) in the above scheme 1. The compound of the embodiment has a partial structure $-C(=CR^f_2)-CR^f=CR^f-$ in the macrocyclic structure, and by using the partial structure, it is possible to produce compounds having various macrocyclic structures.

Hereinafter, specific examples of a macrocyclic structure-containing intermediate compound with $-L^1-C(=CR^f_2)-CR^f=CR^f-L^2-$ in formula (I) will be shown.

[C48]

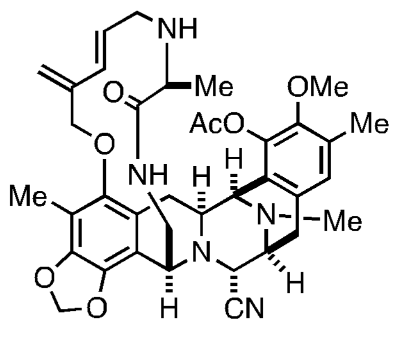

32

,

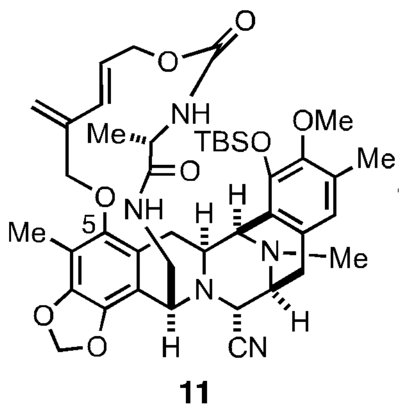

11

[Examples]

Hereinafter, the present invention will be described in more detail with reference to examples, but the present invention is not limited thereto.

In this specification, "room temperature" is generally about 10°C to about 35°C. Unless otherwise specified, "%" is percent by weight.

In this specification, the term "about" can mean ±10%.

The abbreviations used in examples are common abbreviations well known to those skilled in the art. Some abbreviations are shown below.

r.t.: room temperature
Me: methyl
Et: ethyl
iPr: isopropyl
Ac: acetyl
AcOH: acetic acid
MeOH: methanol
Ph: phenyl
MOM: methoxymethyl
TBS: tert-butyl dimethylsilyl
TBAF: tetra-n-butylammonium fluoride
Boc: tert-butoxycarbonyl
Ns: 2-nitrobenzenesulfonyl
Alloc: allyloxycarbonyl
Alloc-OSu: N-(allyloxycarbonyloxy)succinimide
DIPEA: N,N-diisopropylethylamine
DMAP: 4-dimethylaminopyridine
DMF: dimethylformamide
THF: tetrahydrofuran
hexane: hexane
Acetone: acetone
toluene: toluene
hv: light emission
HPLC: high performance liquid chromatography
Grubbs II: second generation Grubbs catalyst (benzylidene{ 1,3-bis(2,4,6-trimethylphenyl)-2-imidazolidinylidene} dichloro(tricyclohexylphosphine)ruthenium)
Grubbs I: first generation Grubbs catalyst (benzylidenebis(tricyclohexylphosphine)dichlororuthenium)
Grubbs cat. M101: dichloro(3-phenyl-1H-inden-1-ylidene)bis(tricyclohexylphosphine)ruthenium (II)
$PCy_3$: tricyclohexylphosphine
reflux: reflux
Allyl: allyl
PINAP: (R)-(+)-4-[2-(diphenylphosphino)-1-naphthalenyl]-n-[-1-phenylethyl]-1-phthalazinamine
dba: dibenzylideneacetone

[Reagent, device, etc.]

Unless otherwise specified, all reactions were performed under a nitrogen atmosphere. For NMR spectrums, a JEOL JNM-ECA 500 ($^1$H/500 MHz, $^{13}$C/125 MHz) spectrometer, a Bruker VSP 500 ($^1$H/500 MHz, $^{13}$C/125 MHz) spectrometer, a Bruker AMX500 ($^1$H/500 MHz, $^{13}$C/125 MHz) spectrometer and a JEOL JNM-ECS400 ($^1$H/400 MHz, $^{13}$C/100 MHz) spectrometer were used. For $^1$H, $^{13}$C-NMR, chloroform, acetonitrile and dimethyl sulfoxide were used as internal standards. $^1$H-NMR data was described as chemical shifts (hydrogen number, multiplicity, and coupling constant). The multiplicity was identified as s (singlet), d (doublet), t (triplet), q (quartet), quin (quintet), m (multiplet), or br (broad). Bruker Daltonics micrOTOF-QII was used for ESI-mass spectrums.

[Example 1]

1. Synthesis of Compound 7 of present invention

According to the following scheme, Compound 7, which is a THIQ compound containing a macrocyclic structure of the present invention, was synthesized in 6 processes from the easily available natural product Cyanosafracin B (Compound 1). First, Alloc-OSu and MOMBr were sequentially reacted to obtain Compound 3 in which the terminal amino group and phenolic hydroxyl group were protected. Phenol Compound 4 was obtained by allowing a photocyclization reaction to progress with visible light emission. This was not isolated and reacted with allyl bromide to obtain Compound 5. Compound 6 was synthesized by constructing a macrocycle according to a ring-closing olefin metathesis reaction using a second generation Grubbs catalyst. The MOM group was removed using trifluoroacetic acid to obtain Compound 7. Hereinafter, reaction conditions for each step will be described in detail.

[C49]

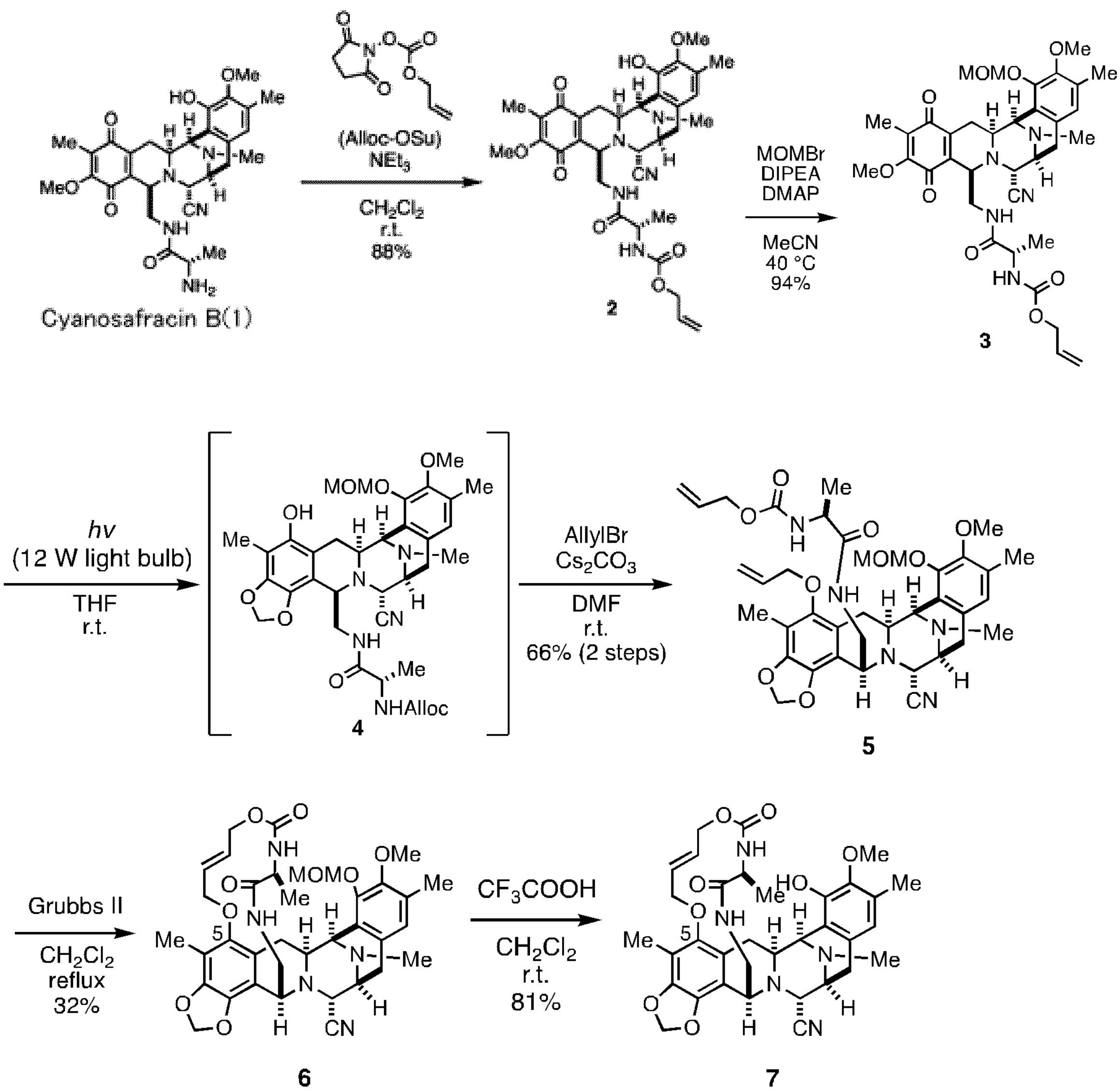


[Synthesis of Compound 2]

[C50]

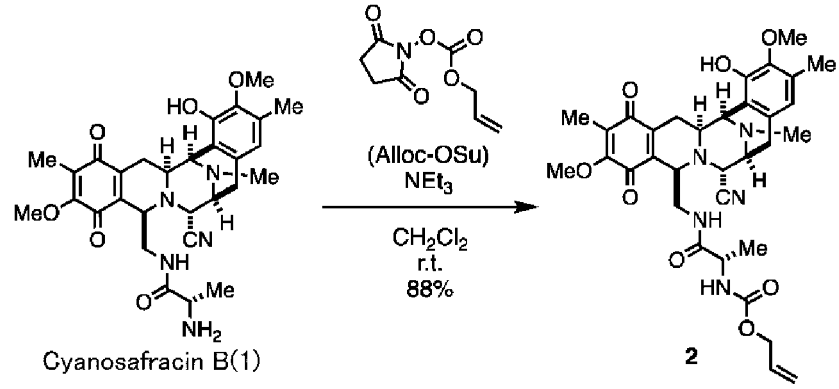

Cyanosafracin B(1)

2

Alloc-OSu (23.6 μL, 0.153 mmol, 1.2 equivalent), and $NEt_3$ (26.6 μL, 0.191 mmol, 1.5 equivalent) were added to a $CH_2Cl_2$ (1.27 mL, 0.10 M) solution of Cyanosafracin B(1) (70.0 mg, 0.127 mmol) under ice cooling. The mixture was stirred at room temperature for 50 minutes. Then, the mixture was diluted with $CH_2Cl_2$ (30 mL) and then quenched with a saturated $NH_4Cl$ aqueous solution (20 mL). After the organic phase and the aqueous phase were separated, the aqueous phase was extracted with $CH_2Cl_2$ (40 mL×2). After the organic phase was mixed, the mixture was washed with a saturated saline, dried with $Na_2SO_4$ and concentrated under a reduced pressure. The crude residue was purified through silica gel column chromatography (hexane/AcOEt), and 2 (70.9 mg, 0.112 mmol, yield 88%) was obtained as a yellow solid.

$^1$H NMR (500 MHz, $CDCl_3$, δ): 0.97 (3H, d, J = 6.3 Hz), 1.77 (1H, dd, J = 16.9, 11.2 Hz), 1.86 (5H, m), 2.28 (3H, s), 2.36 (3H, s), 2.43 (1H, d, J = 18.3 Hz), 2.95 (1H, dt, J = 14.1, 3.3 Hz), 3.04 (1H, dd, J = 18.3, 3.4 Hz), 3.13 (2H, dd, J = 18.0, 7.7 Hz), 3.23 (1H, dt, J = 10.9, 2.9 Hz), 3.38 (1H, d, J = 8.0 Hz), 3.76 (3H, s), 3.80-3.86 (2H, m), 4.00 (4H, m), 4.19 (1H, d, J = 1.7 Hz), 4.43 (2H, m), 4.84 (1H, d, J = 6.9 Hz), 5.03 (1H, d, J = 7.4 Hz), 5.18-5.28 (2H, m), 5.86 (1H, m), 6.27 (1H, s), 6.52 (1H, s).

[Synthesis of Compound 3]

[C51]

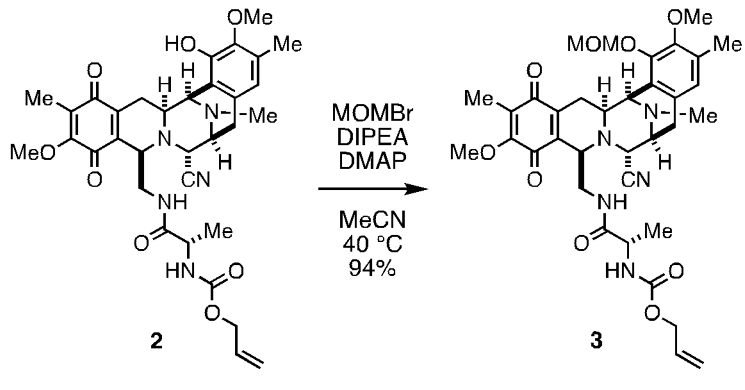

2

3

$iPr_2NEt$ (DIPEA, 633 μL, 3.70 mmol, 15 equivalent), DMAP (3.0 mg, 0.0247 mmol, 0.10 equivalent), and MOMBr (193 μL, 2.47 mmol, 10 equivalent) were added to a solution of Compound 2 (156 mg, 0.247 mmol) in MeCN (2.3 mL, 0.11 M) under ice cooling. The mixture was stirred at 40°C for 4 hours. Quenching was performed with 200 mM $NaH_2PO_4$·NaOH buffer (pH 7.0, 15 mL), and MeCN was distilled off under a reduced pressure. Water (10 mL) was added, extraction with $CH_2Cl_2$ (30 mL×3) was then performed, the organic phase was mixed, and the mixture was then washed with a saturated saline, dried with $Na_2SO_4$ and concentrated under a reduced pressure. The crude residue was purified through silica gel column chromatography (hexane/AcOEt), and Compound 3 (157 mg, 0.231 mmol, yield 94%) was obtained as a yellow solid.

$^1H$ NMR (500 MHz, $CDCl_3$, δ): 1.00 (3H, d, J = 5.7 Hz), 1.87 (3H, s), 2.25 (4H, s), 2.35 (3H, s), 2.46 (1H, d, J = 18.3 Hz), 3.01-3.23 (5H, m), 3.40 (1H, d, J = 8.8 Hz), 3.58 (3H, s), 3.71 (3H, s), 3.88 (2H, m), 4.00-4.02 (4H, m), 4.26 (1H, d, J = 2.3 Hz), 4.36-4.43 (2H, m), 4.77 (1H, br), 5.14-5.17 (3H, m), 5.19-5.30 (3H, m), 5.82 (1H, m), 6.74 (1H, s).

[Synthesis of Compound 5]

[C52]

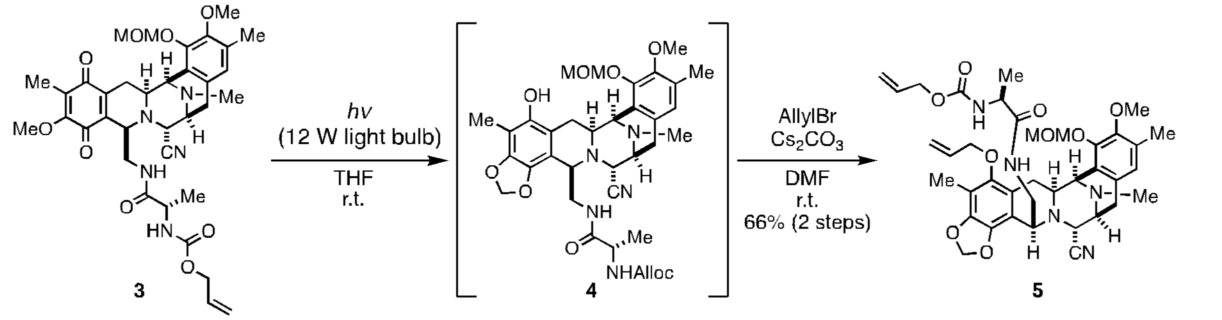


A $CH_2Cl_2$ (10 mL, 0.10 M) solution of Compound 3 (156 mg, 0.230 mmol) was stirred at room temperature for 7.5 hours while emitting visible light using a 12 W household light bulb. The reaction solution was concentrated under a reduced pressure. The crude residue was used in the next reaction without purification.

$Cs_2CO_3$ (300 mg, 0.920 mmol, 4.0 equivalent), and allyl bromide (26.6 μL, 0.191 mmol, 1.5 equivalent) were added to a DMF (11.5 mL, 0.020 M) solution of the crude product of Compound 4 under ice cooling. The mixture was stirred at room temperature for 1.5 hours. After filtration, diluting with $CH_2Cl_2$ (50 mL) was performed and a 200 mM $NaH_2PO_4$·NaOH buffer (pH 7.0, 20 mL) was added. After the organic phase and the aqueous phase were separated, the aqueous phase was extracted with $CH_2Cl_2$ (30 mL×2). After the organic phase was mixed, the mixture was washed with a saturated saline, dried with $Na_2SO_4$ and concentrated under a reduced pressure. The crude residue was purified through silica gel column chromatography (hexane/AcOEt), and Compound 5 (98.9 mg, 0.138 mmol, 2-stage yield 60%) was obtained as a brown solid.

$^1H$ NMR (500 MHz, $CDCl_3$, δ): 0.86-0.92 (4H, m), 1.85 (1H, dd, J = 16.0, 12.1 Hz), 2.11 (3H, s), 2.22 (3H, s), 2.32 (3H, s), 2.64 (1H, d, J = 17.8 Hz), 3.03 (1H, dd, J = 18.3, 8.0 Hz), 3.22-3.25 (2H, m), 3.39 (1H, d, J = 6.9 Hz), 3.49-3.60 (6H, m), 3.69-3.78 (4H, m), 4.02 (1H, s), 4.08-4.12 (2H, m), 4.19-4.23 (2H, m), 4.43 (2H, d, J = 5.2 Hz), 5.08-5.41 (10H, m), 5.79-5.86 (2H, m), 5.96 (1H, d, J = 1.7 Hz), 6.06-6.14 (1H, m), 6.71 (1H, s).

[Synthesis of Compound 6]

[C53]

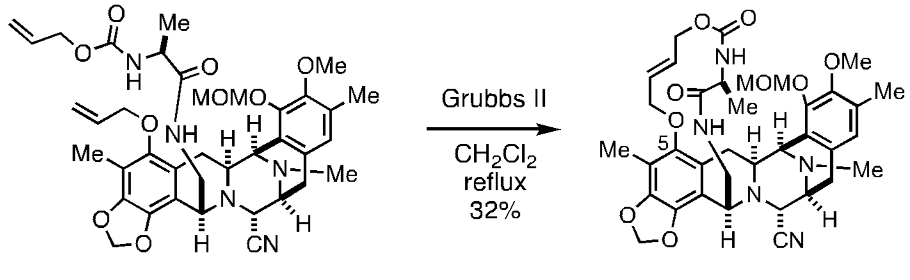


5 6

A $CH_2Cl_2$ (30 mL) solution of a Grubbs second generation catalyst (13.5 mg, 15.9 μmol, 0.20 equivalent) was freeze-deaerated and heated to reflux and a $CH_2Cl_2$ (9.8 mL, final concentration 2.0 mM) solution of Compound 5 (57.2

mg, 79.7 μmol) was then added. After heating to reflux for 2.5 hours, concentration was performed under a reduced pressure. The crude residue was purified using silica gel column chromatography (hexane/AcOEt) and an HPLC system (water/MeCN), and Compound 6 (17.5 mg, 25.4 μmol, yield 32%) was obtained as a brown oily substance.
$^1$H NMR (500 MHz, DMSO-$d_6$, 6): 1.05-1.12 (3H, m), 1.80 (1H, dd, J = 15.5, 12.0 Hz), 2.05-2.10 (3H, m), 2.21 (6H, m), 2.67 (1H, d, J = 18.3 Hz), 2.90-2.94 (1H, m), 2.98-3.10 (7H, m), 3.33- 3.42 (2H, m), 3.57 (3H, m), 3.79-3.86 (4H, m), 4.13 (1H, m), 4.34-4.41 (2H, m), 4.68 (1H, dd, J = 12.9, 5.4 Hz), 5.07 (1H, d, J = 5.7 Hz), 5.19 (1H, d, J = 5.2 Hz), 5.50-5.65 (2H, m), 5.96 (1H, s), 6.00 (1H, s), 6.68 (2H, m). $^{13}$C NMR (125 MHz, DMSO-$d_6$, δ): $^{13}$C NMR (126 MHz, DMSO-D6) δ 9.2, 13.6, 15.2, 17.1, 20.1, 24.2, 26.3, 49.4, 54.1, 55.7, 56.1, 56.2, 56.5, 58.1, 59.2, 62.1, 71.3, 98.3, 100.7, 111.4, 112.7, 117.6, 121.6, 123.3, 124.3, 128.1, 129.2, 129.5, 130.0, 138.0, 143.8, 145.6, 147.7, 153.9, 171.1.

[Synthesis of Compound 7]

[C54]

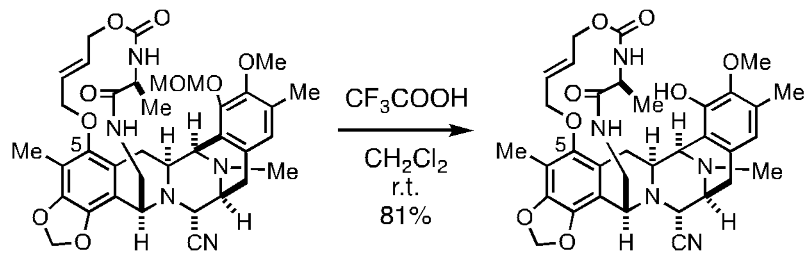


6 7

Trifluoroacetic acid (26.3 μL, 344 μmol, 40 equivalent) was added to a $CH_2Cl_2$ (1.0 mL, 8.6 mM) solution of Compound 6 (5.93 mg, 8.60 μmol) under ice cooling. The mixture was stirred at room temperature for 12.5 hours, and then diluted with $CH_2Cl_2$ (10 mL), and a saturated $NaHCO_3$ aqueous solution (10 mL) was added. After the organic phase and the aqueous phase were separated, the aqueous phase was extracted with $CH_2Cl_2$ (10 mL×2). After the organic phase was mixed, the mixture was washed with a saturated saline, dried with $Na_2SO_4$ and concentrated under a reduced pressure. The crude residue was purified through an HPLC system (water/MeCN), and Compound 7 (4.89 mg, 8.60 μmol, yield 88%) was obtained as a colorless oily substance.
$^1$H NMR (500 MHz, DMSO-$d_6$, δ): 1.02 (3H, d, J = 7.4 Hz), 2.09 (3H, s), 2.17 (3H, s), 2.22 (3H, s), 3.30 (1H, d, J = 8.6 Hz), 3.72 (3H, s), 3.86-3.89 (2H, m), 4.11 (1H, s), 4.37-4.43 (2H, m), 4.52 (1H, s), 4.83 (1H, s), 5.42-5.54 (2H, m), 5.84-5.88 (1H, m), 5.96 (2H, d, J = 17.2 Hz), 6.43 (1H, s), 6.64 (1H, s).

[Example 2]

2. Synthesis of Compounds 13, 14, 16, and 18 of present invention

According to the following scheme, Compounds 13 and 14 were synthesized in 6 processes and Compounds 16 and 18 were synthesized in 7 processes from the natural product Cyanosafracin B (Compound 1). First, Alloc-OSu and TBSCI were sequentially reacted to obtain Compound 8 in which the terminal amino group and phenolic hydroxyl group were protected. Phenol Compound 9 was obtained by allowing a photocyclization reaction to progress with visible light emission. This was not isolated and reacted with propargyl bromide to obtain Compound 10. Compounds 11 and 12 were synthesized by constructing a macrocycle according to a ring-closing enyne metathesis reaction using a first generation Grubbs catalyst. The TBS group was removed using TBAF to obtain Compounds 13 and 14. A [4+2]-cyclization reaction was performed on Compound 12 and the TBS group was then removed to synthesize Compounds 16 and 18. Reaction conditions for each step will be described in detail.

[C55-1]

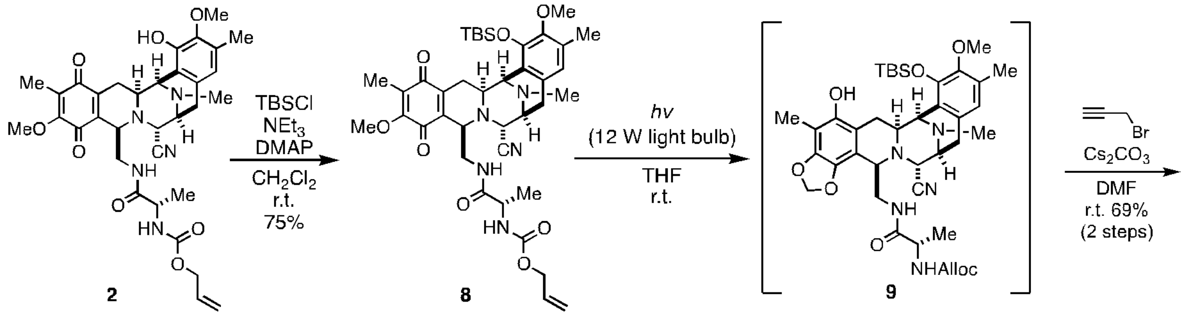
TBSCl
$NEt_3$
DMAP
$CH_2Cl_2$
r.t.
75%
hv
(12 W light bulb)
THF
r.t.
$Cs_2CO_3$
DMF
r.t. 69%
(2 steps)

2 8 9

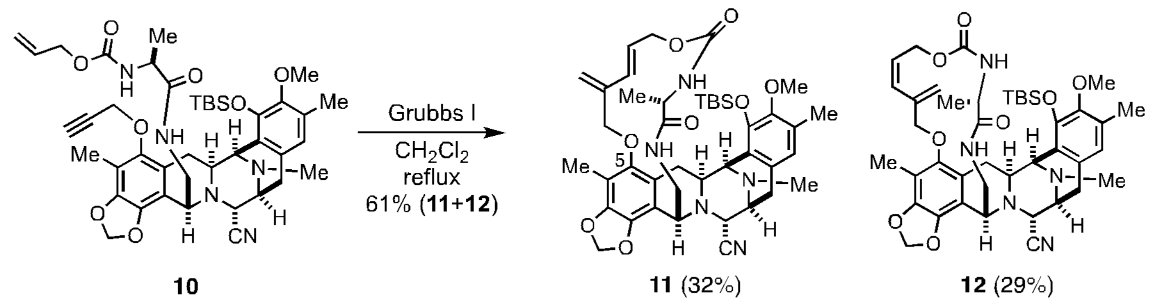
Grubbs I
$CH_2Cl_2$
reflux
61% (**11**+**12**)
**10**
**11** (32%)
**12** (29%)

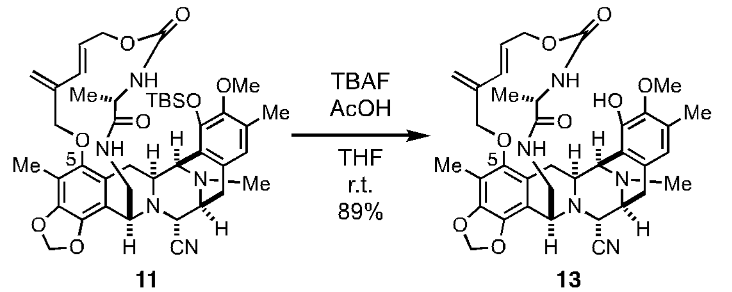
TBAF
AcOH
THF
r.t.
89%
**11**
**13**

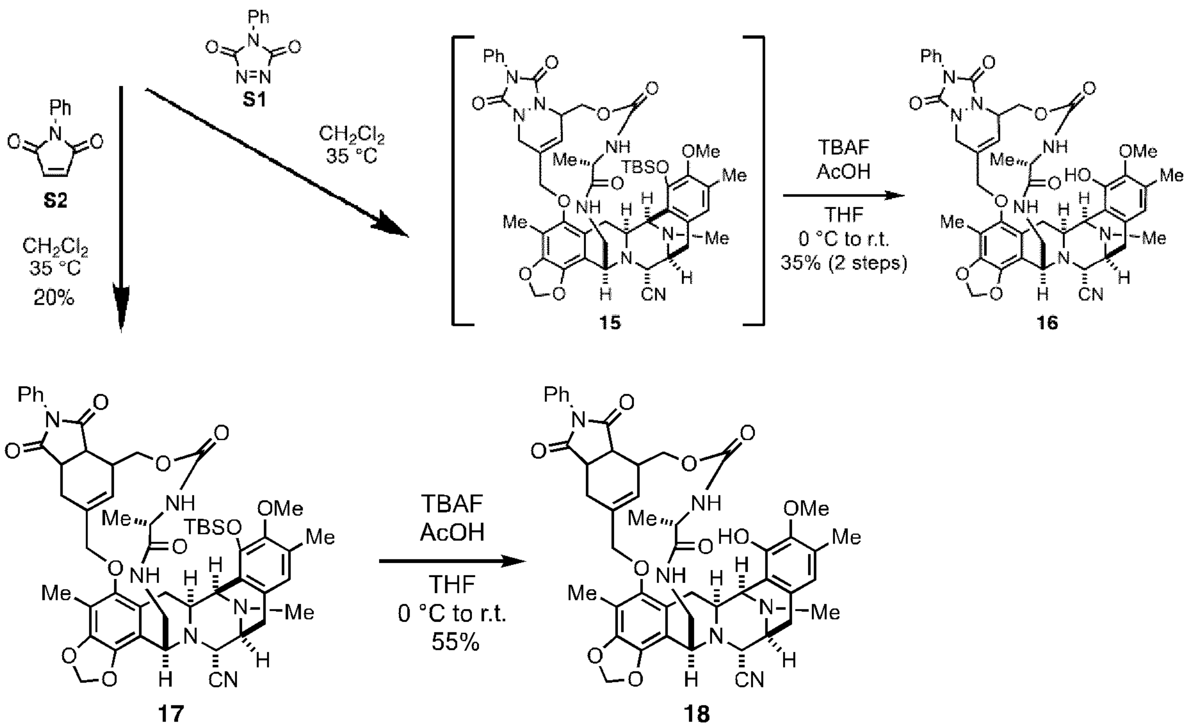
**S1**
$CH_2Cl_2$
35 °C
**S2**
$CH_2Cl_2$
35 °C
20%
**15**
TBAF
AcOH
THF
0 °C to r.t.
35% (2 steps)
**16**
**17**
TBAF
AcOH
THF
0 °C to r.t.
55%
**18**

[C55-2]

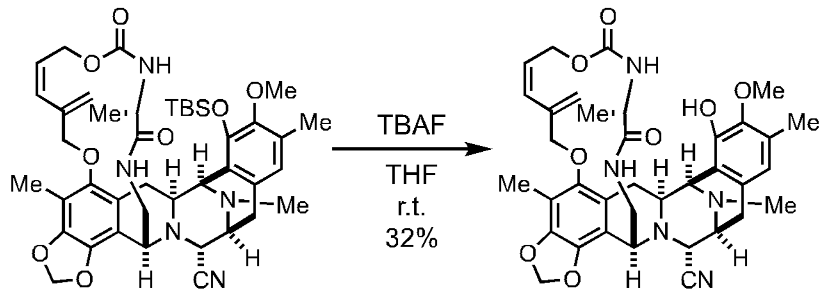


12 14

[Synthesis of Compound 8]

[C56]

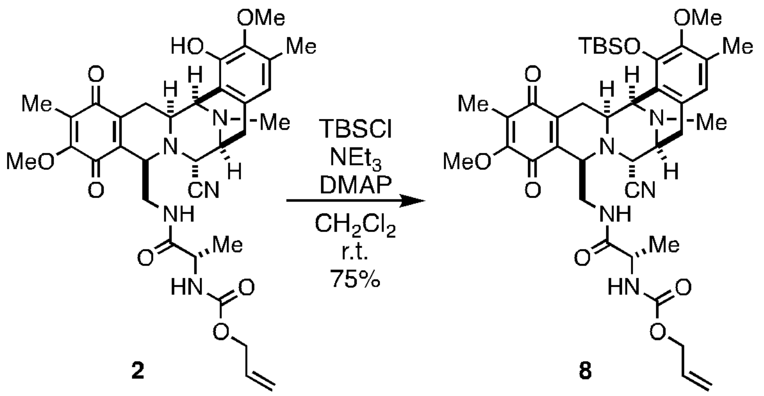


2 8

$NEt_3$ (198 μL, 1.42 mmol, 20 equivalent), DMAP (1.74 mg, 14.2 μmol, 0.20 equivalent), and TBSCl (107 mg, 710 μmol, 10 equivalent) were added to a 2 (45.0 mg, 71.0 μmol) solution in $CH_2Cl_2$ (0.19 mL, 0.10 M) at room temperature. The mixture was stirred at room temperature for 23 hours. Water (30 mL) was added, extraction with $CH_2Cl_2$ (30 mL×3) was then performed, the organic phase was mixed, and the mixture was then washed with a saturated saline, dried with $Na_2SO_4$ and concentrated under a reduced pressure. The crude residue was purified through silica gel column chromatography (hexane/AcOEt), and Compound 8 (39.8 mg, 53.2 μmol, yield 75%) was obtained as a yellow solid.
$^1H$ NMR (400 MHz, $CDCl_3$, δ): 0.11 (3H, s), 0.35 (3H, s), 1.11-1.02 (12H, m), 1.88 (3H, s), 2.26 (3H, s), 2.39-2.45 (4H, m), 2.99-3.21 (5H, m), 3.37 (1H, d, J = 7.8 Hz), 3.56 (3H, s), 3.73-4.04 (7H, m), 4.25 (1H, d, J = 2.7 Hz), 4.36-4.48 (2H, m), 4.79 (1H, s), 5.17-5.27 (3H, m), 5.82 (1H, m), 6.60 (1H, s); HRMS (ESI, m/z): $[M+H]^+$ calcd. for $C_{39}H_{54}N_5O_8Si$, 748.3736; found, 748.3765.

[Synthesis of Compound 10]

[C57]

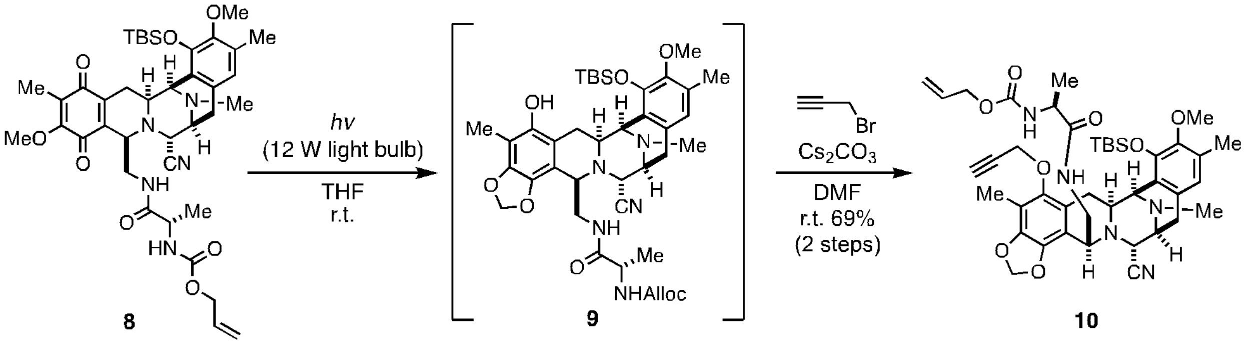


8 9 10

A 8 (12.6 mg, 16.8 μmol) solution in freeze-deaerated THF (3.9 mL, 0.10 M) was stirred at room temperature for 1 hours 30 minutes while emitting blue light. The reaction solution was concentrated under a reduced pressure, and the obtained crude residue was used in the next reaction without purification.

$Cs_2CO_3$ (21.9 mg, 67.2 μmol, 4 equivalent) and propargyl bromide (3.80 μL, 50.4 μmol, 3.0 equivalent) were added to a DMF (0.34 mL, 0.050 M) solution of the crude product of Compound 9. The mixture was stirred at room temperature for 18 hours. After concentration under a reduced pressure, water (30 mL) was added and extraction with ethyl acetate (30 mL×3) was performed. After the organic phase was mixed, the mixture was washed with a saturated saline, dried with $Na_2SO_4$ and concentrated under a reduced pressure. The crude residue was purified through silica gel column chromatography (hexane/AcOEt), and Compound 10 (9.14 mg, 11.6 μmol, 2-stage yield 63%) was obtained as a yellow solid. $^1$H NMR (400 MHz, $CDCl_3$, δ): 0.10 (3H, s), 0.36 (3H, s), 0.91-0.99 (2H, m), 1.08 (9H, s), 1.80-1.89 (2H, m), 2.14 (3H, s), 2.23 (3H, s), 2.35 (3H, s), 2.43 (1H, t, J = 2.3 Hz), 2.52-2.59 (1H, m), 3.04 (1H, q, J = 8.7 Hz), 3.21-3.25 (2H, m), 3.33-3.45 (3H, m), 3.60 (3H, m), 4.01-4.13 (2H, m), 4.18-4.36 (2H, m), 4.41 (2H, d, J = 5.0 Hz), 4.51 (1H, d, J = 15.6 Hz), 5.13-5.31 (4H, m), 5.75-5.86 (2H, m), 5.94 (1H, s), 6.57 (1H, s); HRMS (ESI, m/z): $[M+H]^+$ calcd. for $C_{42}H_{56}N_5O_8Si$, 786.3893; found, 786.3907.

[Synthesis of Compounds 11 and 12]

[C58]

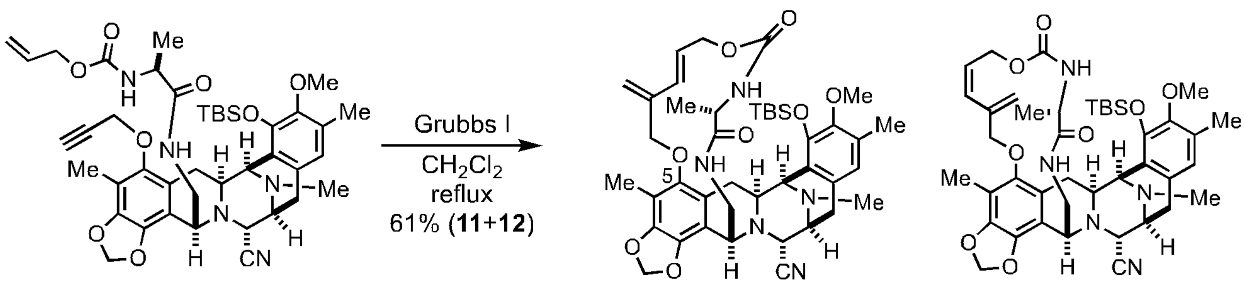


10 **11** (32%) **12** (29%)

A Grubbs first generation catalyst (10.9 mg, 13.2 μmol, 0.20 equivalent) was added to a $CH_2Cl_2$ (6.6 mL, 0.10 M) solution of Compound 10 (51.9 mg, 66.0 μmol). The mixture was heated to reflux for 22 hours. The reaction solution was concentrated under a reduced pressure, the residue was then purified through silica gel column chromatography ($CHCl_3$/acetone), and Compound 11 (E isomer, 16.6 mg, 21.1 μmol, yield 32%), and 12 (Z isomer, 15.1 mg, 19.2 μmol, yield 29%) were obtained as white solids.

11: $^1$H NMR (400 MHz, DMSO-$d_6$, δ): 0.08 (3H, s), 0.33 (3H, s), 1.04 (11H, m), 1.13 (3H, d, J = 7.3 Hz), 1.80-1.86 (1H, m), 2.04 (3H, s), 2.19 (3H, s), 2.26 (3H, s), 2.59 (1H, m), 3.00 (2H, m), 3.32-3.38 (2H, m), 3.45-3.63 (4H, m), 3.84 (1H, m), 4.00-4.28 (3H, m), 4.43-4.53 (2H, m), 4.63 (1H, br), 5.02 (1H, br), 5.37 (1H, s), 5.50 (1H, s), 5.96-6.09 (3H, m), 6.63 (1H, s), 6.90 (1H, br); HRMS (ESI, m/z): $[M-CN]^+$ calcd. for $C_{41}H_{55}N_4O_8Si$, 759.3784; found, 759.3771.
12: $^1$H NMR (400 MHz, DMSO-$d_6$, δ): 0.08 (3H, s), 0.34 (3H, s), 1.03 (13H, m), 2.08 (3H, s), 2.16 (3H, s), 2.24 (3H, s), 2.56 (1H, d, J = 18.3 Hz), 2.89-3.00 (4H, m), 3.31-3.50 (7H, m), 3.88 (1H, s), 4.04-4.21 (2H, m), 4.40 (1H, s),

4.65 (1H, br), 4.83 (1H, br), 5.04 (1H, br), 5.34 (1H, m), 5.52 (1H, s), 5.71 (1H, d, J= 1.1 Hz), 5.99 (2H, m), 6.13 (1H, d, J = 11.0 Hz), 6.61 (1H, s), 7.01 (1H, br); HRMS (ESI, m/z): $[M+H]^+$ calcd. for $C_{42}H_{56}N_5O_8Si$, 786.3893; found, 786.3887.

[Synthesis of Compound 13]

[C59]

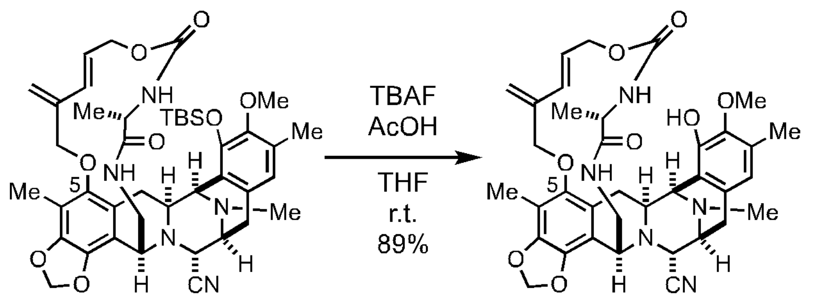


11 13

A mixed solution containing TBAF (1M THF solution, 23.4 μL, 23.4 μmol, 3.0 equivalent)/AcOH (1.34 μL, 23.4 μmol, 3.0 equivalent) was added to a THF (0.78 mL, 0.010 M) solution of Compound 11 (6.14 mg, 7.81 μmol) under ice cooling. The mixture was stirred at room temperature for 2 hours and then concentrated under a reduced pressure. The crude product was passed through STRATA (registered trademark) C18 and eluted with MeCN. After concentration, the residue was purified through an HPLC system, and Compound 13 (4.69 mg, 6.98 μmol, yield 89%) was obtained as a yellow oily substance. $^1$H NMR (400 MHz, DMSO-$d_6$, δ): $^1$H NMR (400 MHz, DMSO-$d_6$, δ): 1.09 (1H, d, J = 6.9 Hz), 1.86 (1H, dd, J = 15.6, 11.9 Hz), 1.99-2.20 (5H, m), 2.55 (1H, m), 2.90-3.15 (3H, m), 3.29-3.36 (2H, m), 3.50-3.64 (4H, m), 3.83 (1H, d, J = 4.6 Hz), 4.02-4.63 (5H, m), 5.08 (1H, m), 5.45 (3H, m), 5.72 (1H, d, J = 1.4 Hz), 5.98 (2H, m), 6.17 (1H, d, J = 15.6 Hz), 6.43 (1H, s), 6.84 (1H, s), 8.44 (1H, s); $^{13}$C NMR (100 MHz, DMSO-$d_6$, δ): 10.1, 15.2, 24.5, 25.9, 40.8, 50.2, 54.3, 54.5, 54.9, 56.0, 56.5, 58.2, 59.7, 63.9, 71.6, 100.9, 111.0, 113.3, 117.4, 118.0, 119.3, 119.7, 121.9, 124.8, 128.3, 130.1, 132.0, 138.0, 142.1, 143.3, 143.8, 147.4, 154.2, 171.9; HRMS (ESI, m/z): $[M-CN]^+$ calcd. for $C_{35}H_{41}N_4O_8$, 645.2919; found, 645.2920.

[Synthesis of Compound 14]

[C60]

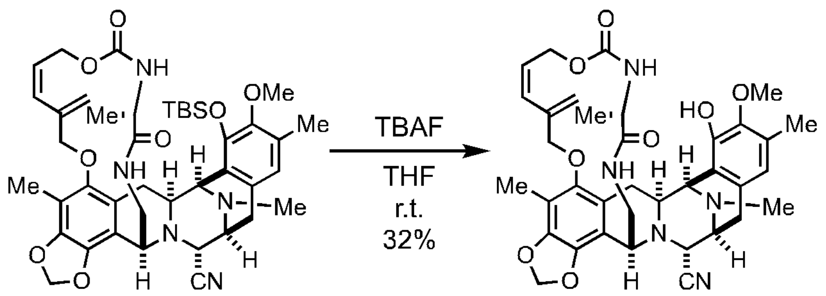


12 14

TBAF (1M THF solution, 59.2 μL, 59.2 μmol, 3.0 equivalent) was added to a THF (2.0 mL, 0.010 M) solution of Compound 12 (15.5 mg, 19.7 μmol). The mixture was stirred at room temperature for 1 hour and 20 minutes and then concentrated under a reduced pressure. The crude product was passed through STRATA (registered trademark) C18

and eluted with MeCN. After concentration, the residue was purified through an HPLC system, Compound 14 (4.24 mg, 6.31 μmol, yield 32%) was obtained as a yellow oily substance. $^1H$ NMR (400 MHz, DMSO-$d_6$, δ): 0.99 (3H, d, J = 6.9 Hz), 2.05-2.08 (4H, m), 2.12-2.14 (6H, m), 2.54 (1H, s), 2.80-3.08 (4H, m), 3.25-3.55 (3H, m), 3.61 (3H, s), 3.87 (1H, s), 4.02-4.09 (2H, m), 4.23-4.39 (2H, m), 4.56 (1H, d, J = 13.3 Hz), 4.82 (1H, br), 5.09 (1H, s), 5.34-5.41 (1H, m), 5.45-5.67 (2H, m), 5.95-6.00 (2H, m), 6.20 (1H, d, J = 11.4 Hz), 6.41 (1H, s), 6.87 (1H, br), 8.35 (1H, s); $^{13}C$ NMR (100 MHz, DMSO-$d_6$, δ): 9.4, 15.2, 24.5, 26.6, 40.9, 50.0, 54.2, 55.2, 55.8, 56.1, 58.3, 59.5, 60.0, 74.7, 100.9, 110.2, 113.6, 117.5, 118.0, 119.1, 120.3, 125.2, 128.4, 129.3, 129.8, 133.0, 137.8, 143.4, 147.4, 170.9; HRMS (ESI, m/z): $[M-CN]^+$ calcd. for $C_{35}H_{41}N_4O_8$, 645.2919; found, 645.2935.

[Synthesis of Compound 16]

[C61]

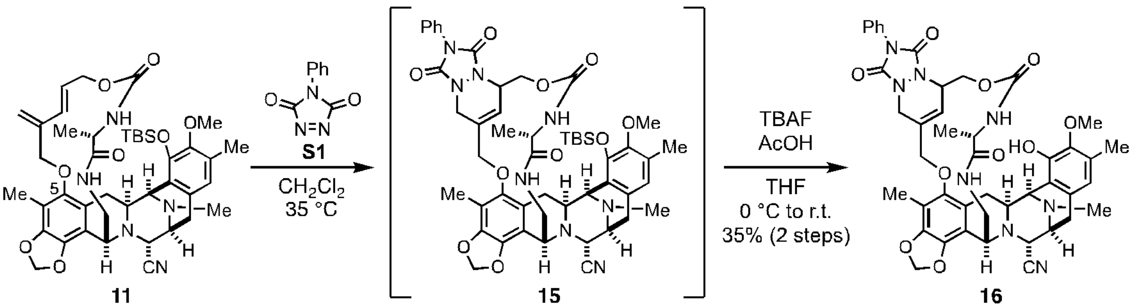


11 15 16

4-phenyl-1,2,4-triazoline-3,5-dione (S1, 15.9 mg, 90.8 μmol, 3.0 equivalent) was added to a $CH_2Cl_2$ (0.61 mL, 0.050 M) solution of Compound 11 (23.8 mg, 30.3 μmol). The mixture was stirred at room temperature for 2 hours and 10 minutes and then concentrated under a reduced pressure. The obtained crude product was used in the next reaction without purification.

A mixed solution containing TBAF (1M THF solution, 90.9 μL, 90.9 μmol, 3.0 equivalent)/AcOH (5.2 μL, 90.9 μmol, 3.0 equivalent) was added to a THF (0.34 mL, 0.050 M) solution of the crude product of Compound 15 under ice cooling. The mixture was stirred at room temperature for 3 hours and water (10 mL) was then added. Extraction with $CH_2Cl_2$ (20 mL×3) was performed, the organic phase was mixed, and the mixture was then washed with a saturated saline, dried with $Na_2SO_4$ and concentrated under a reduced pressure. The crude residue was purified through an HPLC system, and Compound 16 (9.01 mg, 10.6 μmol, 2-stage yield 35%) was obtained as a white solid.
$^1H$ NMR (400 MHz, DMSO-$d_6$, δ): 1.06 (3H, d, J = 6.9 Hz), 1.83-1.98 (4H, m), 2.11-2.25 (7H, m), 2.63 (1H, d, J = 17.4 Hz), 2.78-3.15 (3H, m), 3.25-3.48 (2H, m), 3.55 (3H, s), 3.88 (1H, s), 4.09-4.68 (8H, m), 5.09 (1H, s), 5.71 (1H, d, J = 1.6 Hz), 5.94 (1H, s), 6.03 (1H, s), 6.40 (1H, br), 6.95 (1H, br), 7.37-7.58 (6H, m); $^{13}C$ NMR (100 MHz, DMSO-$d_6$, δ): 9.4, 15.1, 24.1, 40.8, 44.1, 49.5, 52.5, 54.2, 54.5, 55.8, 58.1, 59.6, 61.8, 100.9, 112.0, 113.0, 117.8, 119.9, 123.6, 125.6, 127.7, 128.3, 128.5, 130.2, 131.2, 138.1, 143.1, 143.8, 146.6, 146.9, 151.5, 171.0; HRMS (ESI, m/z): $[M+H]^+$ calcd. for $C_{44}H_{46}N_8O_{10}$, 847.3410; found, 847.3448.

[Synthesis of Compound 17]

[C62]

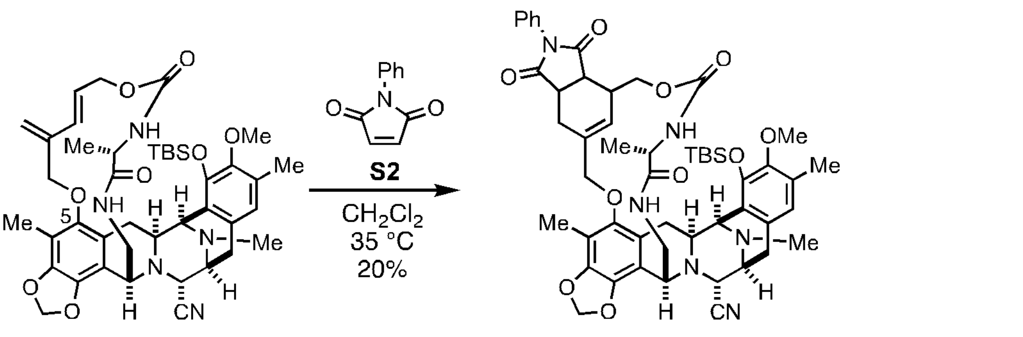


11 17

N-phenylmaleimide (S2, 51.5 mg, 37.9 μmol, 5.0 equivalent) was added to a $CH_2Cl_2$ (3.0 mL, 0.010 M) solution of Compound 11 (21.3 mg, 29.8 μmol). The mixture was stirred at room temperature for 20 hours and then stirred at 35 °C for 15 hours. After concentration under a reduced pressure, the crude product was passed through STRATA (registered trademark) C18 and eluted with MeCN. After concentration, the residue was purified using an HPLC system, and two diastereomers A (2.11 mg, 2.37 μmol, yield 8%) and B (3.19 mg, 3.59 μmol, 12%) of 17 were obtained as white solids. The stereochemistry of three asymmetric carbon atoms generated in this reaction was not determined.

A: $^1H$ NMR (400 MHz, DMSO-$d_6$, δ): 0.05 (3H, s), 0.46 (3H, s), 0.85-0.92 (1H, m), 1.03-1.18 (16H, m), 1.25 (3H, s), 2.06-2.23 (16H, m), 2.63-2.88 (4H, m), 3.42-3.63 (2H, m), 3.78 (4H, s), 4.14-4.25 (2H, m), 4.34-4.43 (2H, m), 5.40 (1H, s), 5.70-5.71 (2H, m), 6.02 (2H, m ), 6.58 (1H, s), 7.08-7.17 (2H, m), 7.36-7.55 (4H, m); HRMS (ESI, m/z): $[M+H]^+$ calcd. for $C_{52}H_{63}N_6O_{10}Si$, 959.4369; found, 959.4417.

B: $^1H$ NMR (400 MHz, DMSO-$d_6$, δ): 0.06 (3H, s), 0.27 (3H, s), 1.00-1.11 (10H, m), 1.15 (3H, d, J = 6.9 Hz), 2.03-2.29 (11H, m), 2.72-2.98 (4H, m), 3.26-3.38 (2H, m), 3.46 (3H, s), 3.80 (1H, s), 4.07 (1H, s), 4.20-4.32 (1H, m), 4.43 (1H, br), 4.57 (1H, br), 5.43 (1H, br), 5.64 (1H, br), 5.96 (1H, s), 6.03 (1H, s), 6.49 (1H, br), 7.09 (3H, d, J = 7.3 Hz), 7.37-7.56 (3H,m), 7.58-7.77 (1H, m); HRMS (ESI, m/z): $[M+H]^+$ calcd. for $C_{52}H_{63}N_6O_{10}Si$, 959.4369; found, 959.4417.

[Synthesis of Compound 18]

[C63]

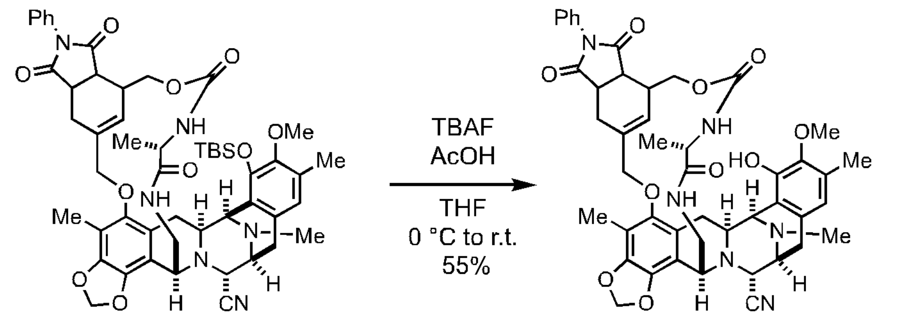


17 18

TBAF (1M THF solution, 6.6 μL, 6.6 μmol, 3.0 equivalent) was added to a THF (0.44 mL, 0.0050 M) solution of Compound 17A (2.11 mg, 2.20 μmol) and the mixture was stirred at room temperature for 2 hours and concentrated under a reduced pressure, and water (10 mL) was then added. Extraction with $CH_2Cl_2$ (10 mL×3) was performed, the organic phase was mixed, and the mixture was then washed with a saturated saline, dried with $Na_2SO_4$ and concentrated under a reduced pressure. The crude product was passed through STRATA (registered trademark) C18 and eluted with MeCN. After concentration, the residue was purified using an HPLC system, and Compound 18 (1.02 mg, 1.21 μmol, yield 55%) was obtained as a yellow oily substance.

$^1H$ NMR (400 MHz, $CDCl_3$, δ): 1.14 (3H, d, J = 6.9 Hz), 1.36 (1H, m), 2.06 (3H, s), 2.12-2.28(6H, m), 2.35 (1H, br), 2.62-3.10 (7H, m), 3.11-3.24 (4H, m), 3.25-3.64 (4H, m), 3.66-3.96 (8H, m), 4.16 (1H, s), 4.39 (1H, d, J = 1.8 Hz), 4.48

(1H, d, J = 11.4 Hz), 5.28 (1H, s), 5.71 (1H, d, J = 1.4 Hz), 5.97 (1H, s), 6.02 (1H, s), 6.43 (1H, s), 6.68 (1H, br), 7.13-7.19 (2H, m), 7.35-7.52 (4H, m), 8.43 (1H, br); $^{13}C$ NMR (100 MHz, DMSO-$d_6$, δ): 9.3, 15.3, 18.7, 24.1, 25.5, 27.3, 40.9, 49.6, 53.9, 54.4, 55.1, 56.3, 58.1, 60.4, 100.9, 111.4, 113.1, 119.7, 126.5, 127.8, 127.9, 128.5, 132.1, 136.8, 138.2, 144.2, 146.5, 148.6, 154.0, 171.4, 176.6, 179.0; HRMS (ESI, m/z): $[M+H]^+$ calcd. for $C_{46}H_{49}N_6O_{10}$, 845.3505; found, 845.3553.

[Example 3]

3. Synthesis of Compounds 28 and 29 of present invention

According to the following scheme, Compound 29 was synthesized in 10 processes from the natural product Cyanosafracin B (Compound 1). First, $Boc_2O$ and $Ac_2O$ were sequentially reacted to obtain Compound 20 in which the terminal amino group and phenolic hydroxyl group were protected. Phenol Compound 21 was obtained by allowing a photocyclization reaction to progress with visible light emission. This was not isolated and reacted with propargyl bromide to obtain Compound 22. TFA was reacted to remove the Boc group, and the primary amine was protected with the Ns group to obtain Compound 24. Compound 26 was synthesized by methylating sulfonamide using iodomethane, reacting benzenethiol, and removing the Ns group. Compound 27 was synthesized by reacting formaldehyde and constructing a macrocycle in the presence of a monovalent copper catalyst. Compound 29 was synthesized by removing the acetyl group using potassium carbonate. Compound 26 was reacted with a copper catalyst in the presence of a ligand and Dimer Compound 28 was also synthesized. Reaction conditions for each step will be described in detail.

[C64]

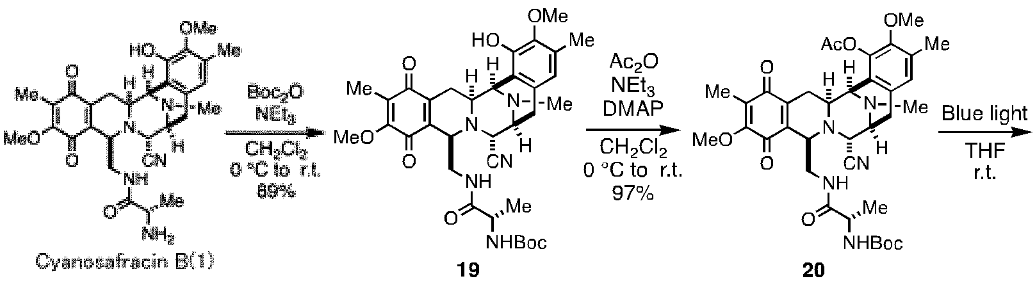


Cyanosafracin B(1)

19

20

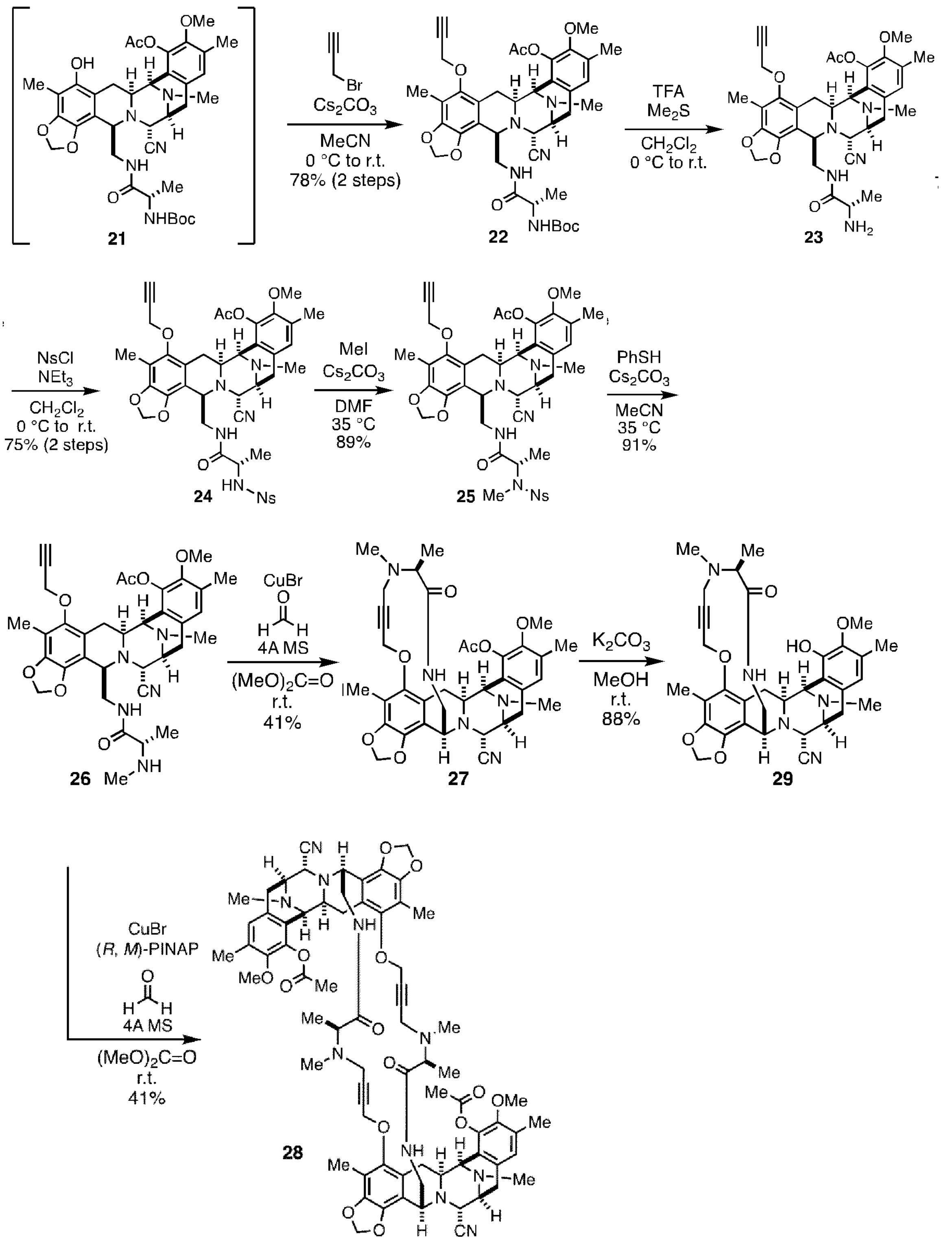


[Synthesis of Compound 19]

[C65]

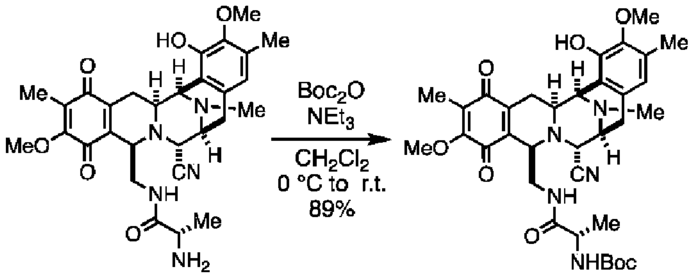


Cyanosafracin B(1) **19**

$Boc_2O$ (439 μL, 1.91 mmol, 1.05 equivalent), and $NEt_3$ (634 μL, 4.55 mmol, 2.5 equivalent) were added to a $CH_2Cl_2$ (18.2 mL, 0.10 M) solution of Compound 1 (1.00 g, 1.82 mmol) under ice cooling. The mixture was stirred at room temperature for 2 hours and then quenched with a saturated $NH_4Cl$ aqueous solution (10 mL). After the organic phase and the aqueous phase were separated, the aqueous phase was extracted with $CH_2Cl_2$ (30 mL×2). After the organic phase was mixed, the mixture was washed with a saturated saline, dried with $Na_2SO_4$ and concentrated under a reduced pressure. The crude residue was purified through silica gel column chromatography ($CH_2Cl_2$/AcOEt), and Compound 19 (1.06 g, 1.63 mmol, yield 89%) was obtained as a yellow solid.
$^1H$ NMR (400 MHz, $CDCl_3$, δ): 0.90 (3H, s), 1.33 (9H, s), 1.76 (1H, m), 1.86 (3H, s), 2.09 (1H, m), 2.22-2.33 (6H, m), 2.45 (1H, d, J = 18.3 Hz), 3.03-3.22 (5H, m), 3.37 (1H, d, J = 7.3 Hz), 3.68-3.80 (4H, m), 3.85 (1H, s), 3.97 (3H, s), 4.04 (1H, d, J = 2.3 Hz), 4.17 (1H, d, J = 2.3 Hz), 4.59 (1H, br), 5.24 (1H, br), 6.25 (1H, s), 6.51 (1H, s).

[Synthesis of Compound 20]

[C66]

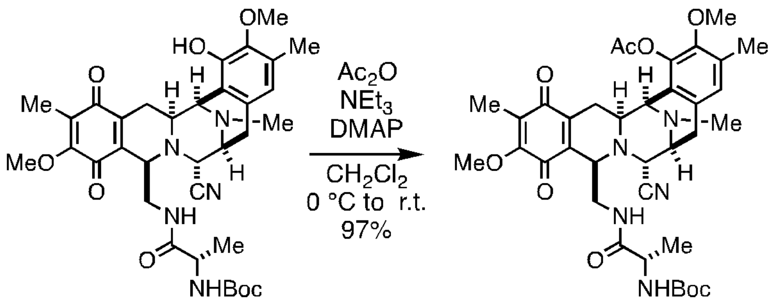


**19** **20**

$NEt_3$ (676 μL, 4.85 mmol, 3.0 equivalent), and $Ac_2O$ (229 μL, 2.42 mmol, 1.5 equivalent) were added to a $CH_2Cl_2$ (16.2 mL, 0.10 M) solution of Compound 19 (1.05 g, 1.62 mmol) under ice cooling. The mixture was stirred at room temperature for 2.5 hours. Under ice cooling, diluting with $CH_2Cl_2$ (10 mL) was performed and quenching was then performed with a saturated $NH_4Cl$ aqueous solution (10 mL). After the organic phase and the aqueous phase were separated, the aqueous phase was extracted with $CH_2Cl_2$ (20 mL×2). After the organic phase was mixed, the mixture was washed with a saturated saline, dried with $Na_2SO_4$ and concentrated under a reduced pressure. The crude residue was purified through silica gel column chromatography (hexane/AcOEt), and Compound 20 (1.09 g, 1.57 mmol, yield 97%) was obtained as a yellow solid.
$^1H$ NMR (500 MHz, $CDCl_3$, δ): 0.90 (3H, d, J = 4.1 Hz), 1.32 (9H, s ), 1.75 (1H, m), 1.87 (3H, s), 2.21-2.34 (6H, m ), 2.41 (3H, s), 2.52 (1H, d, J = 18.3 Hz), 2.91 (1H, m), 3.07-3.17 (4H, m), 3.38-3.40 (1H, m), 3.63-3.78 (5H, m ), 3.84 (1H, s), 3.99 (3H, s), 4.05 (1H, J = 2.3 Hz), 4.51 (1H, br), 5.49 (1H, br), 6.88 (1H, s); HRMS (ESI, m/z): $[M+Na]^+$ calcd. for $C_{36}H_{45}N_5O_9Na$, 714.3109; found, 714.3118.

[Synthesis of Compound 22]

[C67]

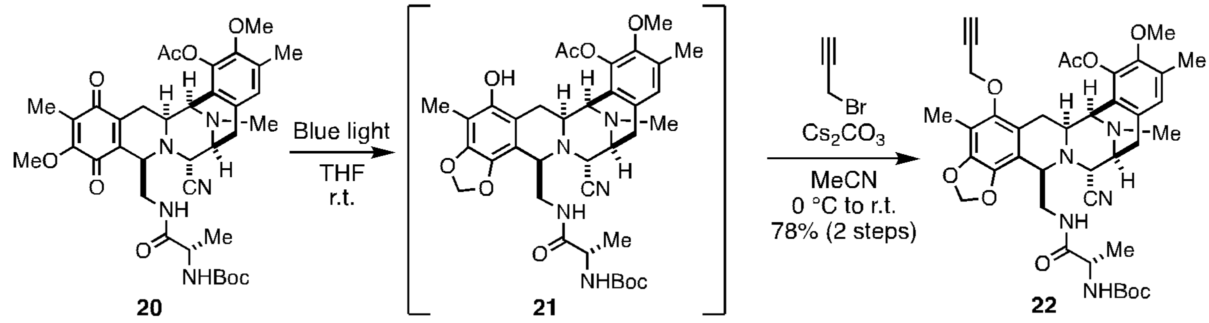

20 21 22

A THF (20.6 mL, 0.050 M) solution of a freeze-deaerated Compound 20 (714 mg, 1.03 mmol) was stirred at room temperature for 1 hours 25 minutes while emitting blue light. The reaction solution was concentrated under a reduced pressure, and the crude residue was used in the next reaction without purification.

$Cs_2CO_3$ (673mg, 2.06 mmol, 2.0 equivalent), and propargyl bromide (117 μL, 1.55 mmol, 1.5 equivalent) were added to a MeCN (10.3 mL) solution of the crude product of Compound 21 under ice cooling. The mixture was stirred at room temperature for 10 hours and 30 minutes. After filtering using celite and washing with $CH_2Cl_2$, the filtrate was concentrated under a reduced pressure. The crude residue was purified twice through silica gel column chromatography ($CH_2Cl_2$ /AcOEt), ($CH_2Cl_2$ /acetone), and Compound 22 (590 mg, 809 μmol, 2-stage yield 78%) was obtained as a brown oily substance.

$^1H$ NMR (400 MHz, $CDCl_3$, δ): 0.88 (3H, d, J = 7.3 Hz), 1.32 (9H, s), 1.95 (1H, dd, J = 15.3, 11.7 Hz), 2.14 (3H, s), 2.25 (7H, m), 2.44 (3H, s), 2.54 (1H, t, J = 2.4 Hz), 2.68 (1H, d, J = 17.9 Hz), 3.03 (1H, q, J = 8.7 Hz), 3.12-3.21 (2H, m), 3.37-3.51 (4H, m), 3.72 (5H, m), 4.00 (1H, br), 4.08 (1H, d, J = 2.3 Hz), 4.33 (1H, dd, J= 15.1, 2.4 Hz), 4.48 (1H, dd, J= 15.1, 2.4 Hz), 4.88 (1H, br), 5.48 (1H, br), 5.86 (1H, d, J = 1.4 Hz), 5.97 (1H, d, J = 1.4 Hz), 6.85 (1H, s); HRMS (ESI, m/z): $[M+H]^+$ calcd. for $C_{39}H_{48}N_5O_9$, 730.3447; found, 730.3456.

[Synthesis of Compound 24]

[C68]

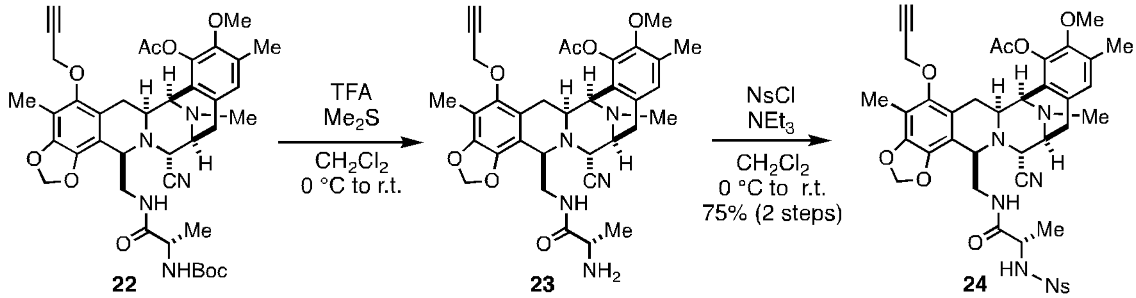

22 23 24

$Me_2S$ (2.36 mL, 31.8 mmol, 30 equivalent) and TFA (1.63 mL, 21.2 mmol, 20 equivalent) were added to a $CH_2Cl_2$ (10.6 mL, 0.10 M) solution of Compound 22 (755 mg, 1.06 mmol) under ice cooling, and the mixture was stirred at room temperature for 18 hours and 20 minutes. Then, after diluting with $CH_2Cl_2$ (20 mL), quenching was performed with a saturated $NaHCO_3$ aqueous solution(20 mL) under ice cooling. After the organic phase and the aqueous phase were separated, the aqueous phase was extracted with $CH_2Cl_2$ (20 mL×2). After the organic phase was mixed, the mixture was washed with a saturated saline, dried with $Na_2SO_4$ and concentrated under a reduced pressure. The obtained crude residue was used in the next reaction without purification.

$NEt_3$ (400 μL, 2.87 mmol, 3.0 equivalent), and 2-nitrobenzenesulfonyl chloride (276 mg, 1.25 mmol, 1.3 equivalent) were added to a $CH_2Cl_2$ (9.57 mL) solution of the crude product of Compound 23 under ice cooling. The mixture was stirred at room temperature for 10 hours. After diluting with $CH_2Cl_2$ (20 mL), quenching was performed with a saturated $NH_4Cl$ aqueous solution (20 mL) under ice cooling, the organic phase and the aqueous phase were separated, and the aqueous phase was then extracted with $CH_2Cl_2$ (30 mL×2).After the organic phase was mixed, the mixture was

washed with a saturated saline, dried with $Na_2SO_4$ and concentrated under a reduced pressure. The crude residue was purified through silica gel column chromatography (hexane/AcOEt), and Compound 24 (647 mg, 0.794 mmol, 2-stage yield 75%) was obtained as a light yellow oily substance.

$^1$H NMR (400 MHz, $CDCl_3$, δ): 0.94 (3H, d, J = 6.9 Hz), 2.00 (1H, dd, J = 15.3, 11.7 Hz), 2.16-2.30 (10H, m), 2.47 (3H, s), 2.52 (1H, t, J = 2.5 Hz), 2.70 (1H, d, J = 18.3 Hz), 2.99-3.39 (6H, m), 3.46-3.55 (2H, m), 3.71-3.82 (5H, m), 3.98 (1H, br), 4.06 (1H, d, J = 2.7 Hz), 4.27 (1H, dd, J = 15.3, 2.5 Hz), 4.55 (1H, dd, J = 15.3, 2.5 Hz), 5.74-5.80 (2H, m), 5.88 (1H, d, J = 1.4 Hz), 5.99 (1H, d, J = 1.4 Hz), 6.87 (1H, s), 7.63-7.84 (5H, m); [M+H] $^+$ calcd. for $C_{40}H_{43}N_6O_{11}S$, 815.2705; found, 815.2732.

[Synthesis of Compound 25]

[C69]

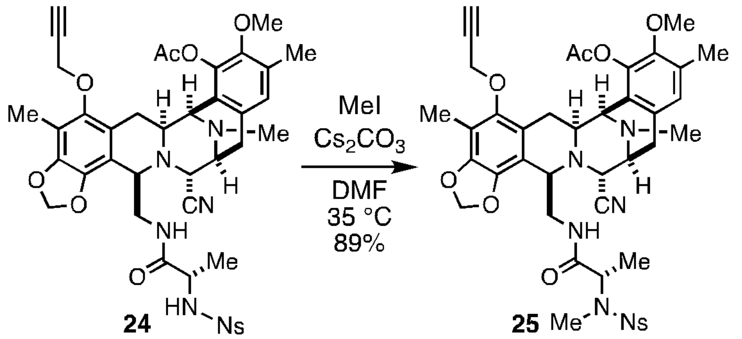


24 25

$K_2CO_3$ (187 mg, 1.35 mmol, 3.0 equivalent), and iodomethane (36.5 μL, 587 μmol, 1.3 equivalent) were added to a DMF (4.51 mL, 0.10 M) solution of Compound 24 (368 mg, 451 μmol) under ice cooling. The mixture was stirred at room temperature for 18 hours and 30 minutes. After diluting with $CH_2Cl_2$ (20 mL), quenching was performed with a saturated $NH_4Cl$ aqueous solution (20 mL) and water (20 mL) under ice cooling, the organic phase and the aqueous phase were separated, and the aqueous layer was then extracted with $CH_2Cl_2$ (30 mL×2).After the organic phase was mixed, the mixture was washed with a saturated saline, dried with $Na_2SO_4$ and concentrated under a reduced pressure. The crude residue was purified through silica gel column chromatography (hexane/$CHCl_3$/acetone), and Compound 25 (334 mg, 0.403 mmol, yield 89%) was obtained as a light yellow oily substance.

$^1$H NMR (400 MHz, $CDCl_3$, δ): 1.08 (3H, d, J = 6.9 Hz), 1.82 (1H, dd, J = 15.1, 11.9 Hz), 2.16-2.23 (9H, m), 2.47 (3H, s), 2.53 (1H, t, J = 2.5 Hz), 2.75 (1H, d, J = 17.9 Hz), 2.92-3.03 (2H, m), 3.15 (1H, dt, J = 11.8, 2.6 Hz), 3.32-3.38 (2H, m), 3.70-3.79 (6H, m), 3.96 (1H, br), 4.18-4.25 (2H, m), 4.54 (1H, dd, J = 15.3, 2.5 Hz), 5.88 (1H, d, J = 1.4 Hz), 6.00 (1H, d, J = 1.4 Hz), 6.16 (1H, q, J = 4.1 Hz), 6.81 (1H, s), 7.53 (1H, dd, J = 7.7, 1.5 Hz), 7.69-7.78 (2H, m), 7.84 (1H, dd, J = 7.7, 1.5 Hz); HRMS (ESI, m/z): [M+H] $^+$ calcd. for $C_{41}H_{45}N_6O_{11}S$, 829.2862; found, 829.2886.

[Synthesis of Compound 26]

[C70]

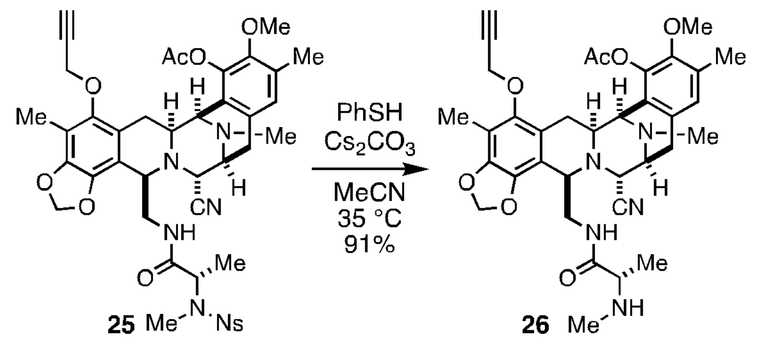


25 26

$Cs_2CO_3$ (104 mg, 321 μmol, 1.5 equivalent), and benzenethiol (26.2 μL, 256 μmol, 1.2 equivalent) were added to a MeCN (2.13 mL, 0.10 M) solution of Compound 25 (177 mg, 214 μmol) under ice cooling. The mixture was stirred at 30°C for 14 hours. Benzenethiol (6.54 μL, 64.1 μmol, 0.30 equivalent) was added and the mixture was additionally stirred at 30°C for 9 hours and 30 minutes. After filtration by a filter, 1 M hydrochloric acid (300 μL) was added, the mixture was concentrated under a reduced pressure, and MeCN was removed. Washing with $CH_2Cl_2$ (30 mL×3) was performed and a saturated $NaHCO_3$ aqueous solution was added to the aqueous layer under ice cooling until the pH reached 7 to 8. Extraction with $CHCl_3$ (30 mL×3) was performed. After the organic phase was mixed, the mixture was dried with $Na_2SO_4$ and concentrated under a reduced pressure to obtain Compound 26 (125 mg, 0.194 mmol, yield 91%) as a colorless oily substance.

$^1H$ NMR (400 MHz, $CDCl_3$, δ): 0.96 (3H, d, J = 6.9 Hz), 1.85 (3H, s), 1.94 (1H, dd, J = 15.3, 11.7 Hz), 2.15 (3H, s,), 2.27 (6H, m), 2.45- 2.51 (4H, m), 2.54 (1H, t, J = 2.5 Hz), 2.75 (1H, d, J = 18.3 Hz), 2.97-3.09 (2H, m), 3.20 (1H, dt, J = 11.4, 2.7 Hz), 3.39 (1H, d, J = 7.8 Hz), 3.52-3.58 (2H, m), 3.70-3.75 (4H, m), 4.00 (1H, br), 4.15 (1H, d, J = 2.3 Hz), 4.26 (1H, dd, J = 15.3, 2.5 Hz), 4.44 (1H, dd, J = 15.3, 2.5 Hz), 5.89 (1H, d, J = 1.4 Hz), 5.97 (1H, d, J = 1.4 Hz), 6.67 (1H, t, J = 6.0 Hz), 6.86 (1H, s); $[M+H]^+$ calcd. for $C_{35}H_{42}N_5O_7$, 644.3079; found, 644.3116.

[Synthesis of Compound 27]

[C71]

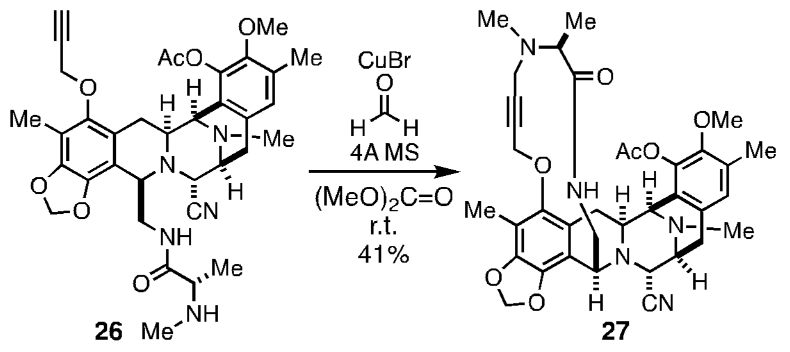


26 27

In the copresence of an activated molecular sieve 4A (722 mg), a dimethyl dicarbonate (4.00 mL) solution of Compound 26 (46.6 mg, 72.4 μmol) and copper bromide (1.04 mg, 7.24 μmol, 0.10 equivalent) were added to a dimethyl dicarbonate (3.24 mL) solution of formaldehyde (21.7 mg, 724 μmol, 10 equivalent). The mixture was stirred at room temperature for 31 hours. After filtration and concentration under a reduced pressure, THF (10 mL), and SiliaMetS (registered trademark) Triamine (22.8 mg) were added, and the mixture was stirred for 18 hours and 30 minutes. After filtration and concentration under a reduced pressure, the crude residue was purified through silica gel column chromatography (hexane/$CHCl_3$/acetone) and Compound 27 (19.5 mg, 29.8 μmol, yield 41%) was obtained as a colorless oily substance.

$^1H$ NMR (400 MHz, $CDCl_3$, δ): 1.01 (3H, d, J = 6.9 Hz), 1.98 (3H, s), 2.14-2.29 (12H, m), 2.37 (4H, m), 2.91 (2H, m),

3.01 (1H, dd, J = 16.5, 2.7 Hz), 3.12-3.16 (2H, m), 3.39 (1H, m), 3.68 (1H, d, J = 2.7 Hz), 3.76 (3H, s), 4.01-4.08 (2H, m), 4.21 (1H, d, J = 2.3 Hz), 4.48 (1H, d, J = 16.0 Hz), 4.70 (1H, d, J = 16.0 Hz), 5.87 (1H, d, J = 1.4 Hz), 6.02 (1H, d, J = 1.4 Hz), 6.65 (1H, d, J = 8.2 Hz), 6.85 (1H, s); HRMS (ESI, m/z): $[M+H]^+$ calcd. for $C_{36}H_{42}N_5O_7$, 656.3079; found, 656.3114.

[Synthesis of Compound 28]

[C72]

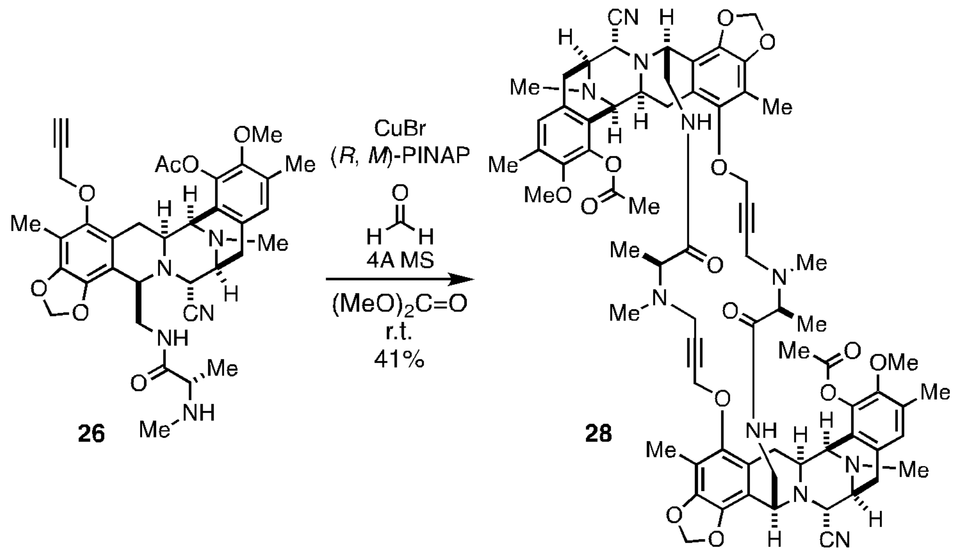


26

28

In the copresence of an activated molecular sieve 4A (311 mg), (R,M)-PINAP (1.04 mg, 1.86 μmol, 0.12 equivalent) was added to a dimethyl dicarbonate (2.6 mL) solution of copper bromide (0.22 mg, 1.55 μmol, 0.10 equivalent), and the mixture was stirred at room temperature for 1 hour and 15 minutes. A dimethyl dicarbonate (0.50 mL) solution of Compound 26 (10.0 mg, 15.5 μmol) and formaldehyde (4.66 mg, 155 μmol, 10 equivalent) were added to the obtained mixture. The mixture was stirred at room temperature for 38 hours. After filtration, SiliaBond (registered trademark) Triamine (9.79 mg) was added and the mixture was stirred for 30 minutes. After filtration and concentration under a reduced pressure, the crude product was passed through Discovery (registered trademark) C18 and eluted with MeCN. After concentration, the residue was purified through an HPLC system, and Compound 28 (1.58 mg, 1.2 μmol, yield 7.8%) was obtained as a colorless oily substance. HRMS (ESI, m/z): $[M+2H]^{2+}$ calcd. for $C_{36}H_{42}N_5O_7$, 656.3079; found, 656.3078.

[Synthesis of Compound 29]

[C73]

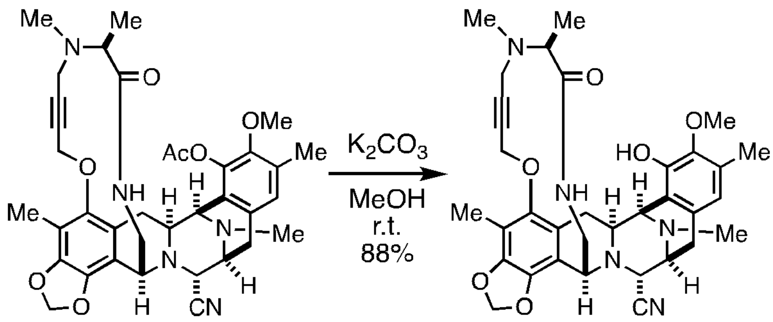


27 29

$K_2CO_3$ (10.4 mg, 75.5 μmol, 5.0 equivalent) was added to a mixed solution containing MeOH (0.48 mL) of Compound 27 (9.90 mg, 15.1 μmol) and water (0.12 mL) under ice cooling, and the mixture was stirred at room temperature for 6 hours and 30 minutes. After diluting with $CH_2Cl_2$ (5.0 mL) and water (5.0 mL), 1 M hydrochloric acid (400 μL) was added and quenching was performed under ice cooling. After the organic phase and the aqueous phase were separated, the aqueous layer was extracted with $CHCl_3$ (20 mL×3). After the organic phase was mixed, the mixture was dried with $Na_2SO_4$ and concentrated under a reduced pressure. The crude product was passed through Discovery (registered trademark) C18 and eluted with MeCN. After concentration, the residue was purified through an HPLC system, and Compound 29 (8.12 mg, 13.2 μmol, yield 88%) was obtained as a colorless oily substance.
$^1H$ NMR (400 MHz, $CDCl_3$, δ): 1.00 (3H, d, J = 6.9 Hz), 1.96 (3H, s), 2.10-2.36 (13H, m), 2.78-2.92 (2H, m), 3.10-3.20 (3H, m), 3.36 (1H, d, J = 7.8 Hz), 3.79 (3H, s), 4.01-4.12 (3H, m), 4.19 (1H, d, J = 2.7 Hz), 4.41 (1H, d, J = 16.0 Hz), 4.66 (1H, d, J = 16.0 Hz), 5.79 (1H, s), 5.85 (1H, d, J = 1.4 Hz), 6.01 (1H, d, J = 1.4 Hz), 6.48 (1H, s), 6.65 (1H, d, J = 8.5 Hz); HRMS (ESI, m/z): $[M+2H]^{2+}$ calcd. for $C_{17}H_{20.5}N_{2.5}O_3$, 307.6523; found, 307.6548.

[Example 4]

4. Synthesis of Compounds 33 and 35 of present invention

According to the following scheme, from the natural product Cyanosafracin B (Compound 1), Compound 33 was synthesized in 10 processes, and Compound 35 was synthesized in 11 processes. Compound 24 was obtained in the same procedure as in Example 3. Compound 30 was synthesized by allylating sulfonamide using allyl bromide. Compound 31 was synthesized by constructing a macrocycle according to a ring-closing enyne metathesis reaction using a first generation Grubbs catalyst. Compound 32 was obtained by reacting benzenethiol and removing the Ns group. Compound 33 was synthesized by removing the acetyl group using potassium carbonate. Compound 32 was reacted with propargyl bromide to obtain Compound 34. Compound 35 was synthesized by removing the acetyl group using potassium carbonate. Reaction conditions for each step will be described in detail.

[C74]

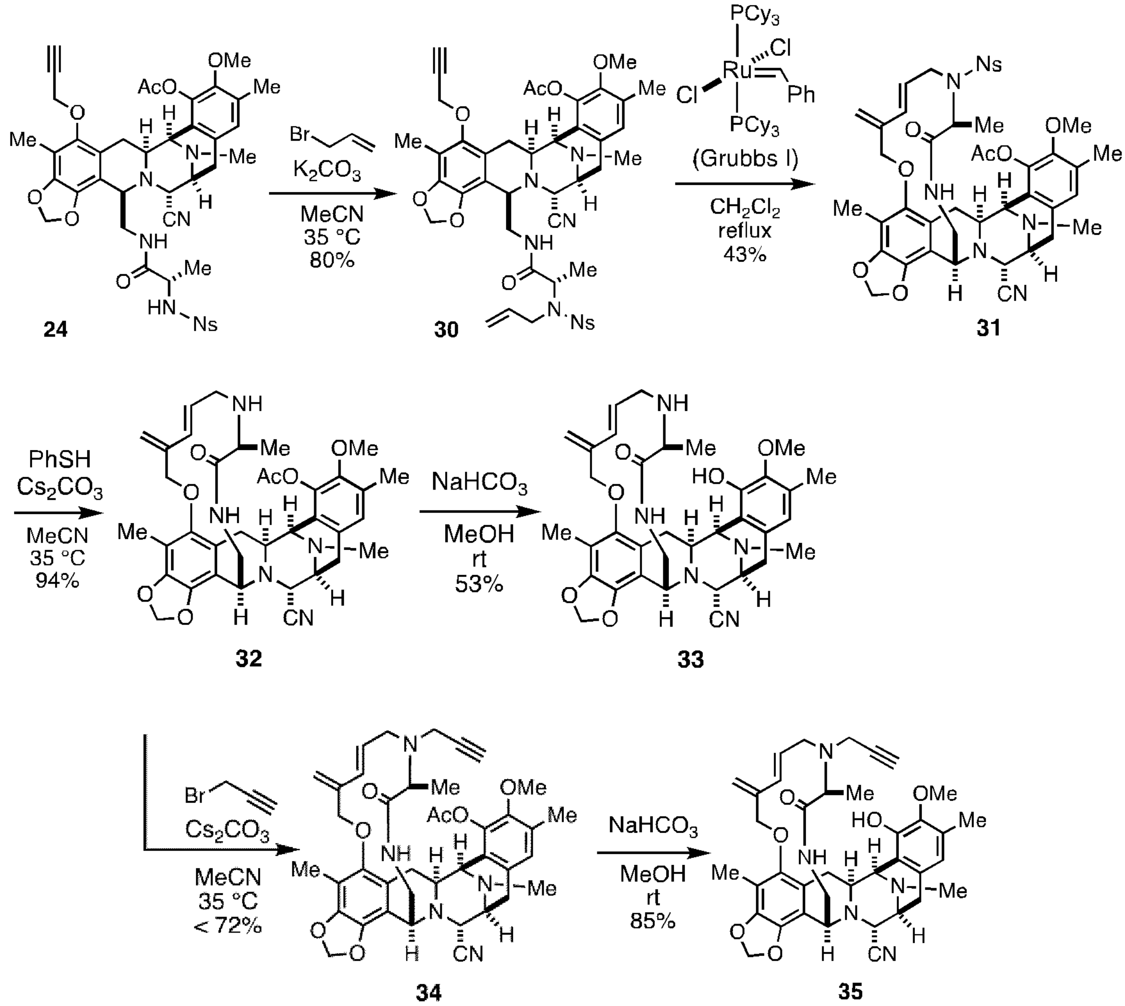


[Synthesis of Compound 30]

[C75]

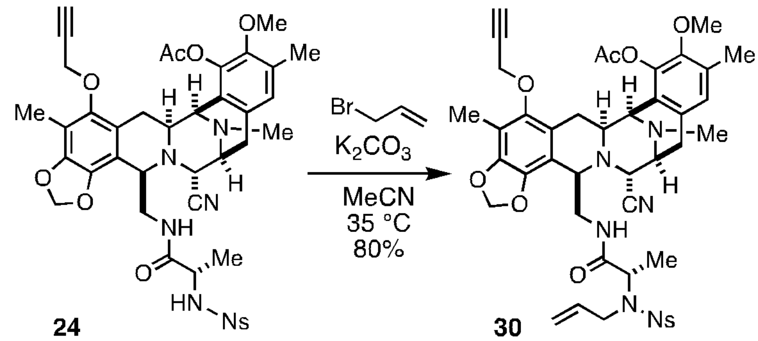


$K_2CO_3$ (148 mg, 1.07 mmol, 3.0 equivalent), and allyl bromide (45.1 μL, 537 μmol, 1.5 equivalent) were added to a DMF (3.58 mL, 0.10 M) solution of Compound 24 (292 mg, 358 μmol) under ice cooling. The mixture was stirred at room temperature for 76 hours. After diluting with $CH_2Cl_2$ (10 mL), quenching was performed with a saturated $NH_4Cl$

aqueous solution (20 mL) and water (20 mL) under ice cooling, the organic phase and the aqueous phase were separated, and the aqueous layer was then extracted with $CH_2Cl_2$ (30 mL×2).After the organic phase was mixed, the mixture was washed with a saturated saline, dried with $Na_2SO_4$ and concentrated under a reduced pressure. The crude residue was purified through silica gel column chromatography (hexane/$CHCl_3$/acetone), and Compound 30 (244 mg, 0.285 mmol, yield 80%) was obtained as a light yellow oily substance. $^1$H NMR (400 MHz, $CDCl_3$, δ): 1.13 (3H, d, J = 6.9 Hz), 1.83 (2H, m), 2.15- 2.24 (10H, m), 2.45 (3H, s), 2.53 (1H, t, J = 2.3 Hz), 2.71 (1H, d, J = 17.9 Hz), 2.94- 3.04 (2H, m), 3.15 (1H, m), 3.31-3.37 (2H, m), 3.62-3.76 (7H, m), 3.79-3.86 (2H, m), 3.97 (1H, br), 4.14 (1H, d, J = 2.3 Hz), 4.22 (1H, dd, J = 15.3, 2.3 Hz), 4.51 (1H, dd, J = 15.6, 2.3 Hz), 4.98-5.08 (2H, m), 5.42-5.52 (1H, m), 5.86 (1H, d, J = 1.4 Hz), 5.97 (1H, d, J = 1.4 Hz), 6.15 (1H, m), 6.79 (1H, s), 7.52 (1H, m), 7.67-7.74 (2H, m), 7.82 (1H, m); HRMS (ESI, m/z): $[M+H]^+$ calcd. for $C_{43}H_{47}N_6O_{11}S$, 855.3018; found, 855.3032.

[Synthesis of Compound 31]

[C76]

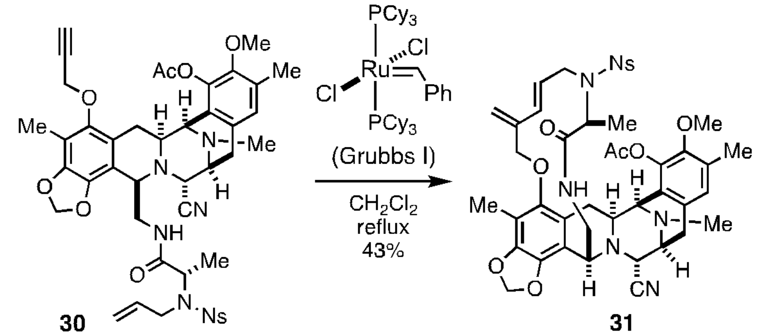


30

31

A $CH_2Cl_2$ (26.4mL) solution of a Grubbs first generation catalyst (70.3 mg, 85.5 μmol, 0.30 equivalent) was heated to reflux, and a $CH_2Cl_2$ (2.00 mL) solution of Compound 30 (244 mg, 285 μmol) was added. The mixture was heated to reflux for 23 hours. The reaction solution was concentrated under a reduced pressure and 1,2-dimethoxyethane (2.85 mL) and SiliaMetS (registered trademark) Thiourea (565 mg) were then added and the mixture was stirred for 10 hours and 30 minutes. After filtration and concentration under a reduced pressure, the residue was purified through silica gel column chromatography (hexane/$CHCl_3$/acetone), and Compound 31 (87.0 mg, 102 μmol, yield 36%) was obtained as a brown solid.

$^1$H NMR (400 MHz, $CDCl_3$, δ): 1.04 (3H, d, J = 6.9 Hz), 2.08 (6H, m), 2.15-2.25 (4H, m), 2.30-2.37 (1H, m), 2.43 (3H, s), 2.56 (1H, d, J = 17.9 Hz), 2.83 (1H, m), 3.02-3.11 (3H, m), 3.20 (1H, dt, J= 11.6, 3.0 Hz), 3.45 (1H, d, J = 9.2 Hz), 3.53-3.60 (1H, m), 3.71-3.76 (5H, m), 4.03-4.13 (3H, m), 4.44 (1H, m), 4.58 (1H, d, J = 13.3 Hz), 4.88 (1H, d, J = 13.3 Hz), 4.94-5.01 (1H, m), 5.22 (1H, s), 5.54 (1H, s), 5.85-6.00 (4H, m), 6.85 (1H, s), 7.54-7.70 (4H, m), 7.78 (1H, m); HRMS (ESI, m/z): $[M+H]^+$ calcd. for $C_{43}H_{47}N_6O_{11}S$, 855.3018; found, 855.3040.

[Synthesis of Compound 32]

[C77]

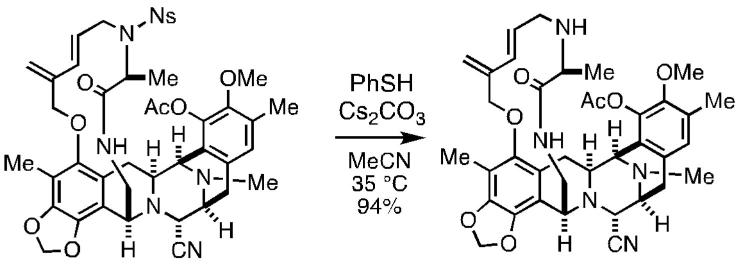


31 32

$Cs_2CO_3$ (35.4 mg, 109 μmol, 3.0 equivalent), and benzenethiol (9.25 μL, 90.7 μmol, 2.5 equivalent) were added to a MeCN (0.72 mL, 0.050 M) solution of Compound 31 (31.0 mg, 36.3 μmol) under ice cooling. The mixture was stirred at 35°C for 15 hours. After diluting with $CH_2Cl_2$ (5.0 mL), 1 M hydrochloric acid (4.0 mL) was added and quenching was performed under ice cooling. Washing with $Et_2O$ (20 mL×2) was performed and a saturated $NaHCO_3$ aqueous solution was added to the aqueous layer under ice cooling until the pH reached 7 to 8.
Extraction with $CHCl_3$ (30 mL×3) was performed, the organic phase was mixed, and the mixture was then dried with $Na_2SO_4$. Concentration was performed under a reduced pressure, and Compound 32 (22.8 mg, 34.1 μmol, yield 94%) was obtained as a colorless oily substance.
$^1$H NMR (400 MHz, $CDCl_3$, δ): 1.10 (3H, d, J = 6.9 Hz), 1.85 (1H, dd, J = 15.6, 11.9 Hz), 2.14-2.35 (12H, m), 2.45 (3H, s), 2.63-2.74 (2H, m), 2.86 (1H, m), 3.05 (2H, m), 3.16 (1H, d, J = 11.9 Hz), 3.34-3.37 (2H, m), 3.57-3.76 (5H, m), 4.00 (2H, d, J = 13.7 Hz), 4.49 (1H, d, J = 11.9 Hz), 4.62 (1H, d, J = 12.4 Hz), 4.98 (1H, s), 5.15-5.21 (1H, m), 5.34 (1H, s), 5.81-5.96 (3H, m), 6.09 (1H, m), 6.88 (1H, s); HRMS (ESI, m/z): $[M+H]^+$ calcd. for $C_{37}H_{44}N_5O_7$, 670.3235; found, 670.3262.

[Synthesis of Compound 33]

[C78]

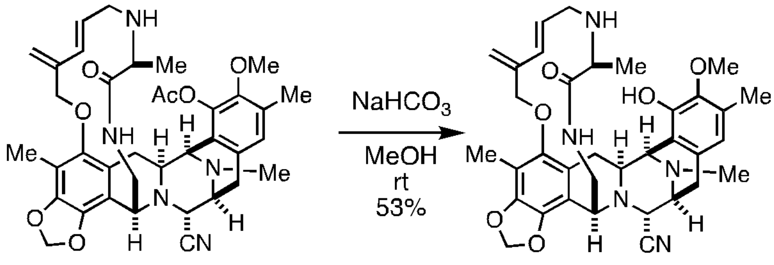


32 33

$K_2CO_3$ (23.6 mg, 171 μmol, 5.0 equivalent) was added to a mixed solution containing MeOH (0.54 mL) of Compound 32 (22.8 mg, 34.1 μmol) and water (0.14 mL) under ice cooling, and the mixture was stirred at room temperature for 5 hours. After diluting with $CH_2Cl_2$ (5.0 mL) and water (5.0 mL), 1 M hydrochloric acid (1.0 mL) was added under ice cooling, and quenching was performed. After the organic phase and the aqueous phase were separated, the aqueous layer was extracted with $CHCl_3$ (20 mL×3). After the organic phase was mixed, the mixture was dried with $Na_2SO_4$ and concentrated under a reduced pressure. The crude product was passed through Discovery (registered trademark) C18 and eluted with MeCN. After concentration, the residue was purified through an HPLC system, and Compound 33 (11.3 mg, 18.0 μmol, yield 53%) was obtained as a colorless oily substance. $^1$H NMR (400 MHz, $CDCl_3$, δ): 1.05 (3H, d, J = 6.9 Hz), 1.98-2.05 (1H, m), 2.14 (3H, s), 2.20 (1H, t, J = 2.3 Hz), 2.26-2.32 (6H, m), 2.58 (1H, d, J = 17.9 Hz), 2.65 (1H, m), 2.74-2.83 (2H, m), 2.93-3.03 (3H, m), 3.09-3.13 (2H, m), 3.36-3.43 (2H, m), 3.73 (1H, q, J = 6.9 Hz), 3.86 (3H, s), 4.00 (1H, d, J = 2.3 Hz), 4.05 (1H, br), 4.13 (1H, d, J = 3.2 Hz), 4.49 (1H, d, J = 11.9 Hz), 4.61 (1H, d, J = 11.9 Hz), 4.93-5.05 (3H, m), 5.84-5.88 (3H, m), 5.97 (1H, d, J = 1.4 Hz), 6.42 (1H, br), 6.55 (1H, s); HRMS (ESI, m/z): $[M+H]^+$ calcd. for $C_{35}H_{42}N_5O_6$, 628.3130; found, 628.3155.

[Synthesis of Compound 34]

[C79]

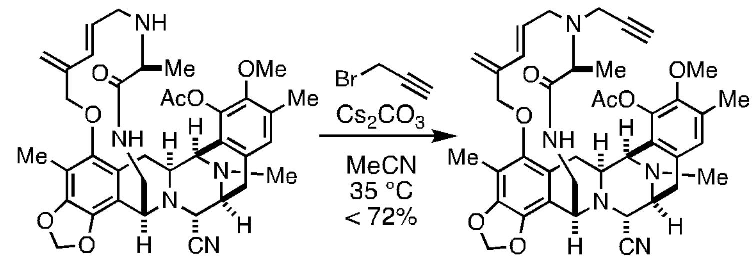


32 34

$Cs_2CO_3$ (25.1 mg, 77.0 μmol, 3.0 equivalent), and propargyl bromide (3.87 μL, 51.4 μmol, 2.0 equivalent) were added to a MeCN (0.51 mL) solution of Compound 32 (17.2 mg, 2.06 mmol) under ice cooling. The mixture was stirred at room temperature for 30 hours and 30 minutes. Propargyl bromide (3.87 μL, 51.4 μmol, 2.0 equivalent) was additionally added and the mixture was additionally stirred for 14 hours. 1 M hydrochloric acid (0.30 mL) was added and quenching was performed under ice cooling, and the aqueous layer was extracted with $CH_2Cl_2$ (30 mL×3). After the organic phase was mixed, the mixture was dried with $Na_2SO_4$ and concentrated under a reduced pressure. The crude residue was purified through silica gel column chromatography (hexane/$CHCl_3$/acetone), and Compound 34 (13.1 mg, 25.7 μmol, yield 72%) was obtained as a light yellow oily substance.
$^1$H NMR (400 MHz, $CDCl_3$, δ): 1.04 (3H, d, J = 6.9 Hz), 2.04 (1H, m), 2.15 (3H, s), 2.19 (1H, t, J = 2.3 Hz), 2.23 (3H, s), 2.34 (3H, s), 2.45 (3H, s), 2.61-2.67 (2H, m), 2.76-2.88 (2H, m), 2.94-3.10 (5H, m), 3.38-3.45 (2H, m), 3.63 (1H, d, J = 2.7 Hz), 3.70 (1H, m), 3.79 (3H, s), 4.00-4.05 (2H, m), 4.52 (1H, d, J = 11.9 Hz), 4.64 (1H, d, J = 11.9 Hz), 4.96-5.02 (2H, m), 5.09 (1H, s), 5.86 (1H, d, J = 1.4 Hz), 5.97-6.01 (2H, m), 6.47 (1H, t, J = 5.3 Hz), 6.93 (1H, s).

[Synthesis of Compound 35]

[C80]

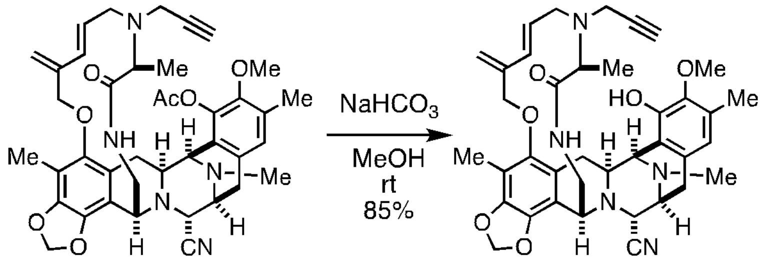


34 35

$K_2CO_3$ (8.03 mg, 58.1 μmol, 5.0 equivalent) was added to a mixed solution containing MeOH (0.46 mL) of Compound 34 (8.23 mg, 11.6 μmol) and water (0.12 mL) under ice cooling, and the mixture was stirred at room temperature for 4 hours and 30 minutes. After diluting with $CH_2Cl_2$ (5.0 mL) and water (5.0 mL), 1 M hydrochloric acid (300 μL) was added and quenching was performed under ice cooling. After the organic phase and the aqueous phase were separated, the aqueous layer was extracted with $CHCl_3$ (20 mL×3). After the organic phase was mixed, the mixture was dried with $Na_2SO_4$ and concentrated under a reduced pressure. The crude product was passed through Discovery (registered trademark) C18 and eluted with MeCN. After concentration, the residue was purified through an HPLC system, and Compound 35 (6.61 mg, 9.93 μmol, yield 85%) was obtained as a colorless oily substance.
$^1$H NMR (400 MHz, $CDCl_3$, δ): 1.00 (3H, d, J = 6.9 Hz), 1.96 (3H, s), 2.10-2.36 (13H, m), 2.78-2.92 (2H, m), 3.10-3.20

(3H, m), 3.36 (1H, d, J = 7.8 Hz), 3.79 (3H, s), 4.01-4.12 (3H, m), 4.19 (1H, d, J = 2.7 Hz), 4.41 (1H, d, J = 16.0 Hz), 4.66 (1H, d, J = 16.0 Hz), 5.79 (1H, s), 5.85 (1H, d, J = 1.4 Hz), 6.01 (1H, d, J = 1.4 Hz), 6.48 (1H, s), 6.65 (1H, d, J = 8.5 Hz); HRMS (ESI, m/z): $[M+2H]^{2+}$ calcd. for $C_{19}H_{21.5}N_{2.5}O_3$, 333.6680; found, 333.6687.

A THIQ compound containing a 14-, 15-, 16-, or 17-membered macrocyclic structure (compound of formula (I)), and also a THIQ dimer compound containing a 28-membered macrocyclic structure (compound of formula (IIIc)) were successfully synthesized efficiently in few processes (6 to 10 processes). These compounds were compounds in which the structure of the nucleic acid alkylation moiety of THIQ compounds having different macrocyclic structures (Yondelis) was maintained and a macrocycle was formed in a manner different from that of Yondelis. Yondelis was synthesized in 24 processes from Cyanosafracin B (Manzanares, I. et al. Org. Lett. 2000, 2, 2545.), and the macrocyclic structure was not limited to the 10-membered ring. It was verified that, according to the production method of the present invention, using Cyanosafracin B as a starting substance, various macrocyclic structures could be synthesized flexibly and efficiently with a greatly reduced number of processes.

[Example 5]

5. Synthesis of Compounds 36 and 37 of present invention

[Synthesis of Compound 36]

[C81]

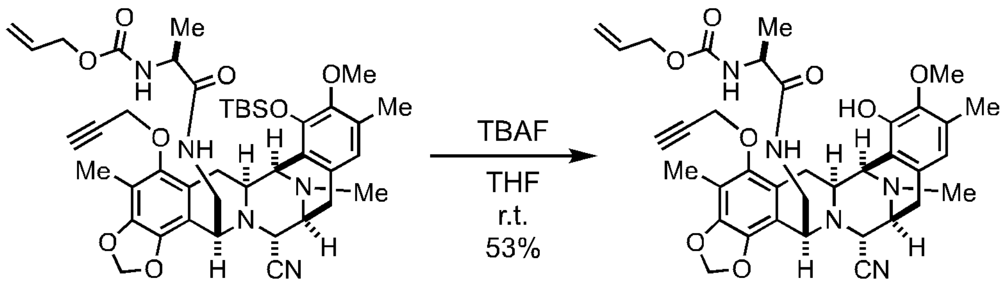


TBAF (1M THF solution, 131 μL, 131 μmol, 3.0 equivalent) was added to a THF (0.80 mL) solution of Compound 10 (34.4 mg, 43.8 μmol). The mixture was stirred at room temperature for 30 minutes and then concentrated under a reduced pressure. Ethyl acetate (10 mL) and a saturated ammonium chloride aqueous solution (30 mL) were added. Extraction with ethyl acetate (10 mL×3) was performed. After the organic phase was mixed, the mixture was dried with $Na_2SO_4$ and concentrated under a reduced pressure. The crude product was passed through STRATA (registered trademark) C18 and eluted with MeCN. After concentration, the residue was purified through an HPLC system, and Compound 36 (15.7 mg, 23.4 μmol, yield 53%) was obtained as a yellow oily substance.

$^1H$ NMR (400 MHz, $CDCl_3$, δ): 0.91 (3H, d, J = 6.9 Hz), 1.96 (1H, m), 2.14 (3H, s), 2.26 (3H, s), 2.31 (3H, s), 2.50 (1H, t, J = 2.3 Hz), 2.58 (1H, d, J = 17.9 Hz), 3.04 (1H, dd, J = 18.1, 8.0 Hz), 3.23-3.32 (2H, m), 3.37-3.44 (3H, m), 3.58-3.64 (1H, m), 3.77 (3H, s), 4.04 (2H, m), 4.16 (1H, d, J = 1.8 Hz), 4.41 (4H, m), 5.15-5.34 (4H, m), 5.77-5.87 (2H, m), 5.96-6.01 (2H, m), 6.48 (1H, s); $^{13}C$ NMR (100 MHz, DMSO-$d_6$, δ): 9.7, 15.9, 19.0, 25.4, 26.7, 40.7, 41.9, 50.1, 55.4, 56.6, 56.9, 59.5, 60.6, 60.9, 65.7, 75.5, 79.4, 101.5, 113.0, 117.3, 117.8, 117.9, 121.0, 121.5, 129.3, 131.0, 132.7, 139.5, 143.2, 144.7, 147.2, 148.0, 155.4, 171.8; HRMS (ESI, m/z): $[M+H]^+$ calcd. for $C_{35}H_{42}N_5O_6$, 628.3130; found, 628.3155.

[Synthesis of Compound 37]

[C82]

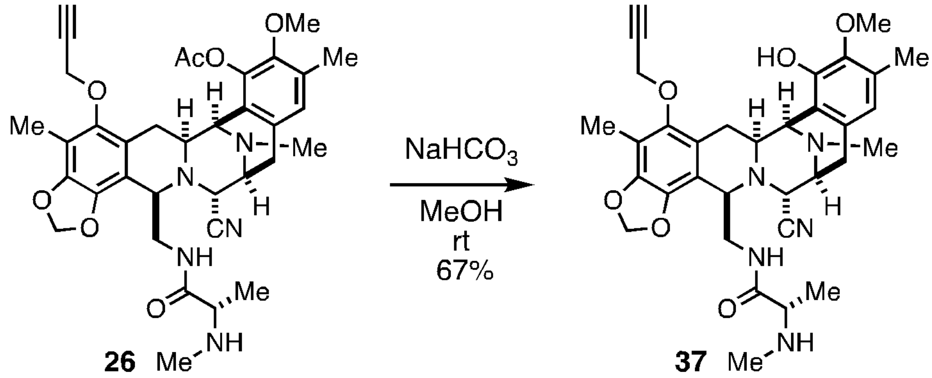


26

37

$K_2CO_3$ (38.3 mg, 277 μmol, 5.0 equivalent) was added to a mixed solution containing MeOH (0.89 mL) of Compound 26 (35.7 mg, 55.4 μmol) and water (0.22 mL), and the mixture was stirred at room temperature for 1 hour and 15 minutes. After filtration, diluting with $CH_2Cl_2$ (5.0 mL) and water (5.0 mL) was performed and 1 M hydrochloric acid (100 μL) was then added, and quenching was performed. After the organic phase and the aqueous phase were separated, the aqueous layer was extracted with $CHCl_3$ (10 mL×3). After the organic phase was mixed, the mixture was dried with $Na_2SO_4$ and concentrated under a reduced pressure. The crude product was passed through Discovery (registered trademark) C18 and eluted with MeCN. After concentration, the residue was purified through an HPLC system, and Compound 37 (22.4 mg, 37.2 μmol, yield 67%) was obtained as a colorless oily substance.
$^1H$ NMR (400 MHz, $CDCl_3$, δ): 0.96 (3H, d, J = 6.9 Hz), 1.82 (3H, s), 1.93 (1H, dd, J = 15.1, 11.0 Hz), 2.13 (3H, d, J = 9.6 Hz), 2.28 (6H,m), 2.44 (1H, q, J = 7.0 Hz), 2.51 (1H, t, J = 2.5 Hz), 2.67 (1H, d, J = 18.3 Hz), 2.99 (1H, dd, J = 18.3, 8.2 Hz), 3.22-3.38 (3H, m), 3.56 (2H, m), 3.76 (3H, s), 4.02 (1H, s), 4.15 (2H, m), 4.37 (2H, m), 5.88 (1H, d, J = 1.4 Hz), 5.96 (1H, d, J = 1.4 Hz), 6.51 (2H, m).

[Example 6]

6. Synthesis of Compounds 42 and 44 of present invention

According to the following scheme, from the natural product Cyanosafracin B (Compound 1), Compound 38 was synthesized in 4 processes in the same manner as in synthesis of 5, and Compounds 42 and 44 were synthesized in the following 3 or 4 processes. First, Compounds 39 and 40 were synthesized by constructing a macrocycle according to a ring-closing metathesis reaction using Grubbs catalyst M101 for Compound 38 in which a protecting group for a phenolic hydroxyl group of Compound 5 was an acetyl group. The isolated Compound 39 was reacted with a 0-valent palladium catalyst to obtain Compound 41. Compound 42 was synthesized by removing the acetyl group of Compound 41. In addition, Compound 43 was obtained according to N-propargylation of Compound 41, and the acetyl group was removed to synthesize Compound 44. Reaction conditions for each step will be described in detail.

[C83]

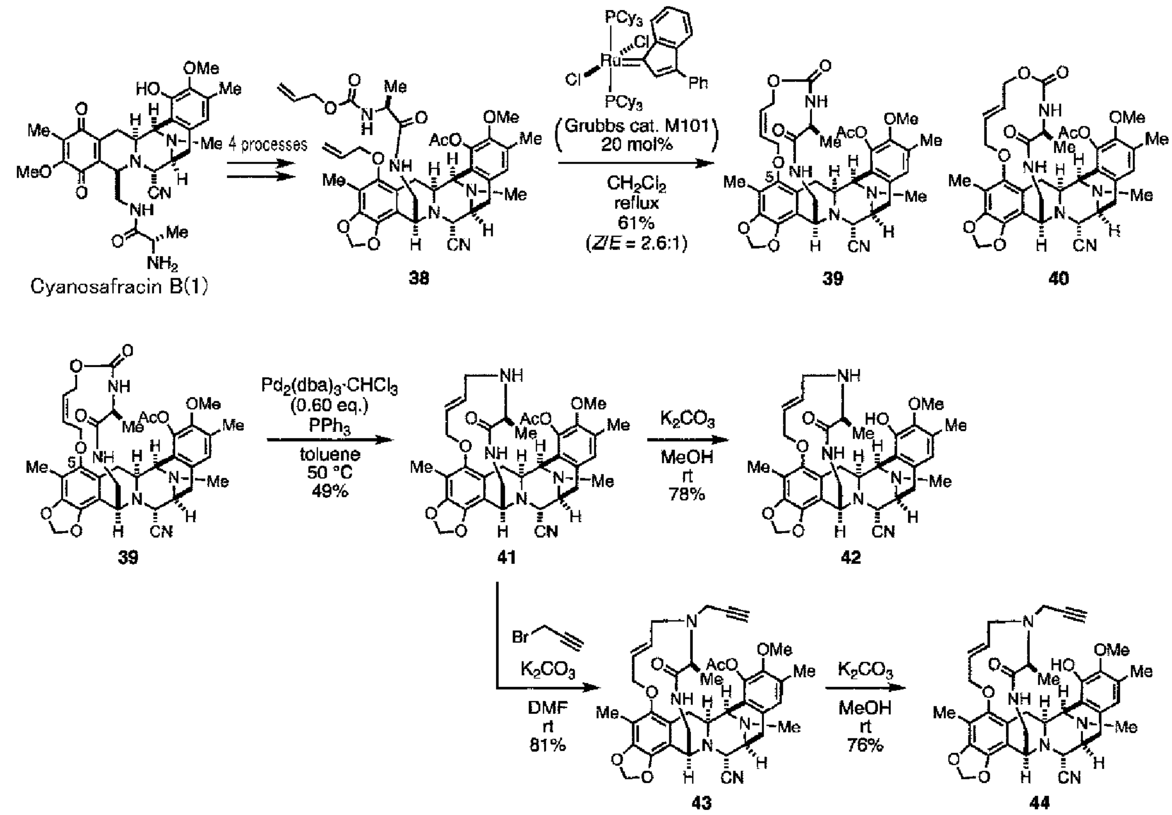


[Synthesis of Compound 48]

[C84]

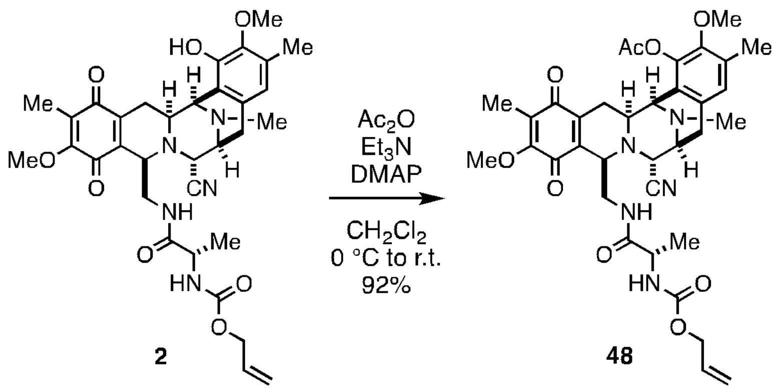


$Ac_2O$ (225 μL, 2.38 mmol, 1.5 equivalent), $NEt_3$ (664 μL, 4.76 mmol, 3 equivalent), and DMAP (19.4 mg, 0.0159 mmol, 0.10 equivalent) were added to a Compound 2 (1.01 g, 1.59 mmol) solution in $CH_2Cl_2$ (15.9 mL, 0.10 M) under ice cooling, and the mixture was stirred at room temperature for 4 hours. Diluting with $CH_2Cl_2$ (10 mL) was performed and a saturated $NH_4Cl$ aqueous solution (10 mL) was added under ice cooling. After the organic phase and the aqueous phase were separated, the aqueous layer was extracted with $CH_2Cl_2$ (20 mL×2).After the organic phase was mixed, the mixture was washed with a saturated saline, dried with $Na_2SO_4$ and concentrated under a reduced pressure. The crude residue was purified through silica gel column chromatography (hexane/AcOEt), and Compound 48 (988 mg, 1.46 mmol, yield 92%) was obtained as a light yellow oily substance.
$^1$H NMR (400 MHz, $CDCl_3$, δ): 0.89 (3H, d, J = 6.9 Hz), 1.60-1.73 (1H, m), 1.83 (3H, s), 2.19-2.24 (6H, m), 2.40-2.51 (4H, m), 2.85 (1H, d, J = 17.4 Hz), 3.02-3.12 (3H, m), 3.36 (2H, m), 3.66-3.81 (6H, m), 3.96-4.03 (4H, m), 4.36-4.45 (2H, m), 4.82 (1H, br), 5.13-5.22 (2H, m), 5.39 (1H, br), 5.74-5.84 (1H, m), 6.83 (1H, s).

[Synthesis of Compound 38]

[C85]

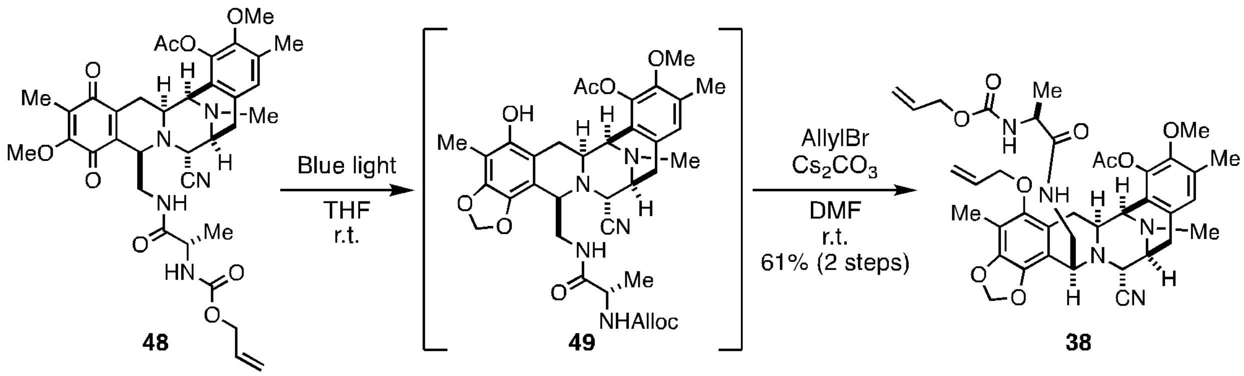


48 49 38

The solution of Compound 48 (446 mg, 0.644 mmol) in freeze-deaerated THF (12.9 mL, 0.05 M) was stirred at room temperature for 3 hours and 30 minutes while emitting blue light. The reaction solution was concentrated under a reduced pressure, and the obtained crude residue was used in the next reaction without purification.

$Cs_2CO_3$ (525 mg, 1.61 mmol, 2.5 equivalent) and propargyl bromide (109 μL, 1.29 mmol, 2.0 equivalent) were added to a MeCN (6.44 mL, 0.10 M) solution of the crude product of Compound 49. The mixture was stirred at room temperature for 12 hours. Diluting with $CH_2Cl_2$ (10 mL) was performed and 1 M hydrochloric acid (5.0 mL) was added and quenching was performed under ice cooling. After the organic phase and the aqueous phase were separated, the aqueous layer was extracted with $CH_2Cl_2$ (30 mL×2).After the organic phase was mixed, the mixture was dried with $Na_2SO_4$ and concentrated under a reduced pressure. The crude residue was purified through silica gel column chromatography ($CHCl_3$/AcOEt), and Compound 38 (289 mg, 0.394 mmol, 2-stage yield 61%) was obtained as a brown solid. $^1H$ NMR (400 MHz, $CDCl_3$, δ): 0.90 (3H, d, J = 6.9 Hz), 1.88-1.95 (1H, m), 2.12 (3H, s), 2.21-2.32 (7H, m), 2.37 (3H, s), 2.68 (1H, d, J = 17.9 Hz), 3.00-3.09 (2H, m), 3.20 (1H, dt, J = 11.9, 2.7 Hz), 3.38-3.56 (4H, m), 3.70-3.75 (5H, m), 4.01 (1H, br), 4.08-4.26 (3H, m), 4.43-4.44 (2H, m), 5.15-5.28 (4H, m), 5.38-5.46 (2H, m), 5.77-5.87 (2H, m), 5.96 (1H, d, J = 1.4 Hz), 6.05-6.13 (1H, m), 6.85 (1H, s).

[Synthesis of Compounds 39 and 40]

[C86]

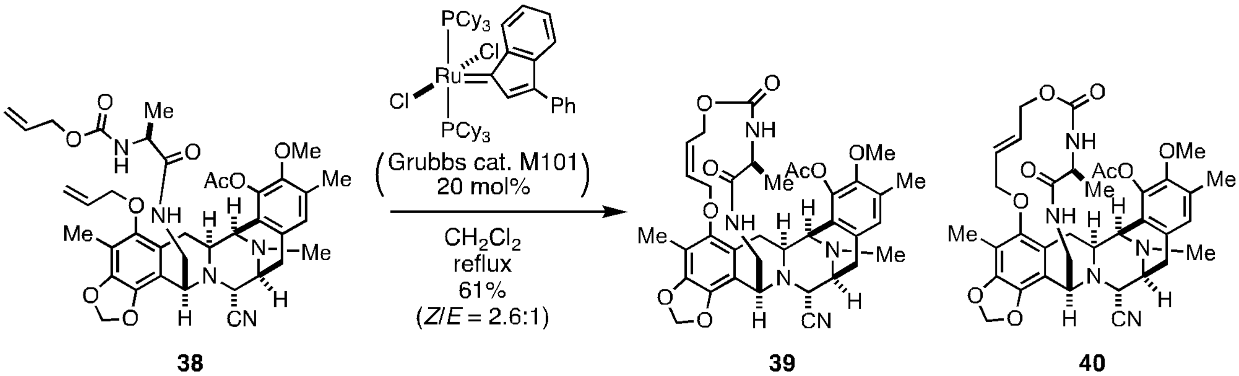


38 39 40

A $CH_2Cl_2$ solution (13 mL) of Compound 38 (308 mg, 430 μmol) was added to a $CH_2Cl_2$ solution (30 mL) of Grubbs catalyst M101 (79.4 mg, 86.0 μmol, 0.20 equivalent). The mixture was heated to reflux for 11 hours. SiliaMetS (registered trademark) Thiourea (569 mg) was added to the reaction solution and the mixture was stirred for 6 hours.

After filtration and concentration under a reduced pressure, the residue was purified through silica gel column chromatography (hexane/AcOEt) and an HPLC system, and Compound 39 (Z isomer, 130 mg, 188 μmol, yield 44%) and Compound 40 (E isomer, 49.4 mg, 71.9 μmol, yield 17%) were obtained as brown solids.

39: $[\alpha]_D^{23}$ -85.6° (c 1.0, $CHCl_3$).; $^1H$ NMR (400 MHz, DMSO-$d_6$, δ): 1.06 (3H, d, J = 7.3 Hz), 2.06-2.24 (9H, m), 2.42 (3H, s), 2.69 (1H, d, J = 17.9 Hz), 2.95-3.36 (8H, m), 3.53-3.83 (6H, m), 4.24-4.41 (3H, m), 4.70 (1H, dd, J = 12.8, 5.5 Hz), 5.50-5.57 (1H, m), 5.71-5.78 (1H, m), 5.97-6.01 (2H, m), 6.86 (2H, br), 8.26 (1H, d, J = 3.2 Hz).; HRMS (ESI, m/z): $[M+H]^+$ calculated for $C_{36}H_{42}N_5O_9$, 688.2977; found, 688.2979.

40: $[\alpha]_D^{23}$ -91.9° (c 1.0, $CHCl_3$); $^1H$ NMR (400 MHz, DMSO-$d_6$, δ): 1.03 (3H, d, J = 7.3 Hz), 2.07-2.20 (9H, m), 2.42 (3H, s), 2.61-2.69 (1H, m), 2.94-3.04 (4H, m), 3.23-3.63 (4H, m), 3.73-3.85 (6H, m), 4.43 (3H, s), 5.54 (1H, d, J = 8.2 Hz), 5.88-6.00 (3H, m), 6.87 (2H, s).; HRMS (ESI, m/z): $[M-CN]^+$ calculated for $C_{35}H_{41}N_4O_9$, 661.2868; found, 661.2870.

[Synthesis of Compound 41]

[C87]

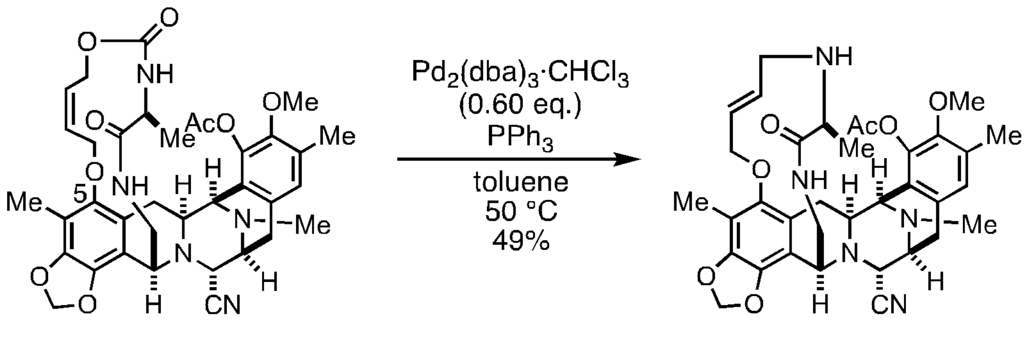


Triphenylphosphine (8.91 mg, 34.0 μmol, 2.4 equivalent), and a toluene solution (400 μL) of Compound 39 (9.74 mg, 14.2 μmol) were sequentially added to a toluene solution (310 μL) of freeze-deaerated $Pd_2(dba)_3$ (7.78 mg, 8.50 μmol, 0.60 equivalent). The mixture was stirred at 50°C for 4.5 hours. SiliaMetS (registered trademark) Thiourea (112 mg) was added to the reaction solution and the mixture was stirred for 11.5 hours. After filtration and concentration under a reduced pressure, the residue was purified through an HPLC system, and Compound 41 (E isomer, 4.47 mg, 6.94 μmol, yield 49%) was obtained as a colorless solid.

41: $^1H$ NMR (400 MHz, $CDCl_3$, δ): 1.09 (3H, d, J = 6.9 Hz), 1.86 (1H, dd, J = 16.0, 6.9 Hz), 2.15-2.31 (11H, m), 2.43 (3H, s), 2.82-2.97 (3H, m), 3.04 (1H, d, J = 13.7 Hz), 3.08-3.16 (1H, m), 3.24 (1H, d, J = 16.5 Hz), 3.36 (1H, d, J = 7.8 Hz), 3.61-3.64 (1H, m), 3.77 (3H, s), 3.91-3.97 (1H, m), 4.02-4.10 (3H, m), 4.63 (1H, dd, J = 11.2, 6.6 Hz), 5.12-5.17 (1H, m), 5.43-5.51 (1H, m), 5.87 (1H, d, J = 1.4 Hz), 6.00 (1H, d, J = 1.4 Hz), 6.77 (1H, d, J = 9.6 Hz), 6.89 (1H, s); HRMS (ESI, m/z): $[M+H]^+$ calculated for $C_{35}H_{42}N_5O_7$, 644.3079; found, 644.3078.

[Synthesis of Compound 42]

[C88]

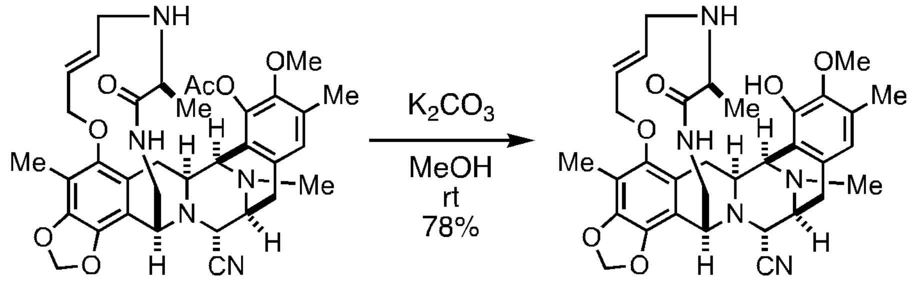


**41** **42**

A $K_2CO_3$ (9.51 mg, 68.8 μmol, 10 equivalent) aqueous solution (138 μL) was added to a MeOH solution (552 μL) of Compound 41 (4.43 mg, 6.88 μmol) under ice cooling, and the mixture was stirred at room temperature for 11 hours. After diluting with $CH_2Cl_2$ (2.0 mL) and water (1.0 mL), 1 M hydrochloric acid (150 μL) was added and quenching was performed under ice cooling. After the organic phase and the aqueous phase were separated, the aqueous layer was extracted with $CHCl_3$ (20 mL×3). After the organic phase was mixed, and the mixture was dried with $Na_2SO_4$ and concentrated under a reduced pressure. The crude product was passed through STRATA (registered trademark) C18 and eluted with MeCN. After concentration, the residue was purified through an HPLC system, and Compound 42 (3.24 mg, 5.38 μmol, yield 78%) was obtained as a colorless solid substance. $^1$H NMR (400 MHz, $CDCl_3$, δ): 1.09 (3H, d, J = 6.9 Hz), 1.63 (1H, dd, J = 15.8, 11.7 Hz), 1.85 (1H, dd, J = 16.0, 6.9 Hz), 2.10-2.21 (4H, m), 2.26-2.34 (7H, m), 2.78 (1H, d, J = 17.9 Hz), 2.90 (1H, dd, J = 18.1, 8.0 Hz), 3.02-3.23 (5H, m), 3.34 (1H, d, J = 8.2 Hz), 3.81-3.94 (5H, m), 4.01-4.10 (5H, m), 4.52-4.62 (1H, m), 5.07-5.11 (1H, m), 5.29-5.44 (1H, m), 5.75-5.89 (2H, m), 5.99 (1H, s), 6.52 (1H, s), 6.69 (1H, d, J = 9.2 Hz).; HRMS (ESI, m/z): $[M+H]^+$ calculated for $C_{33}H_{40}N_5O_6$, 602.2973; found, 602.2962.

[Synthesis of Compound 43]

[C89]

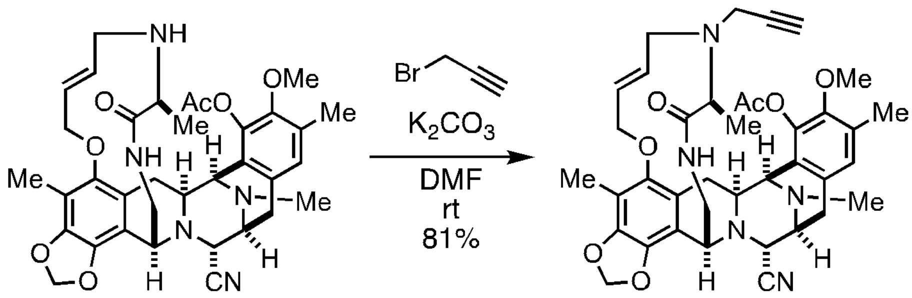


**41** **43**

$K_2CO_3$ (12.6 mg, 90.9 μmol, 15 equivalent), and propargyl bromide (4.56 μL, 60.6 μmol, 10 equivalent) were added to a DMF (0.60 mL) solution of Compound 41 (3.90 mg, 6.06 mmol) under ice cooling. The mixture was stirred at room temperature for 96 hours. The reaction solution was filtered and concentrated under a reduced pressure. The crude residue was purified through an HPLC system, and Compound 43 (3.36 mg, 4.93 μmol, yield 81%) was obtained as a colorless solid substance.

$^1$H NMR (400 MHz, $CDCl_3$, δ): 1.12 (3H, d, J = 6.9 Hz), 1.93-2.01 (1H, m), 2.11-2.20 (4H, m), 2.25-2.35 (7H, m), 2.42-2.48 (3H, m), 2.82-3.12 (8H, m), 3.37 (1H, d, J = 7.3 Hz), 3.61 (1H, d, J = 3.2 Hz), 3.78 (3H, s), 3.95-4.08 (3H, m), 4.18 (1H, d, J = 2.3 Hz), 4.66 (1H, m), 4.95 (1H, dt, J = 10.4, 5.2 Hz), 5.46-5.53 (1H, m), 5.86 (1H, s), 6.00 (1H, s), 6.38 (1H, d, J = 10.1 Hz), 6.88 (1H, s).; HRMS (ESI, m/z): $[M+H]^+$ calculated for $C_{38}H_{44}N_5O_7$, 682.3235; found, 682.3223.

[Synthesis of Compound 44]

[C90]

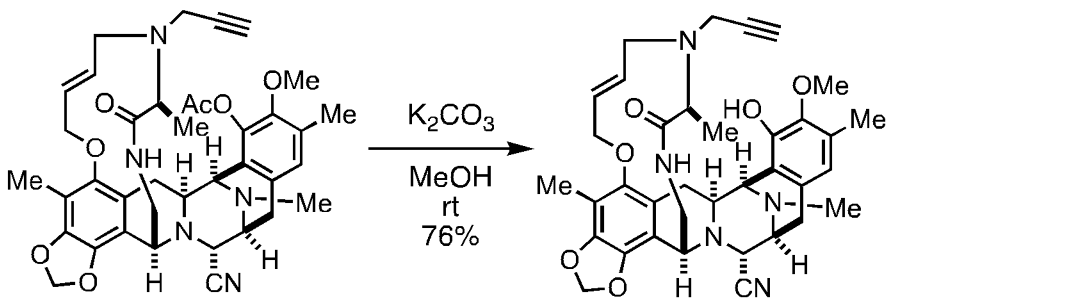


43 44

A $K_2CO_3$ (6.81 mg, 49.3 μmol, 10 equivalent) aqueous solution (197 μL) was added to a MeOH solution (789 μL) of Compound 43 (3.36 mg, 4.93 μmol) under ice cooling, and the mixture was stirred at room temperature for 17.5 hours. After diluting with MeOH (1.0 mL) and water (100 μL), 1 M hydrochloric acid (70 μL) was added and quenching was performed under ice cooling. After concentration under a reduced pressure, the crude product was passed through STRATA (registered trademark) C18 and eluted with MeCN. After concentration, the residue was purified through an HPLC system and Compound 42 (2.41 mg, 3.77 μmol, yield 76%) was obtained as a colorless solid substance.
$^1$H NMR (400 MHz, $CDCl_3$, δ): 1.13 (3H, d, J = 6.9 Hz), 1.92 (1H, dd, J = 16.0, 5.5 Hz), 2.09-2.19 (4H, m), 2.26-2.35 (8H, m), 2.81-3.14 (8H, m), 3.33-3.48 (1H, m), 3.85-3.95 (4H, m), 4.04-4.13 (3H, m), 4.21 (1H, d, J = 2.3 Hz), 4.61 (1H, dd, J = 11.5, 5.8 Hz), 4.88 (1H, dt, J = 10.4, 5.2 Hz), 5.31-5.39 (1H, m), 5.81-5.84 (2H, m), 6.00 (1H, d, J = 1.4 Hz), 6.36 (1H, d, J = 10.5 Hz), 6.50 (1H, s).; HRMS (ESI, m/z): $[M-CN]^+$ calculated for $C_{35}H_{41}N_4O_6$, 613.3021; found, 613.2992.

[Example 7]

7. Synthesis of Compound 47 of the present invention

According to the following scheme, from the natural product Cyanosafracin B (Compound 1), Compound 45 was synthesized in 5 processes in the same manner as in synthesis of Compound 11 and Compound 47 was synthesized in the following 2 processes. First, Compound 46 was obtained by applying a [4+2]-cyclization reaction to Compound 45 in which a protecting group for a phenolic hydroxyl group of Compound 11 was an acetyl group. Then, the acetyl group was removed to synthesize Compound 47. Reaction conditions for each step will be described in detail.

[C91]

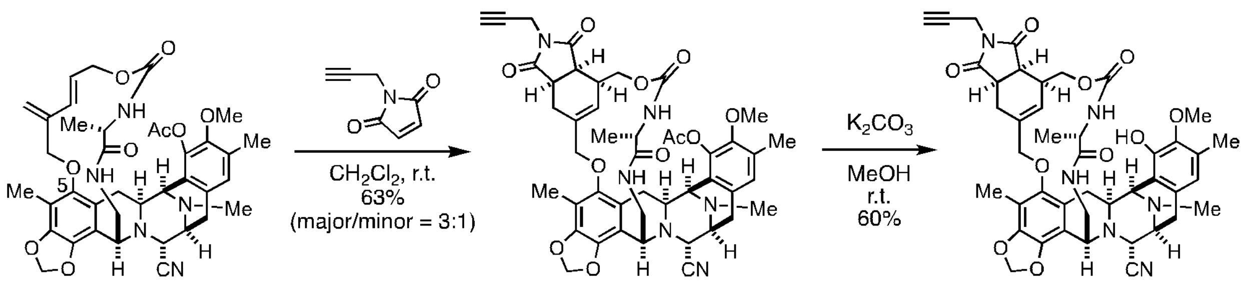


45 46 47

[Synthesis of Compound 50]

[C92]

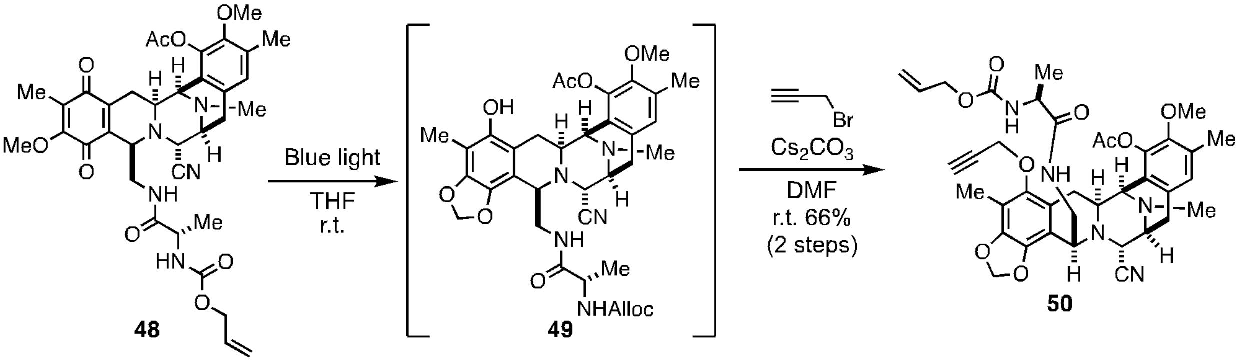


A 48 (512 mg, 0.739 mmol) solution in freeze-deaerated THF (14.8 mL, 0.05 M) was stirred at room temperature for 3 hours and 30 minutes while emitting blue light. The reaction solution was concentrated under a reduced pressure, and the obtained crude residue was used in the next reaction without purification.

Propargyl bromide (111 μL, 1.48 mmol, 2.0 equivalent) and $Cs_2CO_3$ (602 mg, 1.85 mmol, 2.5 equivalent) were added to a MeCN (7.40 mL, 0.10 M) solution of the crude product of Compound 49. The mixture was stirred at room temperature for 11 hours and 30 minutes. Diluting with $CH_2Cl_2$ (10 mL) was performed and 1 M hydrochloric acid (5.0 mL) was added and quenching was performed under ice cooling. After the organic phase and the aqueous phase were separated, the aqueous layer was extracted with $CH_2Cl_2$ (30 mL×2). After the organic phase was mixed, the mixture was dried with $Na_2SO_4$ and concentrated under a reduced pressure. The crude residue was purified through silica gel column chromatography (hexane/AcOEt) and 50 (359 mg, 0.491 mmol, 2-stage yield 66%) was obtained as a brown solid.
$^1$H NMR (400 MHz, $CDCl_3$, δ): 0.88 (3H, d, J = 7.3 Hz), 1.90-2.00 (1H, m), 2.12 (3H, s), 2.19-2.23 (6H, m), 2.42 (3H, s), 2.53 (1H, t, J = 2.3 Hz), 2.65 (1H, d, J = 17.9 Hz), 3.00 (1H, dd, J = 18.3, 8.2 Hz), 3.09-3.18 (2H, m), 3.35-3.51 (4H, m), 3.64-3.76 (4H, m), 3.97 (1H, s), 4.07 (1H, d, J = 2.3 Hz), 4.27-4.49 (4H, m), 5.11-5.21 (3H, m), 5.45 (1H, t, J = 5.7 Hz), 5.73-5.82 (2H, m), 5.93 (1H, d, J = 0.9 Hz), 6.83 (1H, s).

[Synthesis of Compound 45]

[C93]

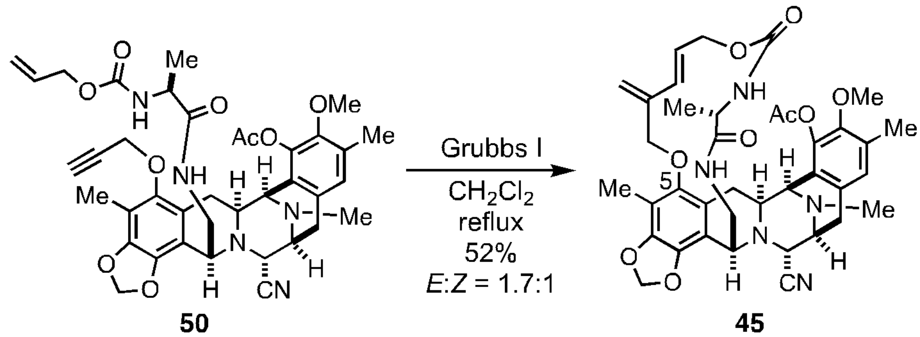


A $CH_2Cl_2$ solution (10 mL) of Compound 50 (287 mg, 401 μmol) was added to a $CH_2Cl_2$ solution (30 mL) of a Grubbs first generation catalyst (99.4 mg, 121 μmol, 0.30 equivalent). The mixture was heated to reflux for 3 hours and 30 minutes. SiliaMetS (registered trademark) Thiourea (798 mg) was added to the reaction solution and the mixture was stirred for 2 hours. After filtration and concentration under a reduced pressure, the residue was purified through silica gel column chromatography (hexane/AcOEt) and an HPLC system, and Compounds 45 (E isomer, 94.6 mg, 188 μmol, yield 33%), and (Z isomer, 55.8 mg, 78.1 μmol, yield 19%) were obtained as brown solids.
$^1$H NMR (400 MHz, DMSO-$d_6$, δ): 1.10 (3H, d, J = 6.9 Hz), 1.72-1.79 (1H, m), 2.09-2.25 (10H, m), 2.35 (3H, s), 2.69 (1H, d, J = 18.3 Hz), 2.93-3.18 (6H, m), 3.33-3.41 (2H, m), 3.53-3.83 (7H, m), 4.30-4.58 (2H, m), 4.74 (1H, br), 5.16-5.28 (1H, m), 5.42 (1H, s), 5.51(1H, s),5.65-5.71 (1H, m), 6.00 (2H, d, J = 17.9 Hz), 6.20 (1H, br), 6.68-6.96 (2H, m).; HRMS

(ESI, m/z): $[M+H]^+$ calculated for $C_{38}H_{44}N_5O_9$, 714.3121; found, 714.3121.

[Synthesis of Compound 46]

[C94]

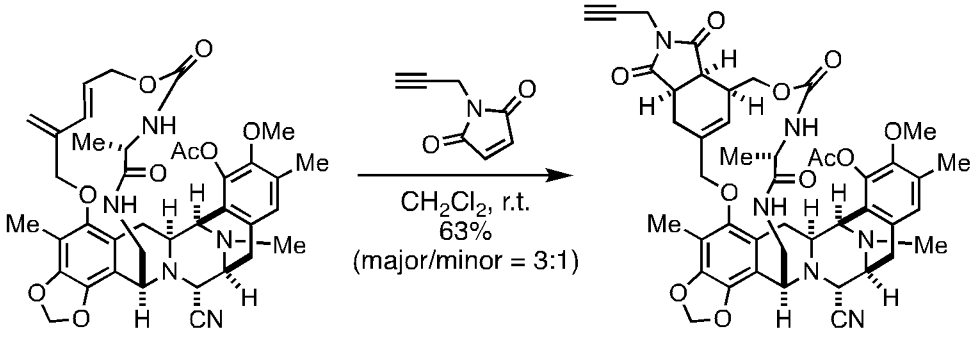


**45** **46**

N-propargylmaleimide (91.7 mg, 679 μmol, 15 equivalent) was added to a $CH_2Cl_2$ (0.226 mL) solution of Compound 45 (32.3 mg, 45.3 μmol) and the mixture was stirred at room temperature for 13 hours. After concentration under a reduced pressure, the residue was purified using a silica gel column, HPLC system, and Compound 46 (18.3 mg, 21.6 μmol, yield 48%) and its diastereomer (6.02 mg, 7.09 μmol, 16%) were obtained as white solids. The absolute configuration of the diastereomer was not determined.
46: $[\alpha]_D^{24}$ -66.6° (c 1.0, $CHCl_3$).; $^1H$ NMR (400 MHz, acetone-$d_6$, δ): 1.26-1.39 (5H, m), 2.03-2.08 (7H, m), 2.23 (3H, s), 2.31-2.48 (4H, m), 2.58-2.65 (4H, m), 2.87-3.03 (9H, m), 3.25-3.50 (4H, m), 3.60-3.83 (7H, m), 3.89-4.16 (6H, m), 4.39-4.49 (3H, m), 5.03 (1H, t, J = 11.0 Hz), 5.31 (1H, s), 5.71 (1H, d, J = 9.6 Hz), 5.88-6.06 (3H, m), 6.92 (1H, s).; HRMS (ESI, m/z): $[M+H]^+$ calculated for $C_{45}H_{49}N_6O_{11}$, 849.3454; found, 849.3474.

[Synthesis of Compound 47]

[C95]

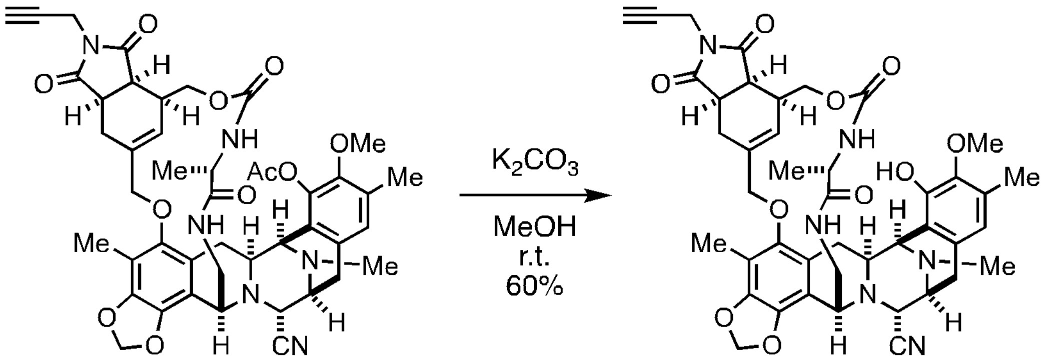


**46** **47**

A $K_2CO_3$ (16.5 mg, 119 μmol, 10 equivalent) aqueous solution (238 μL) was added to a MeOH solution (952 μL) of Compound 46 (10.1 mg, 11.9 μmol) under ice cooling, and the mixture was stirred at room temperature for 50 minutes. After diluting with $CH_2Cl_2$ (2.0 mL) and water (1.0 mL), 1 M hydrochloric acid (150 μL) was added and quenching was performed under ice cooling. After the organic phase and the aqueous phase were separated, the aqueous layer was extracted with $CHCl_3$ (20 mL×3). After the organic phase was mixed, the mixture was dried with $Na_2SO_4$ and

concentrated under a reduced pressure. The crude product was passed through STRATA (registered trademark) C18 and eluted with MeCN. After concentration, the residue was purified through an HPLC system, and Compound 47 (5.73 mg, 7.10 μmol, yield 60%) was obtained as a colorless solid substance.
$^1$H NMR (400 MHz, acetone-$d_6$, δ): 1.20-1.33 (6H, m), 1.42-1.50 (1H, m), 2.14 (1H, s), 2.18-2.31 (3H, m), 2.35 (3H, s), 2.41 (1H, br), 2.64 (1H, s), 2.95-3.02 (2H, m), 3.37-3.48 (3H, m), 3.64-3.71 (2H, m), 3.87-3.96 (5H, m), 4.11 (2H, s), 4.19 (1H, s), 4.37-4.48 (2H, m), 4.99 (1H, t, J = 11.9 Hz), 5.27 (1H, s), 5.67 (1H, d, J = 10.1 Hz), 5.93-5.99 (3H, m), 6.53 (1H, s), 7.80 (1H, s).; HRMS (ESI, m/z): $[M-CN]^+$ calculated. for $C_{43}H_{47}N_6O_{10}$, 807.3348; found, 807.3355.

[Example 8]

8. Verification of DNA alkylating ability

DNA double strands were applied to THIQ compounds containing a macrocyclic structure of the present invention (Compound 7, Compound 13, Compound 14, Compound 29, Compound 36, Compound 37, and Compound 47), analysis was performed using an electrophoresis method, and the DNA alkylating ability was verified. The experiment was performed according to the procedure shown in the literature (Tanifuji, R.; Tsukakoshi, K.; Ikebukuro, K.; Oikawa, H., Oguri, H. Bioorg. Med. Chem. Lett., 2019, 29, 1807.). As a result, it was found that all Compound 7, Compound 13, Compound 14, Compound 29, Compound 36, and Compound 37 exhibited a DNA alkylating ability.
The outline of the DNA alkylation reaction of Compound 7 is shown below.

[C96]

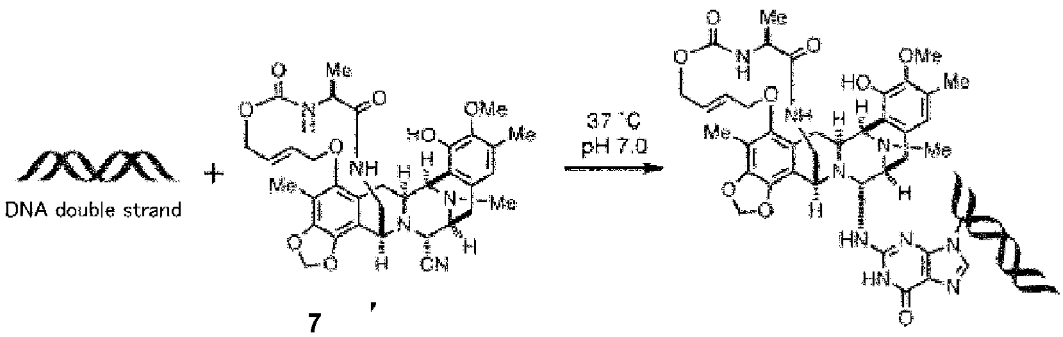

7

[Example 9]

9. Verification of antitumor activity

The THIQ compound containing a macrocyclic structure of the present invention was administered to various cancer cell lines, and the antitumor activity ($GI_{50}$ value) was observed. Specifically, verification was performed according to the literature (Yamori, T.; Matsunaga, A.; Sato, S.; Yamazaki, K.; Komi, A.; Ishizu, K. et al. Cancer Res. 1999, 59, 4042.) by the following procedure.
Cells were plated at an appropriate density in a 96-well plate in RPMI 1640 together with 5% fetal bovine serum, and adhered overnight. The cells were exposed to a drug for 48 hours. Then, according to the sulforhodamine B assay described by Skehan et al (Skehan P., Storeng R., Scudiero D., Monks A., McMahon J., Vistica D., Warren J. T., Bokesch H., Kenney S., Boyd M. R. J. Natl. Cancer Inst. 1990, 82, 1107-1112.), the cell growth rate was measured. The absorbance of the control well (C) and the test well (T) was measured at 525 nm. In addition, the time at which the drug was added was set as time 0, and the absorbance for the test well ($T_0$) was measured. Using these measured values, the cell growth inhibitory effect (% growth = PG) was calculated by the following formula at each concentration of the drug.

$$(T>T_0):\ PG = 100\times[(T - T_0)/(C-T_0)]$$

$$(T<T_0):\ PG = 100\times[(T - T_0)/T]$$

The PG values determined above for each cell line were plotted against the concentration (logarithm) on a

semi-logarithmic graph, and summarized for each visceral cancer. Concentrations at which the dose-response curve of each cell line crossed the horizontal line of PG = 50%, 0%, or -50% were calculated. These concentrations were defined as $LogGI_{50}$, LogTGI, and $LogLC_{50}$. Actually, using two data points of the drug concentration before and after crossing each horizontal line, the drug concentration (logarithmic value) at the intersection of the straight line connecting the two points and the horizontal line for each PG was calculated.

In addition, as comparative values, reference values (NPL: Leal, J. F. M.; Martinez-Diez, M.; Cuevas, C.; Garcia-Fernandez, L. F.; Galmarini, C. M. et al. Br. J. Pharmacol. 2010, 161, 1099-1110) of Ecteinascidin 743 (commercially available from Pharma Mar) and lurbinectedin (commercially available from Pharma Mar) and Cyanosafracin B values were listed.

The obtained results are as follows.

[Table 1]

Table 1: antitumor activity [nM] of Compound 7 ($GI_{50}$ value: 50% growth inhibitory concentration)

| | | 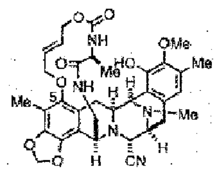 7 | 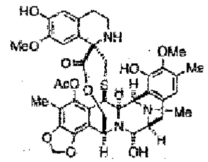 ecteinascidin 743 | 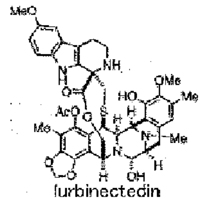 lurbinectedin | 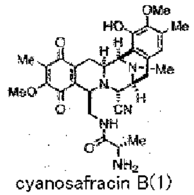 cyanosafracin B(1) |
|---|---|---|---|---|---|
| breast cancer cells | HBC-4 | 28 | | | 59 |
| | BSY-1 | 19 | | | 34 |
| | HBC-5 | 79 | | | 62 |
| | MCF-7 | 17 | 2.6 | 1.7 | 52 |
| | MDA-MB-231 | 32 | 3.9 | 3.4 | 130 |
| central nervous system cancer cells | U251 | 39 | | | 370 |
| | SF-268 | 89 | | | 370 |
| | SF-295 | 240 | | | 500 |
| | SF-539 | 35 | | | 180 |
| | SNB-75 | 61 | | | 150 |
| | SNB-78 | 45 | | | 63 |
| colorectal cancer cells | HCC2998 | 69 | | | 350 |
| | KM-12 | 58 | | | 530 |
| | HT-29 | 58 | 9.8 | 2.4 | 190 |
| | HCT-15 | 310 | | | 390 |
| | HCT-116 | 51 | 4.9 | 6.4 | 290 |

(continued)

Table 1: antitumor activity [nM] of Compound 7 ($GI_{50}$ value: 50% growth inhibitory concentration)

| | | 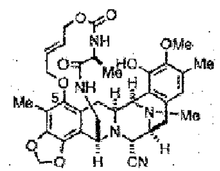 7 | 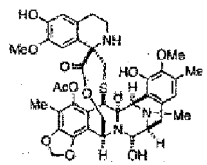 ecteinascidin 743 | 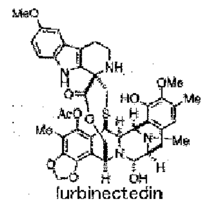 lurbinectedin | 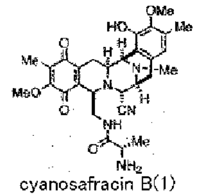 cyanosafracin B(1) |
|---|---|---|---|---|---|
| lung cancer cells | NCI-H23 | 140 | 2.1 | 5.3 | 290 |
| | NCI-H226 | 260 | | | 310 |
| | NCI-H522 | 21 | | | 43 |
| | NCI-H460 | 270 | 1.8 | 1.5 | 400 |
| | A549 | 480 | 10.4 | 1.3 | 1600 |
| | DMS273 | 82 | | | 270 |
| | DMS114 | 14 | | | 35 |
| melanoma cells | LOX-IMVI | 19 | | | 61 |
| cervical cancer cells | OVCAR-3 | 41 | | | 130 |
| | OVCAR-4 | 390 | | | 290 |
| | OVCAR-5 | 240 | | | 390 |
| | OVCAR-8 | 200 | | | 290 |
| | SK-OV-3 | 420 | | | 540 |
| kidney cancer cells | RXF-631L | 240 | | | 400 |
| | ACHN | 300 | | | 320 |

(continued)

| Table 1: antitumor activity [nM] of Compound 7 ($GI_{50}$ value: 50% growth inhibitory concentration) | | | | | |
|---|---|---|---|---|---|
| | | 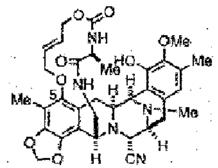<br>**7** | 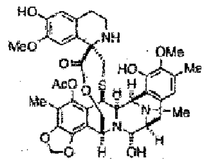<br>ecteinascidin 743 | 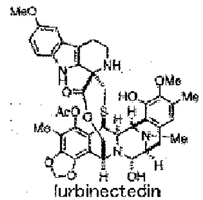<br>lurbinectedin | 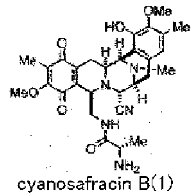<br>cyanosafracin B(1) |
| gastric cancer cells | St-4 | 300 | | | 430 |
| | MKN1 | 36 | | | 52 |
| | MKN-B | 260 | | | 170 |
| | MKN-A | 260 | | | 250 |
| | MKN45 | 47 | | | 300 |
| | MKN74 | 370 | | | 310 |
| prostate cancer cells | DU-145 | 430 | | | 490 |
| | PC-3 | 240 | | | 290 |

[Table 2]

Table 2: antitumor activity [nM] of Compound 13 ($GI_{50}$ value: 50% growth inhibitory concentration)

| | | 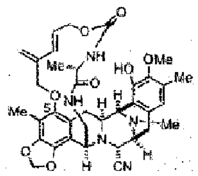 13 | 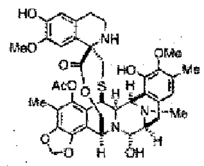 ecteinascidin 743 | 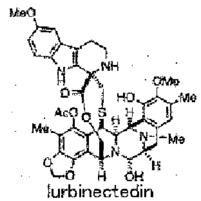 lurbinectedin | 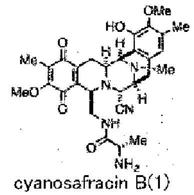 cyanosafracin B(1) |
|---|---|---|---|---|---|
| breast cancer cells | HBC-4 | 120 | | | 59 |
| | HBC-5 | 32 | | | 34 |
| | | 92 | | | 62 |
| | MCF-7 | 50 | 2.6 | 1.7 | 52 |
| | MDA-MB-231 | 95 | 3.9 | 3.4 | 130 |
| central nervous system cancer cells | U251 | 220 | | | 370 |
| | SF-268 | 350 | | | 370 |
| | SF-295 | 490 | | | 500 |
| | SF-539 | 120 | | | 180 |
| | SN8-75 | 120 | | | 150 |
| | SNB-78 | 100 | | | 63 |
| colorectal cancer cells | HCC2998 | 250 | | | 350 |
| | KM-12 | 290 | | | 530 |
| | HT-29 | 200 | 9.8 | 2.4 | 190 |
| | HCT-15 | 1500 | | | 390 |
| | HCT-116 | 220 | 4.9 | 6.4 | 290 |

(continued)

| Table 2: antitumor activity [nM] of Compound 13 ($GI_{50}$ value: 50% growth inhibitory concentration) | | | | | |
|---|---|---|---|---|---|
| | | 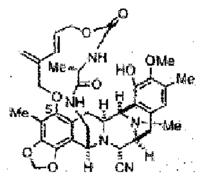 13 | 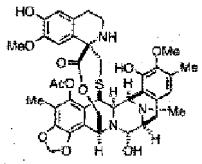 ecteinascidin 743 | 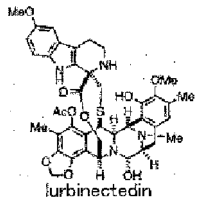 lurbinectedin | 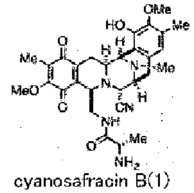 cyanosafracin B(1) |
| lung cancer cells | NCI-H23 | 350 | 2.1 | 5.3 | 290 |
| | NCI-H226 | 530 | | | 310 |
| | NCI-H522 | 40 | | | 43 |
| | NCI-H460 | 620 | 1.8 | 1.5 | 400 |
| | A549 | 3400 | 10.4 | 1.3 | 1600 |
| | DMS273 | 320 | | | 270 |
| | DMS114 | 37 | | | 35 |
| melanoma cells | LOX-IMVI | 57 | | | 61 |
| cervical cancer cells | OVCAR-3 | 290 | | | 130 |
| | OVCAR-4 | 1400 | | | 290 |
| | OVCAR-5 | 580 | | | 390 |
| | OVCAR-8 | 310 | | | 290 |
| | SK-OV-3 | 440 | | | 540 |
| kidney cancer cells | RXF-631L | 460 | | | 400 |
| | ACHN | 520 | | | 320 |

(continued)

Table 2: antitumor activity [nM] of Compound 13 ($GI_{50}$ value: 50% growth inhibitory concentration)

| | | 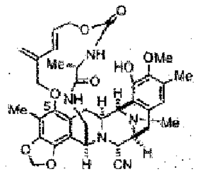 13 | 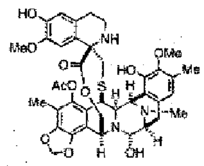 ecteinascidin 743 | 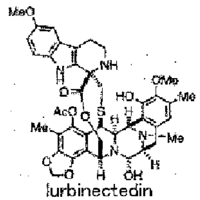 lurbinectedin | 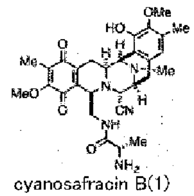 cyanosafracin B(1) |
|---|---|---|---|---|---|
| gastric cancer cells | St-4 | 420 | | | 430 |
| | MKN1 | 220 | | | 52 |
| | MKN-B | 360 | | | 170 |
| | MKN-A | 540 | | | 250 |
| | MKN45 | 250 | | | 300 |
| | MKN74 | 280 | | | 310 |
| prostate cancer cells | DU-145 | 830 | | | 490 |
| | PC-3 | 450 | | | 290 |

[Table 3]

| Table 3: antitumor activity [nM] of Compound 14 ($GI_{50}$ value: 50% growth inhibitory concentration) | | | | | |
|---|---|---|---|---|---|
| | | 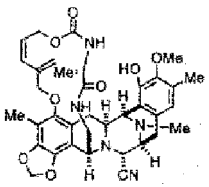 14 | 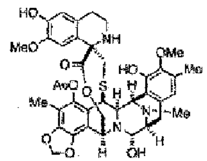 ecteinascidin 743 | 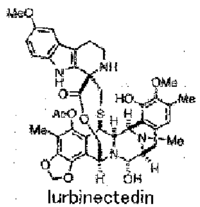 lurbinectedin | 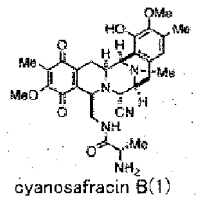 cyanosafracin B(1) |
| breast cancer cells | HBC-4 | 19 | | | 59 |
| | BSY-1 | 10 | | | 34 |
| | HBC-5 | 15 | | | 62 |
| | MCF-7 | 10 | 2.6 | 1.7 | 52 |
| | MDA-MB-231 | 17 | 3.9 | 3.4 | 130 |
| central nervous system cancer cells | U251 | 27 | | | 370 |
| | SF-268 | 39 | | | 370 |
| | SF-295 | 49 | | | 500 |
| | SF-539 | 17 | | | 180 |
| | SNB-75 | 14 | | | 150 |
| | SNB-78 | 34 | | | 63 |
| colorectal cancer cells | HCC2998 | 34 | | | 350 |
| | | | | | |
| | HT-29 | 22 | 9.8 | 2.4 | 190 |
| | HCT-15 | 98 | | | 390 |
| | HCT-116 | 23 | 4.9 | 6.4 | 290 |

(continued)

Table 3: antitumor activity [nM] of Compound 14 ($GI_{50}$ value: 50% growth inhibitory concentration)

| | | 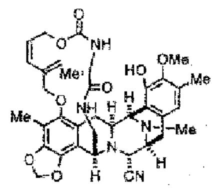 14 | 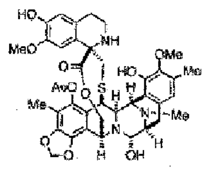 ecteinascidin 743 | 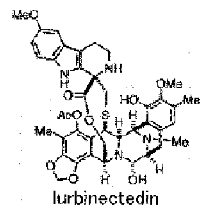 lurbinectedin | 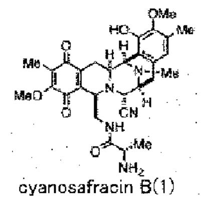 cyanosafracin B(1) |
|---|---|---|---|---|---|
| lung cancer cells | NCI-H23 | 34 | 2.1 | 5.3 | 290 |
| | NCI-H226 | 38 | | | 310 |
| | NCI-H522 | 10 | | | 43 |
| | NCI-H460 | 57 | 1.8 | 1.5 | 400 |
| | A549 | 280 | 10.4 | 1.3 | 1600 |
| | DMS273 | 29 | | | 270 |
| | | | | | |
| melanoma cells | LOX-IMVI | 12 | | | 61 |
| cervical cancer cells | OVCAR-3 | 25 | | | 130 |
| | OVCAR-4 | 38 | | | 290 |
| | OVCAR-5 | 47 | | | 390 |
| | OVCAR-8 | 34 | | | 290 |
| | SK-OV-3 | 59 | | | 540 |
| kidney cancer cells | RXF-631L | 48 | | | 400 |
| | AGHN | 38 | | | 320 |

(continued)

| Table 3: antitumor activity [nM] of Compound 14 ($GI_{50}$ value: 50% growth inhibitory concentration) | | | | | |
|---|---|---|---|---|---|
| | | 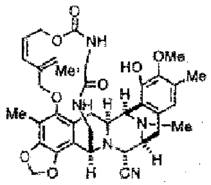 14 | 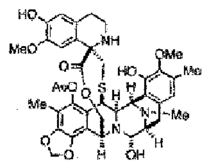 ecteinascidin 743 | 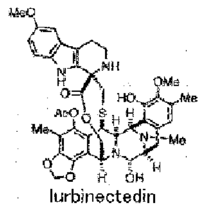 lurbinectedin | 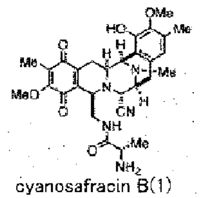 cyanosafracin B(1) |
| gastric cancer cells | St-4 | 36 | | | 430 |
| | | | | | |
| | MKN-B | 34 | | | 170 |
| | MKN-A | 54 | | | 250 |
| | MKN45 | 21 | | | 300 |
| | MKN74 | 41 | | | 310 |
| prostate cancer cells | DU-145 | 52 | | | 490 |
| | PC-3 | 47 | | | 290 |

[Table 4]

| Table 4: antitumor activity [nM] of Compound 16 ($GI_{50}$ value: 50% growth inhibitory concentration) | | | | | |
|---|---|---|---|---|---|
| | | 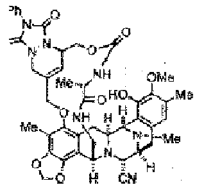 16 | 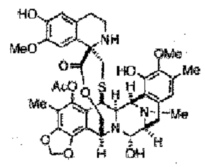 ecteinascidin 743 | 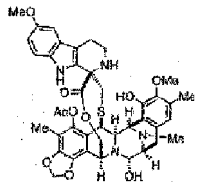 lurbinectedin | 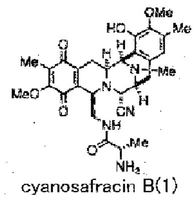 cyanosafracin B(1) |
| breast cancer cells | HBC-4 | 5.2 | | | 59 |
| | BSY-1 | 3.1 | | | 34 |
| | HBC-5 | C6.9 | | | 62 |
| | MCF-7 | 4.4 | 2.6 | 1.7 | 52 |
| | MDA-MB-231 | 6.7 | 3.9 | 3.4 | 130 |
| central nervous system cancer cells | U251 | 5.8 | | | 370 |
| | SF-268 | 13 | | | 370 |
| | SF-295 | 29 | | | 500 |
| | SF-539 | 8.2 | | | 180 |
| | SNB-75 | 9.9 | | | 150 |
| | SNB-78 | 7.5 | | | 63 |
| colorectal cancer cells | HCC2998 | 17 | | | 350 |
| | KM-12 | 14 | | | 530 |
| | HT-29 | 7.3 | 9.8 | 2.4 | 190 |
| | HCT-15 | 43 | | | 390 |
| | HCT-116 | 9.3 | 4.9 | 6.4 | 290 |

(continued)

| Table 4: antitumor activity [nM] of Compound 16 ($GI_{50}$ value: 50% growth inhibitory concentration) | | | | | |
|---|---|---|---|---|---|
| | | 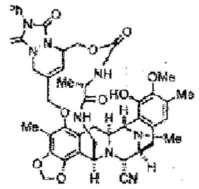 16 | 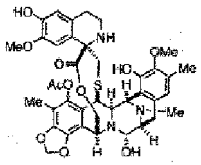 ecteinascidin 743 | 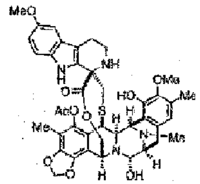 lurbinectedin | 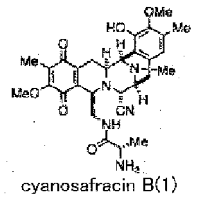 cyanosafracin B(1) |
| lung cancer cells | NCI-H23 | 20 | 2.1 | 5.3 | 290 |
| | NCI-H226 | 25 | | | 310 |
| | NCI-H522 | 2.4 | | | 43 |
| | NCI-H460 | 36 | 1.8 | 1.5 | 400 |
| | A549 | 51 | 10.4 | 1.3 | 1600 |
| | DMS273 | 15 | | | 270 |
| | DMS114 | 3.8 | | | 35 |
| melanoma cells | LOX-IMVI | 3.3 | | | 61 |
| cervical cancer cells | OVCAR-3 | 6.4 | | | 130 |
| | OVCAR-4 | 38 | | | 290 |
| | OVCAR-5 | 31 | | | 390 |
| | QVCAR-8 | 21 | | | 290 |
| | SK-OV-3 | 34 | | | 540 |
| kidney cancer cells | RXF-631L | 35 | | | 400 |
| | ACHN | 25 | | | 320 |

(continued)

| Table 4: antitumor activity [nM] of Compound 16 ($GI_{50}$ value: 50% growth inhibitory concentration) | | | | | |
|---|---|---|---|---|---|
| | | 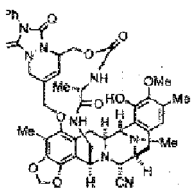 16 | 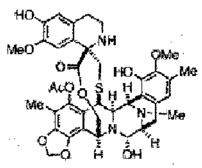 ecteinascidin 743 | 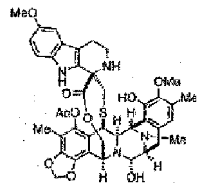 lurbinectedin | 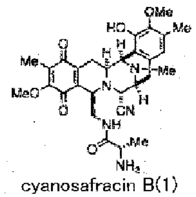 cyanosafracin B(1) |
| gastric cancer cells | St-4 | 37 | | | 430 |
| | MKN1 | 4.8 | | | 52 |
| | MKN-B | 30 | | | 170 |
| | MKN-A | 29 | | | 250 |
| | MKN45 | 7.1 | | | 300 |
| | MKN74 | 32 | | | 310 |
| prostate cancer cells | DU-145 | 41 | | | 490 |
| | PC-3 | 26 | | | 290 |

[Table 5]

| Table 5: antitumor activity [nM] of Compound 18 ($GI_{50}$ value: 50% growth inhibitory concentration) | | | | | |
|---|---|---|---|---|---|
| | | 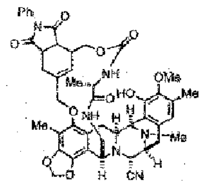 18 | 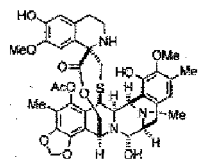 ecteinascidin 743 | 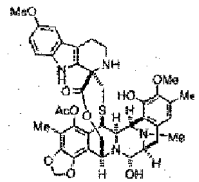 lurbinectedin | 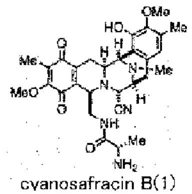 cyanosafracin B(1) |
| breast cancer cells | HBC-4 | 30 | | | 59 |
| | BSY-1 | 19 | | | 34 |
| | HBC-5 | 38 | | | 62 |
| | MCF-7 | 22 | 2.6 | 1.7 | 52 |
| | MDA-MB-231 | 41 | 3.9 | 3.4 | 130 |
| central nervous system cancer cells | U251 | 50 | | | 370 |
| | SF-268 | 74 | | | 370 |
| | SF-295 | 300 | | | 500 |
| | SF-539 | 33 | | | 180 |
| | SNB-75 | 50 | | | 150 |
| | SNB-78 | 40 | | | 63 |
| colorectal cancer cells | HCC2998 | 95 | | | 350 |
| | KM-12 | 100 | | | 530 |
| | HT-29 | 72 | 9.8 | 2.4 | 190 |
| | HCT-15 | 630 | | | 390 |
| | HCT-116 | 74 | 4.9 | 6.4 | 290 |

(continued)

Table 5: antitumor activity [nM] of Compound 18 ($GI_{50}$ value: 50% growth inhibitory concentration)

| | | 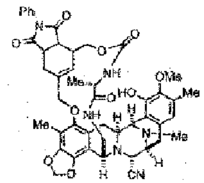 18 | 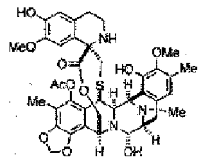 ecteinascidin 743 | 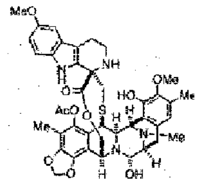 lurbinectedin | 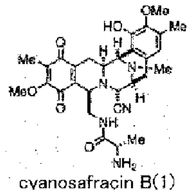 cyanosafracin B(1) |
|---|---|---|---|---|---|
| lung cancer cells | NCI-H23 | 110 | 2.1 | 5.3 | 290 |
| | NCI-H226 | 280 | | | 310 |
| | NCI-H522 | 20 | | | 43 |
| | NCI-H460 | 260 | 1.8 | 1.5 | 400 |
| | A549 | 540 | 10.4 | 1.3 | 1600 |
| | DMS273 | 100 | | | 270 |
| | | 12 | | | 35 |
| melanoma cells | DMS114 LOX-IMVI | 27 | | | 61 |
| cervical cancer cells | OVCAR-3 | 43 | | | 130 |
| | OVCAR-4 | 350 | | | 290 |
| | OVCAR-5 | 280 | | | 390 |
| | OVCAR-8 | 130 | | | 290 |
| | SK-OV-3 | 410 | | | 540 |
| kidney cancer cells | RXF-631L | 310 | | | 400 |
| | ACHN | 240 | | | 320 |

(continued)

Table 5: antitumor activity [nM] of Compound 18 ($GI_{50}$ value: 50% growth inhibitory concentration)

| | | 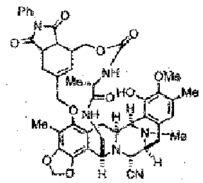 18 | 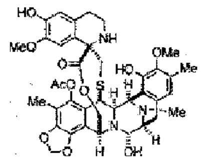 ecteinascidin 743 | 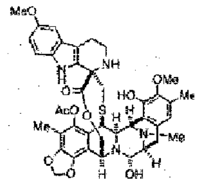 lurbinectedin | 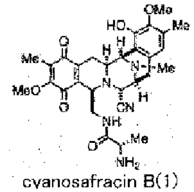 cyanosafracin B(1) |
|---|---|---|---|---|---|
| gastric cancer cells | St-4 | 300 | | | 430 |
| | MKN1 | 50 | | | 52 |
| | MKN-B | 160 | | | 170 |
| | MKN-A | 210 | | | 250 |
| | MKN45 | 78 | | | 300 |
| | MKN74 | 120 | | | 310 |
| prostate cancer cells | DU-145 | 330 | | | 490 |
| | PC-3 | 170 | | | 290 |

[Table 6]

| Table 6: antitumor activity [nM] of Compound 29 ($GI_{50}$ value: 50% growth inhibitory concentration) | | | | | |
|---|---|---|---|---|---|
| | | 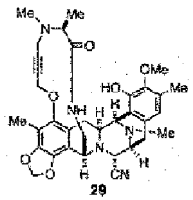 29 | 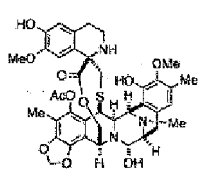 ecteinascidin 743 | 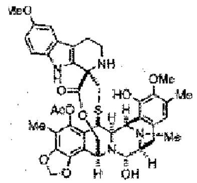 lurbinectedin | 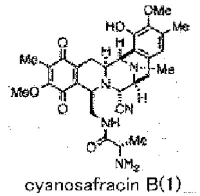 cyanosafracin B(1) |
| breast cancer cells | HBC-4 | 1.5 | | | 59 |
| | BSY-1 | 0.60 | | | 34 |
| | HBC-5 | 1.8 | | | 62 |
| | MCF-7 | 0.67 | 2.6 | 1.7 | 52 |
| | MDA-MB-231 | 2.3 | 3.9 | 3.4 | 130 |
| central nervous system cancer cells | U251 | 1.4 | | | 370 |
| | SF-268 | 2.8 | | | 370 |
| | SF-295 | 3.4 | | | 500 |
| | SF-539 | 1.3 | | | 180 |
| | SNB-75 | 1.3 | | | 150 |
| | SNB-78 | 3.1 | | | 63 |
| colorectal cancer cells | HCC2998 | 2.8 | | | 350 |
| | KM-12 | 3.1 | | | 530 |
| | HT-29 | 2.0 | 9.8 | 2.4 | 190 |
| | HCT-15 | 3.9 | | | 390 |
| | HCT-116 | 2.4 | 4.9 | 6.4 | 290 |

(continued)

Table 6: antitumor activity [nM] of Compound 29 ($GI_{50}$ value: 50% growth inhibitory concentration)

| | | 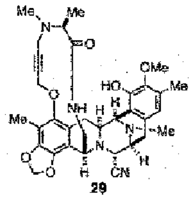 29 | 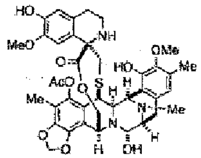 ecteinascidin 743 | 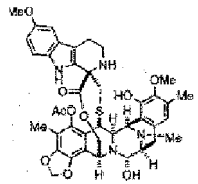 lurbinectedin | 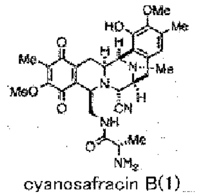 cyanosafracin B(1) |
|---|---|---|---|---|---|
| lung cancer cells | NCI-H23 | 2.6 | 2.1 | 5.3 | 290 |
| | NCI-H226 | 2.5 | | | 310 |
| | NCI-H522 | 0.60 | | | 43 |
| | NCI-H460 | 2.8 | 1.8 | 1.5 | 400 |
| | A549 | 3.4 | 10.4 | 1.3 | 1600 |
| | DMS273 | 1.1 | | | 270 |
| | | 0.89 | | | 35 |
| melanoma cells | DMS114 LOX-IMVI | 0.59 | | | 61 |
| cervical cancer cells | OVCAR-3 | 2.6 | | | 130 |
| | OVCAR-4 | 3.5 | | | 290 |
| | OVCAR-5 | 3.2 | | | 390 |
| | OVCAR-8 | 2.6 | | | 290 |
| | SK-OV-3 | 5.7 | | | 540 |
| kidney cancer cells | RXF-631L | 3.1 | | | 400 |
| | ACHN | 2.2 | | | 320 |

(continued)

Table 6: antitumor activity [nM] of Compound 29 ($GI_{50}$ value: 50% growth inhibitory concentration)

| | | 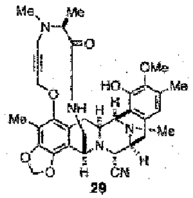 29 | 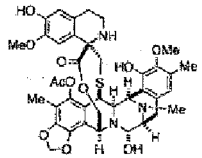 ecteinascidin 743 | 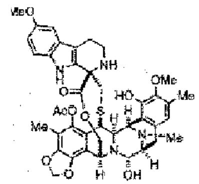 lurbinectedin | 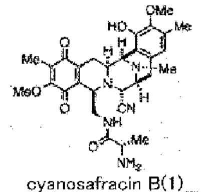 cyanosafracin B(1) |
|---|---|---|---|---|---|
| gastric cancer cells | St-4 | 3.8 | | | 430 |
| | MKN1 | 2.3 | | | 52 |
| | MKN-B | 3.0 | | | 170 |
| | MKN-A | 3.5 | | | 250 |
| | MKN45 | 1.3 | | | 300 |
| | MKN74 | 3.7 | | | 310 |
| prostate cancer cells | DU-145 | 3.5 | | | 490 |
| | PC-3 | 3.0 | | | 290 |

[Table 7]

| Table 7: antitumor activity [nM] of Compound 33 ($GI_{50}$ value: 50% growth inhibitory concentration) | | | | | |
|---|---|---|---|---|---|
| | | 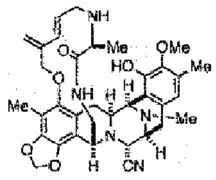 33 | 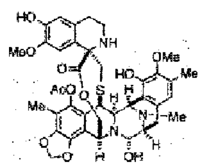 ecteinascidin 743 | 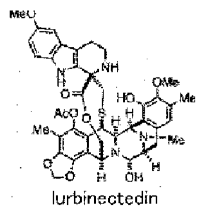 lurbinectedin | 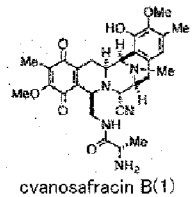 cyanosafracin B(1) |
| breast cancer cells | HBC-4 | 32 | | | 59 |
| | BSY-1 | 18 | | | 34 |
| | HBC-5 | 40 | | | 62 |
| | MCF-7 | 26 | 2.6 | 1.7 | 52 |
| | MDA-MB-231 | 52 | 3.9 | 3.4 | 130 |
| central nervous system cancer cells | U251 | 55 | | | 370 |
| | SF-268 | 110 | | | 370 |
| | SF-295 | 270 | | | 500 |
| | SF-539 | 46 | | | 180 |
| | SNB-75 | 96 | | | 150 |
| | SNB-78 | 83 | | | 63 |
| colorectal cancer cells | HCC2998 | 140 | | | 350 |
| | | 110 | | | 530 |
| | HT-29 | 86 | 9.8 | 2.4 | 190 |
| | HCT-15 | 400 | | | 390 |
| | HCT-116 | 87 | 4.9 | 6.4 | 290 |

(continued)

| Table 7: antitumor activity [nM] of Compound 33 ($GI_{50}$ value: 50% growth inhibitory concentration) | | | | | |
|---|---|---|---|---|---|
| | | 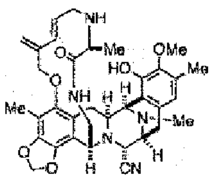 33 | 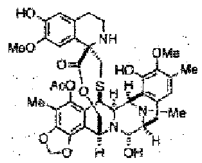 ecteinascidin 743 | 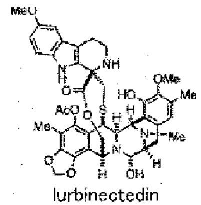 lurbinectedin | 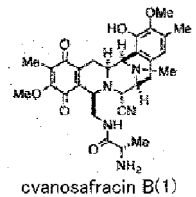 cvanosafracin B(1) |
| lung cancer cells | NCI-H23 | 93 | 2.1 | 5.3 | 290 |
| | NCI-H226 | 260 | | | 310 |
| | NCI-H522 | 25 | | | 43 |
| | NCI-H460 | 260 | 1.8 | 1.5 | 400 |
| | A549 | 360 | 10.4 | 1.3 | 1600 |
| | DMS273 | 140 | | | 270 |
| | DMS114 | 31 | | | 35 |
| melanoma cells | LOX-IMVI | 31 | | | 61 |
| cervical cancer cells | OVCAR-3 | 70 | | | 130 |
| | OVCAR-4 OVCAR-5 | 400 | | | 290 |
| | | 310 | | | 390 |
| | OVCAR-8 | 180 | | | 290 |
| | SK-OV-3 | 500 | | | 540 |
| kidney cancer cells | RXF-631L | 300 | | | 400 |
| | ACHN | 260 | | | 320 |

(continued)

| Table 7: antitumor activity [nM] of Compound 33 ($GI_{50}$ value: 50% growth inhibitory concentration) | | | | | |
|---|---|---|---|---|---|
| | | 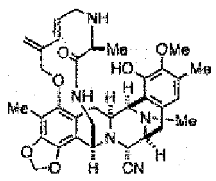 33 | 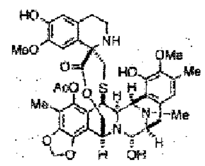 ecteinascidin 743 | 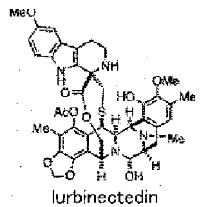 lurbinectedin | 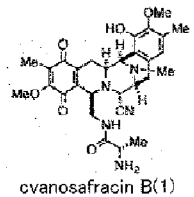 cyanosafracin B(1) |
| gastric cancer cells | St-4 | 270 | | | 430 |
| | MKN1 | 68 | | | 52 |
| | MKN-B | 220 | | | 170 |
| | MKN-A | 330 | | | 250 |
| | MKN45 | 41 | | | 300 |
| | MKN74 | 260 | | | 310 |
| prostate cancer cells | DU-145 | 370 | | | 490 |
| | PC-3 | 190 | | | 290 |

[Table 8]

| Table 8: antitumor activity [nM] of Compound 35 ($GI_{50}$ value: 50% growth inhibitory concentration) | | | | | |
|---|---|---|---|---|---|
| | | 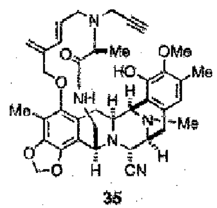 35 | 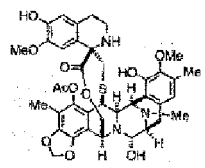 ecteinascidin 743 | 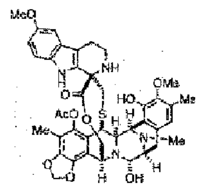 lurbinectedin | 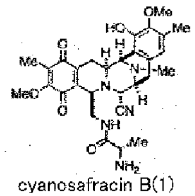 cyanosafracin B(1) |
| breast cancer cells | HBC-4 | 2.2 | | | 59 |
| | BSY-1 | 1.3 | | | 34 |
| | HBC-5 | 2.7 | | | 62 |
| | MCF-7 | 0.87 | 2.6 | 1.7 | 52 |
| | MDA-MB-231 | 2.7 | 3.9 | 3.4 | 130 |
| central nervous system cancer cells | U251 | 2.5 | | | 370 |
| | SF-268 | 3.3 | | | 370 |
| | SF-295 | 3.8 | | | 500 |
| | SF-539 | 2.2 | | | 180 |
| | SNB-75 | 1.7 | | | 150 |
| | SNB-78 | 3.4 | | | 63 |
| colorectal cancer cells | HCC2998 | 3.6 | | | 350 |
| | KM-12 | 3.8 | | | 530 |
| | HT-29 | 3.2 | 9.8 | 2.4 | 190 |
| | HCT-15 | 4.5 | | | 390 |
| | HCT-116 | 2.7 | 4.9 | 6.4 | 290 |

(continued)

Table 8: antitumor activity [nM] of Compound 35 ($GI_{50}$ value: 50% growth inhibitory concentration)

| | | 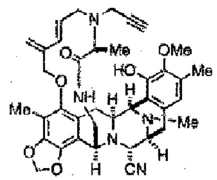 35 | 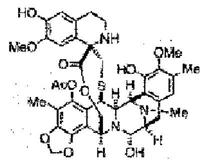 ecteinascidin 743 | 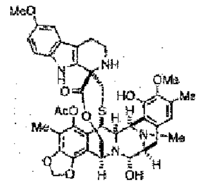 lurbinectedin | 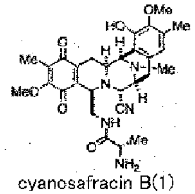 cyanosafracin B(1) |
|---|---|---|---|---|---|
| lung cancer cells | NCI-H23 | 3.5 | 2.1 | 5.3 | 290 |
| | NCI-H226 | 2.7 | | | 310 |
| | NCI-H522 | 1.1 | | | 43 |
| | NCk-H460 | 3.4 | 1.8 | 1.5 | 400 |
| | A549 | 7.2 | 10.4 | 1.3 | 1600 |
| | DMS273 | 2.5 | | | 270 |
| | PMS114 | 1.2 | | | 35 |
| melanoma cells | LOX-IMVI | 1.7 | | | 61 |
| cervical cancer cells | OVCAR-3 | 3.2 | | | 130 |
| | OVCAR-4 | 3.6 | | | 290 |
| | OVCAR-5 | 3.3 | | | 390 |
| | OVCAR-8 | 3.1 | | | 290 |
| | SK-OV-3 | 6.5 | | | 540 |
| kidney cancer cells | RXF-631 | 2.9 | | | 400 |
| | ACHN | 3.1 | | | 320 |

(continued)

Table 8: antitumor activity [nM] of Compound 35 ($GI_{50}$ value: 50% growth inhibitory concentration)

| | | 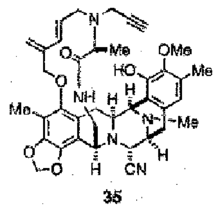 35 | 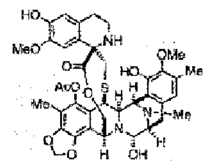 ecteinascidin 743 | 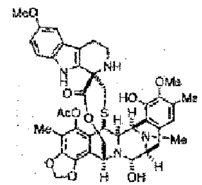 lurbinectedin | 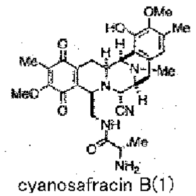 cyanosafracin B(1) |
|---|---|---|---|---|---|
| gastric cancer cells | St-4 | 3.8 | | | 430 |
| | MKN1 | 2.7 | | | 52 |
| | MKN-B | 3.5 | | | 170 |
| | MKN-A | 3.7 | | | 250 |
| | MKN45 | 2.7 | | | 300 |
| | MKN74 | 4.5 | | | 310 |
| prostate cancer cells | DU-145 | 3.8 | | | 490 |
| | PC-3 | 3.3 | | | 290 |

[Table 9]

| Table 9: antitumor activity [nM] of Compound 36 ($GI_{50}$ value: 50% growth inhibitory concentration) | | | | | |
|---|---|---|---|---|---|
| | | 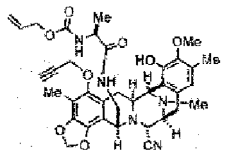 36 | 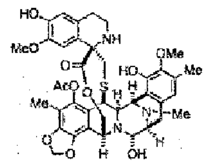 ecteinascidin 743 | 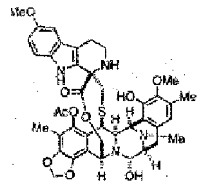 lurbinectedin | 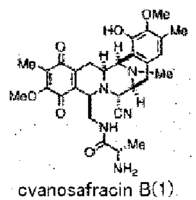 cyanosafracin B(1) |
| breast cancer cells | HBC-4 | 3.5 | | | 59 |
| | BSY-1 | 3.3 | | | 34 |
| | HBC-5 | 7.6 | | | 62 |
| | MCF-7 | 2.9 | 2.6 | 1.7 | 52 |
| | MDA-MB-231 | 4.4 | 3.9 | 3.4 | 130 |
| central nervous system cancer cells | U251 | 4.9 | | | 370 |
| | SF-268 | 6.3 | | | 370 |
| | SF-295 | 17 | | | 500 |
| | SF-539 | 4.3 | | | 180 |
| | SNB-75 | 7 | | | 150 |
| | SNB-78 | 5.7 | | | 63 |
| colorectal cancer cells | HCC2998 | 6.9 | | | 350 |
| | KM-12 | 9 | | | 530 |
| | HT-29 | 5.7 | 9.8 | 2.4 | 190 |
| | HCT-15 | 29 | | | 390 |
| | HCT-116 | 5.3 | 4.9 | 6.4 | 290 |

(continued)

Table 9: antitumor activity [nM] of Compound 36 ($GI_{50}$ value: 50% growth inhibitory concentration)

| | | 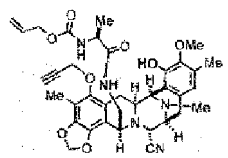 36 | 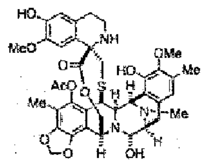 ecteinascidin 743 | 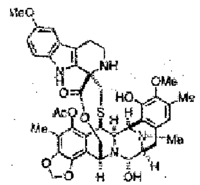 lurbinectedin | 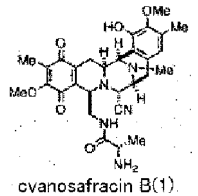 cyanosafracin B(1) |
|---|---|---|---|---|---|
| lung cancer cells | NCI-H23 | 13 | 2.1 | 5.3 | 290 |
| | NCI-H226 | 23 | | | 310 |
| | NCI-H522 | 2.4 | | | 43 |
| | NCI-H460 | 30 | 1.8 | 1.5 | 400 |
| | A549 | 38 | 10.4 | 1.3 | 1600 |
| | DMS273 | 6.8 | | | 270 |
| | DMS114 | 3 | | | 35 |
| melanoma cells | LOX-\|MV\| | 2.8 | | | 61 |
| cervical cancer cells | OVCAR-3 | 4.8 | | | 130 |
| | OVCAR-4 | 25 | | | 290 |
| | OVCAR-5 | 20 | | | 390 |
| | OVCAR-8 | 7.9 | | | 290 |
| | SK-OV-3 | 25 | | | 540 |
| kidney cancer cells | RXF-631L | 39 | | | 400 |
| | ACHN | 22 | | | 320 |

(continued)

Table 9: antitumor activity [nM] of Compound 36 ($GI_{50}$ value: 50% growth inhibitory concentration)

| | | 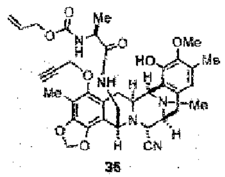 36 | 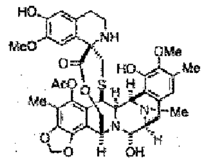 ecteinascidin 743 | 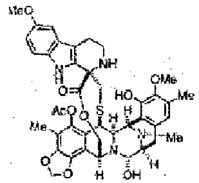 lurbinectedin | 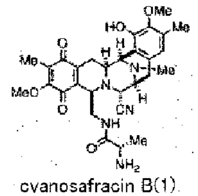 cyanosafracin B(1) |
|---|---|---|---|---|---|
| gastric cancer cells | St-4 | 25 | | | 430 |
| | | 4.1 | | | 52 |
| | MKN1 MKN-B | 25 | | | 170 |
| | MKN-A | 15 | | | 250 |
| | MKN45 | 4.2 | | | 300 |
| | MKN74 | 20 | | | 310 |
| prostate cancer cells | DU-145 | 32 | | | 490 |
| | PC-3 | 17 | | | 290 |

[Table 10]

| Table 10: antitumor activity [nM] of Compound 37 ($GI_{50}$ value: 50% growth inhibitory concentration) | | | | | |
|---|---|---|---|---|---|
| | | 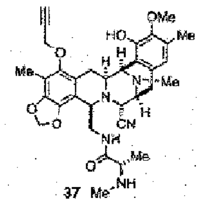 37 | 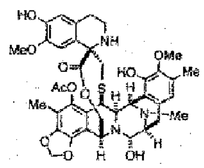 ecteinascidin 743 | 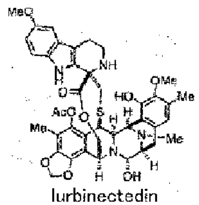 lurbinectedin | 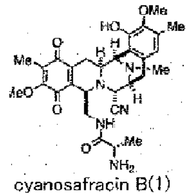 cyanosafracin B(1) |
| breast cancer cells | HBC-4 | 42 | | | 59 |
| | BSY-1 | 26 | | | 34 |
| | HBC-5 | 100 | | | 62 |
| | MCF-7 | 33 | 2.6 | 1.7 | 52 |
| | MDA-MB-231 | 77 | 3.9 | 3.4 | 130 |
| central nervous system cancer cells | U251 | 110 | | | 370 |
| | ST-268 | 250 | | | 370 |
| | SF-295 | 460 | | | 500 |
| | SF-539 | 69 | | | 180 |
| | SNB-75 | 100 | | | 150 |
| | SNB78 | 92 | | | 63 |
| colorectal cancer cells | HCC2998 | 210 | | | 350 |
| | KM-12 | 200 | | | 530 |
| | HT-29 | 93 | 9.8 | 2.4 | 190 |
| | HCT-15 | 480 | | | 390 |
| | HCT-116 | 140 | 4.9 | 6.4 | 290 |

(continued)

| Table 10: antitumor activity [nM] of Compound 37 ($GI_{50}$ value: 50% growth inhibitory concentration) | | | | | |
|---|---|---|---|---|---|
| | | 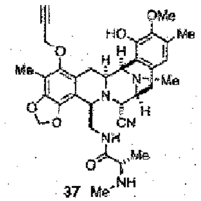 37 | 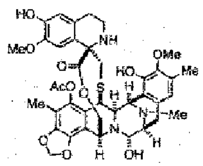 ecteinascidin 743 | 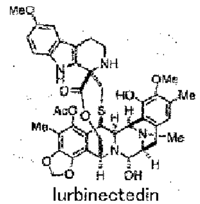 lurbinectedin | 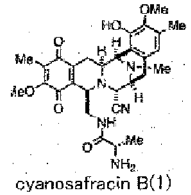 cyanosafracin B(1) |
| lung cancer cells | NCI-H23 | 270 | 2.1 | 5.3 | 290 |
| | NCI-H226 | 280 | | | 310 |
| | NCI-H522 | 28 | | | 43 |
| | NCI-H460 | 410 | 1.8 | 1.5 | 400 |
| | A549 | 1500 | 10.4 | 1.3 | 1600 |
| | DMS273 | 270 | | | 270 |
| | DMS114 | 29 | | | 35 |
| melanoma cells | LOX-\|MV\| | 40 | | | 61 |
| cervical cancer cells | OVCAR-3 | 160 | | | 130 |
| | OVCAR-4 | 450 | | | 290 |
| | OVCAR-5 | 440 | | | 390 |
| | OVCAR-8 | 230 | | | 290 |
| | SK-OV-3 | 560 | | | 540 |
| kidney cancer cells ACHN | RXF-631L | 370 | | | 400 |
| | ACHN | 260 | | | 320 |

(continued)

| Table 10: antitumor activity [nM] of Compound 37 ($GI_{50}$ value: 50% growth inhibitory concentration) | | | | | |
|---|---|---|---|---|---|
| | | 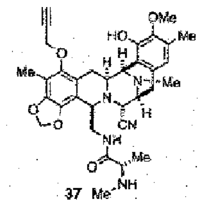 37 | 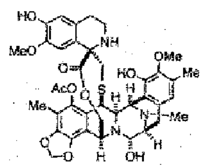 ecteinascidin 743 | 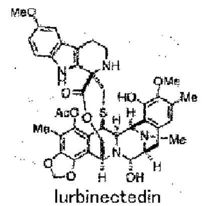 lurbinectedin | 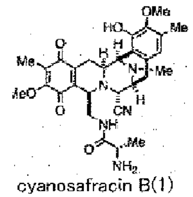 cyanosafracin B(1) |
| gastric cancer cells | St-4 | 400 | | | 430 |
| | MKN1 | 220 | | | 52 |
| | MKN-B | 340 | | | 170 |
| | MKN-A | 370 | | | 250 |
| | MKN45 | 180 | | | 300 |
| | MKN74 | 330 | | | 310 |
| prostate cancer cells | DU-145 | 440 | | | 490 |
| | PC-3 | 350 | | | 290 |

[Table 11]

| Table 11: antitumor activity [nM] of Compound 42 ($GI_{50}$ value: 50% growth inhibitory concentration) | | | | | |
|---|---|---|---|---|---|
| | | 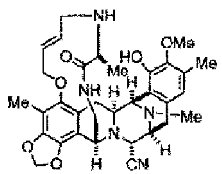 42 | 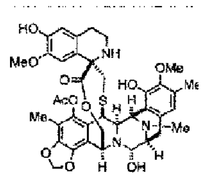 ecteinascidin 743 | 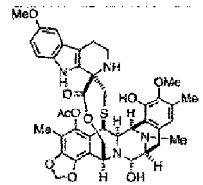 lurbinectedin | 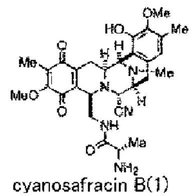 cyanosafracin B(1) |
| breast cancer cells | HBC-4 | <10 | | | 59 |
| | BSY-1 | <10 | | | 34 |
| | HBC-5 | 17 | | | 62 |
| | MCF-7 | <10 | 2.6 | 1.7 | 52 |
| | MDA-MB-231 | <10 | 3.9 | 3.4 | 130 |
| central nervous system cancer cells | U251 | 13 | | | 370 |
| | SF-268 | 27 | | | 370 |
| | SF-295 | 38 | | | 500 |
| | SF-539 | 17 | | | 180 |
| | SNB-75 | <10 | | | 150 |
| | SNB-78 | 11 | | | 63 |
| colorectal cancer cells | HCC2998 | 18 | | | 350 |
| | KM-12 | 19 | | | 530 |
| | HT-29 | 13 | 9.8 | 2.4 | 190 |
| | HCT-15 | 47 | | | 390 |
| | HCT-116 | 21 | 4.9 | 6.4 | 290 |

(continued)

| Table 11: antitumor activity [nM] of Compound 42 ($GI_{50}$ value: 50% growth inhibitory concentration) | | | | | |
|---|---|---|---|---|---|
| | | 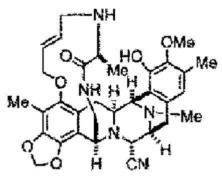 42 | 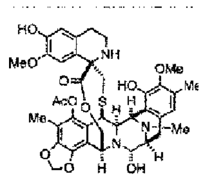 ecteinascidin 743 | 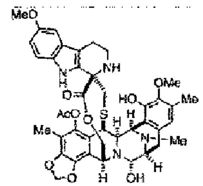 lurbinectedin | 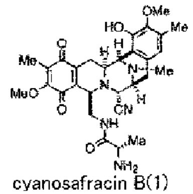 cyanosafracin B(1) |
| lung cancer cells | NCI-H23 | 31 | 2.1 | 5.3 | 290 |
| | NCI-H226 | 30 | | | 310 |
| | NCI-H522 | <10 | | | 43 |
| | NCI-H460 | 22 | 1.8 | 1.5 | 400 |
| | A549 | 110 | 10.4 | 1.3 | 1600 |
| | DMS273 | 16 | | | 270 |
| | DMS114 | <10 | | | 35 |
| melanoma cells | LOX-\|MV\| | <10 | | | 61 |
| cervical cancer cells | OVCAR-3 | 15 | | | 130 |
| | OVCAR-4 | 55 | | | 290 |
| | OVCAR-5 | 47 | | | 390 |
| | OVCAR-8 | 34 | | | 290 |
| | SK-OV-3 | 49 | | | 540 |
| kidney cancer cells | RXF-631L | 34 | | | 400 |
| | ACHN | 42 | | | 320 |

(continued)

Table 11: antitumor activity [nM] of Compound 42 ($GI_{50}$ value: 50% growth inhibitory concentration)

| | | 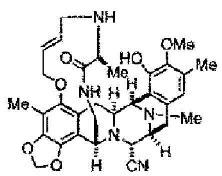 42 | 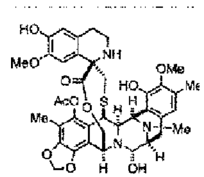 ecteinascidin 743 | 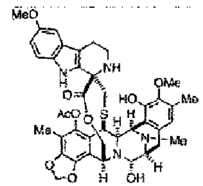 lurbinectedin | 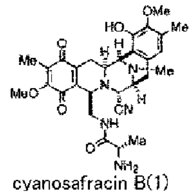 cyanosafracin B(1) |
|---|---|---|---|---|---|
| gastric cancer cells | St-4 | 37 | | | 430 |
| | MKN1 | 18 | | | 52 |
| | MKN-B | 25 | | | 170 |
| | MKN-A | 36 | | | 250 |
| | MKN45 | 16 | | | 300 |
| | MKN74 | 35 | | | 310 |
| prostate cancer cells | DU-145 | 58 | | | 490 |
| | PC-3 | 40 | | | 290 |

[Table 12]

| Table 12: antitumor activity [nM] of Compound 44 ($GI_{50}$ value: 50% growth inhibitory concentration) | | | | | |
|---|---|---|---|---|---|
| | | 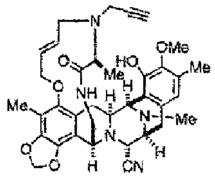 44 | 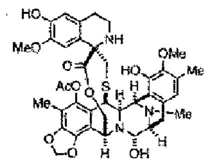 ecteinascidin 743 | 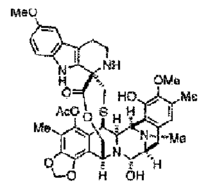 lurbinectedin | 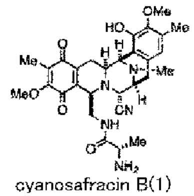 cyanosafracin B(1) |
| breast cancer cells | HBC-4 | <10 | | | 59 |
| | BSY-1 | <10 | | | 34 |
| | HBC-5 | <10 | | | 62 |
| | MCF-7 | <10 | 2.6 | 1.7 | 52 |
| | MDA-MB-231 | <10 | 3.9 | 3.4 | 130 |
| central nervous system cancer cells | U251 | <10 | | | 370 |
| | SF-268 | <10 | | | 370 |
| | SF-295 | <10 | | | 500 |
| | SF-539 | <10 | | | 180 |
| | SNB-75 | <10 | | | 150 |
| | SNB-78 | <10 | | | 63 |
| colorectal cancer cells | HCC2998 | <10 | | | 350 |
| | KM-12 | <10 | | | 530 |
| | HT-29 | <10 | 9.8 | 2.4 | 190 |
| | HCT-15 | <10 | | | 390 |
| | HCT-116 | <10 | 4.9 | 6.4 | 290 |

(continued)

Table 12: antitumor activity [nM] of Compound 44 ($GI_{50}$ value: 50% growth inhibitory concentration)

| | | 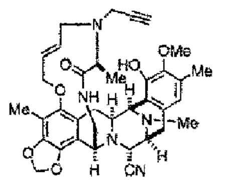 44 | 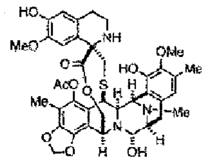 ecteinascidin 743 | 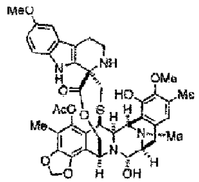 lurbinectedin | 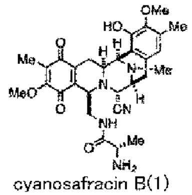 cyanosafracin B(1) |
|---|---|---|---|---|---|
| lung cancer cells | NCI-H23 | <10 | 2.1 | 5.3 | 290 |
| | NCI-H226 | <10 | | | 310 |
| | NCI-H522 | <10 | | | 43 |
| | NCI-H460 | <10 | 1.8 | 1.5 | 400 |
| | A549 | <10 | 10.4 | 1.3 | 1600 |
| | DMS273 | <10 | | | 270 |
| | DMS114 | <10 | | | 35 |
| melanoma cells | LOX-\|MV\| | <10 | | | 61 |
| cervical cancer cells | OVCAR-3 | <10 | | | 130 |
| | OVCAR-4 | <10 | | | 290 |
| | OVCAR-5 | <10 | | | 390 |
| | OVCAR-8 | <10 | | | 290 |
| | SK-OV-3 | <10 | | | 540 |
| kidney cancer cells | RXF-631L | <10 | | | 400 |
| | ACHN | <10 | | | 320 |

(continued)

Table 12: antitumor activity [nM] of Compound 44 ($GI_{50}$ value: 50% growth inhibitory concentration)

| | | 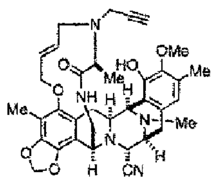 44 | 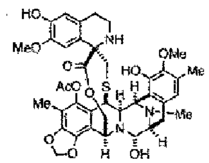 ecteinascidin 743 | 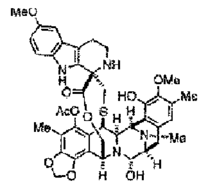 lurbinectedin | 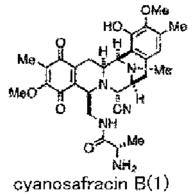 cyanosafracin B(1) |
|---|---|---|---|---|---|
| gastric cancer cells | St-4 | <10 | | | 430 |
| | MKN1 | <10 | | | 52 |
| | MKN-B | <10 | | | 170 |
| | MKN-A | <10 | | | 250 |
| | MKN45 | <10 | | | 300 |
| | MKN74 | <10 | | | 310 |
| prostate cancer cells | DU-145 | <10 | | | 490 |
| | PC-3 | <10 | | | 290 |

[Table 13]

| Table 13: antitumor activity [nM] of Compound 47 ($GI_{50}$ value: 50% growth inhibitory concentration) | | | | | |
|---|---|---|---|---|---|
| | | 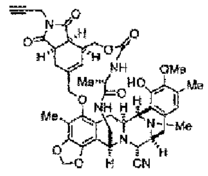 47 | 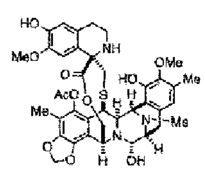 ecteinascidin 743 | 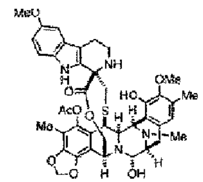 lurbinectedin | 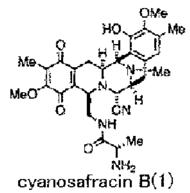 cyanosafracin B(1) |
| breast cancer cells | HBC-4 | 200 | | | 59 |
| | BSY-1 | 150 | | | 34 |
| | HBC-5 | 300 | | | 62 |
| | MCF-7 | 63 | 2.6 | 1.7 | 52 |
| | MDA-MB-231 | 160 | 3.9 | 3.4 | 130 |
| central nervous system cancer cells | U251 | 350 | | | 370 |
| | SF-268 | 620 | | | 370 |
| | SF-295 | 1800 | | | 500 |
| | SF-539 | 290 | | | 180 |
| | SNB-75 | 330 | | | 150 |
| | SNB-78 | 260 | | | 63 |
| colorectal cancer cells | HCC2998 | 410 | | | 350 |
| | KM-12 | 450 | | | 530 |
| | HCT-29 | 490 | 9.8 | 2.4 | 190 |
| | HCT-15 | 2200 | | | 390 |
| | HCT-116 | 450 | 4.9 | 6.4 | 290 |

(continued)

Table 13: antitumor activity [nM] of Compound 47 ($GI_{50}$ value: 50% growth inhibitory concentration)

| | | 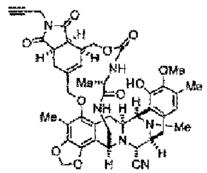 47 | 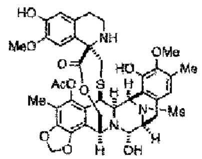 ecteinascidin 743 | 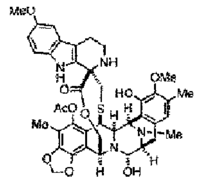 lurbinectedin | 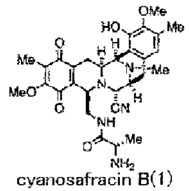 cyanosafracin B(1) |
|---|---|---|---|---|---|
| lung cancer cells | NCI-H23 | 850 | 2.1 | 5.3 | 290 |
| | NCI-H226 | 2300 | | | 310 |
| | NCI-H522 | 29 | | | 43 |
| | NCI-H460 | 630 | 1.8 | 1.5 | 400 |
| | A549 | 3900 | 10.4 | 1.3 | 1600 |
| | DMS273 | 450 | | | 270 |
| | DMS114 | 31 | | | 35 |
| melanoma cells: | LOX-IMVI | 160 | | | 61 |
| cervical cancer cells | OVCAR-3 | 330 | | | 130 |
| | OVCAR-4 | 3500 | | | 290 |
| | OVCAR-5 | 2800 | | | 390 |
| | OVCAR-8 | 1100 | | | 290 |
| | SK-OV-3 | 3900 | | | 540 |
| kidney cancer cells | RXF-631L | 2100 | | | 400 |
| | ACHN | 2800 | | | 320 |

(continued)

| Table 13: antitumor activity [nM] of Compound 47 ($GI_{50}$ value: 50% growth inhibitory concentration) | | | | | |
|---|---|---|---|---|---|
| | | 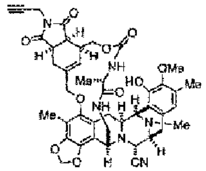 47 | 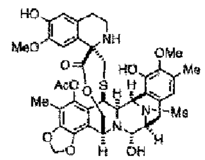 ecteinascidin 743 | 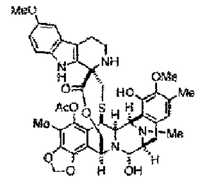 lurbinectedin | 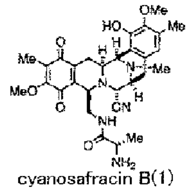 cyanosafracin B(1) |
| gastric cancer cells | St-4 | 2200 | | | 430 |
| | MKN1 | 290 | | | 52 |
| | MKN-B | 730 | | | 170 |
| | MKN-A | 1400 | | | 250 |
| | MKN45 | 470 | | | 300 |
| | MKN74 | 1600 | | | 310 |
| prostate cancer cells | DU-145 | 3900 | | | 490 |
| | PC-3 | 1500 | | | 290 |

Table 1-13 also shows the $GI_{50}$ value of Cyanosafracin B as a comparative example. Based on the literature (Takahashi, N.; Li, W.; Bertino, J. R. et al. Clin. Cancer Res. 2001, 7, 2908-2911.), for the numerical value of Cyanosafracin B, the activity was evaluated by the same procedure as in a compound group for this application. As shown in the above table, it was found that Compounds 7, 13, 14, 16, 18, 29, 33, 35, 42, 44, and 47, which are THIQ compounds containing a macrocyclic structure of the present invention, exhibited better antitumor activity than Cyanosafracin B. In particular, it was found that Compounds 29 and 35 had stronger antitumor activity for a plurality of cell lines than Ecteinascidin 743 or lurbinectedin.

In addition, it was found that Compounds 36 and 37, which are a THIQ alkaloid compound containing no macrocyclic structure (compound having substituents at the 1-position and 5-position of a THIQ framework), also exhibited better antitumor activity than Cyanosafracin B.

The above results verified that the THIQ alkaloid compound of the present invention exhibited an excellent DNA alkylating ability and strong antitumor activity. In addition, since all of the compounds of the present invention synthesized above had functional groups on the macrocycle that could be easily modified, it was possible to further improve the antitumor activity according to tuning of the structure. Therefore, it was apparent that the compounds of the present invention were beneficial for developing novel antitumor drugs.

The scope of the present invention is not limited to the above description, and examples other than the above can be appropriately modified and implemented as long as the gist of the present invention is not impaired. Here, all documents and publications described in this specification are incorporated herein by reference in their entirety regardless of their purpose. In addition, this specification includes content of disclosure of the claims, specification, and drawings of Japanese Patent Application No. 2021-033773 (filed on March 3, 2021), which is the basis of which the priority of this application is claimed.

## Claims

1. A compound represented by the following formula (I) or a pharmaceutically acceptable salt thereof:

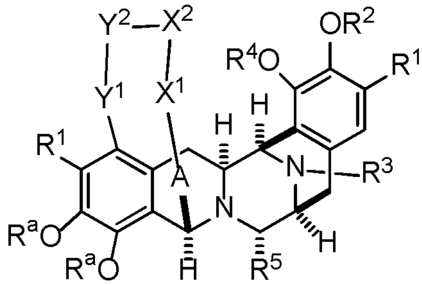

(I)

wherein A is a single bond or an optionally substituted $C_1$-$C_6$ alkylene group;

$X^1$ is a divalent group selected from the group consisting of an optionally substituted alkylene group, an optionally substituted alkenylene group, -$NR^b$C(O)-, -C(O)$NR^b$-, -C(O)O-, -OC(O)-, -$NR^b$C(O)O-, -OC(O)$NR^b$-, -OC(O)O-, a carbonyl group, -C(=S)-, -C(=$NR^b$)-, - $NR^b$-, a sulfonyl group, an ether group, a thioether group, an amino acid residue, and combinations thereof;

$Y^1$ is a divalent group selected from the group consisting of a single bond, an ether group, a thioether group, an optionally substituted $C_1$-$C_6$ alkylene group, and -$NR^b$-;

$Y^2$ is a divalent group selected from the group consisting of an optionally substituted alkylene group, an optionally substituted alkenylene group, -$NR^b$C(O)-, -C(O)$NR^b$-, -C(O)O-, -OC(O)-, -$NR^b$C(O)O-, -OC(O)$NR^b$-, -OC(O)O-, a carbonyl group, -C(=S)-, -C(=$NR^b$)-, - $NR^b$-, a sulfonyl group, an ether group, a thioether group, an amino acid residue, and combinations thereof;

$X^2$ is selected from the group consisting of -$L^1$-C(=$CR^f_2$)-$CR^f$=$CR^f$-$L^2$-, -$L^1$-$CR^f$ $CR^f$-C(=$CR^f_2$)-$L^2$-, -$L^1$-$CR^f$=$CR^f$-$L^2$-, -$L^1$-$CR^f$$CR^f$-$CR^f$=$CR^f$-$L^2$-, -$L^1$-$NR^b$-$CR^f_2$-C≡C-$L^2$-, -$L^1$-C ≡ C-$CR^f_2$-$NR^b$-$L^2$-, -$L^1$-C ≡ C-$L^2$-, and -$L^1$-C ≡ C-C = C-$L^2$-,

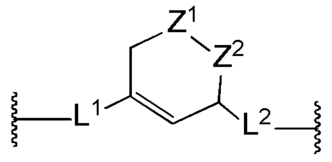

and

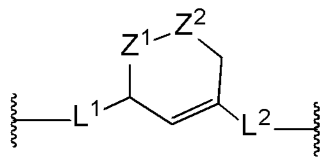

$L^1$ and $L^2$ each independently represent a single bond or a $C_1$-$C_6$ alkylene group,
$R^f$ each independently represents a hydrogen atom, an optionally substituted $C_1$-$C_{20}$ alkyl group or an optionally substituted aryl group,
$Z^1$ and $Z^2$ each independently represent $-NR^c-$ or $-CR^dR^e-$, $R^c$, $R^d$, and $R^e$ are each independently a hydrogen atom or an optionally substituted $C_1$-$C_6$ alkyl group, or $R^c$ and $R^d$ together with $Z^1$ and $Z^2$ to which they are bonded form a 5- or 6-membered ring structure, and the ring structure may be substituted with 1 to 4 substituents;
$R^a$ is each independently a hydrogen atom or an optionally substituted $C_1$-$C_6$ alkyl group or respective $R^a$'s may be taken together to form a ring structure containing an oxygen atom to which they are bonded;
$R^b$ is each independently selected from among a hydrogen atom, an optionally substituted $C_1$-$C_{20}$ alkyl group, an optionally substituted aryl group, an optionally substituted allyl group, a propargyl group, and a nitrogen protecting group,
$R^1$ is a methyl group;
$R^2$ is a hydrogen atom or an optionally substituted $C_1$-$C_6$ alkyl group;
$R^3$ is a methyl group;
$R^4$ is selected from among a hydrogen atom, an optionally substituted $C_1$-$C_6$ alkyl group, an optionally substituted aryl group, an optionally substituted allyl group, a propargyl group, and a protecting group for a phenolic hydroxyl group, and
$R^5$ represents CN, a hydroxyl group or a leaving group containing an oxygen atom, a sulfur atom, a nitrogen atom, or a phosphorus atom.

**2.** The compound according to claim 1, which is represented by the following formula (Ia) or a pharmaceutically acceptable salt thereof:

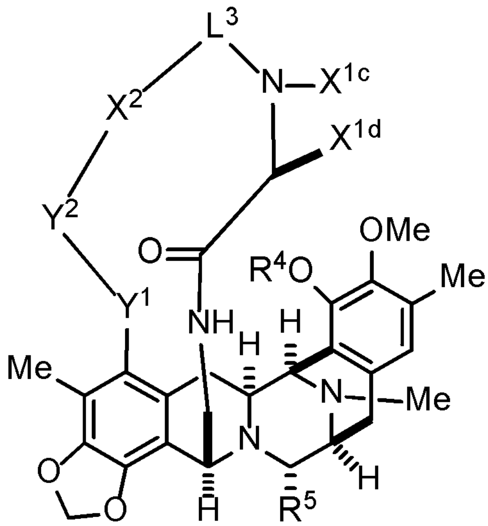

(Ia)

wherein

$X^2$, $Y^1$, $Y^2$, $R^4$, and $R^5$ are as defined in claim 1,
$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,
$X^{1d}$ is selected from among a hydrogen atom, a methyl group, and substituents corresponding to various natural/unnatural amino acid side chains,
$L^3$ is selected from the group consisting of a single bond, an optionally substituted alkylene group, an optionally substituted alkenylene group, a carbonyl group, -C(=S)-, - C(=NR^b)-, -C(O)O-, -C(O)NR^b-, -OC(O)-, -NR^b-, an ether group, a thioether group, and combinations thereof,

$R^b$ is selected from among a hydrogen atom, an optionally substituted $C_1$-$C_{20}$ alkyl group, an optionally substituted aryl group, an optionally substituted allyl group, a propargyl group, and a nitrogen protecting group, and Me represents a methyl group.

**3.** The compound according to claim 1 or 2, which is represented by the following formula (Ic), formula (Id), formula (Ie), formula (If), formula (Ig), or formula (Ih), or a pharmaceutically acceptable salt thereof:

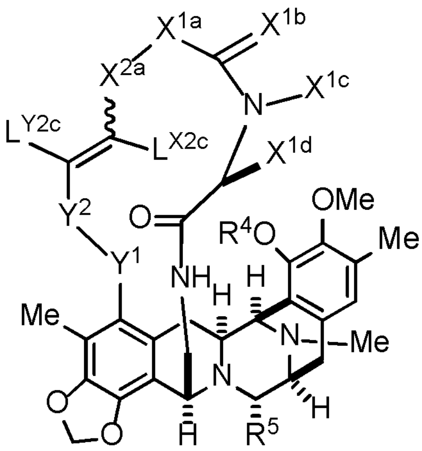

(Ic) ,

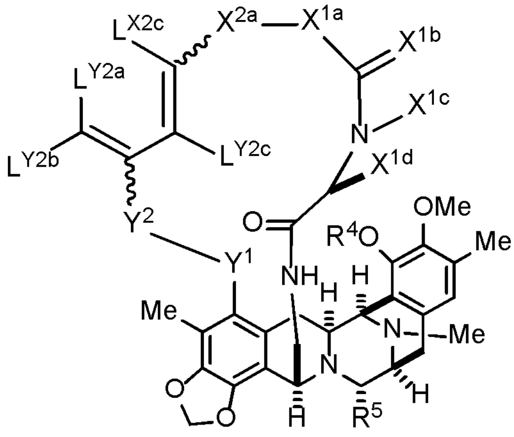

(Id) ,

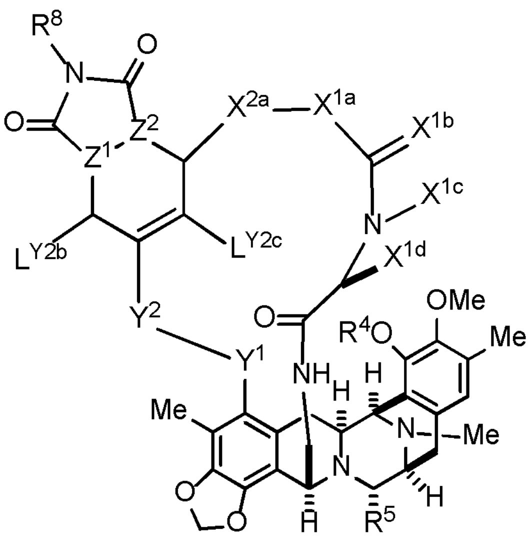

(Ie) ,

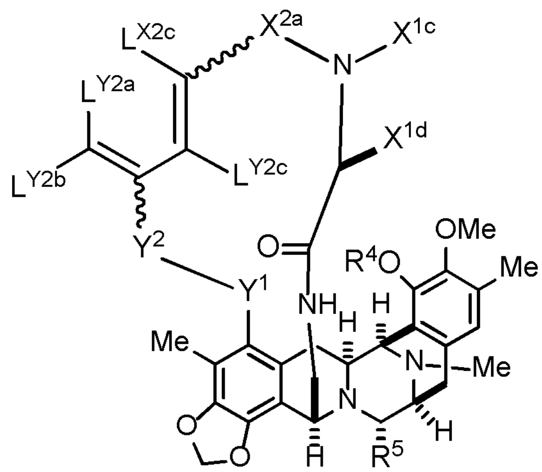

(If) ,

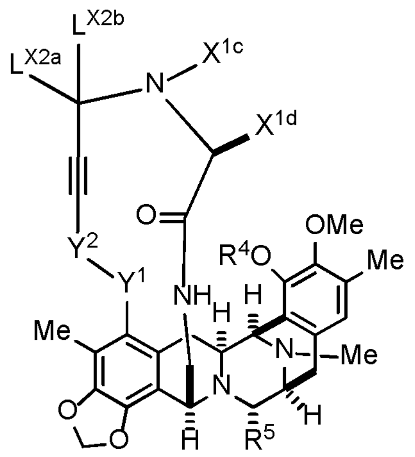

(Ig)

,

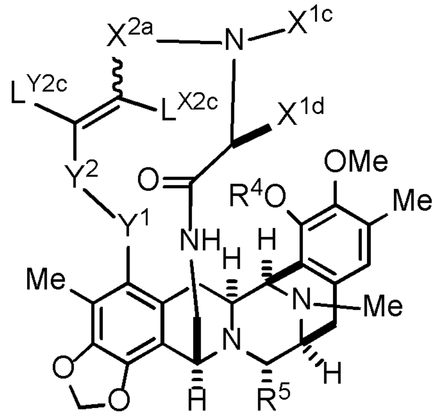

(Ih)

wherein

$X^{1a}$ represents an oxygen atom, a sulfur atom, $-NR^b-$, or an optionally substituted methylene group,
$X^{1b}$ represents an oxygen atom, a sulfur atom, $=NR^b$, or an optionally substituted methylene group,
$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,
$X^{1d}$ is selected from among a hydrogen atom, a methyl group, and substituents corresponding to various natural/unnatural amino acid side chains,
$X^{2a}$ represents an optionally substituted $C_1$-$C_6$ alkylene group,
$L^{X2a}$, $L^{X2b}$ and $L^{X2c}$ each independently represent a hydrogen atom, an optionally substituted $C_1$-$C_{20}$ alkyl group or an optionally substituted aryl group,
$Y^1$ is a divalent group selected from the group consisting of an ether group, a thioether group, an optionally substituted $C_1$-$C_6$ alkylene group, and $-NR^b-$;
$Y^2$ is a divalent group selected from the group consisting of an optionally substituted alkylene group, an optionally substituted alkenylene group, $-NR^bC(O)-$, $-C(O)NR^b-$, $-C(O)O-$, $-OC(O)-$, $-NR^bC(O)O-$, $-OC(O)NR^b-$, $-OC(O)O-$, a carbonyl group, $-C(=S)-$, $-C(=NR^b)-$, $- NR^b-$, a sulfonyl group, an ether group, a thioether group, and an amino acid residue,
$L^{Y2a}$, $L^{Y2b}$ and $L^{Y2c}$ each independently represent a hydrogen atom, an optionally substituted $C_1$-$C_{20}$ alkyl group or an optionally substituted aryl group,
$Z^1$ and $Z^2$ each independently represent N or $CR^e$,
$R^e$ is a hydrogen atom or an optionally substituted $C_1$-$C_6$ alkyl group,
$R^4$ is selected from among a hydrogen atom, an optionally substituted $C_1$-$C_6$ alkyl group, an optionally substituted aryl group, an optionally substituted allyl group, a propargyl group, and a protecting group for a phenolic hydroxyl group,
$R^5$ represents CN, a hydroxyl group, or a leaving group containing an oxygen atom, a sulfur atom, a nitrogen atom, or a phosphorus atom,
$R^8$ represents a hydrogen atom, an optionally substituted aryl group, an optionally substituted $C_1$-$C_{20}$ alkyl group, an optionally substituted allyl group, a propargyl group, or a nitrogen protecting group,
$R^b$ is each independently selected from among a hydrogen atom, an optionally substituted $C_1$-$C_{20}$ alkyl group, an optionally substituted aryl group, an optionally substituted allyl group, a propargyl group, and a nitrogen protecting group, and
Me represents a methyl group.

**4.** The compound according to claim 3 or a pharmaceutically acceptable salt thereof, wherein, in formula (Ic) to formula (Ih),

$X^{1a}$ represents an oxygen atom or $-NR^b-$,
$X^{1b}$ represents an oxygen atom,
$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,
$X^{1d}$ represents a methyl group,
$X^{2a}$ represents a $C_1$-$C_3$ alkylene group,
$L^{X2a}$, $L^{X2b}$ and $L^{X2c}$ each independently represent a hydrogen atom, an optionally substituted $C_1$-$C_8$ alkyl group, or an optionally substituted aryl group,
$Y^1$ represents an ether group;
$Y^2$ represents a $C_1$-$C_3$ alkylene group;
$L^{Y2a}$, $L^{Y2b}$ and $L^{Y2c}$ are each independently selected from among a hydrogen atom, an optionally substituted $C_1$-$C_8$ alkyl group, and an optionally substituted aryl group;
$Z^1$ and $Z^2$ each independently represent N or $CR^e$,
$R^e$ is a hydrogen atom or an optionally substituted $C_1$-$C_6$ alkyl group,
$R^4$ is selected from among a hydrogen atom, an optionally substituted $C_1$-$C_6$ alkyl group, an optionally substituted aryl group, an optionally substituted allyl group, a propargyl group, a propargyl group, and a protecting group for a phenolic hydroxyl group,
$R^5$ represents CN, a hydroxyl group, or a leaving group containing an oxygen atom, a sulfur atom, a nitrogen atom, or a phosphorus atom,
$R^8$ is selected from among a hydrogen atom, an optionally substituted phenyl group, an optionally substituted $C_1$-$C_{20}$ alkyl group, an allyl group, a propargyl group, and a nitrogen protecting group,
$R^b$ is each independently selected from among a hydrogen atom, an optionally substituted $C_1$-$C_{20}$ alkyl group, an optionally substituted aryl group, an optionally substituted allyl group, a propargyl group, and a nitrogen protecting group, and
Me represents a methyl group.

**5.** The compound according to claim 3 or 4 or a pharmaceutically acceptable salt thereof, wherein, in formula (Ic) to formula (Ih),

$X^{1a}$ represents an oxygen atom,
$X^{1b}$ represents an oxygen atom,
$X^{1c}$ is selected from among a hydrogen atom, a methyl group and a propargyl group,
$X^{1d}$ represents a methyl group,
$X^{2a}$ represents a $C_1$-$C_3$ alkylene group,
$L^{X2a}$, $L^{X2b}$ and $L^{X2c}$ each independently represent a hydrogen atom,
$Y^1$ represents an ether group;
$Y^2$ represents a $C_1$-$C_3$ alkylene group;
$L^{Y2a}$, $L^{Y2b}$ and $L^{Y2c}$ each independently represent a hydrogen atom,
$Z^1$ and $Z^2$ each independently represent a nitrogen atom or CH,
$R^4$ represents a hydrogen atom,
$R^5$ represents CN,
$R^8$ represents a hydrogen atom or an optionally substituted phenyl group, and
Me represents a methyl group.

**6.** The compound according to any one of claims 1 to 5, which is selected from the following group, or a pharmaceutically acceptable salt thereof:

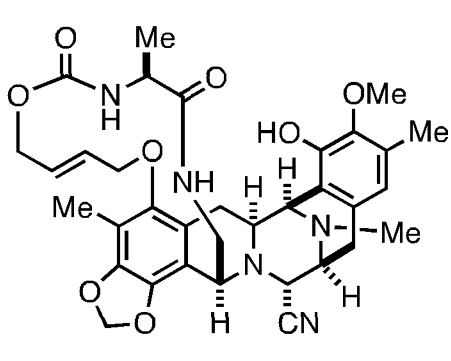
,
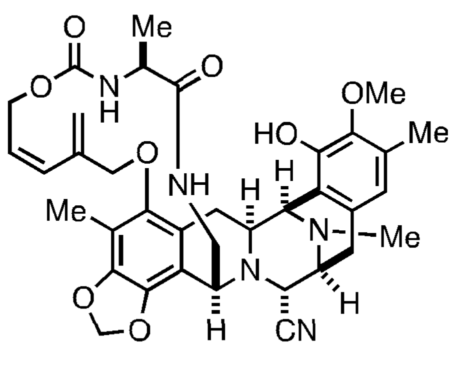
,

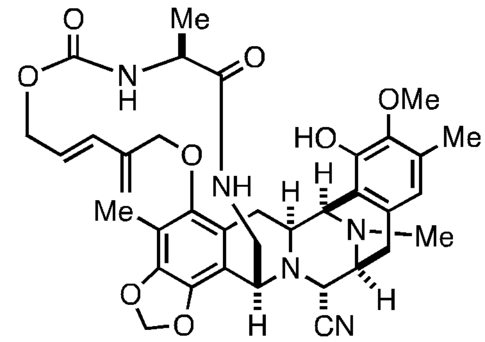

,

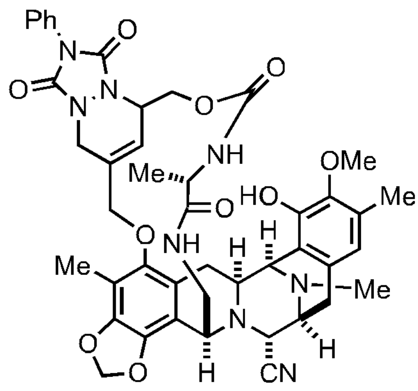

,

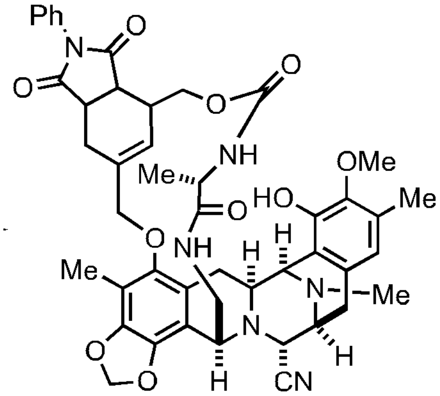

,

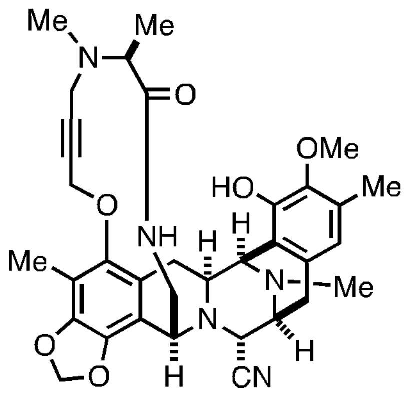

,

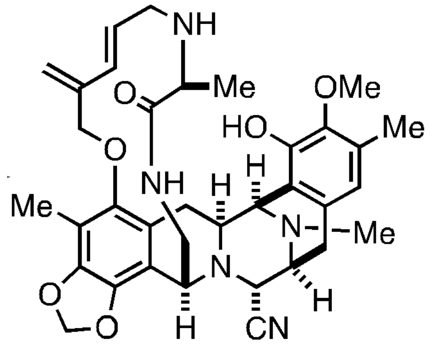

,

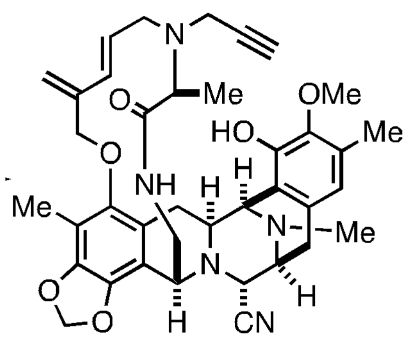

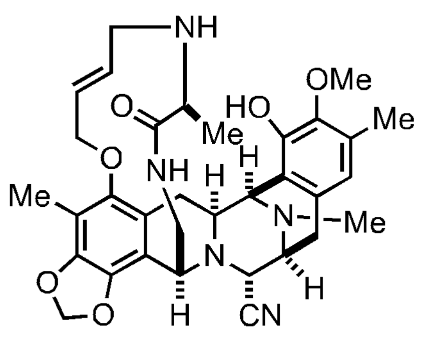

,

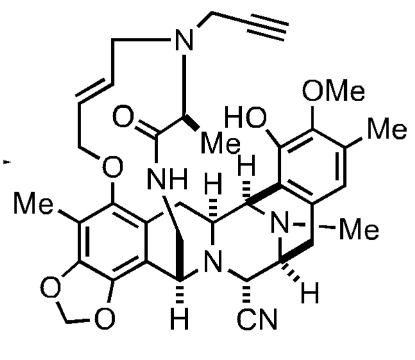

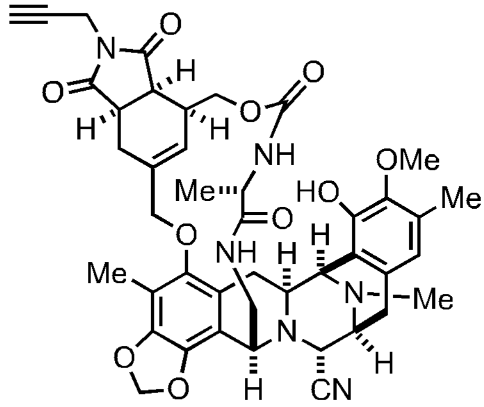

wherein Me represents a methyl group.

**7.** A compound represented by the following formula (IIIc) or a pharmaceutically acceptable salt thereof:

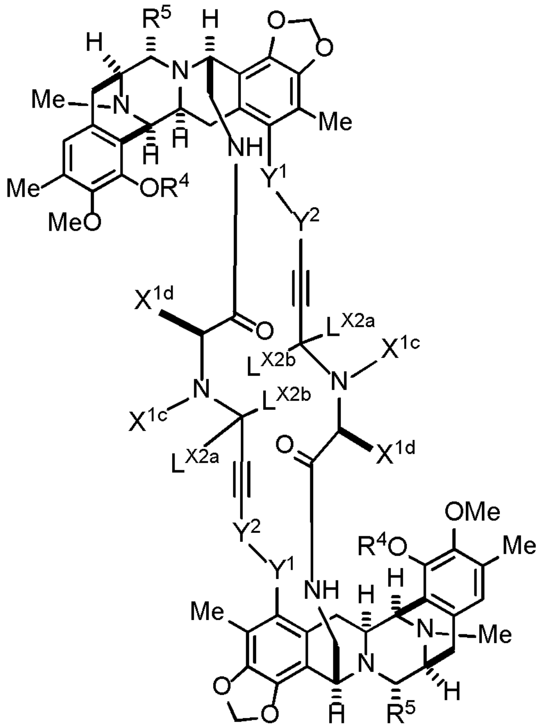

(IIIc)

wherein

$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,
$X^{1d}$ is selected from among a hydrogen atom, a methyl group, and substituents corresponding to various natural/unnatural amino acid side chains,
$L^{X2a}$ and $L^{X2b}$ each independently represent a hydrogen atom, an optionally substituted $C_1$-$C_{20}$ alkyl group or an optionally substituted aryl group,
$Y^1$ is a divalent group selected from the group consisting of an ether group, a thioether group, an optionally substituted $C_1$-$C_6$ alkylene group, and $-NR^b-$;
$Y^2$ is a divalent group selected from the group consisting of an optionally substituted alkylene group, an optionally substituted alkenylene group, $-NR^bC(O)-$, $-C(O)NR^b-$, -C(O)O-, -OC(O)-, $-NR^bC(O)O-$, $-OC(O)NR^b-$, -OC(O)O-, a carbonyl group, -C(=S)-, $-C(=NR^b)-$, $-NR^b-$, a sulfonyl group, an ether group, a thioether group, and an amino

acid residue,

$R^4$ is selected from among a hydrogen atom, an optionally substituted $C_1$-$C_6$ alkyl group, an optionally substituted aryl group, an optionally substituted allyl group, a propargyl group, and a protecting group for a phenolic hydroxyl group,

$R^5$ represents CN, a hydroxyl group, or a leaving group containing an oxygen atom, a sulfur atom, a nitrogen atom, or a phosphorus atom,

$R^b$ is selected from among a hydrogen atom, an optionally substituted $C_1$-$C_{20}$ alkyl group, an optionally substituted aryl group, an optionally substituted allyl group, a propargyl group, and a nitrogen protecting group, and Me represents a methyl group.

**8.** The compound according to claim 7, which is the following compound, or a pharmaceutically acceptable salt thereof:

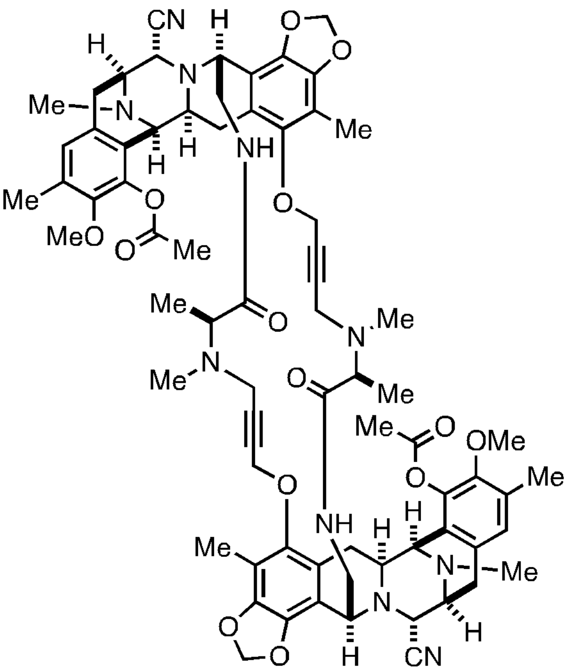

wherein Me represents a methyl group.

**9.** A compound represented by the following formula (II) or a pharmaceutically acceptable salt thereof:

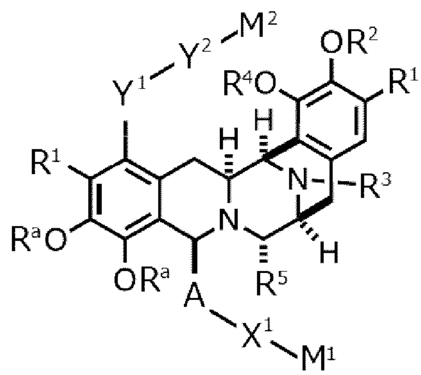

(II)

wherein

A is a single bond or an optionally substituted $C_1$-$C_6$ alkylene group;

$X^1$ is a divalent group selected from the group consisting of an optionally substituted alkylene group, an optionally substituted alkenylene group, $-NR^bC(O)-$, $-C(O)NR^b-$, -C(O)O-, -OC(O)-, $-NR^bC(O)O-$, $-OC(O)NR^b-$, -OC(O)O-, a carbonyl group, -C(=S)-, $-C(=NR^b)-$, $-NR^b-$, a sulfonyl group, an ether group, a thioether group, an amino

acid residue, and combinations thereof;

$Y^1$ is a divalent group selected from the group consisting of a single bond, an ether group, a thioether group, an optionally substituted $C_1$-$C_6$ alkylene group, and $-NR^b-$;

$Y^2$ is a divalent group selected from the group consisting of an optionally substituted alkylene group, an optionally substituted alkenylene group, $-NR^bC(O)-$, $-C(O)NR^b-$, -C(O)O-, -OC(O)-, $-NR^bC(O)O-$, $-OC(O)NR^b-$, -OC(O)O-, a carbonyl group, -C(=S)-, $-C(=NR^b)-$, - $NR^b-$, a sulfonyl group, an ether group, a thioether group, an amino acid residue, and combinations thereof;

$M^1$ is selected from among a hydrogen atom, an optionally substituted alkyl group, an optionally substituted alkenyl group, an optionally substituted alkynyl group, $-N(R^b)_2$, an optionally substituted alkylene-$N(R^b)_2$, a hydroxyl group, a carbonyl group, a thiol group, and a halogen atom;

$M^2$ is selected from among a hydrogen atom, an optionally substituted alkyl group, an optionally substituted alkenyl group, an optionally substituted alkynyl group, $-N(R^b)_2$, an optionally substituted alkylene-$N(R^b)_2$, a hydroxyl group, a carbonyl group, a thiol group, and a halogen atom;

$R^b$ is each independently selected from among a hydrogen atom, an optionally substituted $C_1$-$C_6$ alkyl group, an optionally substituted aryl group, an optionally substituted allyl group, a propargyl group, and a nitrogen protecting group,

$R^a$ is each independently a hydrogen atom or an optionally substituted $C_1$-$C_6$ alkyl group, or respective $R^a$'s may be taken together to form a ring structure containing an oxygen atom to which they are bonded;

$R^1$ is a methyl group;

$R^2$ is a hydrogen atom or an optionally substituted $C_1$-$C_6$ alkyl group;

$R^3$ is a methyl group;

$R^4$ is selected from among a hydrogen atom, an optionally substituted $C_1$-$C_6$ alkyl group, an optionally substituted aryl group, an optionally substituted allyl group, a propargyl group, and a protecting group for a phenolic hydroxyl group, and

$R^5$ represents CN, a hydroxyl group or a leaving group containing an oxygen atom, a sulfur atom, a nitrogen atom, or a phosphorus atom.

**10.** The compound according to claim 9, which is represented by the following formula (IIc), formula (IId), formula (IIe), or formula (IIf) or a pharmaceutically acceptable salt thereof:

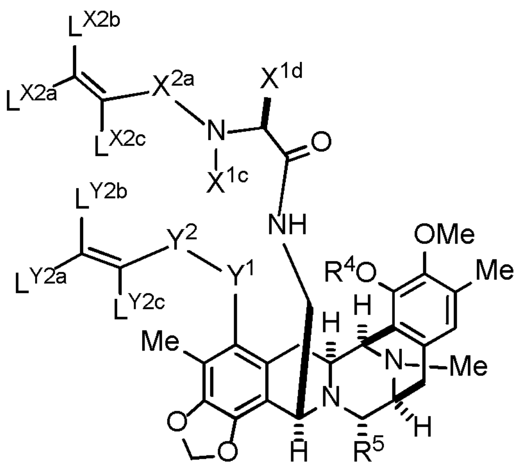

(IIc)

,

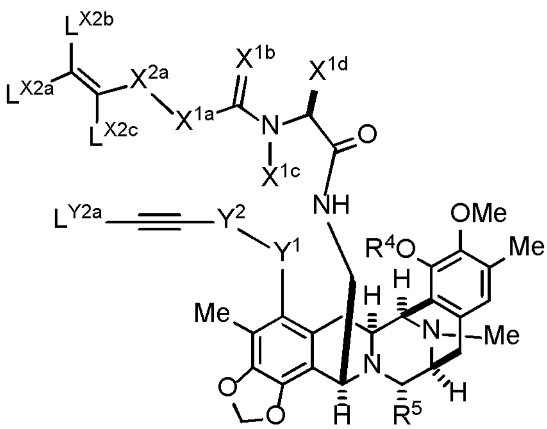

(IId)

,

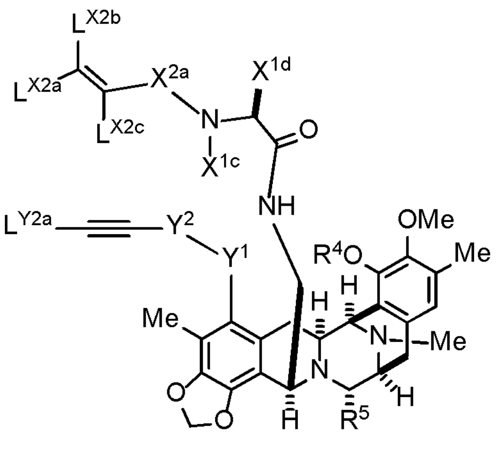

(IIe)

,

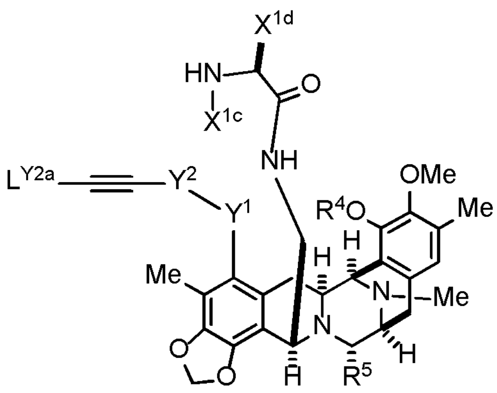

(IIf)

wherein

$X^{1a}$ represents an oxygen atom, a sulfur atom, $-NR^b-$, or an optionally substituted methylene group,
$X^{1b}$ represents an oxygen atom, a sulfur atom, $=NR^b$, or an optionally substituted methylene group,
$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,
$X^{1d}$ is selected from among a hydrogen atom, a methyl group, and substituents corresponding to various natural/unnatural amino acid side chains,
$X^{2a}$ represents an optionally substituted $C_1$-$C_6$ alkylene group,
$L^{X2a}$, $L^{X2b}$ and $L^{X2c}$ each independently represent a hydrogen atom, an optionally substituted $C_1$-$C_{20}$ alkyl group or an optionally substituted aryl group,
$Y^1$ is a divalent group selected from the group consisting of an ether group, a thioether group, an optionally substituted $C_1$-$C_6$ alkylene group, and $-NR^b-$;
$Y^2$ is a divalent group selected from the group consisting of an optionally substituted alkylene group, an optionally substituted alkenylene group, $-NR^bC(O)-$, $-C(O)NR^b-$, -C(O)O-, -OC(O)-, $-NR^bC(O)O-$, $-OC(O)NR^b-$, -OC(O)O-, a carbonyl group, -C(=S)-, $-C(=NR^b)-$, $- NR^b-$, a sulfonyl group, an ether group, a thioether group, and an amino acid residue,
$L^{Y2a}$, $L^{Y2b}$ and $L^{Y2c}$ each independently represent a hydrogen atom, an optionally substituted $C_1$-$C_{20}$ alkyl group or an optionally substituted aryl group,
$Z^1$ and $Z^2$ each independently represent N or $CR^e$,
$R^e$ is a hydrogen atom or an optionally substituted $C_1$-$C_6$ alkyl group,
$R^4$ is selected from among a hydrogen atom, an optionally substituted $C_1$-$C_6$ alkyl group, an optionally substituted aryl group, an optionally substituted allyl group, a propargyl group, and a protecting group for a phenolic hydroxyl group,
$R^5$ represents CN, a hydroxyl group, or a leaving group containing an oxygen atom, a sulfur atom, a nitrogen atom, or a phosphorus atom,
$R^b$ is selected from among a hydrogen atom, an optionally substituted $C_1$-$C_{20}$ alkyl group, an optionally substituted aryl group, an optionally substituted allyl group, a propargyl group, and a nitrogen protecting group, and Me represents a methyl group.

**11.** The compound according to claim 10 or a pharmaceutically acceptable salt thereof, wherein, in formula (IIc) to formula (IIf),

$X^{1a}$ represents an oxygen atom or $-NR^b-$,
$X^{1b}$ represents an oxygen atom,
$X^{1c}$ is selected from among a hydrogen atom, an optionally substituted $C_1$-$C_{20}$ alkyl group, an optionally substituted aryl group, an optionally substituted allyl group, a propargyl group, and a nitrogen protecting group,
$X^{1d}$ represents a methyl group,
$X^{2a}$ represents a $C_1$-$C_3$ alkylene group,
$L^{X2a}$, $L^{X2b}$ and $L^{X2c}$ each independently represent a hydrogen atom, an optionally substituted $C_1$-$C_8$ alkyl group,

or an optionally substituted aryl group,
$Y^1$ represents an ether group;
$Y^2$ represents a $C_1$-$C_3$ alkylene group;
$L^{Y2a}$, $L^{Y2b}$ and $L^{Y2c}$ are each independently selected from among a hydrogen atom, an optionally substituted $C_1$-$C_8$ alkyl group, and an optionally substituted aryl group;
$R^4$ is selected from among a hydrogen atom, an optionally substituted $C_1$-$C_6$ alkyl group, an aryl group, an allyl group, a propargyl group, a propargyl group, and a protecting group for a phenolic hydroxyl group,
$R^5$ represents CN, a hydroxyl group, or a leaving group containing an oxygen atom, a sulfur atom, a nitrogen atom, or a phosphorus atom,
$R^b$ is each independently selected from among a hydrogen atom, an optionally substituted $C_1$-$C_{20}$ alkyl group, an optionally substituted aryl group, an optionally substituted allyl group, a propargyl group, and a nitrogen protecting group, and
Me represents a methyl group.

**12.** The compound according to claim 10 or 11 or a pharmaceutically acceptable salt thereof, wherein, in formula (IIc) to formula (IIf),

$X^{1a}$ represents an oxygen atom,
$X^{1b}$ represents an oxygen atom,
$X^{1c}$ is selected from among a hydrogen atom, a methyl group, a propargyl group and a nitrogen protecting group,
$X^{1d}$ represents a methyl group,
$X^{2a}$ represents a $C_1$-$C_3$ alkylene group,
$L^{X2a}$, $L^{X2b}$ and $L^{X2c}$ each independently represent a hydrogen atom,
$Y^1$ represents an ether group;
$Y^2$ represents a $C_1$-$C_3$ alkylene group;
$L^{Y2a}$, $L^{Y2b}$ and $L^{Y2c}$ each independently represent a hydrogen atom,
$R^4$ represents a hydrogen atom or a protecting group for a phenolic hydroxyl group,
$R^5$ represents CN, and
Me represents a methyl group.

**13.** The compound according to claim 9, which is selected from the following group:

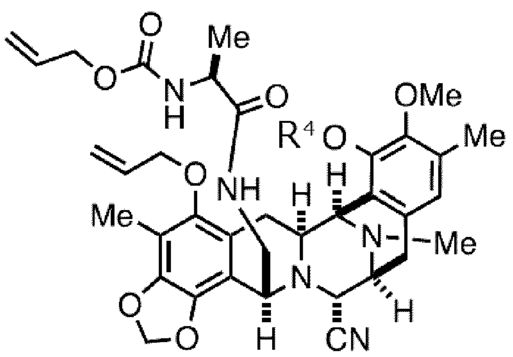
,
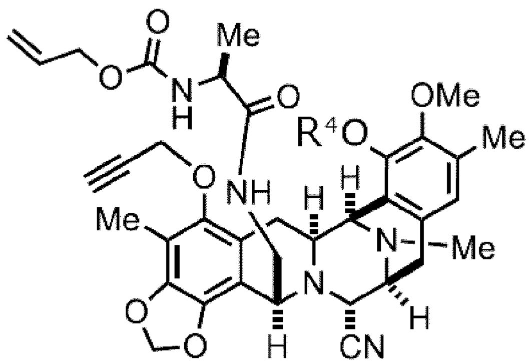
,

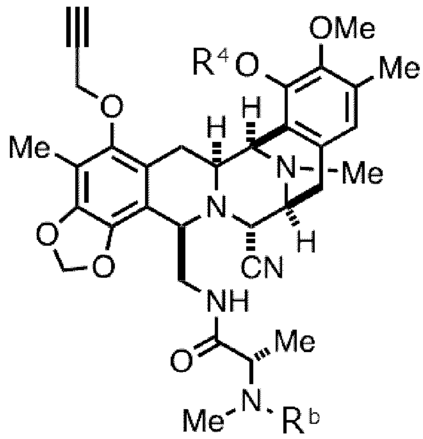

,

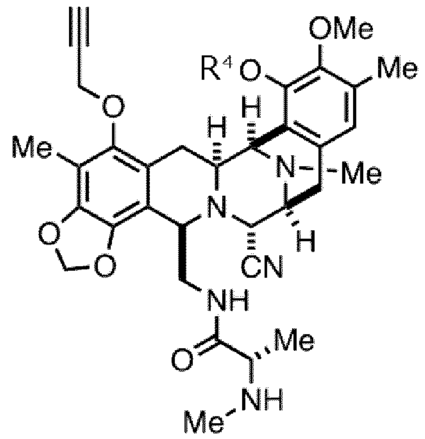

,

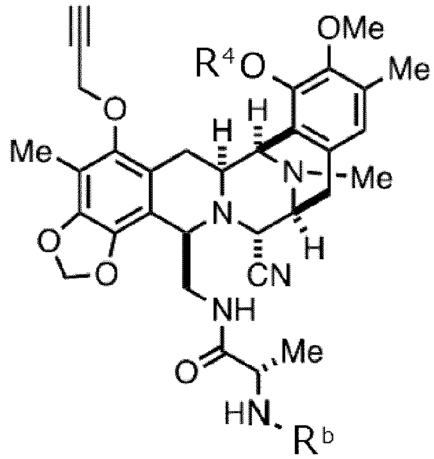

,

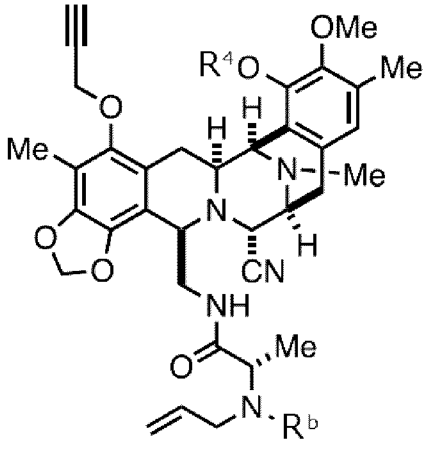

wherein Me represents a methyl group,

$R^4$ represents a hydrogen atom or a protecting group for a phenolic hydroxyl group, and
$R^b$ is each independently selected from among a hydrogen atom, an optionally substituted $C_1$-$C_{20}$ alkyl group, an optionally substituted aryl group, an optionally substituted allyl group, a propargyl group, and a nitrogen protecting group.

**14.** A pharmaceutical composition comprising the compound according to any one of claims 1 to 13 or a pharmaceutically acceptable salt thereof, and a pharmaceutically acceptable carrier.

**15.** A DNA alkylating agent comprising the compound according to any one of claims 1 to 13 or a pharmaceutically acceptable salt thereof.

**16.** An anti-cancer agent comprising the compound according to any one of claims 1 to 13 or a pharmaceutically acceptable salt thereof.

**17.** The anti-cancer agent according to claim 16,
wherein a target disease is selected from the group consisting of breast cancer, brain tumor, colorectal cancer, lung cancer, ovarian cancer, and gastric cancer.

**18.** A method for producing a DNA alkylating agent or anti-cancer agent having a tetrahydroisoquinoline framework using the compound according to any one of claims 9 to 13.

**19.** A use of the compound according to any one of claims 9 to 13, for producing a DNA alkylating agent or anti-cancer agent having a tetrahydroisoquinoline framework.

**20.** A method for producing a tetrahydroisoquinoline alkaloid compound containing a macrocyclic structure, the method including any one step of the following steps (A) to (C):

step (A): subjecting a compound represented by the following formula (IIc) to a ring-closing olefin metathesis reaction in the presence of a ruthenium catalyst or a tungsten catalyst to obtain a compound represented by formula (Ic);

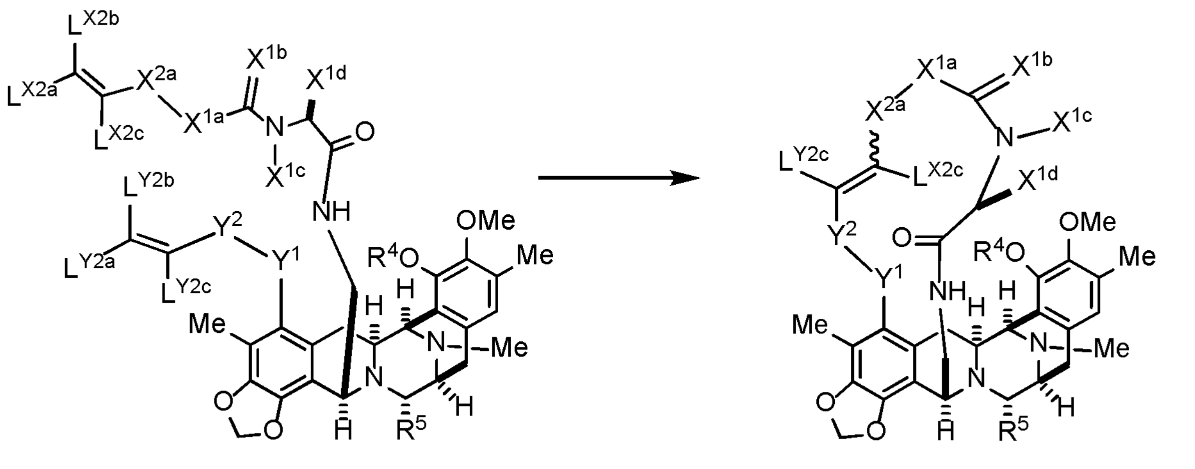

(IIc) (Ic)

step (B): subjecting a compound represented by the following formula (IId) to a ring-closing enyne metathesis reaction in the presence of a ruthenium catalyst or a tungsten catalyst to obtain a compound represented by formula (Id);

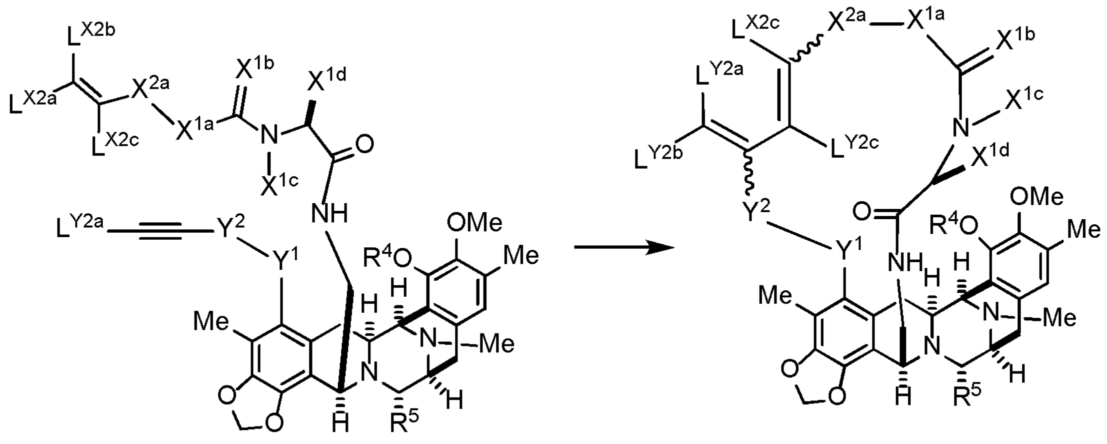

(IId) (Id)

step (C): subjecting a compound represented by the following formula (IIe) to a ring-closing enyne metathesis reaction in the presence of a ruthenium catalyst or a tungsten catalyst to obtain a compound represented by formula (If);

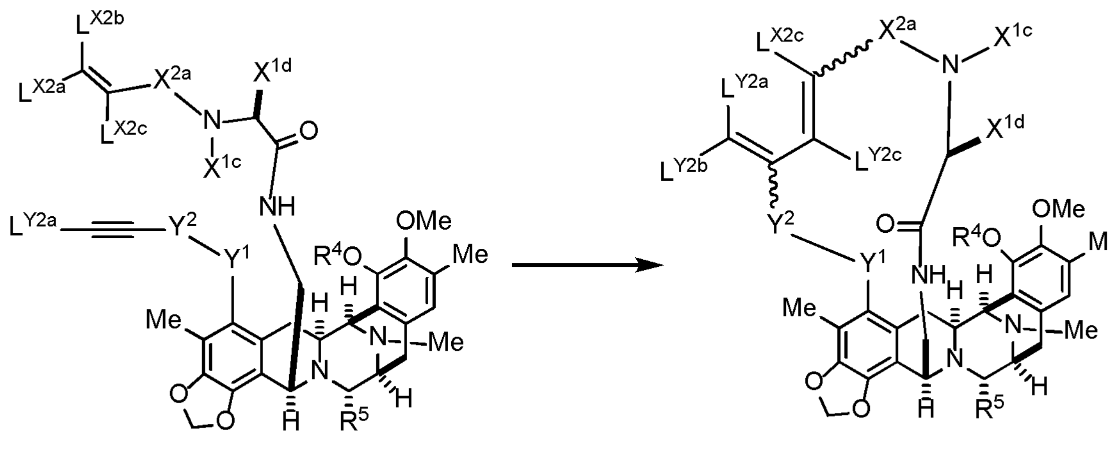

(IIe) (If)

wherein $X^{1a}$, $X^{1b}$, $X^{1c}$, $X^{1d}$, $X^{2a}$, $L^{X2a}$, $L^{X2b}$ $L^{X2c}$, $Y^1$, $Y^2$, $L^{Y2a}$, $L^{Y2b}$ $L^{Y2c}$, $R^4$, $R^5$ and Me are as defined in claim 10, and
$R^4$ represents a protecting group for a phenolic hydroxyl group.

**21.** A method for producing a tetrahydroisoquinoline alkaloid compound containing a macrocyclic structure, the method including the following step (D):
step (D): reacting a compound represented by formula (Id) with a compound represented by formula (IVa) to obtaining a compound represented by formula (Ie):

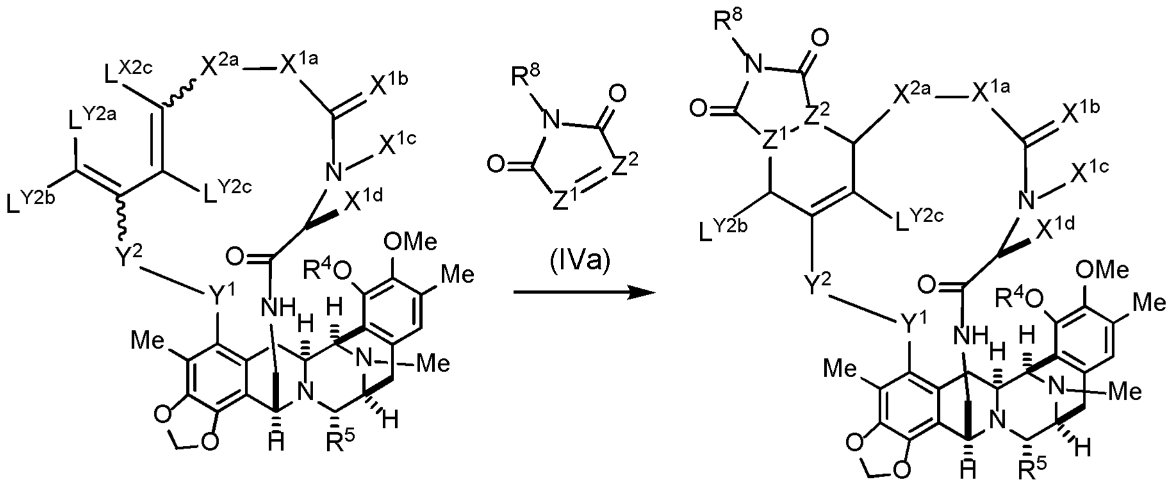

(IVa)

(Id) (Ie)

wherein $X^{1a}$, $X^{1b}$, $X^{1c}$, $X^{1d}$, $X^{2a}$, $L^{X2c}$, $Y^1$, $Y^2$, $L^{Y2a}$, $L^{Y2b}$, $L^{Y2c}$, $R^4$, $R^5$, $R^8$, $Z^1$, $Z^2$ and Me are as defined in claim 10, and $R^4$ represents a protecting group for a phenolic hydroxyl group.

**22.** A method for producing a tetrahydroisoquinoline alkaloid compound containing a macrocyclic structure, the method including the following step (E):
step (E): reacting a compound represented by the following formula (IIf) with a compound represented by formula (IVb) in the presence of a copper catalyst to obtain a compound represented by formula (Ig):

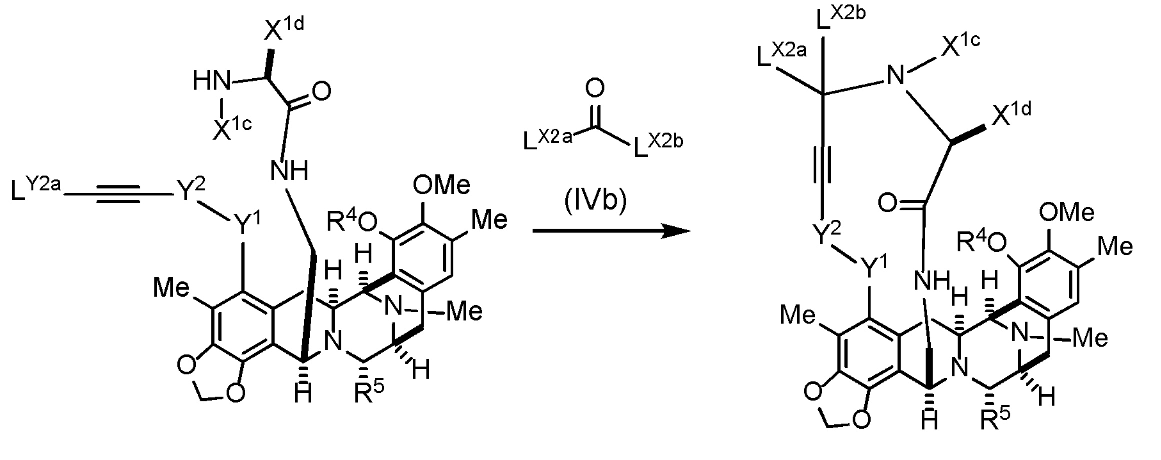


(IIf) (Ig)

wherein $X^{1c}$, $X^{1d}$, $L^{X2a}$, $L^{X2b}$, $Y^1$, $Y^2$, $L^{Y2a}$, $R^4$, $R^5$, and Me are as defined in claim 10, and $R^4$ represents a protecting group for a phenolic hydroxyl group.

**23.** A method for producing a tetrahydroisoquinoline alkaloid compound containing a macrocyclic structure, the method including the following step (F):
step (F): reacting a compound represented by the following formula (IIf) with a compound represented by formula (IVb) in the presence of a copper catalyst and a ligand to obtain a compound represented by formula (IIIc):

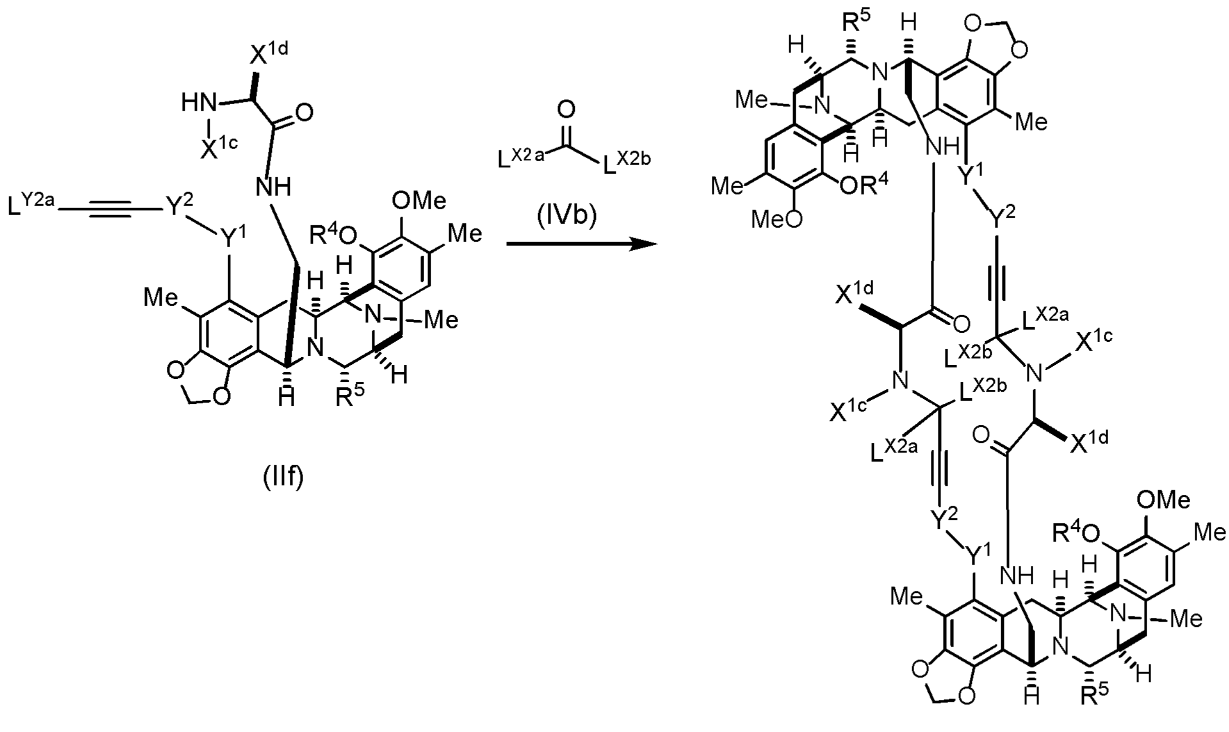


(IIf)

(IIIc)

wherein $X^{1c}$, $X^{1d}$, $L^{X2a}$, $L^{X2b}$, $Y^2$, $L^{Y2a}$, $R^4$, $R^5$, and Me are as defined in claim 10, and $R^4$ represents a protecting group for a phenolic hydroxyl group.

**INTERNATIONAL SEARCH REPORT**

International application No.
**PCT/JP2022/008664**

**A. CLASSIFICATION OF SUBJECT MATTER**

***C07D 498/22***(2006.01)i; ***A61K 31/4995***(2006.01)i; ***A61P 35/00***(2006.01)i; ***C07D 491/22***(2006.01)i
FI: C07D498/22 CSP; A61P35/00; C07D491/22; A61K31/4995

According to International Patent Classification (IPC) or to both national classification and IPC

**B. FIELDS SEARCHED**

Minimum documentation searched (classification system followed by classification symbols)

C07D498/22; A61K31/4995; A61P35/00; C07D491/22

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | Organic Letters. 2000, 2(16), 2545-2548, DOI 10.1021/ol0062502 in particular, fig. 1 | 1-8, 14-23 |
| A | JP 2013-526599 A (PHARMA MRS, S.A.) 24 June 2013 (2013-06-24) in particular, examples | 1-8, 14-23 |
| A | JP 2009-513618 A (PHARMA MRS, S.A.) 02 April 2009 (2009-04-02) in particular, examples | 1-8, 14-23 |
| A | JP 2005-523275 A (PHARMA MAR SAU) 04 August 2005 (2005-08-04) in particular, examples | 1-8, 14-23 |
| A | JP 2003-533532 A (PHARMA MRS, S.A.) 11 November 2003 (2003-11-11) in particular, examples | 1-8, 14-23 |
| A | Bioorganic & Medicinal Chemistry Letters. 2019, 29(14), 1807-1811 in particular, fig. 2 | 1-8, 14-23 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 May 2022** | **24 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/008664**

**C. DOCUMENTS CONSIDERED TO BE RELEVANT**

| Category* | Citation of document, with indication, where appropriate, of the relevant passages | Relevant to claim No. |
|---|---|---|
| X | 細野絵理奈，谷藤涼，大栗博毅, サフラマイシン骨格を基盤としたマクロ環含有DNAアルキル化リガンドの設計と合成, 日本化学会第１００春季年会(2020), 05 March 2020, vol. 100th, 2H6-06, https://confit.atlas.jp/guide/event/csj100th/subject/2H6-06/date?cryptoId= in particular, p. 2H6-06, (HOSONO, Erina. TANIFUJI, Ryo. OGURI, Hiroki. Design and synthesis of saframycin-type DNA alkylating ligands bearing a macrocyclic skeleton. The 100th CSJ Annual Meeting (2020).) | 1-8, 14-23 |
| P, X | 細野絵理奈，谷藤涼，大栗博毅, シアノサフラシン骨格へのマクロ環構築と機能評価, 日本化学会第１０１春季年会(2021), 04 March 2021, vol. 101th, A22-2pm-06, https://confit.atlas.jp/guide/event-img/csj101st/A22-2pm-06/public/pdf?type=in in particular, p. A22-2pm-06, (HOSONO, Erina. TANIFUJI, Ryo. OGURI, Hiroki. Installation of macrocyclic frameworks on cyanosafracin scaffold and functional evaluation. The 101st CSJ Annual Meeting (2021).) | 1-8, 14-23 |

Form PCT/ISA/210 (second sheet) (January 2015)

INTERNATIONAL SEARCH REPORT

International application No.
PCT/JP2022/008664

**Box No. III Observations where unity of invention is lacking (Continuation of item 3 of first sheet)**

This International Searching Authority found multiple inventions in this international application, as follows:

Document 1: Organic Letters. 2000, 2(16), 2545-2548, DOI 10.1021/ol0062502
Document 2: JP 2013-526599 A (PHARMA MRS, S.A.) 24 June 2013 (2013-06-24)
Document 3: JP 2009-513618 A (PHARMA MRS, S.A.) 02 April 2009 (2009-04-02)
Document 4: JP 2005-523275 A (PHARMA MAR SAU) 04 August 2005 (2005-08-04)
Document 5: JP 2003-533532 A (PHARMA MRS, S.A.) 11 November 2003 (2003-11-11)

(Invention 1) All of claims 1-8 and 20-23, and parts of claims 14-19
Claims 1-8 and 20-23 have the special technical features of: the compound, set forth in claim 1, having a crosslinked ring structure represented by formula (I); and a production method therefor, and are thus classified as invention 1.
In addition, parts of claims 14-19 dependent on any of claims 1-8 have the aforementioned special technical features, and are thus classified as invention 1.

(Invention 2) All of claims 9-13, and parts of claims 14-19
Claims 9-13 share, with claim 1 classified as invention 1, the common technical feature of a tetrahydroisoquinoline alkaloid compound having a predetermined structure such as -R1, -OR2, -OR4, -R3, -R5, -AX1, -Y1-Y2, R1, or -ORa. However, said technical feature is disclosed in documents 1-5 and the like, and does not make a contribution over the prior art, and thus cannot be said to be a special technical feature.
Moreover, there are no other same or corresponding special technical features between these inventions.
Further, claims 9-13 are not dependent on claim 1. Furthermore, claims 9-13 are not substantially identical to or similarly closely related to any of the claims classified as invention 1.
Accordingly, claims 9-13 cannot be classified as invention 1, and are classified as invention 2.
In addition, parts of claims 14-19 dependent on any of claims 9-13 are classified as invention 2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **all of claims 1-8 and 20-23, and parts of claims 14-19**

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2022/008664**

| Patent document cited in search report | Publication date (day/month/year) | Patent family member(s) | Publication date (day/month/year) |
|---|---|---|---|
| JP 2013-526599 A | 24 June 2013 | US 2013/0066067 A1<br>in particular, examples<br>WO 2011/147828 A1<br>EP 2576569 A1<br>CN 103038240 A<br>CN 107033164 A | |
| JP 2009-513618 A | 02 April 2009 | US 2009/0076016 A1<br>in particular, examples<br>WO 2007/052076 A2<br>EP 1968592 A2<br>KR 10-2008-0064959 A<br>CN 101300011 A | |
| JP 2005-523275 A | 04 August 2005 | US 2007/0093658 A1<br>in particular, examples<br>US 2009/0163507 A1<br>US 2010/0216987 A1<br>WO 2003/066638 A2<br>EP 1472261 A2<br>EP 2305689 A1<br>CN 1646539 A | |
| JP 2003-533532 A | 11 November 2003 | US 2003/0216397 A1<br>in particular, examples<br>US 2004/0019056 A1<br>US 2006/0019960 A1<br>US 2006/0111570 A1<br>US 2008/0045713 A1<br>US 2008/0051407 A1<br>US 2008/0146580 A1<br>US 2004/0002602 A1<br>WO 2000/069862 A2<br>WO 2001/087894 A1<br>WO 2001/077115 A1<br>WO 2001/087895 A1<br>EP 1185536 A2<br>EP 1280809 A1<br>EP 1287004 A1<br>EP 1289999 A1<br>EP 1496060 A1<br>CN 1436193 A<br>KR 10-0777464 B1<br>KR 10-0886496 B1<br>KR 10-0830717 B1<br>CN 1441805 A<br>CN 1440414 A<br>CN 1360588 A<br>KR 10-0834601 B1<br>JP 2003-533533 A<br>JP 2003-530402 A<br>JP 2002-544280 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021033773 A


### Non-patent literature cited in the description


- **SCOTT, J. D. ; WILLIAMS, R. M.** *Chem. Rev.,* 2002, vol. 102, 1669
- **LE, V. H. ; INAI, M. ; WILLIAMS, R. M. ; KAN, T.** *Nat. Prod. Rep.,* 2015, vol. 32, 328
- **DANIELE G. SOARES ; MIRIANA S. MACHADO ; CELINE J. ROCCA et al.** *Mol. Cancer. Ther.,* 2011, vol. 10, 1481-1489
- **BRADLEY J. PETEK ; ROBIN L. JONES.** *Molecules,* 2014, vol. 19, 12328-12335
- **MARGREY, K. A. ; LEVENS, A. ; NICEWICZ, D. A.** *Angew. Chem. Int. Ed.,* 2017, vol. 56, 15644
- **YAMORI, T. ; MATSUNAGA, A. ; SATO, S. ; YAMAZAKI, K. ; KOMI, A. ; ISHIZU, K. et al.** *Cancer Res.,* 1999, vol. 59, 4042
- **SKEHAN P. ; STORENG R. ; SCUDIERO D. ; MONKS A. ; MCMAHON J. ; VISTICA D. ; WARREN J. T. ; BOKESCH H. ; KENNEY S. ; BOYD M. R.** *J. Natl. Cancer Inst.,* 1990, vol. 82, 1107-1112
- **LEAL, J. F. M. ; MARTINEZ-DIEZ, M. ; CUEVAS, C. ; GARCIA-FERNANDEZ, L. F. ; GALMARINI, C. M. et al.** *Br. J. Pharmacol.,* 2010, vol. 161, 1099-1110
- **TAKAHASHI, N. ; LI, W. ; BERTINO, J. R. et al.** *Clin. Cancer Res.,* 2001, vol. 7, 2908-2911